# EUROPEAN PATENT APPLICATION

(11) **EP 4 487 869 A2**
(43) Date of publication of application: **08.01.2025**
(21) Application number: 24200084.2
(22) Date of filing: 21.04.2017
(51) Int. Cl.: A61K 39/00

(54) **RNA ENCODING A TUMOR ANTIGEN**

(30) Priority: 22.04.2016 WO PCT/EP2016/059111
(62) Divisional of application: 20186753.8
(71) Applicant: CureVac SE, 72076 Tübingen (DE)
(72) Inventor: FOTIN-MLECZEK, Mariola, 72076 Tübingen (DE); HOERR, Ingmar, 72076 Tübingen (DE)
(74) Representative: Graf von Stosch Patentanwaltsgesellschaft mbH

(57) **Abstract**

The present invention relates to an RNA encoding a tumor antigen. In particular, the present invention relates to RNA suitable for treatment and/or prophylaxis of cancer and related diseases. The present invention concerns such novel RNA as well as compositions, vaccines and kits comprising the RNA. Furthermore, the present invention relates to the RNA, compositions, vaccines or kits as disclosed herein for use in the treatment and/or prophylaxis of cancer and related diseases.

## Description

The present invention relates to an RNA encoding a tumor antigen. In particular, the present invention relates to RNA suitable for treatment and/or prophylaxis of cancer and related diseases. The present invention concerns such novel RNA as well as compositions, vaccines and kits comprising the RNA. Furthermore, the present invention relates to the RNA, compositions, vaccines or kits as disclosed herein for use in the treatment and/or prophylaxis of cancer and related diseases.

Apart from cardiovascular diseases and infectious diseases, the occurrence of cancer diseases is one of the most frequent causes of death in modern society and is in most cases associated with considerable costs in terms of therapy and subsequent rehabilitation measures. The treatment of cancer diseases is greatly dependent, for example, on the type of tumor that occurs, on the age, the distribution of cancer cells in the patient to be treated, etc. Cancer therapy is nowadays conventionally carried out by the use of radiation therapy or chemotherapy in addition to invasive operations. However, such conventional therapies typically place extraordinary stress on the immune system and can be used in some cases to only a limited extent. In addition, most of these conventional therapies require long intervals between the individual treatments to allow for regeneration of the immune system.

Therefore, supplementary strategies have been investigated in recent years in addition to such "conventional treatments" to avoid or at least reduce the impact on the immune system by such treatments. One such supplementary treatment in particular includes gene therapeutic approaches or genetic vaccination, which already have been found to be highly promising for treatment or for supporting such conventional therapies.

Gene therapy and genetic vaccination are methods of molecular medicine, which have already been proven in the therapy and prevention of certain diseases and generally exhibit a considerable effect on daily medical practice, in particular on the treatment of diseases as mentioned above. Both methods, gene therapy and genetic vaccination, are based on the introduction of nucleic acids into the patient's cells or tissue and subsequent processing of the information coded for by the nucleic acid that has been introduced into the cells or tissue, that is to say the (protein) expression of the desired polypeptides.

In gene therapy approaches, typically DNA is used even though RNA is also known in recent developments. Importantly, in all these gene therapy approaches mRNA functions as messenger for the sequence information of the encoded protein, irrespectively if DNA, viral RNA or mRNA is used.

In general RNA is considered an unstable molecule: RNases are ubiquitous and notoriously difficult to inactivate. Furthermore, RNA is also chemically more labile than DNA. Also for that reason, many gene therapy approaches normally use DNA to transfer the coding information into the cell which is then transcribed into mRNA. carrying the naturally occurring elements of an mRNA. particularly the 5'-CAP structure and the 3' poly(A) sequence to ensure expression of the encoded therapeutic or antigenic protein.

However, in many cases expression systems based on the introduction of such nucleic acids into the patient's cells or tissue and the subsequent expression of the desired polypeptides coded for by these nucleic acids do not exhibit the desired, or even the required, level of expression which may allow for an efficient therapy, irrespective as to whether DNA or RNA is used.

In the prior art, different attempts have hitherto been made to increase the yield of the expression of an encoded protein, in particular by use of improved expression systems, both *in vitro* and/or *in vivo.* Methods for increasing expression described generally in the prior art are conventionally based on the use of expression vectors or cassettes containing specific promoters and corresponding regulation elements. As these expression vectors or cassettes are typically limited to particular cell systems, these expression systems have to be adapted for use in different cell systems. Such adapted expression vectors or cassettes are then usually transfected into the cells and typically treated in dependence of the specific cell line. Therefore, preference is given primarily to those nucleic acid molecules which are able to express the encoded proteins in a target cell by systems inherent in the cell, independent of promoters and regulation elements which are specific for particular cell types. In this context, there can be distinguished between mRNA stabilizing elements and elements which increase translation efficiency of the mRNA.

mRNAs which are optimized in their coding sequence and which are in general suitable for such a purpose are described in application WO 02/098443 (CureVac GmbH). For example, WO 02/098443 describes mRNAs that are stabilised in general form and optimised for translation in their coding regions. WO 02/098443 further discloses a method for determining sequence modifications. WO 02/098443 additionally describes possibilities for substituting adenine and uracil nucleotides in mRNA sequences in order to increase the guanine/cytosine (G/C) content of the sequences. According to WO 02/098443, such substitutions and adaptations for increasing the G/C content can be used for gene therapeutic applications but also genetic vaccines in the treatment of cancer or infectious diseases. In this context. WO 02/098443 generally mentions sequences as a base sequence for such modifications, in which the modified mRNA codes for at least one biologically active peptide or polypeptide, which is translated in the patient to be treated, for example, either not at all or inadequately or with faults. Alternatively, WO 02/098443 proposes mRNAs coding for antigens e.g. tumour antigens or viral antigens as a base sequence for such modifications.

In a further approach to increase the expression of an encoded protein the application WO 2007/036380 describes the positive effect of long poly(A) sequences (particularly longer than 120 bp) and the combination of at least two 3' untranslated regions of the beta globin gene on mRNA stability and translational activity.

However, even though some of these prior art documents try to provide quite efficient tools for gene therapy approaches and additionally improved mRNA stability and translational activity, there still remains the problem of a generally lower stability of RNA-based applications versus DNA vaccines and DNA based gene therapeutic approaches. Accordingly, there still exists a need in the art to provide improved tools for gene therapy approaches and genetic vaccination or as a supplementary therapy for conventional treatments as discussed above, which allow for better provision of encoded proteins *in vivo,* e.g. via a further improved mRNA stability and/or translational activity. preferably for gene therapy and genetic vaccination.

Furthermore despite of all progress in the art. efficient expression of an encoded peptide or protein in cell-free systems, cells or organisms (recombinant expression) is still a challenging problem.

It is thus an object of the present invention to provide a tumor vaccine. In particular, it is an object to provide a system for expressing a tumor antigen, preferably by stabilization of the mRNA and/or an increase of the translational efficiency of such an mRNA with respect to such nucleic acids known from the prior art. It is a further object of the invention to provide such a system, which allows treatment and/or prophylaxis of cancer by stimulating the immune system in a safe and effective manner. It is thus a particular object of the present invention to provide a cancer vaccine or a composition for treatment or prophylaxis of cancer by stimulating the immune system.

The object underlying the present invention is solved by the claimed subject matter.

The present application is filed together with a sequence listing in electronic format. The information contained in the electronic format of the sequence listing is incorporated herein by reference in its entirety.

For the sake of clarity and readability the following definitions are provided. Any technical feature mentioned for these definitions may be read on each and every embodiment of the invention. Additional definitions and explanations may be specifically provided in the context of these embodiments.

Adaptive immune response: The adaptive immune response is typically understood to be an antigen-specific response of the immune system. Antigen specificity allows for the generation of responses that are tailored, for example, to specific pathogens or pathogen-infected cells. The ability to mount these tailored responses is usually maintained in the body by "memory cells". Should a pathogen infect the body more than once, these specific memory cells are used to quickly eliminate it. In this context, the first step of an adaptive immune response is the activation of naive antigen-specific T cells or different immune cells able to induce an antigen-specific immune response by antigen-presenting cells. This occurs in the lymphoid tissues and organs through which naive T cells are constantly passing. The three cell types that may serve as antigen-presenting cells are dendritic cells, macrophages, and B cells. Each of these cells has a distinct function in eliciting immune responses. Dendritic cells may take up antigens by phagocytosis and macropinocytosis and may become stimulated by contact with e.g. a foreign antigen to migrate to the local lymphoid tissue, where they differentiate into mature dendritic cells. Macrophages ingest particulate antigens such as bacteria and are induced by infectious agents or other appropriate stimuli to express MHC molecules. The unique ability of B cells to bind and internalize soluble protein antigens via their receptors may also be important to induce T cells. MHC-molecules are. typically, responsible for presentation of an antigen to T-cells. Therein, presenting the antigen on MHC molecules leads to activation of T cells, which induces their proliferation and differentiation into armed effector T cells. The most important function of effector T cells is the killing of infected cells by CD8+ cytotoxic T cells and the activation of macrophages by Th1 cells, which together make up cell-mediated immunity, and the activation of B cells by both Th2 and Thl cells to produce different classes of antibody, thus driving the humoral immune response. T cells recognize an antigen by their T cell receptors which do not recognize and bind the antigen directly, but instead recognize short peptide fragments e.g. of pathogen-derived protein antigens, e.g. so-called epitopes, which are bound to MHC molecules on the surfaces of other cells.

Adaptive immune system: The adaptive immune system is essentially dedicated to eliminate or prevent pathogenic growth. It typically regulates the adaptive immune response by providing the vertebrate immune system with the ability to recognize and remember specific pathogens (to generate immunity), and to mount stronger attacks each time the pathogen is encountered. The system is highly adaptable because of somatic hypermutation (a process of accelerated somatic mutations), and V(D)J recombination (an irreversible genetic recombination of antigen receptor gene segments). This mechanism allows a small number of genes to generate a vast number of different antigen receptors, which are then uniquely expressed on each individual lymphocyte. Because the gene rearrangement leads to an irreversible change in the DNA of each cell, all of the progeny (offspring) of such a cell will then inherit genes encoding the same receptor specificity, including the Memory 8 cells and Memory T cells that are the keys to long-lived specific immunity.

Adjuvant/adjuvant component: An adjuvant or an adjuvant component in the broadest sense is typically a pharmacological and/or immunological agent that may modify, e.g. enhance, the effect of other agents, such as a drug or vaccine. It is to be interpreted in a broad sense and refers to a broad spectrum of substances. Typically, these substances are able to increase the immunogenicity of antigens. For example, adjuvants may be recognized by the innate immune systems and, e.g., may elicit an innate immune response. "Adjuvants" typically do not elicit an adaptive immune response. Insofar, "adjuvants" do not qualify as antigens. Their mode of action is distinct from the effects triggered by antigens resulting in an adaptive immune response.

Antigen: In the context of the present invention, the term "antigen" typically refers to a substance, which is capable of being recognized by the immune system, preferably by the adaptive immune system, and which is capable of eliciting an antigen-specific immune response, e.g. by formation of antibodies and/or antigen-specific T cells as part of an adaptive immune response. Typically, an antigen may be or may comprise a peptide or protein, which may be presented by the MHC to T-cells. In the sense of the present invention an antigen may be the product of translation of a provided nucleic acid molecule, preferably an RNA comprising at least one coding sequence as defined herein. In this context. also fragments, variants and derivatives of an antigen, such as a peptide or a protein, comprising at least one epitope are understood as antigens.

Artificial nucleic acid molecule: An artificial nucleic acid molecule may typically be understood to be a nucleic acid molecule, e.g. a DNA or an RNA. that does not occur naturally. In other words, an artificial nucleic acid molecule may be understood as a non-natural nucleic acid molecule. Such nucleic acid molecule may be non-natural due to its individual sequence (which does not occur naturally) and/or due to other modifications, e.g. structural modifications of nucleotides, which do not occur naturally. An artificial nucleic acid molecule may be a DNA molecule, an RNA molecule or a hybrid-molecule comprising DNA and RNA portions. Typically, artificial nucleic acid molecules may be designed and/or generated by genetic engineering methods to correspond to a desired artificial sequence of nucleotides (heterologous sequence). In this context an artificial sequence is usually a sequence that may not occur naturally, i.e. it differs from the wild type sequence by at least one nucleotide. The term "wild type" may be understood as a sequence occurring in nature. Further, the term "artificial nucleic acid molecule" is not restricted to mean "one single molecule" but is, typically, understood to comprise an ensemble of identical molecules. Accordingly, it may relate to a plurality of identical molecules contained in an aliquot.

Bicistronic RNA, multicistronic RNA: A bicistronic or multicistronic RNA is typically an RNA, preferably an mRNA. that typically may have two (bicistronic) or more (multicistronic) open reading frames (ORF). An open reading frame in this context is a sequence of codons that is translatable into a peptide or protein.

Carrier / polymeric carrier: A carrier in the context of the invention may typically be a compound that facilitates transport and/or complexation of another compound (cargo). A polymeric carrier is typically a carrier that is formed of a polymer. A carrier may be associated to its cargo by covalent or non-covalent interaction. A carrier may transport nucleic acids, e.g. RNA or DNA, to the target cells. The carrier may - for some embodiments - be a cationic component.

Cationic component: The term "cationic component" typically refers to a charged molecule, which is positively charged (cation) at a pH value typically from 1 to 9, preferably at a pH value of or below 9 (e.g. from 5 to 9). of or below 8 (e.g. from 5 to 8). of or below 7 (e.g. from 5 to 7), most preferably at a physiological pH, e.g. from 7.3 to 7.4. Accordingly, a cationic component may be any positively charged compound or polymer, preferably a cationic peptide or protein, which is positively charged under physiological conditions, particularly under physiological conditions *in vivo,* A "cationic peptide or protein" may contain at least one positively charged amino acid. or more than one positively charged amino acid, e.g. selected from Arg. His, Lys or Orn. Accordingly, "polycationic" components are also within the scope exhibiting more than one positive charge under the conditions given.

5'-cap: A 5'-cap is an entity, typically a modified nucleotide entity, which generally "caps" the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g. m7GpppN, wherein N is the terminal 5' nucleotide of the nucleic acid carrying the 5'-cap, typically the 5'-end of an RNA. Further examples of 5'cap structures include glyceryl, inverted deoxy abasic residue (moiety), 4',5' methylene nucleotide, 1-(beta-D-erythrofuranosyl) nucleotide, 4'-thio nucleotide, carbocyclic nucleotide. 1,5-anhydrohexitol nucleotide, L-nucleotides, alpha-nucleotide, modified base nucleotide, threo-pentofuranosyl nucleotide, acyclic 3',4'-seco nucleotide, acyclic 3.4-dihydroxybutyl nucleotide, acyclic 3,5 dihydroxypentyl nucleotide, 3'-3'-inverted nucleotide moiety, 3'-3'-inverted abasic moiety, 3'-2'-inverted nucleotide moiety. 3'-2'-inverted abasic moiety, 1,4-butanediol phosphate, 3'-phosphoramidate, hexylphosphate, aminohexyl phosphate, 3'-phosphate, 3'phosphorothioate. phosphorodithioate, or bridging or non-bridging methylphosphonate moiety.

Cellular immunity/cellular immune response: Cellular immunity relates typically to the activation of macrophages, natural killer cells (NK), antigen-specific cytotoxic T-lymphocytes, and the release of various cytokines in response to an antigen. In more general terms, cellular immunity is not based on antibodies, but on the activation of cells of the immune system. Typically, a cellular immune response may be characterized e.g. by activating antigen-specific cytotoxic T-lymphocytes that are able to induce apoptosis in cells, e.g. specific immune cells like dendritic cells or other cells, displaying epitopes of foreign antigens on their surface. Such cells may be virus-infected or infected with intracellular bacteria, or cancer cells displaying tumor antigens. Further characteristics may be activation of macrophages and natural killer cells, enabling them to destroy pathogens and stimulation of cells to secrete a variety of cytokines that influence the function of other cells involved in adaptive immune responses and innate immune responses.

DNA: DNA is the usual abbreviation for deoxy-ribonucleic acid. It is a nucleic acid molecule, i.e. a polymer consisting of nucleotides. These nucleotides are usually deoxy-adenosine-monophosphate, deoxy-thymidine-monophosphate, deoxy-guanosine-monophosphate and deoxy-cytidine-monophosphate monomers which are - by themselves - composed of a sugar moiety (deoxyribose), a base moiety and a phosphate moiety, and polymerise by a characteristic backbone structure. The backbone structure is, typically, formed by phosphodiester bonds between the sugar moiety of the nucleotide, i.e. deoxyribose, of a first and a phosphate moiety of a second, adjacent monomer. The specific order of the monomers, i.e. the order of the bases linked to the sugar/phosphate-backbone. is called the DNA sequence. DNA may be single stranded or double stranded. In the double stranded form, the nucleotides of the first strand typically hybridize with the nucleotides of the second strand, e.g. by A/T-base-pairing and G/C-base-pairing.

Epitope: In the context of the present invention, the term 'epitope' typically refers to a fragment of an antigen or a variant of an antigen, wherein said fragment is presented by an MHC complex. Such a fragment comprising or consisting of an epitope as used herein may typically comprise from about 5 to about 20 amino acids. An epitope may also be referred to herein as 'antigen determinant'. Epitopes can be distinguished in T cell epitopes and B cell epitopes. T cell epitopes or parts of the proteins in the context of the present invention may comprise fragments preferably having a length of about 6 to about 20 or even more amino acids, e.g. fragments as processed and presented by MHC class I molecules, preferably having a length of about 8 to about 10 amino acids, e.g. 8, 9, or 10, (or even 11. or 12 amino acids), or fragments as processed and presented by MHC class 11 molecules, preferably having a length of about 13 or more amino acids, e.g. 13, 14, 15, 16, 17, 18, 19, 20 or even more amino acids, wherein these fragments may be selected from any part of the amino acid sequence. These fragments are typically recognized by T cells in form of a complex consisting of the peptide fragment and an MHC molecule, i.e. the fragments are typically not recognized in their native form. B cell epitopes are typically fragments located on the outer surface of (native) protein or peptide antigens as defined herein, preferably having 5 to 15 amino acids, more preferably having 5 to 12 amino acids, even more preferably having 6 to 9 amino acids, which may be recognized by antibodies, i.e. in their native form. Such epitopes of proteins or peptides may furthermore be selected from any of the herein mentioned variants of such proteins or peptides. In this context, antigenic determinants can be conformational or discontinuous epitopes, which are composed of segments of the proteins or peptides as defined herein that are discontinuous in the amino acid sequence of the proteins or peptides as defined herein but are brought together in the three-dimensional structure or continuous or linear epitopes, which are composed of a single polypeptide chain.

Fragment of a sequence: A fragment of a sequence may typically be a shorter portion of a full-length sequence of e.g. a nucleic acid molecule or an amino acid sequence. Accordingly, a fragment, typically, consists of a sequence that is identical to the corresponding stretch within the full-length sequence. A preferred fragment of a sequence in the context of the present invention, consists of a continuous stretch of entities, such as nucleotides or amino acids corresponding to a continuous stretch of entities in the molecule the fragment is derived from, which represents at least 20%. preferably at least 30%. more preferably at least 40%, more preferably at least 50%, even more preferably at least 60%. even more preferably at least 70%, and most preferably at least 80% of the total (i.e. full-length) molecule from which the fragment is derived.

G/C modified: A Eye-modified nucleic acid may typically be a nucleic acid, preferably an artificial nucleic acid molecule as defined herein, based on a modified wild-type sequence comprising a preferably increased number of guanosine and/or cytosine nucleotides as compared to the wild-type sequence. Such an increased number may be generated by substitution of codons containing adenosine or thymidine nucleotides by codons containing guanosine or cytosine nucleotides. If the enriched G/C content occurs in a coding region of DNA or RNA, it makes use of the degeneracy of the genetic code. Accordingly, the codon substitutions preferably do not alter the encoded amino acid residues, but exclusively increase the G/C content of the nucleic acid molecule. As used herein, the term 'G/C modification' comprises, in particular, the modifications of the number of guanosine and/or cytosine nucleotides in the RNA according to the invention, such as GC optimization of sequences, adaptation of sequences to human codon usage, codon optimization, or C-optimization of sequences.

Gene therapy: Gene therapy may typically be understood to mean a treatment of a patient's body or isolated elements of a patient's body, for example isolated tissues/cells, by nucleic acids encoding a peptide or protein. It typically may comprise at least one of the steps of a) administration of a nucleic acid, preferably an RNA as defined herein. directly to the patient - by whatever administration route - or *in vitroto* isolated cells/tissues of the patient, which results in transfection of the patient's cells either *in vivo*/ *ex vivo or in vitro,* b) transcription and/or translation of the introduced nucleic acid molecule; and optionally c) re-administration of isolated, transfected cells to the patient, if the nucleic acid has not been administered directly to the patient.

Genetic vaccination: Genetic vaccination may typically be understood to be vaccination by administration of a nucleic acid molecule encoding an antigen or an immunogen or fragments thereof. The nucleic acid molecule may be administered to a subject's body or to isolated cells of a subject. Upon transfection of certain cells of the body or upon transfection of the isolated cells, the antigen or immunogen may be expressed by those cells and subsequently presented to the immune system, eliciting an adaptive, i.e. antigen-specific immune response. Accordingly, genetic vaccination typically comprises at least one of the steps of a) administration of a nucleic acid, preferably an artificial nucleic acid molecule as defined herein, to a subject. preferably a patient, or to isolated cells of a subject. preferably a patient, which usually results in transfection of the subject's cells either *in vivo* or *in vitro,* b) transcription and/or translation of the introduced nucleic acid molecule: and optionally c) re-administration of isolated, transfected cells to the subject. preferably the patient, if the nucleic acid has not been administered directly to the patient.

Heterologous sequence: Two sequences are typically understood to be 'heterologous' if they are not derivable from the same gene. I.e.. although heterologous sequences may be derivable from the same organism, they naturally (in nature) do not occur in the same nucleic acid molecule, such as in the same mRNA.

Humoral immunity/humoral immune response: Humoral immunity refers typically to antibody production and optionally to accessory processes accompanying antibody production. A humoral immune response may be typically characterized, e.g., by Th2 activation and cytokine production, germinal center formation and isotype switching, affinity maturation and memory cell generation. Humoral immunity also typically may refer to the effector functions of antibodies, which include pathogen and toxin neutralization, classical complement activation, and opsonin promotion of phagocytosis and pathogen elimination.

Immunogen: In the context of the present invention, an immunogen may be typically understood to be a compound that is able to stimulate an immune response. Preferably, an immunogen is a peptide, polypeptide, or protein. In a particularly preferred embodiment, an immunogen in the sense of the present invention is the product of translation of a provided nucleic acid molecule, preferably an artificial nucleic acid molecule as defined herein. Typically, an immunogen elicits at least an adaptive immune response.

Immunostimulatory composition: In the context of the invention, an immunostimulatory composition may be typically understood to be a composition containing at least one component which is able to induce an immune response or from which a component, which is able to induce an immune response, is derivable. Such immune response may be preferably an innate immune response or a combination of an adaptive and an innate immune response. Preferably, an immunostimulatory composition in the context of the invention contains at least one artificial nucleic acid molecule, more preferably an RNA, for example an mRNA molecule. The immunostimulatory component, such as the mRNA may be complexed with a suitable carrier. Thus, the immunostimulatory composition may comprise an mRNA/carrier-complex. Furthermore, the immunostimulatory composition may comprise an adjuvant and/or a suitable vehicle for the immunostimulatory component, such as the mRNA.

Immune response: An immune response may typically be a specific reaction of the adaptive immune system to a particular antigen (so called specific or adaptive immune response) or an unspecific reaction of the innate immune system (so called unspecific or innate immune response), or a combination thereof.

Immune system: The immune system may protect organisms from infection. If a pathogen succeeds in passing a physical barrier of an organism and enters this organism, the innate immune system provides an immediate, but non-specific response. If pathogens evade this innate response, vertebrates possess a second layer of protection, the adaptive immune system. Here, the immune system adapts its response during an infection to improve its recognition of the pathogen. This improved response is then retained after the pathogen has been eliminated, in the form of an immunological memory, and allows the adaptive immune system to mount faster and stronger attacks each time this pathogen is encountered. According to this, the immune system comprises the innate and the adaptive immune system. Each of these two parts typically contains so called humoral and cellular components.

Immunostimulatory RNA: An immunostimulatory RNA (isRNA) in the context of the invention may typically be an RNA that is able to induce an innate immune response. It usually does not have an open reading frame and thus does not provide a peptide-antigen or immunogen but elicits an immune response e.g. by binding to a specific kind of Toll-like-receptor (TLR) or other suitable receptors. However, of course also mRNAs having an open reading frame and coding for a peptide/protein may induce an innate immune response and, thus, may be immunostimulatory RNAs.

Innate immune system: The innate immune system, also known as non-specific (or unspecific) immune system, typically comprises the cells and mechanisms that defend the host from infection by other organisms in a non-specific manner. This means that the cells of the innate system may recognize and respond to pathogens in a generic way, but unlike the adaptive immune system, it does not confer long-lasting or protective immunity to the host. The innate immune system may be, e.g., activated by ligands of Toll-like receptors (TLRs) or other auxiliary substances such as lipopolysaccharides. TNF-alpha, CD40 ligand, or cytokines, monokines, lymphokines, interleukins or chemokines. IL-I, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27. IL-28, IL-29, IL-30. 11-31, IL-32, IL-33, IFN-alpha, IFN-beta, IFN-gamma, GM-CSF, G-CSF, M-CSF, LT-beta, TNF-alpha, growth factors, and hGH. a ligand of human Toll-like receptor TLRI, TLR2. TLR3, TLR4, TLR5, TLR6, TLR7. TLR8, TLR9, TLR10, a ligand of murine Toll-like receptor TLR1, TLR2, TLR3, TLR4, TLR5, TLR6, TLR7, TLR8, TLR9, TLR10, TLR!!. TLR12 or TLR13, a ligand of a NOD-like receptor, a ligand of a RIG-1 like receptor, an immunostimulatory nucleic acid, an immunostimulatory RNA (isRNA), a CpG-DNA, an antibacterial agent, or an anti-viral agent. The pharmaceutical composition according to the present invention may comprise one or more such substances. Typically, a response of the innate immune system includes recruiting immune cells to sites of infection, through the production of chemical factors, including specialized chemical mediators, called cytokines: activation of the complement cascade; identification and removal of foreign substances present in organs, tissues, the blood and lymph, by specialized white blood cells; activation of the adaptive immune system; and/or acting as a physical and chemical barrier to infectious agents.

Cloning site: A cloning site is typically understood to be a segment of a nucleic acid molecule, which is suitable for insertion of a nucleic acid sequence, e.g., a nucleic acid sequence comprising an open reading frame. Insertion may be performed by any molecular biological method known to the one skilled in the art. e.g. by restriction and ligation. A cloning site typically comprises one or more restriction enzyme recognition sites (restriction sites). These one or more restrictions sites may be recognized by restriction enzymes which cleave the DNA at these sites. A cloning site which comprises more than one restriction site may also be termed a multiple cloning site (MCS) or a polylinker.

Nucleic acid molecule: A nucleic acid molecule is a molecule comprising, preferably consisting of nucleic acid components. The term nucleic acid molecule preferably refers to DNA or RNA molecules. It is preferably used synonymous with the term "polynucleotide". Preferably, a nucleic acid molecule is a polymer comprising or consisting of nucleotide monomers, which are covalently linked to each other by phosphodiester-bonds of a sugar/phosphate-backbone. The term "nucleic acid molecule" also encompasses modified nucleic acid molecules, such as base-modified, sugar-modified or backbone-modified etc. DNA or RNA molecules.

Open reading frame: An open reading frame (ORF) in the context of the invention may typically be a sequence of several nucleotide triplets, which may be translated into a peptide or protein. An open reading frame preferably contains a start codon, i.e. a combination of three subsequent nucleotides coding usually for the amino acid methionine (ATG), at its 5'-end and a subsequent region, which usually exhibits a length which is a multiple of 3 nucleotides. An ORF is preferably terminated by a stop-codon (e.g., TAA, TAG, TGA). Typically, this is the only stop-codon of the open reading frame. Thus, an open reading frame in the context of the present invention is preferably a nucleotide sequence, consisting of a number of nucleotides that may be divided by three, which starts with a start codon (e.g. ATG) and which preferably terminates with a stop codon (e.g., TAA, TGA, or TAG). The open reading frame may be isolated or it may be incorporated in a longer nucleic acid sequence, for example in a vector or an mRNA. An open reading frame may also be termed "(protein) coding region" or, preferably, "coding sequence".

Peptide: A peptide or polypeptide is typically a polymer of amino acid monomers, linked by peptide bonds. It typically contains less than 50 monomer units. Nevertheless, the term peptide is not a disclaimer for molecules having more than 50 monomer units. Long peptides are also called polypeptides, typically having between 50 and 600 monomeric units.

Pharmaceutically effective amount: A pharmaceutically effective amount in the context of the invention is typically understood to be an amount that is sufficient to induce a pharmaceutical effect, such as an immune response, altering a pathological level of an expressed peptide or protein. or substituting a lacking gene product, e.g.. in case of a pathological situation.

Protein: A protein typically comprises one or more peptides or polypeptides. A protein is typically folded into 3-dimensional form, which may be required for the protein to exert its biological function.

Poly(A) sequence: A poly(A) sequence, also called poly(A) tail or 3'-poly(A) tail, is typically understood to be a sequence of adenosine nucleotides. e.g., of up to about 400 adenosine nucleotides, e.g. from about 20 to about 400, preferably from about 50 to about 400. more preferably from about 50 to about 300, even more preferably from about 50 to about 250. most preferably from about 60 to about 250 adenosine nucleotides. As used herein, a poly(A) sequence may also comprise about 10 to 200 adenosine nucleotides, preferably about 10 to 100 adenosine nucleotides, more preferably about 40 to 80 adenosine nucleotides or even more preferably about 50 to 70 adenosine nucleotides. A poly(A) sequence is typically located at the 3'end of an mRNA. In the context of the present invention, a poly(A) sequence may be located within an mRNA or any other nucleic acid molecule, such as, e.g., in a vector, for example, in a vector serving as template for the generation of an RNA, preferably an mRNA, e.g., by transcription of the vector.

Polyadenylation: Polyadenylation is typically understood to be the addition of a poly(A) sequence to a nucleic acid molecule, such as an RNA molecule, e.g. to a premature mRNA. Polyadenylation may be induced by a so-called polyadenylation signal. This signal is preferably located within a stretch of nucleotides at the 3'-end of a nucleic acid molecule, such as an RNA molecule, to be polyadenylated. A polyadenylation signal typically comprises a hexamer consisting of adenine and uracil/thymine nucleotides, preferably the hexamer sequence AAUAAA. Other sequences, preferably hexamer sequences, are also conceivable. Polyadenylation typically occurs during processing of a pre-mRNA (also called premature-mRNA). Typically, RNA maturation (from pre-mRNA to mature mRNA) comprises the step of polyadenylation.

Restriction site: A restriction site, also termed restriction enzyme recognition site, is a nucleotide sequence recognized by a restriction enzyme. A restriction site is typically a short, preferably palindromic nucleotide sequence, e.g. a sequence comprising 4 to 8 nucleotides.

A restriction site is preferably specifically recognized by a restriction enzyme. The restriction enzyme typically cleaves a nucleotide sequence comprising a restriction site at this site. In a double-stranded nucleotide sequence, such as a double-stranded DNA sequence, the restriction enzyme typically cuts both strands of the nucleotide sequence.

RNA, mRNA: RNA is the usual abbreviation for ribonucleic-acid. It is a nucleic acid molecule, i.e. a polymer consisting of nucleotides. These nucleotides are usually adenosine-monophosphate, uridine-monophosphate, guanosine-monophosphate and cytidine-monophosphate monomers which are connected to each other along a so-called backbone. The backbone is formed by phosphodiester bonds between the sugar, i.e. ribose, of a first and a phosphate moiety of a second, adjacent monomer. The specific succession of the monomers is called the RNA-sequence. Usually RNA may be obtainable by transcription of a DNA-sequence, e.g., inside a cell. In eukaryotic cells, transcription is typically performed inside the nucleus or the mitochondria. In vivo, transcription of DNA usually results in the so-called premature RNA which has to be processed into so-called messenger-RNA. usually abbreviated as mRNA. Processing of the premature RNA, e.g. in eukaryotic organisms, comprises a variety of different posttranscriptional-modifications such as splicing, 5'-capping, polyadenylation. export from the nucleus or the mitochondria and the like. The sum of these processes is also called maturation of RNA. The mature messenger RNA usually provides the nucleotide sequence that may be translated into an amino-acid sequence of a particular peptide or protein. Typically, a mature mRNA comprises a 5'-cap, a 5'-UTR, an open reading frame, a 3'-UTR and a poly(A) sequence. Aside from messenger RNA, several non-coding types of RNA exist which may be involved in regulation of transcription and/or translation.

Sequence of a nucleic acid molecule: The sequence of a nucleic acid molecule is typically understood to be the particular and individual order, i.e. the succession of its nucleotides. The sequence of a protein or peptide is typically understood to be the order, i.e. the succession of its amino acids.

Sequence identity: Two or more sequences are identical if they exhibit the same length and order of nucleotides or amino acids. The percentage of identity typically describes the extent to which two sequences are identical, i.e. it typically describes the percentage of nucleotides that correspond in their sequence position with identical nucleotides of a reference-sequence. For determination of the degree of identity, the sequences to be compared are considered to exhibit the same length, i.e. the length of the longest sequence of the sequences to be compared. This means that a first sequence consisting of 8 nucleotides is 80% identical to a second sequence consisting of 10 nucleotides comprising the first sequence. In other words, in the context of the present invention, identity of sequences preferably relates to the percentage of nucleotides of a sequence which have the same position in two or more sequences having the same length. Gaps are usually regarded as non-identical positions, irrespective of their actual position in an alignment.

Stabilized nucleic acid molecule: A stabilized nucleic acid molecule is a nucleic acid molecule, preferably a DNA or RNA molecule that is modified such, that it is more stable to disintegration or degradation, e.g., by environmental factors or enzymatic digest, such as by an exo- or endonuclease degradation, than the nucleic acid molecule without the modification. Preferably, a stabilized nucleic acid molecule in the context of the present invention is stabilized in a cell, such as a prokaryotic or eukaryotic cell, preferably in a mammalian cell, such as a human cell. The stabilization effect may also be exerted outside of cells, e.g. in a buffer solution etc., for example, in a manufacturing process for a pharmaceutical composition comprising the stabilized nucleic acid molecule.

Transfection: The term "transfection" refers to the introduction of nucleic acid molecules, such as DNA or RNA (e.g. mRNA) molecules, into cells, preferably into eukaryotic cells. In the context of the present invention, the term "transfection" encompasses any method known to the skilled person for introducing nucleic acid molecules into cells, preferably into eukaryotic cells, such as into mammalian cells. Such methods encompass, for example, electroporation, lipofection, e.g. based on cationic lipids and/or liposomes, calcium phosphate precipitation, nanoparticle based transfection, virus based transfection, or transfection based on cationic polymers, such as DEAE-dextran or polyethylenimine etc. Preferably, the introduction is non-viral.

Vaccine: A vaccine is typically understood to be a prophylactic or therapeutic material providing at least one antigen, preferably an immunogen. The antigen or immunogen may be derived from any material that is suitable for vaccination. For example, the antigen or immunogen may be derived from a pathogen. such as from bacteria or virus particles etc., or from a tumor or cancerous tissue. The antigen or immunogen stimulates the body's adaptive immune system to provide an adaptive immune response.

Vector: The term "vector" refers to a nucleic acid molecule, preferably to an artificial nucleic acid molecule. A vector in the context of the present invention is suitable for incorporating or harboring a desired nucleic acid sequence, such as a nucleic acid sequence comprising an open reading frame. Such vectors may be storage vectors, expression vectors, cloning vectors, transfer vectors etc. A storage vector is a vector, which allows the convenient storage of a nucleic acid molecule, for example, of an mRNA molecule. Thus, the vector may comprise a sequence corresponding, e.g., to a desired mRNA sequence or a part thereof, such as a sequence corresponding to the coding sequence and the 3'-UTR of an mRNA. An expression vector may be used for production of expression products such as RNA. e.g. mRNA, or peptides, polypeptides or proteins. For example, an expression vector may comprise sequences needed for transcription of a sequence stretch of the vector, such as a promoter sequence, e.g. an RNA polymerase promoter sequence. A cloning vector is typically a vector that contains a cloning site, which may be used to incorporate nucleic acid sequences into the vector. A cloning vector may be, e.g., a plasmid vector or a bacteriophage vector. A transfer vector may be a vector, which is suitable for transferring nucleic acid molecules into cells or organisms, for example, viral vectors. A vector in the context of the present invention may be, e.g., an RNA vector or a DNA vector. Preferably, a vector is a DNA molecule. Preferably, a vector in the sense of the present application comprises a cloning site, a selection marker, such as an antibiotic resistance factor, and a sequence suitable for multiplication of the vector, such as an origin of replication. Preferably, a vector in the context of the present application is a plasmid vector.

Vehicle: A vehicle is typically understood to be a material that is suitable for storing, transporting, and/or administering a compound, such as a pharmaceutically active compound. For example, it may be a physiologically acceptable liquid, which is suitable for storing, transporting, and/or administering a pharmaceutically active compound.

3'-untranslated region (3'-UTR): Generally, the term "3'-UTR" refers to a part of the artificial nucleic acid molecule, which is located 3' (i.e. "downstream") of an open reading frame and which is not translated into protein. Typically, a 3'-UTR is the part of an mRNA which is located between the protein coding region (open reading frame (ORF) or coding sequence (CDS)) and the poly(A) sequence of the mRNA. In the context of the invention, the term 3'-UTR may also comprise elements, which are not encoded in the template, from which an RNA is transcribed, but which are added after transcription during maturation, e.g. a poly(A) sequence. A 3'-UTR of the mRNA is not translated into an amino acid sequence. The 3'-UTR sequence is generally encoded by the gene. which is transcribed into the respective mRNA during the gene expression process. The genomic sequence is first transcribed into pre-mature mRNA. which comprises optional introns. The pre-mature mRNA is then further processed into mature mRNA in a maturation process. This maturation process comprises the steps of 5'capping, splicing the pre-mature mRNA to excise optional introns and modifications of the 3'-end. such as polyadenylation of the 3'-end of the pre-mature mRNA and optional endo-/ or exonuclease cleavages etc. In the context of the present invention, a 3'-UTR corresponds to the sequence of a mature mRNA, which is located between the stop codon of the protein coding region, preferably immediately 3' to the stop codon of the protein coding region, and the poly(A) sequence of the mRNA. The term "corresponds to" means that the 3'-UTR sequence may be an RNA sequence, such as in the mRNA sequence used for defining the 3'-UTR sequence, or a DNA sequence, which corresponds to such RNA sequence. In the context of the present invention, the term "a 3'-LITR of a gene", such as "a 3'-UTR of a ribosomal protein gene", is the sequence, which corresponds to the 3'-UTR of the mature mRNA derived from this gene, i.e. the mRNA obtained by transcription of the gene and maturation of the pre-mature mRNA. The term "3'-UTR of a gene" encompasses the DNA sequence and the RNA sequence (both sense and antisense strand and both mature and immature) of the 3'-UTR.

5'-untranslated region (5'-UTR): A 5'-UTR is typically understood to be a particular section of messenger RNA (mRNA). It is located 5' of the open reading frame of the mRNA. Typically, the 5'-UTR starts with the transcriptional start site and ends one nucleotide before the start codon of the open reading frame. The 5'-UTR may comprise elements for controlling gene expression, also called regulatory elements. Such regulatory elements may be, for example, ribosomal binding sites. The 5'-UTR may be post-transcriptionally modified, for example by addition of a 5'-CAP. In the context of the present invention, a 5'-LITR corresponds to the sequence of a mature mRNA. which is located between the 5'-CAP and the start codon. Preferably, the 5'-UTR corresponds to the sequence, which extends from a nucleotide located 3' to the 5'-CAP, preferably from the nucleotide located immediately 3' to the 5'-CAP, to a nucleotide located 5' to the start codon of the protein coding region, preferably to the nucleotide located immediately 5' to the start codon of the protein coding region. The nucleotide located immediately 3' to the 5'-CAP of a mature mRNA typically corresponds to the transcriptional start site. The term "corresponds to" means that the 5'-UTR sequence may be an RNA sequence, such as in the mRNA sequence used for defining the 5'-UTR sequence, or a DNA sequence, which corresponds to such RNA sequence. In the context of the present invention, the term "a 5'-UTR of a gene" is the sequence, which corresponds to the 5'-UTR of the mature mRNA derived from this gene, i.e. the mRNA obtained by transcription of the gene and maturation of the pre-mature mRNA. The term "5'-UTR of a gene" encompasses the DNA sequence and the RNA sequence of the 5'-UTR. By the inventive embodiments such a 5' -UTR may be provided 5' -terminal to the coding sequence. Its length is typically less than 500, 400, 300. 250 or less than 200 nucleotides. In other embodiments its length may be in the range of at least 10, 20, 30 or 40, preferably up to 100 or 150, nucleotides.

5'Terminal Oliqopyrimidine Tract (TOP): The 5'terminal oligopyrimidine tract (TOP) is typically a stretch of pyrimidine nucleotides located in the 5' terminal region of a nucleic acid molecule, such as the 5' terminal region of certain mRNA molecules or the 5' terminal region of a functional entity, e.g. the transcribed region, of certain genes. The sequence starts with a cytidine, which usually corresponds to the transcriptional start site, and is followed by a stretch of usually about 3 to 30 pyrimidine nucleotides. For example, the TOP may comprise 3, 4, 5, 6, 7, 8, 9, 10, 11, 12, 13, 14, 15, 16, 17, 18, 19, 20, 21, 22, 23, 24, 25, 26, 27, 28, 28, 30 or even more nucleotides. The pyrimidine stretch and thus the 5' TOP ends one nucleotide 5' to the first purine nucleotide located downstream of the TOP. Messenger RNA that contains a 5'terminal oligopyrimidine tract is often referred to as TOP mRNA. Accordingly, genes that provide such messenger RNAs are referred to as TOP genes. TOP sequences have, for example, been found in genes and mRNAs encoding peptide elongation factors and ribosomal proteins.

TOP motif: In the context of the present invention, a TOP motif is a nucleic acid sequence which corresponds to a 5'TOP as defined above. Thus, a TOP motif in the context of the present invention is preferably a stretch of pyrimidine nucleotides having a length of 3-30 nucleotides. Preferably, the TOP-motif consists of at least 3 pyrimidine nucleotides, preferably at least 4 pyrimidine nucleotides, preferably at least 5 pyrimidine nucleotides, more preferably at least 6 nucleotides, more preferably at least 7 nucleotides, most preferably at least 8 pyrimidine nucleotides, wherein the stretch of pyrimidine nucleotides preferably starts at its 5'end with a cytosine nucleotide. In TOP genes and TOP mRNAs, the TOP-motif preferably starts at its 5'end with the transcriptional start site and ends one nucleotide 5' to the first purin residue in said gene or mRNA. A TOP motif in the sense of the present invention is preferably located at the 5'end of a sequence, which represents a 5'UTR, or at the 5'end of a sequence, which codes for a 5'UTR. Thus, preferably, a stretch of 3 or more pyrimidine nucleotides is called "TOP motif" in the sense of the present invention if this stretch is located at the 5'end of a respective sequence, such as the artificial nucleic acid molecule, the 5'UTR element of the artificial nucleic acid molecule, or the nucleic acid sequence which is derived from the 5'UTR of a TOP gene as described herein. In other words, a stretch of 3 or more pyrimidine nucleotides, which is not located at the 5'-end of a 5'UTR or a 5'UTR element but anywhere within a 5'UTR or a 5'UTR element, is preferably not referred to as "TOP motif".

TOP gene: TOP genes are typically characterised by the presence of a 5' terminal oligopyrimidine tract. Furthermore, most TOP genes are characterized by a growth-associated translational regulation. However, also TOP genes with a tissue specific translational regulation are known. As defined above, the 5'UTR of a TOP gene corresponds to the sequence of a 5'UTR of a mature mRNA derived from a TOP gene. which preferably extends from the nucleotide located 3' to the 5'-CAP to the nucleotide located 5' to the start codon. A 5'UTR of a TOP gene typically does not comprise any start codons, preferably no upstream AUGs (uAUGs) or upstream open reading frames (uORFs). Therein, upstream AUGs and upstream open reading frames are typically understood to be AUGs and open reading frames that occur 5' of the start codon (AUG) of the open reading frame that should be translated. The 5`UTRs of TOP genes are generally rather short. The lengths of 5'UTRs of TOP genes may vary between 20 nucleotides up to 500 nucleotides, and are typically less than about 200 nucleotides, preferably less than about 150 nucleotides, more preferably less than about 100 nucleotides. Exemplary 5'UTRs of TOP genes in the sense of the present invention are the nucleic acid sequences extending from the nucleotide at position 5 to the nucleotide located immediately 5' to the start codon (e.g. the ATG) in the sequences according to SEQ ID NOs. 1-1363 of the patent application WO2013/143700, whose disclosure is incorporated herewith by reference. In this context, a particularly preferred fragment of a 5'UTR of a TOP gene is a 5'UTR of a TOP gene lacking the 5'TOP motif. The terms "5'UTR of a TOP gene" or "5'-T0P UTR" preferably refer to the 5'UTR of a naturally occurring TOP gene.

The present invention provides an RNA comprising at least one coding sequence encoding a tumor antigen, or a fragment or variant of a tumor antigen.

In the context of the present invention, the tumor antigen encoded by the at least one coding sequence of the RNA is preferably an antigen as defined herein, which is derived from or associated with a tumor or a cancer disease. Preferably, the tumor is a malignant tumor. The tumor antigen is preferably an antigen associated with a cancer disease. A tumor antigen as used herein is typically derived from a tumor cell, preferably a mammalian tumor cell. A tumor antigen is preferably located in or on the surface of a tumor cell derived from a mammalian, preferably from a human, tumor, such as a systemic or a solid tumor.

Tumor antigens may also be selected from proteins, which are overexpressed in tumor cells compared to a normal cell. Furthermore, the expression 'tumor antigen' also includes an antigen expressed in cells, which are (were) not themselves (or originally not themselves) degenerated but are associated with the supposed tumor. Antigens, which are connected with tumor-supplying vessels or (re)formation thereof, in particular those antigens, which are associated with neovascularization, e.g. growth factors, such as VEGF, bFGF etc., are also included herein. Further antigens connected with a tumor include antigens from cells or tissues typically embedding the tumor. Further, some substances (usually proteins or peptides) are expressed in patients suffering (knowingly or not-knowingly) from a cancer disease and they occur in increased concentrations in the body fluids of said patients. Some substances may also referred to herein as 'tumor antigens', without being antigens in the strictest sense of an immune response inducing substance. The class of tumor antigens can be divided further into tumor-specific antigens (TSAs) and tumor-associated-antigens (TAAs), both of which are comprised by the expression as used herein. TSAs can only be presented by tumor cells and never by normal "healthy" cells. They typically result from a tumor specific mutation. TAAs, which are more common, are usually presented by both tumor and healthy cells. These antigens are recognized and the antigen-presenting cell can be destroyed by cytotoxic T cells. Additionally, tumor antigens can also occur on the surface of the tumor in the form of, e.g., a mutated receptor. In this case, they can be recognized by antibodies.

As used herein, the term 'tumor antigen' or 'antigen' preferably refers to any one of the antigens provided in Table 1 herein. The at least one coding sequence of the RNA according to the invention thus preferably encodes a tumor antigen selected from the antigens provided in Table 1, or a fragment or variant thereo.

The inventors surprisingly found that the RNA according to the invention is capable of providing expression of the tumor antigen encoded in the at least one coding region upon administration of the RNA to cells or to a patient. It was unexpectedly found that an improved immune response can advantageously be obtained by using the RNA according to the invention as a vaccine against a tumor antigen as defined herein.

Preferably, the at least one coding sequence of the RNA according to the invention encodes a tumor antigen, preferably as defined herein, or a fragment or variant thereof, wherein the tumor antigen is preferably selected from the group consisting of IA01_HLA-A/m; IA02; 5T4; ACRBP; AFP; AKAP4; alpha-actinin-_4/m; alpha-methylacyl-coenzyme_A_racemase; ANDR; ART-4; ARTCI/m; AURKB; B2MG; B3GN5; B4GNI; B7H4: BAGE-1; BASI; BCL-2: bcr/abl: beta-catenin/m: BING-4: BIRC7; BRCAI/m; BY55: calreticulin: CAMEL: CASP-8/m; CASPA: cathepsin_B; cathepsin_L; CD1A; CD1B; CD1C; CD1D; CD1E; CO20; CD22; CD276; CD33; CD3E: CD3Z; CD44_Isofarm_1; CD44_Isofarm_ 6; CD4; CD52: CD55; CD5B; CD80; CD86; CD8A; CDC27/m; CDE3D; CDK4/m: CDKN2A/m; CEA: CEAM6; CH3L2: CLCA2: CML28: CML66; COA-1/m; coactosin-like_protein: collagen.XXIII; COX-2; CP1B1; CSAG2; CT45A1; CT55; CT-_9/8RD6; CTAG2_Isoform_LAGE-1A; CTAG2_Isoform_LAGE-IB; CTCFL: Cten; cyclin_B1; cγclin_D1; cyp-B: DAM-10; DEPIA; E7; EFIA2; EFTUD2/m; EGFR; EGLN3; ELF2/m; EMMPRIN; EpCam: EphA2: EphAS; ErbB3; ERBB4; ERG; ETVG; EWS: EZH2: FABP7: FCGR3A_Version_1; FCER3A_Version_2; FGF5: FGFR2; fibronectin; FDS; FOXP3; FUT1; G250: GAGE-1; GAGE-2; GAGE-3; GAGE-4; GAGE-5; GAGE-6; GAGE7b; GAGE-8_(GAGE-2D); GASR; GnT-V; GPC3; GPNMB/m: GRM3; HAGE; hepsin; Her2/neu: HLA-A2/m: hameobox_NKX3.1; HOM-TES-85; HPG1; HS71A; HS710; HST-2; hTERT; iCE: IF2B3: IL10; IL-13Ra2; IL2-RA: IL2-RB: IL2-RG; IL-5; IMP3: ITA5; ITB1; ITB6; kallikrein-2; kallikrein-3; kallikrein-4; KI20A; KIAA0205; KIF2C; KK-LC-1; LDLR; LGMN; LIRB2; LYGK: MAGA5; MAGAB; MAGAB; MAGE-A10; MAGE-A12; MAGE-A1: MAGE-A2: MAGE-A3: MAGE-A4; MAGE-A6; MAGE-A9: MAGE-B10: MAGE-B16; MAGE-B17; MAGE-_B1; MAGE-B2: MAGE-B3: MAGE-84; MAGE-B5; MAGE-B6; MAGE-C1; MAGE-E2; MAGE-C3; MAGE-D1; MAGE-02; MAGE-D4; MAGE-_E1; MAGE-E1_(MAGE1): MAGE-E2; MAGE-FI: MAGE-H1; MAGEL2: mammaglabin_A; MART-1/melan-A; MART-2; MCI_R: M-CSF: mesothelin; MITF; MMP1_1; MMP7; MUC-1; MUM-11m: MUM-2/m; MYCN; MYO1A; MYO1B: MYO1C; MYO1D: MYDIE; MYO1F; MYO1G; MYO1H; NA17; NA88-A; Neo-PAP; NFYC/m; NGEP; NPM: NRCAM; NSE: NUF2: NY-ESO-1; OA1; OGT; OS-9; osteocalcin; osteopontin; p53; PAGE-4; PAI-1; PAI-2; PAP; PATE: PAX3; PAX5; PD1L1; PDCD1; PDEF; PECA1; PGCB: PGFRB: Pim-1_-Kinase; Pin-1; PLACI; PMEL; PML: POTEF; POTE: PRAME; PRDX5/m; PRM2; prostein; proteinase-3; PSA; PSB9; PSCA; PSGR; PSM: PTPRC; RAB8A; RAGE-1: RARA; RASH: RASK; RASN; RGS5: RHAMM/CD168; RHOC; RSSA; RUI: RU2; RUNXI; S-100; SAGE: SART-_1: SART-2: SART-3; SEPR: SERPINB5; SIA7F; SIA8A: SIAT9; SIRT2/m: SOX10; SP17; SPNXA; SPXN3; SSX-1; SSX-2; SSX3: SSX-4; ST1A1; STAG2; STAMP-1; STEAP-1; Survivin-20; survivin: SYCP1; SYT-SSX-1; SYT-SSX-2; TARP: TCRg; TF2AA; TGFB1; TGFR2; TGM-4; TIE2; TKTLI; TPI/m: TRGV11; TRGV9; TRPC1; TRP-p8; TSG10; TSPYI; TVC_(TRGV3); TX101; tyrosinase; TYRPI; TYRP2: UPA; VEGFRI; WT1; and XAGEI.

It is further preferred that the at least one coding sequence of the RNA of the present invention encodes a tumor antigen, or a fragment or variant thereof, wherein the tumor antigen is an antigen selected from the antigens listed in Table 1. Therein, each row corresponds to an antigen as identified by the respective gene name (first column 'Gene Name') and the database accession number of the corresponding protein (second column 'Protein Accession No.'). The third column ('A') in Table 1 indicates the SEQ ID NO: corresponding to the respective amino acid sequence as provided herein. The SEQ ID NO: corresponding to the nucleic acid sequence of the wild type RNA encoding the antigen is indicated in the fourth column ('8'). The fifth column ('C') provides the SEQ ID NO:'s corresponding to modified nucleic acid sequences of the RNAs as described herein that encode the antigen preferably having the amino acid sequence as defined by the SEQ ID NO: indicated in the third column ('A') or by the database entry indicated in the second column ('Protein Accession No.').

For example, the at least one coding region of the RNA according to the invention may encode the antigen '5T4' (see Table 1, third row), as referenced in the UniProtKB database under 'Q13641' (see Table 1, third row, second column). The full-length amino acid sequence of the 5T4 antigen as used herein is indicated in the third column and is defined by SED ID NO: 3. In the case of an RNA encoding 5T4 or a fragment or variant thereof, the at least one coding region of the RNA may thus comprise or consist of, for example, the nucleic acid sequence according to SEQ ID NO: 507 (see Table 1, third row. fourth column) or a fragment or variant thereof, or, alternatively, any one of the nucleic acid sequences according to SEQ ID NOs: 1011; 1515; 2019; 2523; 3027; 3531; 4035, or a fragment or variant of any one of these sequences.

**Table 1: List of tumor antigens**

| **Gene Name** | **Accession No.** (UniProtKB. NCBI, Genbank or Pubmed) | **A** | **B** | **C** |
|---|---|---|---|---|
| HLA-A | P30443 | 1 | 505 | 1009; 1513: 2017; 2521; 3025; 3529; 4033 |
| HLA-A | P01892 | 2 | 506 | 1010; 1514; 2018; 2522; 3026; 3530; 4034 |
| TPBG | Q13641 | 3 | 507 | 1011; 1515; 2019; 2523; 3027; 3531; 4035 |
| ACRBP | Q8MEB7 | 4 | 508 | 1012; 1516; 2020; 2524; 3028; 3532: 4036 |
| AFP | P02771 | 5 | 509 | 1013; 1517; 2021; 2525; 3029; 3533; 4037 |
| AKAP4 | Q5JQC9 | 6 | 510 | 1014; 1518; 2022; 2526; 3030; 3534; 4038 |
| ACTN4 | B4DSX0 | 7 | 511 | 1015; 1519; 2023; 2527; 3031; 3535; 4039 |
| ACTN4 | B4E337 | 8 | 512 | 1016; 1520; 2024: 2528; 3032: 3536; 4040 |
| ACTN4 | 043707 | 9 | 513 | 1017; 1521; 2025: 2529; 3033; 3537: 4041 |
| AMACR | A0A024RE16 | 10 | 514 | 1018; 1522; 2026; 2530; 3034; 3538; 4042 |
| AMACR | A8KAC3 | 11 | 515 | 1018; 1523; 2027; 2531: 3035; 3539; 4043 |
| AR | P10275 | 12 | 516 | 1020: 1524; 2028; 2532; 3036; 3540; 4044 |
| NOB1 | Q9ULX3 | 13 | 517 | 1021; 1525; 2029; 2533; 3037; 3541; 4045 |
| ART1 | P52961 | 14 | 518 | 1022; 1526; 2030; 2534; 3038: 3542; 4046 |
| AURKB | Q96GD4 | 15 | 519 | 1023; 1527; 2031; 2535: 3039; 3543: 4047 |
| B2M | P61769 | 16 | 520 | 1024; 1528; 2032; 2536: 3040; 3544; 4048 |
| B3GNT5 | Q9BYG0 | 17 | 521 | 1025; 1529; 2033; 2537; 3041: 3545; 4049 |
| B4GALNT1 | Q00973 | 18 | 522 | 1026: 1530: 2034; 2538; 3042; 3546; 4050 |
| VTCN1 | Q7Z7D3 | 19 | 523 | 1027; 1531; 2035; 2539; 3043: 3547; 4051 |
| BAGE | Q13072 | 20 | 524 | 1028: 1532; 2036; 2540; 3044; 3548; 4052 |
| BSG | P35613 | 21 | 525 | 1029; 1533; 2037; 2541; 3045: 3549; 4053 |
| bc12 | A9QXG9 | 22 | 526 | 1030; 1534; 2038; 2542; 3046; 3550; 4054 |
| BCR/ABL | A9UEZ4 | 23 | 527 | 1031; 1535; 2039; 2543; 3047: 3551: 4055 |
| BCR/ABL | A9UEZ7 | 24 | 528 | 1032: 1536; 2040: 2544; 3048: 3552; 4056 |
| BCR/ABL | A9UEZ8 | 25 | 529 | 1033: 1537; 2041; 2545; 3048; 3553; 4057 |
| BCR/ABL | A9UEZ9 | 26 | 530 | 1034; 1538; 2042; 2546; 3050; 3554; 4058 |
| BCR/ABL | A9UF00 | 27 | 531 | 1035; 1538; 2043; 2547; 3051; 3555; 4059 |
| BCR/ABL | A9UF01 | 28 | 532 | 1036: 1540; 2044; 2548; 3052; 3556; 4060 |
| BCR/ABL | A9UF03 | 29 | 533 | 1037; 1541; 2045; 2549; 3053; 3557; 4081 |
| BCR/ABL | A9UF04 | 30 | 534 | 1038: 1542: 2046: 2550; 3054; 3558; 4062 |
| BCR/ABL | A9UF05 | 31 | 535 | 1039; 1543; 2047; 2551; 3055; 3559; 4063 |
| BCR/ABL | A9UF06 | 32 | 536 | 1040; 1544; 2048; 2552; 3056; 3560; 4064 |
| BCR/ABL | A90F08 | 33 | 537 | 1041: 1545: 2049: 2553; 3057: 3561; 4065 |
| CTNNB1 | P35222 | 34 | 538 | 1042; 1546; 2050; 2554; 3058; 3562; 4066 |
| CTNNBL1 | Q8WYA6 | 35 | 539 | 1043; 1547; 2051; 2555; 3059; 3563; 4067 |
| WDR46 | O15213 | 36 | 540 | 1044; 1548; 2052; 2556; 3060; 3564; 4068 |
| BIRC7 | Q96CA5 | 37 | 541 | 1045; 1549; 2053: 2557; 3061; 3565: 4068 |
| BRCA1 | A0A024R1V0 | 38 | 542 | 1046; 1550; 2054; 2558; 3062; 3566; 4070 |
| BRCAI | A0A024R1V7 | 39 | 543 | 1047; 1551; 2055; 2559; 3063; 3567; 4071 |
| BRCAI | A0A024R1ZE | 40 | 544 | 1048; 1552: 2056; 2560; 3064; 3568; 4072 |
| BRCAI | A0A068BFX7 | 41 | 545 | 1049; 1553; 2057; 2561; 3065; 3569; 4073 |
| BRCAI | C6YB45 | 42 | 546 | 1050; 1554; 2058: 2562; 3066: 3570; 4074 |
| BRCAI | C6YB47 | 43 | 547 | 1051; 1555; 2059; 2563: 3067: 3571; 4075 |
| BRCAI | G3XAC3 | 44 | 548 | 1052: 1556; 2060; 2564: 3068: 3572; 4076 |
| CD160 | D95971 | 45 | 549 | 1053; 1557; 2061; 2565; 3069; 3573; 4077 |
| CALR | B4DHRI | 46 | 550 | 1054; 1558; 2062; 2566; 3070; 3574; 4078 |
| CALR | 84E2Y9 | 47 | 551 | 1055; 1559; 2063: 2567: 3071; 3575: 4078 |
| CALR | P27797 | 48 | 552 | 1056: 1560; 2064; 2568; 3072: 3576: 4080 |
| CALR3 | Q96L12 | 49 | 553 | 1057; 1561; 2065; 2569; 3073; 3577; 4081 |
| CAMEL | 095987 | 50 | 554 | 1058; 1562; 2066; 2570; 3074; 3578; 4082 |
| CASP8 | Q14790 | 51 | 555 | 1059; 1563; 2067; 2571; 3075; 3579: 4083 |
| CASP10 | Q92851-4 | 52 | 556 | 1060; 1564; 2068; 2572: 3076: 3580; 4084 |
| CTSB | A0A024R374 | 53 | 557 | 1061; 1565; 2069; 2573: 3077; 3581; 4085 |
| CTSB | P07858 | 54 | 558 | 1062; 1566; 2070; 2574; 3078: 3582; 4086 |
| CTSL | A0A024R276 | 55 | 559 | 1063; 1567; 2071; 2575; 3079; 3583; 4087 |
| CTSL | P07711 | 56 | 560 | 1064: 1568; 2072; 2576; 3080; 3584: 4088 |
| CTSL | Q9HBQ7 | 57 | 561 | 1065; 1569; 2073; 2577; 3081; 3585; 4089 |
| CDIA | P06126 | 58 | 562 | 1066; 1570; 2074; 2578; 3082; 3586; 4090 |
| CDIB | P29016 | 59 | 563 | 1067; 1571; 2075; 2579; 3083; 3587; 4091 |
| CDIC | P29017 | 60 | 564 | 1068; 1572; 2076; 2580; 3084; 3588; 4092 |
| CDID | P15813 | 61 | 565 | 1069; 1573; 2077; 2581: 3085; 3589; 4093 |
| CDIE | P15812 | 62 | 566 | 1070; 1574; 2078; 2582; 3086; 3590; 4094 |
| MS4A1 | P11836 | 63 | 567 | 1071; 1575; 2079: 2583: 3087; 3591; 4095 |
| CD22 | O60926 | 64 | 568 | 1072; 1576; 2080; 2584; 3088; 3592: 4096 |
| CD22 | P20273 | 65 | 569 | 1073; 1577; 2081; 2585; 3089; 3593; 4097 |
| CD22 | Q0EAF5 | 66 | 570 | 1074; 1578; 2082; 2580; 3090; 3594; 4098 |
| CD276 | Q5ZPR3 | 67 | 571 | 1075; 1579: 2083; 2587: 3091; 3595: 4099 |
| C033 | B4DF51 | 68 | 572 | 1076; 1580; 2084; 2588: 3092; 3596; 4100 |
| CD33 | P20138 | 69 | 573 | 1077; 1581; 2085; 2589; 3093; 3597; 4101 |
| CD33 | Q546G0 | 70 | 574 | 1078; 1582; 2086; 2590; 3094; 3598: 4102 |
| CD3E | P07766 | 71 | 575 | 1079: 1583; 2087; 2591; 3095; 3599; 4103 |
| CD247 | P20963 | 72 | 576 | 1080; 1584; 2088; 2592; 3096; 3600; 4104 |
| CD44 | P16070 | 73 | 577 | 1081; 1585; 2089; 2593; 3097; 3601; 4105 |
| CD44 | P16070-6 | 74 | 578 | 1082; 1586; 2090: 2594; 3098; 3602; 4106 |
| CD4 | P01730 | 75 | 579 | 1083: 1587: 2091; 2595: 3099; 3603; 4107 |
| CD52 | P31358 | 76 | 580 | 1084; 1588; 2092; 2596: 3100; 3604; 4108 |
| CDW52 | Q61BD0 | 77 | 581 | 1085; 1589; 2093; 2597; 3101; 3605; 4109 |
| HEL-S-171mP | V9HWN9 | 78 | 582 | 1086; 1590; 2094; 2598; 3102; 3606; 4110 |
| CD55 | BIAP15 | 79 | 583 | 1087; 1591; 2095; 2599; 3103; 3607; 4111 |
| CD55 | D3DT85 | 80 | 584 | 1088; 1592; 2096; 2600; 3104; 3608; 4112 |
| CD55 | D3DT86 | 81 | 585 | 1089; 1593; 2087; 2601; 3105; 3609; 4113 |
| CD55 | P08174 | 82 | 586 | 1080; 1594; 2098; 2602: 3106; 3610; 4114 |
| NCAMI | P13591 | 83 | 587 | 1091; 1595; 2099; 2603; 3107; 3611; 4115 |
| CD80 | A0N0P2 | 84 | 588 | 1092; 1596; 2100; 2604; 3108; 3612; 4116 |
| CD80 | P33681 | 85 | 588 | 1093: 1597; 2101; 2605; 3109; 3613; 4117 |
| CD86 | P42081 | 86 | 590 | 1094; 1598; 2102: 2606; 3110; 3614; 4118 |
| CD8A | P01732 | 87 | 591 | 1095; 1599; 2103; 2607; 3111; 3615; 4119 |
| CDC27 | G5EA36 | 88 | 592 | 1096: 1600; 2104; 2608: 3112; 3616; 4120 |
| CDC27 | P30260 | 89 | 593 | 1097: 1601; 2105; 2609; 3113; 3617; 4121 |
| TNFRSF8 | P28908 | 90 | 594 | 1098; 1602; 2106; 2610; 3114; 3618; 4122 |
| CDK4 | A0A024RBB6 | 91 | 595 | 1099: 1603: 2107: 2611; 3115; 3619; 4123 |
| CDK4 | P11802 | 92 | 596 | 1100; 1604; 2108; 2612; 3116; 3620; 4124 |
| CDK4 | Q6LC83 | 93 | 597 | 1101; 1905; 2109; 2613; 3117; 3621; 4125 |
| CDK4 | Q96BE9 | 94 | 598 | 1102; 1606; 2110; 2914; 3118; 3622: 4126 |
| CDKN2A | DILYX3 | 95 | 599 | 1103: 1607; 2111; 2615: 3119; 3623: 4127 |
| CDKN2A | G3XAG3 | 96 | 600 | 1104; 1608; 2112; 2616; 3120; 3624; 4128 |
| CDKN2A | K7PML8 | 97 | 601 | 1105; 1609: 2112; 2617; 3121; 3625; 4129 |
| CDKN2A | L8E941 | 98 | 602 | 1106; 1610; 2114; 2618; 3122; 3626; 4130 |
| CDKN2A | Q8N726 | 99 | 603 | 1107; 1611; 2115; 2619; 3123: 3627; 4131 |
| CEACAM5 | NP_004354 | 100 | 604 | 1108; 1612; 2116; 2020; 3124; 3628; 4132 |
| CEACAM6 | P40199 | 101 | 605 | 1108; 1613; 2117; 2621; 3125; 3629; 4133 |
| CHI3L2 | Q15782 | 102 | 606 | 1110; 1614; 2118: 2622: 3126: 3630; 4134 |
| CLCA2 | Q9UQC9 | ID3 | 607 | 1111; 1615: 2119; 2623; 3127: 3631; 4135 |
| EXOSC5 | Q9NQT4 | 104 | 608 | 1112; 1916; 2120; 2624; 3128; 3632; 4136 |
| NUDCDI | Q96RS6 | 105 | 609 | 1113; 1617; 2121; 2625; 3129; 3633; 4137 |
| UBXNII | Q5T124 | 106 | 610 | 1114: 1618; 2122; 2626: 3130; 3634: 4138 |
| CDTL1 | Q14019 | 107 | 611 | 1115; 1619; 2123; 2627; 3131; 3635: 4139 |
| COL23A1 | L8EAS4 | 108 | 612 | 1116; 1620; 2124; 2628; 3132: 3636; 4140 |
| CDL23A1 | Q86Y22 | 109 | 613 | 1117; 1621; 2125; 2629: 3133: 3637: 4141 |
| COX-2 | Q6ZYK7 | 110 | 614 | 1118; 1622; 2126; 2630; 3134; 3638; 4142 |
| CYPIB1 | Q16G78 | 111 | 615 | 1119; 1623; 2127: 2631; 3135; 3639; 4143 |
| CSAG2 | Q9Y5P2-2 | 112 | 616 | 1120; 1624; 2128; 2632; 3136; 3640; 4144 |
| CSAG2 | Q9Y5P2 | 113 | 617 | 1121: 1625: 2129; 2633: 3137; 3541; 4145 |
| Cf45A) | U5HYN5 | 114 | 618 | 1122; 1626; 2130: 2634; 3138; 3642; 4146 |
| CT55 | Q8WUE5 | 115 | 619 | 1123; 1627; 2131: 2635: 3139: 3643; 4147 |
| BRDT | Q58F21 | 116 | 620 | 1124: 1628; 2132; 2636; 3140; 3644: 4148 |
| CTAG2 | O75638-2 | 117 | 621 | 1125; 1629; 2133; 2637; 3141; 3645; 4149 |
| CTAG2 | O75638 | 118 | 622 | 1126; 1630; 2134; 2638: 3142; 3646: 4150 |
| CTCFL | Q8NI51 | 119 | 623 | 1127: 1631; 2135; 2639; 3143; 3647; 4151 |
| TNS4 | Q8IZW8 | 120 | 624 | 1128: 1632; 2136; 2640; 3144; 3648: 4152 |
| CCNB1 | P14635 | 121 | 625 | 1129; 1633; 2137; 2941; 3145; 3649; 4153 |
| CCND1 | P24385 | 122 | 626 | 1130: 1634: 2138; 2642; 3146; 3650; 4154 |
| PP1B | P23284 | 123 | 627 | 1131; 1935; 2139; 2643: 3147; 3651; 4155 |
| MAGEB1 | P43366 | 124 | 628 | 1132; 1636; 2140; 2644: 3148; 3652; 4156 |
| DEPD1 | Q5TB30 | 125 | 629 | 1133; 1637; 2141; 2645: 3149; 3653; 4157 |
| E7 | P03129 | 126 | 630 | 1134; 1638: 2142: 2646; 3150; 3654; 4158 |
| E7 | P06788 | 127 | 631 | 1135; 1639; 2143: 2647; 3151; 3655; 4159 |
| E7 | P17387 | 128 | 632 | 1136; 1640; 2144; 2648: 3152; 3656; 4160 |
| E7 | P06429 | 129 | 633 | 1137; 1641; 2145; 2649; 3153; 3657: 4161 |
| E7 | P27230 | 130 | 634 | 1138; 1642; 2145; 2650; 3154; 3658: 4162 |
| E7 | P24837 | 131 | 635 | 1139: 1643; 2147; 2651; 3155; 3659; 4163 |
| E7 | P21736 | 132 | 636 | 1140; 1644: 2148; 2652; 3156; 3660; 4164 |
| E7 | P26558 | 133 | 637 | 1141; 1645; 2149; 2653; 3157; 3661; 4165 |
| E7 | P36831 | 134 | 638 | 1142; 1646; 2150; 2654; 3158; 3662; 4166 |
| E7 | P36833 | 135 | 639 | 1143: 1647; 2151; 2655: 3159; 3663; 4167 |
| E7 | Q9QCZ1 | 130 | 640 | 1144; 1648: 2152: 2656: 3160; 3664: 4168 |
| ORF | Q81965 | 137 | 641 | 1145; 1649; 2153; 2657: 3161; 3665; 4169 |
| E7 | Q180856 | 138 | 642 | 1146; 1650; 2154; 2658; 3162; 3666; 4170 |
| EEF1A2 | Q05639 | 139 | 643 | 1147; 1651; 2155; 2659; 3163; 3667; 4171 |
| EFTUD2 | Q15029 | 140 | 644 | 1148: 1652: 2156; 2660; 3164; 3668; 4172 |
| EGFR | A0A0B4JIY5 | 141 | 645 | 1149: 1653; 2157; 2661; 3165; 3668; 4173 |
| EGFR | E7BSV0 | 142 | 646 | 1150; 1654; 2158; 2662: 3166; 3670; 4174 |
| EGFR | LOR6G1 | 143 | 647 | 1151; 1655; 2159; 2663; 3167; 3671; 4175 |
| EGFR | P00533-2 | 144 | 648 | 1152; 1656; 2160; 2664; 3168; 3672: 4176 |
| EGFR | P00533 | 145 | 649 | 1153; 1657; 2101; 2665; 3159; 3673: 4177 |
| EGFR | Q147T7 | 146 | 650 | 1154; 1658: 2162; 2666; 3170; 3674; 4178 |
| EGFR | Q50408 | 147 | 651 | 1155; 1659: 2163; 2667; 3171; 3675; 4179 |
| EGFR | Q8NDU8 | 148 | 652 | 1156; 1660; 2164; 2668; 3172; 3676: 4180 |
| EGLN3 | Q9H6Z9 | 149 | 653 | 1157: 1661; 2165; 2669; 3173; 3677; 4181 |
| ELF2 | B7Z720 | 150 | 654 | 1158; 1662; 2166; 2670; 3174; 3678; 4182 |
| hEMMPRIN | Q54A51 | 151 | 655 | 1159; 1663; 2167; 2671; 3175; 3679; 4183 |
| EPCAM | P16422 | 152 | 656 | 1160; 1664; 2168; 2672; 3176; 3680; 4184 |
| EPHA2 | P29317 | 153 | 657 | 1161; 1665; 2169; 2673: 3177; 3681; 4185 |
| EPHA3 | P29320 | 154 | 658 | 1162; 1666; 2170; 2674; 3178; 3682: 4186 |
| EPHA3 | Q6P4R6 | 155 | 659 | 1163; 1667; 2171; 2675; 3179; 3683; 4187 |
| ERBB3 | 83KWG5 | 156 | 660 | 1164; 1668; 2172; 2676; 3180: 3684; 4188 |
| ERB83 | B4DGQ7 | 157 | 661 | 1165; 1669; 2173; 2677: 3181; 3685; 4189 |
| ERBB4 | Q15303 | 158 | 662 | 1166; 1670; 2174; 2678; 3182; 3686; 4190 |
| ERG | P11308 | 159 | 663 | 1167; 1671; 2175; 2679; 3183; 3687: 4191 |
| ETV6 | P41212 | 160 | 664 | 1168; 1672; 2176; 2680; 3184; 3688; 4192 |
| EWSRI | Q01844 | 161 | 665 | 1169; 1673; 2177; 2681; 3185; 3689; 4183 |
| EZH2 | F2YMM1 | 162 | 666 | 1170; 1674; 2178; 2682: 3186: 3690; 4194 |
| EZH2 | G3XAL2 | 163 | 667 | 1171; 1675: 2179; 2683: 3187: 3691; 4195 |
| EZH2 | L0R855 | 164 | 668 | 1172; 1676; 2180; 2684; 3188; 3692: 4196 |
| EZH2 | Q15910 | 165 | 669 | 1173; 1677; 2181: 2685: 3188; 3693; 4197 |
| EZH2 | S4S3R8 | 166 | 670 | 1174; 1678; 2182: 2686; 3190: 3694; 4198 |
| FABP7 | O15540 | 167 | 671 | 1175; 1679; 2183; 2687; 3191; 3695; 4199 |
| FCGR3A | P08637 | 168 | 672 | 1176: 1680; 2184; 2688; 3192: 3696; 4200 |
| FCGR3A | CCDS1232.1 | 169 | 673 | 1177; 1681; 2185: 2689; 3193: 3697: 4201 |
| FGF5 | P12034 | 170 | 674 | 1178; 1682; 2186; 2690; 3194; 3698: 4202 |
| FGF5 | Q60518 | 171 | 675 | 1179; 1683; 2187; 2691; 3195; 3699; 4203 |
| FGFR2 | P21802 | 172 | 676 | 1180; 1684; 2188; 2692; 3196; 3700; 4204 |
| LRFN4 | A0A024R516 | 173 | 677 | 1181; 1685; 2189: 2693; 3197; 3701; 4205 |
| ITGA5 | A0A024RB01 | 174 | 678 | 1182; 1686; 2190; 2694; 3198; 3702; 4206 |
| FNDC3A | A0A024R0T9 | 175 | 679 | 1183; 1687; 2191; 2695; 3199; 3703; 4207 |
| FNDC3A | A0A024R0V5 | 176 | 680 | 1184; 1688: 2192: 2696; 3200; 3704; 4208 |
| FANKI | A6NH44 | 177 | 681 | 1185; 1689; 2193: 2697; 3201; 3705; 4209 |
| A8K6A5 | A8K6A5 | 178 | 682 | 1186; 1690; 2194; 2698; 3202; 3706; 4210 |
| B2R627 | B2R627 | 179 | 683 | 1187; 1691; 2195; 2699; 3203: 3707; 4211 |
| B3KXM5 | B3KXM5 | 180 | 684 | 1188: 1692: 2196; 2700: 3204; 3708; 4212 |
| B4DIC5 | B4D1C5 | 181 | 685 | 1189; 1693; 2197; 2701; 3205: 3709; 4213 |
| B4DN21 | B4DN21 | 182 | 686 | 1190; 1694; 2198; 2702; 3206; 3710; 4214 |
| B4DS98 | B4DS98 | 183 | 687 | 1191; 1695; 2199; 2703; 3207; 3711; 4215 |
| B4DTH2 | B4DTH2 | 184 | 688 | 1192; 1686: 2200: 2704; 3208: 3712; 4216 |
| B4DTK1 | B4DTK1 | 185 | 689 | 1193; 1697; 2201; 2705; 3209; 3713; 4217 |
| B4DU16 | B4DU16 | 186 | 690 | 1194; 1698; 2202; 2706; 3210; 3714; 4218 |
| B7Z3W5 | B7Z3W5 | 187 | 691 | 1195; 1699: 2203; 2707; 3211; 3715; 4219 |
| B7Z939 | B7Z939 | 188 | 692 | 1196; 1700; 2204; 2708; 3212; 3716; 4220 |
| FNDC3A | G5E9X3 | 189 | 693 | 1197; 1701; 2205; 2709; 3213; 3717; 4221 |
| Q9H382 | Q9H382 | 190 | 694 | 1198; 1702: 2206; 2710: 3214: 3718; 4222 |
| FOS | P01100 | 191 | 695 | 1199; 1703: 2207: 2711; 3215; 3719; 4223 |
| FOXP3 | Q9BZS1 | 192 | 696 | 1200; 1704; 2208; 2712: 3216; 3720; 4224 |
| FUT1 | P19526 | 193 | 697 | 1201; 1705; 2209; 2713: 3217; 3721; 4225 |
| CA9 | Q16790 | 194 | 698 | 1202; 1706; 2210; 2714; 3218; 3722: 4226 |
| GAGE1 | AAA82744 | 195 | 699 | 1203; 1707; 2211: 2715; 3219; 3723; 4227 |
| GAGE2A | Q6NT46 | 196 | 700 | 1204; 1708; 2212; 2716; 3220; 3724; 4228 |
| GAGE3 | Q13067 | 197 | 701 | 1205; 1709; 2213; 2717: 3221: 3725: 4229 |
| GAGE4 | Q13068 | 198 | 702 | 1206; 1710; 2214; 2718; 3222; 3726; 4230 |
| GAGE5 | Q13069 | 199 | 703 | 1207: 1711; 2215; 2719; 3223; 3727: 4231 |
| GAGE6 | Q13070 | 200 | 704 | 1208; 1712; 2216; 2720; 3224; 3728; 4232 |
| GAGE7 | 076087 | 201 | 705 | 1209; 1713; 2217; 2721; 3225; 3729; 4233 |
| GAGE2D | Q9UEU5 | 202 | 706 | 1210; 1714; 2218; 2722; 3226; 3730; 4234 |
| CCKBR | P32239 | 203 | 707 | 1211; 1715; 2219; 2723; 3227; 3731; 4235 |
| MGATS | Q09328 | 204 | 708 | 1212; 1716; 2220; 2724: 3228: 3732; 4236 |
| GPC3 | I6QTG3 | 205 | 709 | 1213; 1717; 2221; 2725; 3229; 3733; 4237 |
| GPC3 | P51654 | 206 | 710 | 1214: 1718; 2222; 2726; 3230; 3734; 4238 |
| GPC3 | Q81YG2 | 207 | 711 | 1215: 1719; 2223; 2727; 3231; 3735; 4239 |
| GPNMB | A0A024RA55 | 208 | 712 | 1216; 1720; 2224: 2728; 3232; 3736; 4240 |
| GPNMB | Q14956 | 209 | 713 | 1217; 1721; 2225; 2729; 3233: 3737; 4241 |
| GPNMB | Q81XJ5 | 210 | 714 | 1218; 1722; 2226: 2730; 3234: 3738; 4242 |
| GPNMB | Q96F58 | 211 | 715 | 1219; 1723; 2227; 2731; 3235; 3739: 4243 |
| GRM3 | Q14832 | 212 | 716 | 1220; 1724: 2228: 2732; 3236; 3740; 4244 |
| D0X43 | Q9NXZ2 | 213 | 717 | 1221:1725: 2229; 2733: 3237; 3741; 4245 |
| HPN | B2ZDQ2 | 214 | 718 | 1222; 1726; 2230; 2734: 3238; 3742; 4246 |
| HPN | P05981 | 215 | 719 | 1223; 1727; 2231; 2735; 3239; 3743; 4247 |
| ERBB2 | B4DTR1 | 216 | 720 | 1224; 1728; 2232: 2736; 3240; 3744: 4248 |
| ERBB2 | L8E8G2 | 217 | 721 | 1225; 1729; 2233: 2737: 3241; 3745; 4249 |
| ERBB2 | P04626 | 218 | 722 | 1226; 1730; 2234; 2738; 3242; 3746; 4250 |
| HER-2 | Q9UK79 | 219 | 723 | 1227: 1731; 2235: 2739; 3243; 3747; 4251 |
| HLA-A2 | Q95387 | 220 | 724 | 1228; 1732; 2236: 2740; 3244; 3748: 4252 |
| HLA-A2 | Q9MYF8 | 221 | 725 | 1229: 1733; 2237; 2741; 3245; 3740; 4253 |
| NKX3-1 | Q99801 | 222 | 726 | 1230; 1734; 2238; 2742; 3246; 3750: 4254 |
| LUZP4 | B2RBQ6 | 223 | 727 | 1231: 1735; 2239; 2743: 3247; 3751; 4255 |
| LUZP4 | Q9P127 | 224 | 728 | 1232: 1736; 2240; 2744; 3248: 3752: 4256 |
| NDRGI | I2543784 | 225 | 729 | 1233; 1737; 2241: 2745; 3249; 3753; 4257 |
| HSPAIA | P0DMV8 | 226 | 730 | 1234: 1738; 2242; 2746; 3250; 3754; 4258 |
| HSPAIB | P0DMV9 | 227 | 731 | 1235; 1739: 2243; 2747; 3251; 3755; 4259 |
| FGF6 | P10767 | 228 | 732 | 1236; 1740; 2244; 2748; 3252; 3756; 4260 |
| hTERT | O94807 | 229 | 733 | 1237; 1741: 2245: 2749; 3253: 3757; 4261 |
| CES2 | O00748 | 230 | 734 | 1238: 1742: 2246: 2750; 3254; 3758: 4262 |
| IGF2BP3 | O00425 | 231 | 735 | 1239; 1743; 2247; 2751; 3255; 3759; 4263 |
| IL10 | P22301 | 232 | 736 | 1240: 1744: 2248: 2752; 3256: 3760; 4264 |
| IL13RA2 | Q14627 | 233 | 737 | 1241;1745: 2249; 2753; 3257: 3761; 4265 |
| IL2RA | P01589 | 234 | 738 | 1242: 1746; 2250; 2754; 3258; 3762: 4266 |
| IL2RB | P14784 | 235 | 739 | 1243: 1747; 2251; 2755; 3259; 3763; 4267 |
| IL2RG | P31785 | 236 | 740 | 1244; 1748; 2252: 2756; 3260; 3764; 4268 |
| IL5 | P05113 | 237 | 741 | 1245; 1749; 2253; 2757: 3261; 3765; 4269 |
| IMP3 | Q9NV31 | 238 | 742 | 1246; 1750; 2254; 2758; 3262; 3766; 4270 |
| ITGA5 | P08648 | 239 | 743 | 1247: 1751; 2255; 2759; 3263; 3767; 4271 |
| ITGB1 | P05556 | 240 | 744 | 1248; 1752; 2256: 2760; 3264; 3768: 4272 |
| ITGB6 | P18564 | 241 | 745 | 1249; 1753: 2257: 2761; 3265: 3769; 4273 |
| KLK2 | A0A024R4J4 | 242 | 746 | 1250; 1754; 2258: 2762; 3266; 3770; 4274 |
| KLK2 | A0A024R4N3 | 243 | 747 | 1251; 1755; 2259; 2763; 3267; 3771; 4275 |
| KLK2 | B0AZU9 | 244 | 748 | 1252: 1756; 2260; 2764; 3268: 3772: 4276 |
| KLK2 | B4DU77 | 245 | 749 | 1253: 1757: 2261; 2765: 3269; 3773; 4277 |
| KLK2 | P20151 | 246 | 750 | 1254; 1758: 2262: 2766; 3270: 3774; 4278 |
| KLK2 | Q6T774 | 247 | 751 | 1255; 1759; 2263: 2767; 3271; 3775; 4279 |
| KLK2 | Q6T775 | 248 | 752 | 1256: 176D; 2264; 2768; 3272: 3776; 4280 |
| KLK3 | P07288-1 | 4558 | 4561 | 4564; 4567; 4570; 4573; 4576; 4579: 4582; 4590 |
| KLK3 | P07288-2 | 4559 | 4562 | 4565; 4568; 4571; 4574; 4577; 4580; 4583 |
| KLK3 | P07288-3 | 4560 | 4563 | 4566; 4569; 4572; 4575; 4578; 4581; 4584 |
| KLK4 | A0A0C4DFQ5 | 249 | 753 | 1257; 1761; 2265; 2769; 3273: 3777; 4281 |
| KLK4 | Q5BQA0 | 250 | 754 | 1258; 1762; 2266: 2770; 3274; 3778; 4282 |
| KLK4 | Q96PT0 | 251 | 755 | 1259; 1763; 2267: 2771: 3275; 3779; 4283 |
| KLK4 | Q96PT1 | 252 | 756 | 1260; 1764; 2268; 2772; 3276; 3780; 4284 |
| KLK4 | Q9Y5K2 | 253 | 757 | 1261; 1765; 2269; 2773; 3277; 3781; 4285 |
| KIF20A | O95235 | 254 | 758 | 1262; 1766; 2270; 2774; 3278: 3782; 4286 |
| LPGAT1 | Q92604 | 255 | 759 | 1263; 1767: 2271; 2775; 3279; 3783; 4287 |
| KIF2C | Q99661 | 256 | 760 | 1264; 1768; 2272; 2776; 3280; 3784: 4288 |
| CT83 | Q5H943 | 257 | 761 | 1265; 1769: 2273; 2777; 3281; 3785; 4289 |
| LDLR | P01130 | 258 | 762 | 1266; 1770; 2274: 2778; 3282: 3786; 4290 |
| LGMN | Q99538 | 259 | 763 | 1267; 1771; 2275; 2779: 3283; 3787; 4291 |
| LILRB2 | Q8N423 | 260 | 764 | 1268; 1772; 2276; 2780; 3284; 3788: 4292 |
| LY6K | Q17RY6 | 261 | 765 | 1269; 1773; 2277; 2781; 3285; 3789; 4293 |
| MAGEA5 | P43359 | 262 | 766 | 1270: 1774: 2278; 2782: 3286; 3790; 4294 |
| MAGEA8 | P43361 | 263 | 767 | 1271; 1775; 2279; 2783; 3287; 3791: 4295 |
| MAGEA11 | P43364 | 264 | 768 | 1272; 1776; 2280: 2784; 3288; 3782; 4296 |
| MAGEA10 | A0A024RC14 | 265 | 769 | 1273: 1777; 2281; 2785; 3289; 3793; 4297 |
| MAGEA12 | P43365 | 266 | 770 | 1274: 1778: 2282; 2786: 3290; 3794: 4298 |
| MAGEA1 | P43355 | 267 | 771 | 1275; 1779; 2283; 2787; 3291; 3795; 4299 |
| MAGEA2 | P43356 | 268 | 772 | 1276; 1780; 2284; 2788; 3292; 3796: 4300; 4588 |
| MAGEA3 | P43357 | 269 | 773 | 1277: 1781; 2285; 2789; 3293: 3797; 4301; 4587 |
| MAGEA4 | A0A024RC12 | 270 | 774 | 1278; 1782; 2286: 2790; 3294; 3798; 4302 |
| MAGEA4 | P43358 | 271 | 775 | 1279; 1783; 2287; 2791; 3295; 3799: 4303 |
| MAGEA4 | Q1RN33 | 272 | 776 | 1280: 1784; 2288: 2792; 3296: 3800: 4304 |
| MAGEA6 | A8K072 | 273 | 777 | 1281; 1785: 2289; 2793; 3297: 3801; 4305 |
| MAGEA6 | P43360 | 274 | 778 | 1282; 1786; 2290: 2794; 3298; 3802; 4306 |
| MAGEA6 | Q6FH15 | 275 | 779 | 1283; 1787; 2291; 2795; 3299; 3803; 4307 |
| MAGEA8 | P43362 | 276 | 780 | 1284; 1788; 2292; 2796; 3300: 3804; 4308 |
| MAGEBID | Q96LZ2 | 277 | 781 | 1285: 1789; 2293; 2797: 3301; 3805; 4309 |
| MAGEB16 | A2A368 | 278 | 782 | 1286: 1790; 2294; 2798; 3302; 3806; 4310 |
| MAGEB17 | A8MXT2 | 279 | 783 | 1287; 1791; 2295; 2799; 3303; 3807; 4311 |
| MAGE-B1 | Q96TG1 | 280 | 784 | 1288: 1792; 2296; 2800; 3304; 3808; 4312 |
| MAGEB2 | O15479 | 281 | 785 | 1289; 1793; 2297; 2801; 3305; 3809; 4313 |
| MAGEB3 | O15480 | 282 | 786 | 1290; 1794; 2298: 2802; 3306; 3810: 4314 |
| MAGEB4 | O15481 | 283 | 787 | 1291; 1795: 2299; 2803; 3307; 3811; 4315 |
| MAGEB5 | Q9BZ81 | 284 | 788 | 1282; 1796; 2300; 2804; 3308: 3812; 4316 |
| MAGEB6 | Q8N7X4 | 285 | 789 | 1293; 1797; 2301; 2805; 3309: 3813; 4317 |
| MAGEC1 | O60732 | 286 | 790 | 1294; 1798: 2302; 2806; 3310; 3814: 4318 |
| MAGEC2 | Q9UBF1 | 287 | 791 | 1295; 1799; 2303: 2807: 3311; 3815; 4319; 4585 |
| MAGEC3 | Q8TD91 | 288 | 792 | 1296: 1800; 2304; 2808; 3312: 3816; 4320 |
| MAGED1 | Q9Y5V3 | 289 | 793 | 1297; 1801; 2305; 2809: 3313; 3817; 4321 |
| MAGED2 | Q9UNF1 | 290 | 794 | 1298; 1802; 2306: 2810; 3314: 3818; 4322 |
| MAGED4 | Q96JG8 | 291 | 795 | 1299; 1803; 2307; 2811; 3315: 3819; 4323 |
| MAGEE1 | Q61A17 | 292 | 796 | 1300; 1804; 2308; 2812; 3316; 3820; 4324 |
| MAGEE1 | Q9HC15 | 293 | 797 | 1301; 1805; 2309; 2813; 3317; 3821; 4325 |
| MAGEE2 | Q8TD80 | 294 | 798 | 1302; 1806; 2310: 2814; 3318; 3822; 4326 |
| MAGEFI | Q9HAY2 | 295 | 799 | 1303: 1807: 2311; 2815: 3319; 3823: 4327 |
| MAGEHI | Q9H213 | 296 | 800 | 1304; 1808; 2312; 2816; 3320: 3824; 4328 |
| MAGEL2 | Q9UJ55 | 297 | 801 | 1305; 1809; 2313; 2817; 3321; 3825; 4328 |
| SCGB2A2 | Q13296 | 298 | 802 | 1306; 1810; 2314; 2818; 3322: 3826; 4330 |
| SCGB2A2 | Q6NX70 | 299 | 803 | 1307; 1811; 2315; 2819; 3323; 3827; 4331 |
| MLANA | Q16655 | 300 | 804 | 1308; 1812; 2316; 2820; 3324; 3828: 4332 |
| HHAT | Q5YTY9 | 301 | 805 | 1309; 1813; 2317; 2821; 3325; 3829: 4333 |
| MCIR | Q01726 | 302 | 806 | 1310; 1814; 2318; 2822; 3326; 3830; 4334 |
| MCIR | U1JUL4 | 303 | 807 | 1311; 1815; 2319; 2823; 3327; 3831; 4335 |
| MCIR | Q1JUL6 | 304 | 808 | 1312; 1816; 2320: 2824; 3328: 3832; 4336 |
| MCIR | Q1JUL8 | 305 | 809 | 1313; 1817: 2321; 2825; 3329; 3833: 4337 |
| MCIR | Q1JUL9 | 306 | 810 | 1314; 1818; 2322; 2826; 3330; 3834; 4338 |
| MCIR | Q1JUM0 | 307 | 811 | 1315; 1819; 2323; 2827; 3331: 3835; 4339 |
| MCIR | Q1JUM2 | 308 | 812 | 1316; 1820; 2324; 2828: 3332; 3836; 4340 |
| MCIR | Q1JUM3 | 309 | 813 | 1317: 1821; 2325; 2829; 3333: 3837; 4341 |
| MCIR | Q1JUM4 | 310 | 814 | 1318; 1822; 2326; 2830; 3334; 3838; 4342 |
| MCIR | Q1JUM5 | 311 | 815 | 1319; 1823: 2327; 2831; 3335; 3839: 4343 |
| MCIR | Q6UR92 | 312 | 816 | 1320; 1824; 2328; 2832; 3336; 3840; 4344 |
| MCIR | Q6UR94 | 313 | 817 | 1321; 1825; 2329: 2833: 3337: 3841; 4345 |
| MCIR | Q6UR95 | 314 | 818 | 1322; 1826; 2330; 2834; 3338: 3842; 4346 |
| MCIR | Q6UR96 | 315 | 819 | 1323; 1827; 2331; 2835; 3339; 3843; 4347 |
| MCIR | Q6UR97 | 316 | 820 | 1324; 1828; 2332; 2836; 3340: 3844: 4348 |
| MCIR | Q6UR98 | 317 | 821 | 1325: 1829: 2333: 2837; 3341; 3845; 4349 |
| MCIR | Q6UR99 | 318 | 822 | 1326; 1830; 2334; 2838; 3342; 3846; 4350 |
| MCIR | Q6URA0 | 319 | 823 | 1327: 1831; 2335: 2839; 3343; 3847; 4351 |
| MCIR | Q86YW1 | 320 | . 824 | 1328: 1832: 2336: 2840; 3344; 3848; 4352 |
| MCIR | V9Q5S2 | 321 | 825 | 1329; 1833; 2337; 2841; 3345; 3849; 4353 |
| MCIR | V9Q671 | 322 | 826 | 1330: 1834: 2338: 2842: 3346; 3850: 4354 |
| MCIR | V9Q783 | 323 | 827 | 1331;1835; 2339; 2843: 3347; 3851; 4355 |
| MCIR | V9Q7F1 | 324 | 828 | 1332: 1836; 2340; 2844: 3348: 3852; 4356 |
| MCIR | V9Q8N1 | 325 | 829 | 1333: 1837; 2341; 2845; 3349; 3853; 4357 |
| MCIR | V9Q977 | 326 | 830 | 1334: 1838; 2342; 2846; 3350; 3854; 4358 |
| MCIR | V9Q9P5 | 327 | 831 | 1335: 1839; 2343: 2847: 3351; 3855; 4359 |
| MCIR | V9Q9R8 | 328 | 832 | 1336; 1840; 2344; 2848: 3352; 3856; 4360 |
| MCIR | V9QAE0 | 329 | 833 | 1337: 1841; 2345: 2849; 3353; 3857: 4361 |
| MCIR | V9QAR2 | 330 | 834 | 1338: 1842; 2346; 2850: 3354; 3858: 4362 |
| MCIR | V9QAW3 | 331 | 835 | 1339: 1843; 2347; 2851; 3355; 3859; 4363 |
| MCIR | V9QB02 | 332 | 836 | !340: 1844; 2348; 2852: 3356; 3860; 4364 |
| MCIR | V9QB58 | 333 | 837 | 1341; 1845; 2349; 2853; 3357; 3861; 4365 |
| MCIR | V9QBY6 | 334 | 838 | 1342; 1846; 2350; 2854; 3358; 3862; 4366 |
| MCIR | V9QC17 | 335 | 839 | 1343: 1847: 2351: 2855; 3359: 3863: 4367 |
| MCIR | V9QC66 | 336 | 840 | 1344: 1848; 2352; 2856; 3360; 3864; 4368 |
| MCIR | V9QCQ4 | 337 | 841 | 1345; 1849; 2353; 2857; 3361; 3865: 4369 |
| MCIR | V9QDF4 | 338 | 842 | 1346; 1850: 2354; 2858: 3362; 3866; 4370 |
| MCIR | V9QDN7 | 339 | 843 | 1347; 1851; 2355; 2859: 3363; 3867; 4371 |
| MCIR | V9QDQ6 | 340 | 844 | 1348; 1852; 2356; 2860; 3364: 3868; 4372 |
| CSFI | P09603 | 341 | 845 | 1349; 1853; 2357; 2861: 3365; 3869; 4373 |
| MSLN | Q13421 | 342 | 846 | 1350: 1854; 2358: 2862; 3366; 3870; 4374 |
| MIFF | O75030-8 | 343 | 847 | 1351:1855; 2359; 2863; 3367; 3871; 4375 |
| MIFF | O75030-9 | 344 | 848 | 1352; 1856; 2360; 2864: 3368; 3872:4376 |
| MITF | O75030 | 345 | 849 | 1353; 1857; 2361; 2865; 3369; 3873; 4377 |
| MMP11 | B3KQS8 | 346 | 850 | 1354; 1858; 2362; 2866; 3370; 3874; 4378 |
| MMP7 | P09237 | 347 | 851 | 1355; 1859: 2363: 2867; 3371; 3875; 4379 |
| MUC1 | AAA60019 | 348 | 852 | 1356; 1850; 2364; 2868; 3372; 3876: 4380 |
| MUC1 | NP_116242 | 349 | 853 | 1357: 1861; 2365; 2869; 3373; 3877; 4381 |
| TRAPPC) | Q9Y5R8 | 350 | 854 | 1358; 1862; 2366: 2870; 3374; 3878; 4382 |
| MYCN | P04198 | 351 | 855 | 1359; 1863; 2367; 2871; 3375; 3879; 4383 |
| MYDIA | Q9UBC5 | 352 | 856 | 1360; 1864; 2368; 2872: 3376; 3880; 4384 |
| MYDIB | O43795 | 353 | 857 | 1361; 1865; 2369; 2873; 3377: 3881; 4385 |
| MYOIC | O00158 | 354 | 858 | 1362; 1866; 2370; 2874; 3378; 3882; 4386 |
| MYOID | O94832 | 355 | 859 | 1363; 1867; 2371; 2875; 3379; 3883; 4387 |
| MYOIE | Q12965 | 356 | 860 | 1364; 1868; 2372; 2876; 3380; 3884; 4388 |
| MYOIF | O00160 | 357 | 861 | 1365; 1869; 2373; 2877: 3381; 3885; 4389 |
| MYO1G | B011T2 | 358 | 862 | 1366; 1870; 2374: 2878: 3382; 3886; 4390 |
| MYO1H | NP_001094891 | 359 | 863 | 1367; 1871; 2375; 2879; 3383; 3887; 4331 |
| MGAT5B | Q3V5L5 | 360 | 864 | 1368; 1872; 2376; 2880; 3384; 3888; 4392 |
| VENTXPI | I0790436 | 361 | 865 | 1369; 1873; 2377; 2881; 3385; 3889; 4393 |
| PAPOLG | Q9BWT3 | 362 | 866 | 1370; 1874; 2378; 2882; 3386; 3890; 4394 |
| NFYC | Q13952 | 363 | 867 | 1371; 1875; 2379; 2883; 3387; 3891; 4395 |
| AN07 | Q61WH7 | 364 | 868 | 1372; 1876; 2380; 2884; 3388; 3892; 4396 |
| NPM1 | P06748 | 365 | 869 | 1373; 1877; 2381; 2885; 3389; 3893; 4397 |
| NRCAM | Q92823 | 366 | 870 | 1374: 1878; 2382; 2886; 3390; 3894; 4398 |
| EN02 | P09104 | 367 | 871 | 1375; 1879; 2383; 2887; 3391; 3895; 4399 |
| NUF2 | Q9BZD4 | 368 | 872 | 1376; 1880; 2384; 2888; 3392; 3896; 4400 |
| CTAG1A | P78358 | 369 | 873 | 1377; 1881; 2385; 2889; 3393: 3897; 4401; 4586 |
| GPR143 | P51810 | 370 | 874 | 1378; 1882; 2386; 2890; 3394; 3898; 4402 |
| DGT | D15294 | 371 | 875 | 1379; 1883; 2387; 2891; 3395; 3899; 4403 |
| OS9 | B4DH11 | 372 | 876 | 1380: 1884; 2388; 2892: 3396; 3900; 4404 |
| OS9 | B4E321 | 373 | 877 | 1381; 1885; 2389; 2893; 3397; 3901; 4405 |
| OS9 | B7Z8E7 | 374 | 878 | 1382; 1886; 2390; 2894; 3398: 3902; 4406 |
| OS9 | Q13438 | 375 | 879 | 1383; 1887; 2391: 2895; 3399; 3903: 4407 |
| BGLAP | P02818 | 376 | 880 | 1384: 1888: 2392; 2896; 3400; 3904: 4408 |
| SPP) | A0A024RDE2 | 377 | 881 | 1385; 1889; 2393: 2897; 3401; 3905; 4408 |
| SPPI | A0A024RDE6 | 378 | 882 | 1386; 1890; 2394; 2898; 3402: 3906; 4410 |
| SPPI | A0A024RDJ0 | 379 | 883 | 1387; 1891; 2395; 2899; 3403; 3907:4411 |
| SPP1 | B7Z351 | 380 | 884 | 1388; 1892; 2396; 2900; 3404; 3908; 4412 |
| SPPI | F2YQ21 | 381 | 885 | 1389; 1893; 2397; 2901; 3405; 3909; 4413 |
| SPP1 | P10451 | 382 | 886 | 1390; 1894; 2398; 2902; 3406; 3910; 4414 |
| TP53 | P04637 | 383 | 887 | 1391; 1895; 2399; 2903; 3407; 3911; 4415 |
| PAGE4 | 060829 | 384 | 888 | 1392; 1896; 2400; 2904; 3408; 3912: 4416 |
| SERPINE1 | P05121 | 385 | 889 | 1393; 1897; 2401; 2905; 3409; 3913; 4417 |
| SERPINB2 | P05120 | 386 | 890 | 1394; 1898; 2402; 2906; 3410; 3914; 4418 |
| REG3A | Q06141 | 387 | 891 | 1395; 1899; 2403; 2907; 3411; 3915; 4419 |
| PAP | Q53S56 | 388 | 892 | 1396: 1900; 2404; 2908; 3412; 3916; 4420 |
| PATE1 | Q8WXA2 | 389 | 893 | 1387: 1901: 2405; 2909: 3413; 3917; 4421 |
| PAX3 | P23760 | 390 | 894 | 1398; 1902; 2406; 2910: 3414; 3918; 4422 |
| PAX5 | Q02548 | 391 | 895 | 1399; 1903; 2407; 2911; 3415; 3919: 4423 |
| CD274 | Q9NZQ7 | 392 | 896 | 1400: 1904; 2408; 2912; 3416: 3920; 4424 |
| PDCD1 | Q15116 | 393 | 897 | 1401: 1905; 2409; 2913; 3417; 3921; 4425 |
| SPDEF | O95238 | 394 | 898 | 1402; 1906; 2410; 2914; 3418; 3922; 4426 |
| PECAMI | P16284 | 395 | 899 | 1403; 1907; 2411: 2915; 3419; 3923; 4427 |
| BCAN | Q96GW7 | 396 | 900 | 1404; 1908; 2412; 2916; 3420; 3924: 4428 |
| PDGFRB | P09619 | 397 | 901 | 1405: 1909; 2413: 2917; 3421; 3925; 4429 |
| PIM1 | A0A024RD25 | 398 | 902 | 1406; 1910; 2414: 2918: 3422; 3926: 4430 |
| PINIPI | O15428 | 399 | 903 | 1407; 1911; 2415; 2919; 3423; 3927; 4431 |
| PIN1 | Q13526 | 400 | 904 | 1408; 1912; 2416; 2920; 3424; 3928; 4432 |
| PIN1 | Q49AR7 | 401 | 905 | 1409; 1913; 2417: 2921; 3425; 3929; 4433 |
| PLAC1 | Q9HBJ0 | 402 | 906 | 1410; 1914: 2418; 2922: 3426; 3930; 4434 |
| PMEL | P40967 | 403 | 907 | 1411; 1915; 2419; 2923; 3427; 3931; 4435; 4591 |
| PML | P29590 | 404 | 908 | 1412; 1916; 2420; 2924: 3428; 3932: 4436 |
| POTEF | A5A3E0 | 405 | 909 | 1413; 1917; 2421; 2925; 3429: 3933; 4437 |
| POTED | Q86YR6 | 406 | 910 | 1414; 1918; 2422; 2926; 3430: 3934; 4438 |
| PRAME | A0A024RIE6 | 407 | 911 | 1415; 1919; 2423; 2927; 3431; 3935; 4439 |
| PRAME | P78395 | 408 | 912 | 1416; 1920; 2424; 2928; 3432; 3936; 4440 |
| PRDX5 | P30044 | 409 | 913 | 1417; 1921; 2425; 2929; 3433: 3937; 4441 |
| PRM2 | P04554 | 410 | 914 | 1418; 1922; 2426; 2930; 3434: 3938; 4442 |
| SLC45A3 | Q96JT2 | 411 | 915 | 1419; 1923; 2427; 2931; 3435; 3939: 4443 |
| PRTN3 | D6CHE9 | 412 | 916 | 1420; 1924; 2428; 2932; 3436; 3940; 4444 |
| PRTN3 | P24158 | 413 | 917 | 1421; 1925; 2429; 2933; 3437; 3941: 4445 |
| NPEPPS | P55786 | 414 | 918 | 1422: 1926; 2430; 2934; 3438: 3942; 4446 |
| PSMB9 | P28065 | 415 | 919 | 1423; 1927; 2431: 2935; 3439; 3943: 4447 |
| PSCA | D3DW16 | 416 | 920 | 1424; 1928; 2432; 2936; 3440; 3944: 4448 |
| PSCA | O43653 | 417 | 921 | 1425; 1929; 2433; 2937; 3441; 3945; 4449 |
| OR51E2 | Q9H255 | 418 | 922 | 1426; 1930; 2434; 2938: 3442; 3946; 4450 |
| FOLH1 | Q04609 | 419 | 923 | 1427: 1931: 2435: 2939: 3443; 3947; 4451; 4588 |
| PTPRC | NP_002829 | 420 | 924 | 1428; 1932: 2436: 2940; 3444; 3948; 4452 |
| RAB8A | P61006 | 421 | 925 | 1429: 1933; 2437; 2941; 3445; 3949; 4453 |
| MOK | Q9UQ07 | 422 | 926 | 1430; 1934; 2438; 2942; 3446; 3950; 4454 |
| RARA | P10276 | 423 | 927 | 1431: 1935; 2439; 2943; 3447; 3951; 4455 |
| HRAS | P01112 | 424 | 928 | 1432: 1936; 2440: 2944; 3448; 3952; 4456 |
| KRAS | P01116 | 425 | 929 | 1433: 1937; 2441; 2945: 3449: 3953; 4457 |
| NRAS | P01111 | 426 | 930 | 1434: 1938; 2442: 2946; 3450; 3954; 4458 |
| RGS5 | 015539 | 427 | 931 | 1435; 1939; 2443: 2947; 3451; 3955; 4459 |
| HMMR | 075330 | 428 | 932 | 1436; 1940; 2444; 2948; 3452: 3956; 4460 |
| RHOC | P08134 | 429 | 933 | 1437: 1941; 2445: 2949; 3453; 3957; 4461 |
| RPSA | P08865 | 430 | 934 | 1438: 1942: 2446; 2950; 3454: 3958; 4462 |
| SFMBT1 | Q9UHJ3 | 431 | 935 | 1439: 1943; 2447; 2951; 3455; 3959: 4463 |
| DCDC2 | Q9UHG0 | 432 | 936 | 1440; 1944; 2448; 2952; 3456; 3960; 4464 |
| RUNXI | Q01196 | 433 | 937 | 1441:1945; 2449: 2953; 3457; 3961: 4465 |
| HEL-S-100 | V9HW39 | 434 | 938 | 1442: 1946; 2450; 2954; 3458: 3962: 4466 |
| SAGEI | Q9NXZ1 | 435 | 939 | 1443: 1947; 2451; 2955; 3459; 3963; 4467 |
| SART1 | O43290 | 436 | 940 | 1444; 1948; 2452; 2956; 3460; 3964; 4468 |
| DSE | Q9UL01 | 437 | 941 | 1445: 1949; 2453: 2957; 3461; 3965; 4469 |
| SART3 | Q15020 | 438 | 942 | 1446: 1950: 2454; 2958; 3462; 3966: 4470 |
| FAP | Q12884 | 439 | 943 | 1447; 1951; 2455: 2959; 3463; 3967; 4471 |
| SERPINB5 | P36952 | 440 | 944 | 1448; 1952; 2456; 2960; 3464; 3968: 4472 |
| ST6GALNAC6 | 0969X2 | 441 | 945 | 1449: 1953: 2457: 2961; 3465; 3969; 4473 |
| ST8SIA1 | 092185 | 442 | 946 | 1450:1954; 2458; 2962; 3466; 3970; 4474 |
| ST3GAL5 | Q9UNP4 | 443 | 947 | 1451: 1955; 2459; 2963; 3467: 3971; 4475 |
| SIRT2 | A0A024R0G8 | 444 | 948 | 1452; 1956; 2460: 2964; 3468; 3972; 4476 |
| SIRT2 | Q8IXJ6 | 445 | 949 | 1453: 1957: 2461; 2965; 3469: 3973: 4477 |
| S0X10 | P56693 | 446 | 950 | 1454: 1958; 2462; 2966; 3470; 3974; 4478 |
| SPA17 | Q15506 | 447 | 951 | 1455: 1959; 2463: 2967; 3471; 3975: 4479 |
| SPANXA1 | Q9NS26 | 448 | 952 | 1456: 1960; 2464: 2968; 3472; 3976; 4480 |
| SPANXN3 | Q5MJ09 | 449 | 953 | 1457; 1961; 2465; 2969; 3473; 3977; 4481 |
| SSX1 | Q16384 | 450 | 954 | 1458: 1962; 2466; 2970; 3474: 3978; 4482 |
| SSX2 | Q16385 | 451 | 955 | 1459: 1963; 2467; 2971; 3475; 3979; 4483 |
| SSX3 | Q99909 | 452 | 956 | 1460: 1904; 2468; 2972: 3476: 3980; 4484 |
| SSX4 | O60224 | 453 | 957 | 1461;1965; 2469; 2973; 3477; 3981; 4485 |
| SULTIA1 | P50225 | 454 | 958 | 1462: 1966: 2470; 2974; 3478: 3982; 4486 |
| STAG2 | Q8N304-2 | 455 | 959 | 1463; 1967; 2471: 2975: 3479; 3983; 4487 |
| STEAP2 | Q8NFT2 | 456 | 960 | 1464; 1968; 2472; 2976; 3480; 3984; 4488 |
| STEAP1 | ADAD24RA63 | 457 | 961 | 1465; 1969; 2473: 2977; 3481; 3985; 4489 |
| STEAP1 | Q9UHE8 | 458 | 962 | 1466: 1970: 2474; 2978; 3482; 3986; 4490 |
| BIRC5 | O15392-2 | 459 | 963 | 1467; 1971; 2475; 2979; 3483; 3987; 4491 |
| BIRC5 | O15392 | 460 | 964 | 1468: 1972; 2476: 2980: 3484; 3988; 4492 |
| SYCP1 | A0A024R012 | 461 | 965 | 1469; 1973: 2477; 2981; 3485; 3989; 4493 |
| SYCPI | B7ZLS9 | 462 | 966 | 1470; 1974; 2478: 2982; 3486; 3990; 4494 |
| SYCP1 | QI5431 | 463 | 967 | 1471: 1975; 2479: 2983; 3487: 3991; 4495 |
| SYCP1 | Q3MHC4 | 464 | 968 | 1472; 1976; 2480: 2984; 3488; 3992; 4496 |
| SYT-SSX1 | A4PIV7 | 465 | 969 | 1473; 1977: 2481; 2985; 3489; 3993; 4497 |
| SYT-SSX1 | A4PIV8 | 466 | 970 | 1474; 1978; 2482: 2986; 3490; 3994: 4498 |
| SYT-SSX2 | A4PIV9 | 467 | 971 | 1475: 1979: 2483: 2987; 3491; 3995; 4499 |
| SYT-SSX2 | A4PIWO | 468 | 972 | 1476: 1980; 2484: 2988; 3492; 3996; 4500 |
| TARP | Q0VGM3 | 469 | 973 | 1477: 1981; 2485: 2989; 3493; 3997; 4501 |
| TCRg | A2JGV3 | 470 | 974 | 1478; 1982: 2486: 2990; 3494; 3998: 4502 |
| GTF2A1 | P52655 | 471 | 975 | 1479; 1983; 2487; 2991; 3495; 3999; 4503 |
| TGFB1 | P01137 | 472 | 976 | 1480: 1984: 2488: 2992; 3496; 4000; 4504 |
| TGFBR2 | P37173 | 473 | 977 | 1481; 1985; 2489; 2993; 3497; 4001: 4505 |
| TGM4 | B2R7D1 | 474 | 978 | 1482: 1986; 2490; 2994; 3498; 4002; 4506 |
| TEK | Q02763 | 475 | 979 | 1483; 1987; 2491; 2995: 3499: 4003; 4507 |
| TKTLI | P51854 | 476 | 980 | 1484: 1988; 2492; 2996; 3500; 4004: 4508 |
| TPII | P60174 | 477 | 981 | 1485; 1989; 2493; 2997; 3501; 4005: 4509 |
| TRGVII | Q99601 | 478 | 982 | 1486: 1990; 2494; 2998; 3502; 4006; 4510 |
| LOC402482 | A4DIX2 | 479 | 983 | 1487; 1991; 2495; 2999; 3503; 4007; 4511 |
| TRGV9 | Q99603 | 480 | 984 . | 1488; 1992; 2496; 3000; 3504: 4008; 4512 |
| TRGV9 | Q99604 | 481 | 985 | 1489; 1993; 2497; 3001; 3505: 4009; 4513 |
| TRPC1 | P48995 | 482 | 986 | 1490: 1994: 2498; 3002; 3506; 4010; 4514 |
| TRPM8 | Q7Z2W7 | 483 | 987 | 1491:1995; 2499; 3003: 3507: 4011; 4515 |
| TSGAI0 | Q9BZW7 | 484 | 988 | 1482: 1996; 2500; 3004; 3508; 4012; 4516 |
| TSPY1 | Q01534 | 485 | 989 | 1493: 1997: 2501: 3005: 3509; 4013; 4517 |
| TRGV3 | M13231.1 | 486 | 990 | 1494; 1998; 2502; 3006: 3510; 4014; 4518 |
| TEXIO1 | Q9BY14-2 | 487 | 991 | 1495: 1999; 2503; 3007; 3511; 4015; 4519 |
| TYR | A0A024DBG7 | 488 | 992 | 1496: 2000: 2504: 3008: 3512; 4016; 4520 |
| TYR | L8BD82 | 489 | 993 | 1497; 2001; 2505; 3009: 3513; 4017; 4521 |
| TYR | L8B086 | 490 | 994 | 1498; 2002: 2506; 3010: 3514; 4018; 4522 |
| TYR | L8B089 | 491 | 995 | 1499; 2003; 2507; 3011; 3515; 4019; 4523 |
| TRP-2 | O75767 | 492 | 996 | 1500; 2004; 2508: 3012; 3516; 4020; 4524 |
| TYR | P14679 | 493 | 997 | 1501; 2005: 2509; 3013; 3517: 4021: 4525 |
| TYR | U3M8N0 | 494 | 998 | 1502: 2006; 2510; 3014; 3518; 4022; 4526 |
| TYR | U3M905 | 495 | 999 | 1503; 2007; 2511; 3015; 3519; 4023; 4527 |
| TYR | U3M9J2 | 496 | 1000 | 1504: 2008: 2512; 3016; 3520: 4024; 4528 |
| TYRP1 | P17643 | 497 | 1001 | 1505; 2009; 2513; 3017: 3521; 4025: 4529 |
| DCT | P40126 | 498 | 1002 | 1506; 2010; 2514; 3018; 3522; 4026; 4530 |
| PRAPI | Q96NZ9 | 499 | 1003 | 1507; 2011; 2515; 3019; 3523; 4027; 4531 |
| VEGFRI | B5A924 | 500 | 1004 | 1508: 2012: 2516; 3020; 3524; 4028; 4532 |
| WTI | A0A0H5AUY0 | 501 | 1005 | 1509; 2013; 2517: 3021; 3525; 4029; 4533 |
| WTI | P19544 | 502 | 1006 | 1510; 2014; 2518; 3022; 3526; 4030; 4534 |
| WTI-AS | Q06250 | 503 | 1007 | 1511; 2015; 2519; 3023; 3527; 4031; 4535 |
| XAGEIA | Q9HD64 | 504 | 1008 | 1512; 2016; 2520; 3024; 3528: 4032; 4536 |

| | | | | |
|---|---|---|---|---|
| Legend to Table 1: Column 'A': amino acid sequence SEQ ID NO:; column 'B': RNA sequence SEQ ID NO: (wild type RNA); column 'C': RNA sequence SEQ ID NO: (modified RNA). | | | | |

It is thus further preferred that the at least one coding sequence of the RNA of the present invention encodes a tumor antigen, or a fragment or variant thereof, wherein the tumor antigen is an antigen selected from the antigens listed in Table 1. preferably an antigen selected from the antigens defined by the database accession number provided under the respective column in Table 1. More preferably, the at least one coding sequence of the RNA according to the invention comprises or consists any one of the nucleic acid sequences provided in Table 1, or a fragment or variant of any one of these sequences, preferably as defined herein.

According to a preferred embodiment, the present invention concerns an RNA comprising at least one coding sequence encoding a tumor antigen, or a fragment or variant of a tumor antigen, wherein the tumor antigen preferably comprises or consists of any one of the amino acid sequences defined in the third column (column 'A') of Table 1, or a fragment or variant of any one of these sequences. In other words, the at least one coding sequence preferably encodes a tumor antigen comprising or consisting of an amino acid sequence selected from the group consisting of SEQ ID NDs: 1 - 504, or a fragment or variant of any one of said amino acid sequences.

The at least one coding sequence of the RNA according to the invention preferably comprises a nucleic acid sequence encoding a full-length tumor antigen or a full-length variant of a tumor antigen as defined herein. The term 'full-length (tumor) antigen' or 'full-length variant of a (tumor) antigen' as used herein typically refers to an antigen that substantially comprises the entire amino acid sequence of the naturally occuring tumor antigen. As used herein, the term 'full-length (tumor) antigen' preferably relates to the full-length sequence of an antigen indicated in Table 1. More preferably, the term 'full-length (tumor) antigen' preferably refers to an amino acid sequence as defined by any one of the SEQ ID NO:'s listed under the third column ('A') of Table 1 or to an amino acid provided in the database under the respective database accession number.

Alternatively, the at least one coding sequence of the RNA according to the invention may also comprise a nucleic acid sequence encoding a fragment of a tumor antigen or a fragment of a variant of a tumor antigen as defined herein.

In the context of the present invention, a 'fragment' of an antigen or of a variant thereof may comprise a sequence of an antigen or of a variant thereof as defined above, which is, with regard to its amino acid sequence (or its encoded nucleic acid sequence), N-terminally, C-terminally and/or intrasequentially truncated compared to the amino acid sequence of the naturally occuring protein or a variant thereof (or its encoded nucleic acid sequence). Such truncation may thus occur either on the amino acid level or on the nucleic acid level, respectively. A sequence identity with respect to such a fragment as defined herein therefore preferably refers to the entire antigen or a variant thereof as defined herein or to the entire (coding) nucleic acid sequence of such an antigen or of a variant thereof.

A fragment of an antigen or of a variant thereof in the context of the present invention may furthermore comprise a sequence of an antigen or of a variant thereof as defined herein, which has a length of about 5 to about 20 or even more amino acids and which is preferably processed and presented by an MHC complex. Preferably, a fragment of an antigen or of a variant thereof in the context of the present invention may furthermore comprise a sequence of an antigen or of a variant thereof as defined above, which has a length of about 6 to about 20 or even more amino acids, e.g. a fragment as processed and presented by MHC class I molecules, preferably having a length of about 8 to about 10 amino acids, e.g. 8, 9, or 10, (or even 6, 7. 11, or 12 amino acids), or a fragment as processed and presented by MHC class II molecules, preferably having a length of about 13 or more amino acids, e.g. 13, 14, 15, 16, 17, 18, 19, 20 or even more amino acids, wherein the fragment may be selected from any part of the amino acid sequence. These fragments are typically recognized by T-cells in the form of a complex consisting of the peptide fragment and an MHC molecule, i.e. the fragments are typically not recognized in their native form.

Preferably, a fragment or a variant of an antigen is a functional fragment or a functional variant, which comprises at least one functional epitope of the corresponding antigen capable of inducing an adaptive immune response in a subject. Such a functional epitope may be a confirmed epitope by any biological assay or a predicted epitope by any possibility to predict epitopes (e.g. computer analysis of the antigen).

A fragment of an antigen or of a variant thereof as defined herein preferably comprises at least one epitope of the antigen or of the variant thereof. An epitope (also called "antigen determinant") in the context of the present invention is typically a fragment located on the outer surface of a (native) antigen or of a variant thereof as defined herein, preferably having 5 to 20 amino acids, more preferably having 5 to 15 or 5 to 12 amino acids, even more preferably having 6 to 9 amino acids, which may be recognized by antibodies or B-cell receptors, i.e. in their native form. Such an epitopeof an antigen or of a variant thereof may furthermore be selected from any of the herein mentioned variants of such antigens. In this context, an antigenic determinant can be a conformational or discontinous epitope, which is composed of segments of an antigen or of a variant thereof as defined herein that are discontinuous in the amino acid sequence of the antigen or of the variant as defined herein but are brought together in the three-dimensional structure. Alternatively, an antigenic determinant may also be a continuous or linear epitope. which is composed of a single polypeptide chain.

According to a preferred embodiment of the invention, the RNA comprises at least one coding sequence encoding a variant of a tumor antigen as defined herein, or a fragment of a variant of a tumor antigen.

In certain embodiments of the present invention, a 'variant' of a tumor antigen or a fragment thereof as defined herein may be encoded by the RNA comprising at least one coding sequence as defined herein, wherein the amino acid sequence encoded by the at least one coding sequence differs in at least one amino acid residue from the naturally occuring amino acid sequence. In this context, the 'change' in at least one amino acid residue may consist, for example, in a mutation of an amino acid residue to another amino acid, a deletion or an insertion. More preferably, the term 'variant' as used in the context of the amino acid sequence encoded by the at least one coding sequence of the RNA according to the invention comprises any homolog, isoform or transcript variant of the tumor antigen or a fragment thereof as defined herein, wherein the homolog, isoform or transcript variant is preferably characterized by a degree of identity or homology, respectively, as defined herein.

Preferably, a variant of a tumor antigen or a fragment thereof may be encoded by the RNA comprising at least one coding sequence as defined herein, wherein at least one amino acid residue of the amino acid sequence encoded by the at least one coding sequence is substituted. Substitutions, wherein amino acids, which originate from the same class, are exchanged for one another, are called conservative substitutions. In particular, these are amino acids having aliphatic side chains, positively or negatively charged side chains, aromatic groups in the side chains or amino acids, the side chains of which can form hydrogen bridges, e.g. side chains which have a hydroxyl function. By conservative constitution, e.g. an amino acid having a polar side chain may be replaced by another amino acid having a corresponding polar side chain. or, for example, an amino acid characterized by a hydrophobic side chain may be substituted by another amino acid having a corresponding hydrophobic side chain (e.g. serine (threonine) by threonine (serine) or leucine (isoleucine) by isoleucine (leucine)). In a preferred embodiment, a variant of a tumor antigen or a fragment thereof may be encoded by the RNA according to the invention, wherein at least one amino acid residue of the amino acid sequence encoded by the at least one coding sequence comprises at least one conservative substitution compared to the respective naturally occuring sequence. These amino acid sequences as well as their encoding nucleic acid sequences in particular are comprised by the term 'variant' as defined herein.

Insertions, deletions and/or non-conservative substitutions are also possible, in particular, at those sequence positions, which cause no modification to the three-dimensional structure or do not affect the binding region. Modifications to a three-dimensional structure by insertion(s) or deletion(s) can easily be determined e.g. using CD spectra (circular dichroism spectra) (Urry, 1985, Absorption, Circular Dichroism and ORD of Polypeptides, in: Modern Physical Methods in Biochemistry, Neuberger et al. (ed.). Elsevier, Amsterdam).

In order to determine the percentage, to which two sequences (nucleic acid sequences, e.g. RNA or mRNA sequences as defined herein. or amino acid sequences, preferably the amino acid sequence encoded by the RNA according to the invention) are identical, the sequences can be aligned in order to be subsequently compared to one another. For this purpose, e.g. gaps can be inserted into the sequence of the first sequence and the component at the corresponding position of the second sequence can be compared. If a position in the first sequence is occupied by the same component as is the case at a corresponding position in the second sequence, the two sequences are identical at this position. The percentage, to which two sequences are identical, is a function of the number of identical positions divided by the total number of positions. The percentage, to which two sequences are identical, can be determined using a mathematical algorithm. A preferred, but not limiting, example of a mathematical algorithm, which can be used is the algorithm of Karlin et al. (1993), PNAS USA. 90:5873-5877 or Altschul et al. (1997), Nucleic Acids Res., 25:3389-3402. Such an algorithm is integrated, for example, in the BLAST program. Sequences, which are identical to the sequences of the present invention to a certain extent, can be identified by this program.

A fragment of a tumor antigen or a variant thereof encoded by the at least one coding sequence of the RNA according to the invention may typically comprise an amino acid sequence having a sequence identity of at least 5%. 10%. 20%, 30%, 40%, 50%, 60%, 70%, 80%, 85%, 86%. 87%, 88%, 89%, 90%. 91%, 92%, 93%, 94%. 95%, 96%, 97%, 98%, or 99%, preferably of at least 70%, more preferably of at least 80%, even more preferably at least 85%, even more preferably of at least 90% and most preferably of at least 95% or even 97%. with an amino acid sequence of the respective naturally occuring full-length antigen or a variant thereof.

More preferably, a fragment of a tumor antigen or a variant thereof encoded by the at least one coding sequence of the RNA according to the invention may typically comprise or consist of an amino acid sequence having a sequence identity of at least 5%, 10%. 20%, 30%, 40%, 50%, 60%. 70%. 80%. 85%, 86%. 87%, 88%. 89%. 90%, 91%, 92%, 93%, 94%. 95%. 96%. 97%, 98%, or 99%, preferably of at least 70%, more preferably of at least 80%, even more preferably at least 85%, even more preferably of at least 90% and most preferably of at least 95% or even 97%, with an amino acid sequence of an antigen selected from the antigens indicated in Table I or a variant thereof. Even more preferably, a fragment of a tumor antigen or a variant thereof encoded by the at least one coding sequence of the RNA according to the invention may typically comprise or consist of an amino acid sequence having a sequence identity of at least 5%, 10%. 20%. 30%. 40%. 50%, 60%, 70%, 80%, 85%, 86%, 87%, 88%, 89%. 80%. 91%. 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with any one of the amino acid sequences defined in the third column (column 'A') of Table 1, or a fragment or variant of any one of these sequences.

Most preferably, a fragment of a tumor antigen or a variant thereof encoded by the at least one coding sequence of the RNA according to the invention typically comprises or consists of an amino acid sequence having a sequence identity of at least 80% with any one of the amino acid sequences defined in the third column (column 'A') of Table 1, or a fragment or variant of any one of these sequences.

Preferably, the tumor antigen encoded by the at least one coding sequence of the RNA is an antigen as defined herein, which is encoded by a nucleic acid sequence comprising or consisting of any one of the nucleic acid sequences defined in Table 1, preferably in the fourth or fifth column (column 'B' or 'C', respectively) of Table 1, or a fragment or variant of any one of these sequences. In other words, the tumor antigen encoded by the at least one coding sequence of the RNA is preferably an antigen as defined herein, which is preferably encoded by a nucleic acid sequence comprising or consisting of a nucleic acid sequence selected from the group consisting of SEQ ID NOs: 505 - 4033; 4561 - 4591 or a fragment or variant of any of these sequence.

In a preferred embodiment, the present invention thus provides an RNA comprising at least one coding sequence, wherein the coding sequence comprises or consists any one of the nucleic acid sequences defined in Table 1, preferably in the fourth or fifth column (column 'B' or 'C', respectively) of Table 1, or a fragment or variant of any one of these sequences.

In certain embodiments, the RNA according to the invention, preferably the at least one coding sequence of the RNA according to the invention, may comprise or consist of a fragment of a nucleic acid sequence encoding a tumor antigen or a fragment or variant thereof as defined herein. Preferably, the at least one coding sequence of the RNA according to the invention comprises or consists of a fragment, preferably as defined herein, of any one of the nucleic acid sequences defined in Table 1, preferably in the fourth or fifth column (column 'B' or 'C', respectively) of Table 1, or a fragment or variant of any one of these sequences.

In this context, a 'fragment of a nucleic acid sequence' is preferably a nucleic acid sequence encoding a fragment of a tumor antigen or of a variant thereof as described herein. More preferably, the expression 'fragment of a nucleic acid sequence' refers to a nucleic acid sequence having a sequence identity of at least 5%, 10%, 20%, 30%, 40%, 50%, 60%, 70%, 80%. 85%, 86%, 87%, 88%, 89%, 90%, 91%, 92%, 93%, 94%, 95%, 96%, 97%, 98%, or 99°/, preferably of at least 70%, more preferably of at least 80%, even more preferably at least 85%, even more preferably of at least 90% and most preferably of at least 95% or even 97%, with a respective full-length nucleic acid sequence.

In another preferred embodiment, the RNA according to the invention, preferably the at least one coding sequence of the RNA according to the invention, may comprise or consist of a variant of a nucleic acid sequence as defined herein, preferably of a nucleic acid sequence encoding a tumor antigen or a fragment thereof as defined herein. The expression 'variant of a nucleic acid sequence' as used herein in the context of a nucleic acid sequence encoding a tumor antigen or a fragment thereof. typically refers to a nucleic acid sequence, which differs by at least one nucleic acid residue from the respective naturally occuring nucleic acid sequence encoding a tumor antigen or a fragment thereof. More preferably, the expression 'variant of a nucleic acid sequence' refers to a nucleic acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%. preferably of at least 70%. more preferably of at least 80%. even more preferably at least 85%, even more preferably of at least 90% and most preferably of at least 95% or even 97%, with a nucleic acid sequence. from which it is derived.

Preferably, the RNA according to the invention, more preferably the at least one coding sequence of the RNA according to the invention, encodes a variant of a tumor antigen or a fragment thereof, preferably as defined herein.

In a preferred embodiment, the RNA according to the invention, more preferably the at least one coding sequence of the RNA according to the invention, comprises or consists of a variant of a nucleic acid sequence encoding a tumor antigen or a fragment thereof as defined herein, wherein the variant of the nucleic acid sequence encodes an amino acid sequence comprising at least one conservative substitution of an amino acid residue.

In another embodiment, the RNA according to the invention, more preferably the at least one coding sequence of the RNA according to the invention, comprises or consists of a variant of a nucleic acid sequence encoding a tumor antigen or a fragment thereof as defined herein, wherein the nucleic acid sequence of the variant differs from the respective naturally occuring nucleic acid sequence in at least one nucleic acid residue, preferably without resulting - due to the degenerated genetic code - in an alteration of the encoded amino acid sequence, i.e. the amino acid sequence encoded by the variant or at least part thereof may preferably not differ from the naturally occuring amino acid sequence in one or more mutation(s) within the above meaning.

Furthermore, a 'variant' of a nucleic acid sequence encoding a tumor antigen or a fragment or variant thereof as defined herein, may also comprise DNA sequences, which correspond to RNA sequences as defined herein and may also comprise further RNA sequences, which correspond to DNA sequences as defined herein. Those skilled in the art are familiar with the translation of an RNA sequence into a DNA sequence (or vice versa) or with the creation of the complementary strand sequence (i.e. by substitution of U residues with T residues and/or by constructing the complementary strand with respect to a given sequence).

According to a preferred embodiment, the at least one coding sequence of the RNA according to the invention comprises or consists of a nucleic acid sequence having a sequence identity of at least 5%, 10%, 20%. 30%, 40%, 50%, 60%, 70%. 80%, 85%, 86%, 87%, 88%, 89%, 90%, 91%, 92%, 93%. 94%, 95%, 96%, 97%, 98%, or 99%, preferably of at least 70%, more preferably of at least 80%, even more preferably at least 85%, even more preferably of at least 90% and most preferably of at least 95% or even 97%, with a nucleic acid sequence encoding a naturally occuring full-length tumor antigen as defined herein, or a variant thereof.

In a further preferred embodiment, the at least one coding sequence of the RNA according to the invention comprises or consists of a nucleic acid sequence identical to or having a sequence identity of at least 5%, 10%, 20%, 30%, 40%, 50%, 60%, 70%, 80%, 85%, 86%, 87%, 88%, 80%, 90%, 91%. 92%, 93%, 94%, 95%, 06%, 97%, 98%, or 99%, preferably of at least 70%, more preferably of at least 80%, even more preferably at least 85%, even more preferably of at least 90% and most preferably of at least 95% or even 97%. with any one of the nucleic acid sequences defined in Table 1, preferably in the fourth or fifth column (column 'B' or 'C', respectively) of Table 1, or a fragment or variant thereof. According to a particularly preferred embodiment, the at least one coding sequence of the RNA according to the invention comprises or consists of a nucleic acid sequence having a sequence identity of at least 80% with any one of the nucleic acid sequences defined in Table 1, preferably in the fourth or fifth column (column '8' or 'C', respectively) of Table 1, or a fragment or variant of any one of these sequences.

In a further preferred embodiment, the at least one coding sequence of the RNA according to the invention comprises or consists of a nucleic acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 85%, 96%, 97%, 98%, or 99%, preferably of at least 70%, more preferably of at least 80%, even more preferably at least 85%, even more preferably of at least 90% and most preferably of at least 95% or even 97%, with any one of the nucleic acid sequences defined in the fourth (column 'B') of Table 1, or a fragment or variant of any one of these sequences. In other words, the at least one coding sequence of the RNA according to the invention preferably comprises or consists of a nucleic acid sequence having a sequence identity of at least 5%, 10%, 20%. 30%, 40%, 50%, 60%. 70%. 80%, 85%, 86%, 87%, 88%, 89%, 90%, 91%, 92%. 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with a nucleic acid sequence selected from the group consisting of SEQ ID NOs: 505 - 1008; 4561 - 4563, or a fragment or variant of any one of said nucleic acid sequences. According to a particularly preferred embodiment, the at least one coding sequence of the RNA according to the invention comprises or consists of a nucleic acid sequence having a sequence identity of at least 80% with any one of the nucleic acid sequences defined in the fourth (column 'B') of Table 1, or a fragment or variant of any one of these sequences.

According to certain embodiments of the present invention, the RNA is mono-, bi-, or multicistronic, preferably as defined herein. The coding sequences in a bi- or multicistronic RNA preferably encode distinct tumor antigens as defined herein or a fragment or variant thereof. Preferably, the coding sequences encoding two or more antigens may be separated in the bi- or multicistronic RNA by at least one IRES (internal ribosomal entry site) sequence, as defined below. Thus, the term "encoding two or more antigens" may mean, without being limited thereto, that the bi- or even multicistronic RNA, may encode e.g. at least two, three, four, five, six or more (preferably different) antigens of the antigens or their fragments or variants within the definitions provided herein. More preferably, without being limited thereto, the bi- or even multicistronic mRNA. may encode, for example, at least two, three, four, five, six or more (preferably different) antigens as defined herein or their fragments or variants as defined herein. In this context, a so-called IRES (internal ribosomal entry site) sequence as defined above can function as a sole ribosome binding site, but it can also serve to provide a bi- or even multicistronic mRNA as defined above, which encodes several antigens, which are to be translated by the ribosomes independently of one another. Examples of IRES sequences, which can be used according to the invention, are those from picornaviruses (e.g. FMDV), pestiviruses (CFFV), polioviruses (PV), encephalomyocarditis viruses (ECMV), foot and mouth disease viruses (FMDV), hepatitis C viruses (HCV), classical swine fever viruses (CSFV), mouse leukoma virus (MLV), simian immunodeficiency viruses (SIV) or cricket paralysis viruses (CrPV).

According to a further embodiment the at least one coding sequence of the RNA according to the invention may encode at least two. three, four, five, six, seven, eight and more antigens (or fragments and derivatives thereof) as defined herein linked with or without an amino acid linker sequence, wherein said linker sequence can comprise rigid linkers, flexible linkers, cleavable linkers (e.g., self-cleaving peptides) or a combination thereof. Therein, the antigens may be identical or different or a combination thereof. Particular antigen/epitope combinations can be encoded by said mRNA encoding at least two antigens as explained herein (also referred to herein as 'multi-antigen-constructs/mRNA').

Preferably, the at least one coding sequence of the RNA according to the invention comprises at least two. three, four. five, six, seven, eight or more nucleic acid sequences identical to or having a sequence identity of at least 5%. 10%, 20%, 30%, 40%, 50%, 60%. 70%, 80%, 85%, 86%, 87%, 88%. 89%, 90%, 91%, 92%, 93%, 94%, 85%, 96%, 97%, 98%, or 99%, preferably of at least 70%, more preferably of at least 80%, even more preferably at least 85%, even more preferably of at least 90% and most preferably of at least 95% or even 97%, with any one of the nucleic acid sequences disclosed in Table 1 herein, or a fragment or variant of any one of said nucleic acid sequences.

Preferably, the RNA comprising at least one coding sequence as defined herein typically comprises a length of about 50 to about 20000. or 100 to about 20000 nucleotides, preferably of about 250 to about 20000 nucleotides, more preferably of about 500 to about 10000, even more preferably of about 500 to about 5000.

The RNA according to the invention may further be single stranded or double stranded. When provided as a double stranded RNA, the RNA according to the invention preferably comprises a sense and a corresponding antisense strand.

In a preferred embodiment, the RNA comprising at least one coding sequence as defined herein is an mRNA, a viral RNA or a replicon RNA.

According to a further embodiment, the RNA, preferably an mRNA. according to the invention is a modified RNA, preferably a modified RNA as described herein. In this context, a modification as defined herein preferably leads to a stabilization of the RNA according to the invention. More preferably, the invention thus provides a stabilized RNA comprising at least one coding sequence as defined herein.

According to one embodiment, the RNA of the present invention may thus be provided as a "stabilized mRNA", that is to say as an RNA that is essentially resistant to in vivo degradation (e.g. by an exo- or endo-nuclease). Such stabilization can be effected, for example, by a modified phosphate backbone of the RNA of the present invention. A backbone modification in connection with the present invention is a modification in which phosphates of the backbone of the nucleotides contained in the RNA are chemically modified. Nucleotides that may be preferably used in this connection contain e.g. a phosphorothioate-modified phosphate backbone, preferably at least one of the phosphate oxygens contained in the phosphate backbone being replaced by a sulfur atom. Stabilized RNAs may further include, for example: non-ionic phosphate analogues, such as, for example, alkyl and aryl phosphonates, in which the charged phosphonate oxygen is replaced by an alkyl or aryl group, or phosphodiesters and alkylphosphotriesters, in which the charged oxygen residue is present in alkylated form. Such backbone modifications typically include, without implying any limitation, modifications from the group consisting of methylphosphonates, phosphoramidates and phosphorothioates (e.g. cytidine-5'-0-(1-thiophosphate)).

In the following, specific modifications are described, which are preferably capable of "stabilizing" the RNA as defined herein.

### Chemical modifications:

The term "RNA modification" as used herein may refer to chemical modifications comprising backbone modifications as well as sugar modifications or base modifications.

In this context, a modified RNA as defined herein may contain nucleotide analogues/modifications, e.g. backbone modifications, sugar modifications or base modifications. A backbone modification in connection with the present invention is a modification, in which phosphates of the backbone of the nucleotides contained in an RNA as defined herein are chemically modified. A sugar modification in connection with the present invention is a chemical modification of the sugar of the nucleotides of the RNA as defined herein. Furthermore, a base modification in connection with the present invention is a chemical modification of the base moiety of the nucleotides of the RNA. In this context, nucleotide analogues or modifications are preferably selected from nucleotide analogues, which are applicable for transcription and/or translation.

### Sugar Modifications:

The modified nucleosides and nucleotides, which may be incorporated into a modified RNA as described herein, can be modified in the sugar moiety. For example, the 2' hydroxyl group (OH) can be modified or replaced with a number of different "oxy" or "deoxy" substituents. Examples of "oxy" -2' hydroxyl group modifications include, but are not limited to, alkoxy or aryloxy (-OR, e.g., R = H, alkyl, cycloalkyl, aryl, aralkyl, heteroaryl or sugar); polyethylene glycols (PEG), -O(CH₂CH₂O)nCH₂CH₂OR: "locked" nucleic acids (LNA) in which the 2' hydroxyl is connected, e.g., by a methylene bridge, to the 4' carbon of the same ribose sugar; and amino groups (-O-amino, wherein the amino group, e.g., NRR, can be alkylamino, dialkylamino, heterocyclyl, arylamino, diarylamino, heteroarylamino, or diheteroaryl amino, ethylene diamine, polyamino) or aminoalkoxy.

"Deoxy" modifications include hydrogen, amino (e.g. NH₂: alkylamino, dialkylamino, heterocyclyl, arylamino, diaryl amino, heteroaryl amino, diheteroaryl amino, or amino acid); or the amino group can be attached to the sugar through a linker, wherein the linker comprises one or more of the atoms C, N. and O.

The sugar group can also contain one or more carbons that possess the opposite stereochemical configuration than that of the corresponding carbon in ribose. Thus, a modified RNA can include nucleotides containing, for instance, arabinose as the sugar.

### Backbone Modifications:

The phosphate backbone may further be modified in the modified nucleosides and nucleotides, which may be incorporated into a modified RNA as described herein. The phosphate groups of the backbone can be modified by replacing one or more of the oxygen atoms with a different substituent. Further, the modified nucleosides and nucleotides can include the full replacement of an unmodified phosphate moiety with a modified phosphate as described herein. Examples of modified phosphate groups include, but are not limited to, phosphorothioate. phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulfur. The phosphate linker can also be modified by the replacement of a linking oxygen with nitrogen (bridged phosphoroamidates). sulfur (bridged phosphorothioates) and carbon (bridged methylene-phosphonates).

### Base Modifications:

The modified nucleosides and nucleotides, which may be incorporated into a modified RNA as described herein can further be modified in the nucleobase moiety. Examples of nucleobases found in RNA include, but are not limited to, adenine, guanine, cytosine and uracil. For example, the nucleosides and nucleotides described herein can be chemically modified on the major groove face. In some embodiments, the major groove chemical modifications can include an amino group, a thiol group, an alkyl group, or a halo group.

In particularly preferred embodiments of the present invention, the nucleotide analogues/modifications are selected from base modifications, which are preferably selected from 2-amino-6-chloropurineriboside-5'-triphosphate, 2-Aminopurine-riboside-5'-triphosphate; 2-aminoadenosine-5'-triphosphate, 2'-Amino-2'-deoxycytidine-triphosphate, 2-thiocytidine-5'-triphosphate, 2-thiouridine-5'-triphosphate, 2'-Fluorothymidine-5'-triphosphate, 2'-O-Methyl-inosine-5'-triphosphate 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-Bromo-2'-deoxycytidine-5'-triphosphate, 5-Bromo-2'-deoxyuridine-5'-triphosphate, 5-iodocytidine-5'-triphosphate, 5-lodo-2'-deoxycytidine-5'-triphosphate. 5-iodouridine-5'-triphosphate, 5-lodo-2'-deoxyuridine-5'-triphosphate, 5-methylcytidine-5'-triphosphate, 5-methyluridine-5'-triphosphate, 5-Propynyl-2'-deoxycytidine-5'-triphosphate, 5-Propynyl-2'-deoxyuridine-5'-triphosphate, 6-azacytidine-5'-triphosphate, 6-azauridine-5'-triphosphate, 6-chloropurineriboside-5'-triphosphate, 7-deazaadenosine-5'-triphosphate, 7-deazaguanosine-5'-triphosphate. 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, or puromycin-5'-triphosphate. xanthosine-5'-triphosphate. Particular preference is given to nucleotides for base modifications selected from the group of base-modified nucleotides consisting of 5-methylcytidine-5'-triphosphate. 7-deazaguanosine-5'-triphosphate. 5-bromocytidine-5'-triphosphate, and pseudouridine-5'-triphosphate.

In some embodiments, modified nucleosides include pyridin-4-one ribonucleoside, 5-aza-uridine, 2-thio-5-aza-uridine, 2-thiouridine, 4-thio-pseudouridine, 2-thio-pseudouridine. 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine. 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, and 4-methoxy-2-thio-pseudouridine.

In some embodiments, modified nucleosides include 5-aza-cytidine, pseudoisocytidine. 3-methyl-cytidine. N4-acetylcytidine, 5-formylcytidine, N4-methylcytidine, 5-hydroxymethylcytidine, 1-methyl-pseudoisacytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl- 1-deaza-pseudoisocytidine. 1-methyl-1-deaza-pseudoisocytidine, zebularine. 5-aza-zebularine. 5-methyl-zebularine, 5-aza-2-thio-zebularine, 2-thio-zebularine, 2-methoxy-cytidine, 2-methoxy-5-methyl-cytidine, 4-methoxy-pseudoisocytidine, and 4-methoxy-1-methyl-pseudoisocytidine .

In other embodiments, modified nucleosides include 2-aminopurine. 2, 6-diaminopurine, 7-deaza-adenine, 7-deaza-8-aza-adenine, 7-deaza-2-aminopurine, 7-deaza-8-aza-2-aminopurine, 7-deaza-2,6-diaminapurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-NB-threonyl carbamoyladenosine, N6,N6-dimethyladenosine, 7-methyladenine, 2-methylthio-adenine, and 2-methoxy-adenine.

In other embodiments, modified nucleosides include inosine, 1-methyl-inosine, wyosine, wybutasine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine. 1-methylguanosine, N2-methylguanosine, N2,N2-dimethylguanosine, 8-oxo-guanosine, 7-methyl-8-oxo-guanosine. 1-methyl-6-thio-guanosine. N2-methyl-6-thio-guanosine, and N2,N2-dimethyl-6-thio-guanosine.

In some embodiments, the nucleotide can be modified on the major groove face and can include replacing hydrogen on C-5 of uracil with a methyl group or a halo group. In specific embodiments, a modified nucleoside is 5'-0-(1-thiophosphate)-adenosine, 5'-O-(1-thiophosphate)-cytidine, 5'-O-(1-thiophosphate)-guanosine, 5'-O-(1-thiophosphate)-uridine or 5'-O-(1-thiophosphate)-pseudouridine.

In further specific embodiments, a modified RNA may comprise nucleoside modifications selected from 6-aza-cytidine, 2-thio-cytidine, α-thio-cytidine. Pseudo-iso-cytidine, 5-aminoallyl-uridine, 5-iodo-uridine, N1-methyl-pseudouridine, 5,6-dihydrouridine, α-thio-uridine, 4-thio-uridine, 6-aza-uridine, 5-hydroxy-uridine, deoxy-thymidine, 5-methyl-uridine, Pyrrolo-cytidine, inosine, α-thia-guanosine, 6-methyl-guanosine, 5-methyl-cytdine, 8-oxo-guanosine, 7-deaza-guanosine, N1-methyl-adenosine, 2-amino-6-Chloro-purine. N6-methyl-2-amino-purine. Pseudo-iso-cytidine. 6-Chloro-purine, N6-methyl-adenosine, α-thio-adenosine, 8-azido-adenosine, 7-deaza-adenosine.

### Lipid modification:

According to a further embodiment, a modified RNA as defined herein can contain a lipid modification. Such a lipid-modified RNA typically comprises an RNA as defined herein. Such a lipid-modified RNA as defined herein typically further comprises at least one linker covalently linked with that RNA, and at least one lipid covalently linked with the respective linker. Alternatively, the lipid-modified RNA comprises at least one RNA as defined herein and at least one (bifunctional) lipid covalently linked (without a linker) with that RNA. According to a third alternative, the lipid-modified RNA comprises an RNA molecule as defined herein, at least one linker covalently linked with that RNA. and at least one lipid covalently linked with the respective linker, and also at least one (bifunctional) lipid covalently linked (without a linker) with that RNA. In this context, it is particularly preferred that the lipid modification is present at the terminal ends of a linear RNA sequence.

### G/C content modification:

According to another embodiment, the RNA of the present invention, preferably an mRNA. may be modified, and thus stabilized, by modifying the guanosine/cytosine (G/C) content of the RNA. preferably of the at least one coding sequence of the RNA of the present invention.

In a particularly preferred embodiment of the present invention, the G/C content of the coding region of the RNA of the present invention is modified, particularly increased, compared to the G/C content of the coding region of the respective wild-type RNA, i.e. the unmodified RNA. The amino acid sequence encoded by the RNA is preferably not modified as compared to the amino acid sequence encoded by the respective wild-type RNA. This modification of the RNA of the present invention is based on the fact that the sequence of any RNA region to be translated is important for efficient translation of that RNA. Thus, the composition of the RNA and the sequence of various nucleotides are important. In particular, sequences having an increased G (guanosine)/C (cytosine) content are more stable than sequences having an increased A (adenosine)/U (uracil) content. According to the invention, the codons of the RNA are therefore varied compared to the respective wild-type RNA. while retaining the translated amino acid sequence, such that they include an increased amount of G/C nucleotides. In respect to the fact that several codons code for one and the same amino acid (so-called degeneration of the genetic code), the most favourable codons for the stability can be determined (so-called alternative codon usage). Depending on the amino acid to be encoded by the RNA, there are various possibilities for modification of the RNA sequence, compared to its wild-type sequence. In the case of amino acids, which are encoded by codons, which contain exclusively G or C nucleotides. no modification of the codon is necessary. Thus, the codons for Pro (CCC or CCG), Arg (CGC or CGG), Ala (GCC or GCG) and Gly (GGC or GGG) require no modification, since no A or U is present. In contrast, codons which contain A and/or U nucleotides can be modified by substitution of other codons, which code for the same amino acids but contain no A and/or U. Examples of these are: the codons for Pro can be modified from ECU or CCA to CCC or CCG: the codons for Arg can be modified from CGU or CGA or AGA or AGG to CGC or CGG; the codons for Ala can be modified from GCU or GCA to GCC or GCG; the codons for Gly can be modified from GGU or GGA to GGC or GGG. In other cases, although A or U nucleotides cannot be eliminated from the codons, it is however possible to decrease the A and U content by using codons which contain a lower content of A and/or U nucleotides. Examples of these are: the codons for Phe can be modified from UUU to UUC; the codons for Leu can be modified from UUA, UUG, CUU or CUA to CUC or CUG; the codons for Ser can be modified from UCU or UCA or AGU to UCC, UCG or AGC; the codon for Tyr can be modified from UAU to UAC; the codon for Cys can be modified from UGU to UGC: the codon for His can be modified from CAU to CAC: the codon for Gln can be modified from CAA to CAG; the codons for Ile can be modified from AUU or AHA to AUC; the codons for The can be modified from ACU or ACA to ACC or ACG; the codon for Asn can be modified from AAU to AAC; the codon for Lys can be modified from AAA to AAG; the codons for Val can be modified from GUU or GLIA to GUC or GUG; the codon for Asp can be modified from GAU to GAC; the codon for Glu can be modified from GAA to GAG; the stop codon UAA can be modified to CAG or UGA. In the case of the codons for Met (AUG) and Trp (GGG), on the other hand, there is no possibility of sequence modification. The substitutions listed above can be used either individually or in all possible combinations to increase the G/C content of the at least one mRNA of the composition of the present invention compared to its particular wild-type mRNA (i.e. the original sequence). Thus, for example, all codons for The occurring in the wild-type sequence can be modified to ACC (or ACG). Preferably, however, for example, combinations of the above substitution possibilities are used:
substitution of all codons coding for The in the original sequence (wild-type mRNA) to ACC (or ACG) and substitution of all codons originally coding for Ser to GCC (or UCG or AGC); substitution of all codons coding for lie in the original
sequence to AUC and
substitution of all codons originally coding for Lys to AAG and
substitution of all codons originally coding for Tyr to UAC; substitution of all codons coding for Val in the original sequence to GUC (or GGG) and
substitution of all codons originally coding for Glu to GAG and
substitution of all codons originally coding for Ala to GCC (or GCG) and
substitution of all codons originally coding for Arg to CGC (or CGG): substitution of all codons coding for Val in the original sequence to GUC (or GUG) and
substitution of all codons originally coding for Glu to GAG and
substitution of all codons originally coding for Ala to GCC (or GCG) and
substitution of all codons originally coding for Gly to GGC (or GGG) and
substitution of all codons originally coding for Asn to AAC; substitution of all codons coding for Val in the original sequence to GUC (or GGG) and
substitution of all codons originally coding for Phe to UUC and
substitution of all codons originally coding for Cys to UGC and
substitution of all codons originally coding for Leu to CUG (or CUC) and
substitution of all codons originally coding for Gln to CAG and
substitution of all codons originally coding for Pro to CCC (or CCG); etc.

Preferably, the G/C content of the coding region of the RNA of the present invention is increased by at least 7%, more preferably by at least 15%, particularly preferably by at least 20%, compared to the G/C content of the coding region of the wild-type RNA, which codes for an antigen as defined herein or a fragment or variant thereof. According to a specific embodiment at least 5%, 10%, 20%. 30%, 40%, 50%, 60%, more preferably at least 70%, even more preferably at least 80% and most preferably at least 90%. 95% or even 100% of the substitutable codons in the region coding for an antigen as defined herein or a fragment or variant thereof or the whole sequence of the wild type RNA sequence are substituted, thereby increasing the GC/content of said sequence. In this context, it is particularly preferable to increase the G/C content of the RNA of the present invention. preferably of the at least one coding region of the RNA according to the invention, to the maximum (i.e. 100% of the substitutable codons) as compared to the wild-type sequence. According to the invention, a further preferred modification of the RNA of the present invention is based on the finding that the translation efficiency is also determined by a different frequency in the occurrence of tRNAs in cells. Thus, if so-called "rare codons" are present in the RNA of the present invention to an increased extent, the corresponding modified RNA sequence is translated to a significantly poorer degree than in the case where codons coding for relatively "frequent" tRNAs are present. According to the invention, in the modified RNA of the present invention, the region which codes for an antigen as defined herein or a fragment or variant thereof is modified compared to the corresponding region of the wild-type RNA such that at least one codon of the wild-type sequence, which codes for a tRNA which is relatively rare in the cell, is exchanged for a codon, which codes for a tRNA which is relatively frequent in the cell and carries the same amino acid as the relatively rare tRNA. By this modification, the sequences of the RNA of the present invention is modified such that codons for which frequently occurring tRNAs are available are inserted. In other words, according to the invention, by this modification all codons of the wild-type sequence, which code for a tRNA which is relatively rare in the cell, can in each case be exchanged for a codon, which codes for a tRNA which is relatively frequent in the cell and which, in each case, carries the same amino acid as the relatively rare tRNA. Which tRNAs occur relatively frequently in the cell and which, in contrast, occur relatively rarely is known to a person skilled in the art; cf. e.g. Akashi. Curr. Opin. Genet. Dev. 2001, 11(6): 660-666. The codons, which use for the particular amino acid the tRNA which occurs the most frequently, e.g. the Gly codon, which uses the tRNA, which occurs the most frequently in the (human) cell, are particularly preferred. According to the invention, it is particularly preferable to link the sequential G/C content which is increased, in particular maximized, in the modified RNA of the present invention, with the "frequent" codons without modifying the amino acid sequence of the protein encoded by the coding region of the RNA. This preferred embodiment allows provision of a particularly efficiently translated and stabilized (modified) RNA of the present invention. The determination of a modified RNA of the present invention as described above (increased G/C content: exchange of tRNAs) can be carried out using the computer program explained in WO 02/088443 - the disclosure content of which is included in its full scope in the present invention. Using this computer program, the nucleotide sequence of any desired RNA can be modified with the aid of the genetic code or the degenerative nature thereof such that a maximum G/C content results, in combination with the use of codons which code for tRNAs occurring as frequently as possible in the cell, the amino acid sequence coded by the modified RNA preferably not being modified compared to the non-modified sequence. Alternatively, it is also possible to modify only the G/C content or only the codon usage compared to the original sequence. The source code in Visual Basic 6.0 (development environment used: Microsoft Visual Studio Enterprise 6.0 with Servicepack 3) is also described in WO 02/098443. In a further preferred embodiment of the present invention, the A/U content in the environment of the ribosome binding site of the RNA of the present invention is increased compared to the A/U content in the environment of the ribosome binding site of its respective wild-type mRNA. This modification (an increased A/U content around the ribosome binding site) increases the efficiency of ribosome binding to the RNA. An effective binding of the ribosomes to the ribosome binding site (Kozak sequence: SEQ ID NO: 4557; the AUG forms the start codon) in turn has the effect of an efficient translation of the RNA. According to a further embodiment of the present invention, the RNA of the present invention may be modified with respect to potentially destabilizing sequence elements. Particularly, the coding region and/or the 5' and/or 3' untranslated region of this RNA may be modified compared to the respective wild-type RNA such that it contains no destabilizing sequence elements, the encoded amino acid sequence of the modified RNA preferably not being modified compared to its respective wild-type RNA. It is known that, for example in sequences of eukaryotic RNAs, destabilizing sequence elements (DSE) occur, to which signal proteins bind and regulate enzymatic degradation of RNA in vivo. For further stabilization of the modified RNA, optionally in the region which encodes an antigen as defined herein or a fragment or variant thereof, one or more such modifications compared to the corresponding region of the wild-type RNA can therefore be carried out, so that no or substantially no destabilizing sequence elements are contained there. According to the invention, DSE present in the untranslated regions (3'- and/or 5'-UTR) can also be eliminated from the RNA of the present invention by such modifications. Such destabilizing sequences are e.g. AU-rich sequences (AURES), which occur in 3'-UTR sections of numerous unstable RNAs (Caput et al., Proc. Natl. Acad. Sci. USA 1986, 83: 1670 to 1674). The RNA of the present invention is therefore preferably modified compared to the respective wild-type RNA such that the RNA of the present invention contains no such destabilizing sequences. This also applies to those sequence motifs which are recognized by possible endonucleases, e.g. the sequence GAACAAG, which is contained in the 3'-UTR segment of the gene encoding the transferrin receptor (Binder et al., EMBO J. 1994, 13: 1969 to 1980). These sequence motifs are also preferably removed in the RNA of the present invention.

According to a preferred embodiment, the present invention provides an RNA as defined herein comprising at least one coding sequence, wherein the coding sequence comprises or consists of any one of the (modified) nucleic acid sequences defined in the fifth column (column 'C') of Table 1, or of a fragment or variant of any one of these sequences. In other words, the at least one coding sequence preferably comprises or consists of a nucleic acid sequence selected from the group consisting of SEQ ID NOs: 1009 - 4033; 4564 - 4591 or a fragment or variant of any one of these nucleic acid sequences.

In a further preferred embodiment, the at least one coding sequence of the RNA according to the invention comprises or consists of a nucleic acid sequence identical to or having a sequence identity of at least 5%, 10%, 20%, 30%, 40%, 50%, 60%, 70%, 80%, 85%, 86%, 87%, 88%, 89%, 90%, 91%, 92%, 93%, 94%, 95%. 96%, 97%, 98%, or 99%, preferably of at least 70%, more preferably of at least 80%, even more preferably at least 85%, even more preferably of at least 90% and most preferably of at least 95% or even 97%, with any one of the (modified) nucleic acid sequences defined in the fifth column (column 'C') of Table 1, or of a fragment or variant of any one of these sequences.

According to a particularly preferred embodiment, the at least one coding sequence of the RNA according to the invention comprises or consists of a nucleic acid sequence having a sequence identity of at least 80% with any one of the (modified) nucleic acid sequences defined in the fifth column (column 'C') of Table 1, or of a fragment or variant of any one of these sequences.

### GC optimized sequences:

In a preferred embodiment, the present invention provides an RNA comprising at least one coding sequence, wherein the coding sequence comprises or consists of a nucleic acid sequence selected from the group consisting of SEQ ID NOs: 1009 -1512; 3025 - 3528; 3529 - 4032; 4033 - 4033, 4564 - 4566; 4576 - 4591 or a fragment or variant of any one of these nucleic acid sequences.

According to a further embodiment, the at least one coding sequence of the RNA according to the invention comprises or consists of a nucleic acid sequence having a sequence identity of at least 5%, 10%, 20%, 30%, 40%, 50%, 60%, 70%, 80%, 85%, 86%, 87%, 88%, 89%, 90%, 91%. 92%, 93%, 94%, 95%, 96%, 97%, 98%, or 99%, preferably of at least 70%. more preferably of at least 80%, even more preferably at least 85%, even more preferably of at least 90% and most preferably of at least 95% or even 97%, with a nucleic acid sequence selected from the group consisting of SEQ ID NOs: 1009 - 1512; 3025 - 3528: 3529 - 4032; 4033 - 4033, 4564 - 4566; 4576 - 4591 or a fragment or variant of any one of these nucleic acid sequences.

### Sequences adapted to human codon usage:

According to the invention, a further preferred modification of the RNA of the present invention is based on the finding that codons encoding the same amino acid typically occur at different frequencies. According to the invention, in the modified RNA of the present invention, the coding sequence (coding region) as defined herein is preferably modified compared to the corresponding region of the respective wild-type RNA such that the frequency of the codons encoding the same amino acid corresponds to the naturally occurring frequency of that codon according to the human codon usage as e.g. shown in Table 2.

For example, in the case of the amino acid alanine (Ala) present in an amino acid sequence encoded by the at least one coding sequence of the RNA according to the invention, the wild type coding sequence is preferably adapted in a way that the codon "GCC" is used with a frequency of 0.40, the codon "GET" is used with a frequency of 0.28, the codon "GCA" is used with a frequency of 0.22 and the codon "GCG" is used with a frequency of 0.10 etc. (see Table 2).

**Table 2: Human codon usage table**

| **Amin oacid** | **codon** | **fraction** | **/1000** |
|---|---|---|---|
| **Ala** | GCG | 0.10 | 7.4 |
| **Ala** | GCA | 0.22 | 15.8 |
| **Ala** | GCT | 0.28 | 18.5 |
| **Ala** | GCC* | 0.40 | 27.7 |
| **Cys** | TGT | 0.42 | 10.6 |
| **Cys** | TGC* | 0.58 | 12.6 |
| **Asp** | GAT | 0.44 | 21.8 |
| **Asp** | GAC* | 0.56 | 25.1 |
| **Glu** | GAG* | 0.59 | 39.6 |
| **Glu** | GAA | 0.41 | 29.0 |
| **Phe** | TTT | 0.43 | 17.6 |
| **Phe** | TTC* | 0.57 | 20.3 |
| **Gly** | GGG | 0.23 | 16.5 |
| **Gly** | GGA | 0.26 | 16.5 |
| **Gly** | GGT | 0.18 | 10.8 |
| **Gly** | GGC* | 0.33 | 22.2 |
| **His** | CAT | 0.41 | 10.9 |
| **His** | CAC* | 0.59 | 15.1 |
| **Ile** | ATA | 0.14 | 7.5 |
| **Ile** | ATT | 0.35 | 16.0 |
| **Ile** | ATC* | 0.52 | 20.8 |
| **Lys** | AAG* | 0.60 | 31.9 |
| **Lys** | AAA | 0.40 | 24.4 |
| **Leu** | TTG | 0.12 | 12.9 |
| **Leu** | TTA | 0.06 | 7.7 |
| **Leu** | CTG* | 0.43 | 39.6 |
| **Leu** | CTA | 0.07 | 7.2 |
| **Leu** | CTT | 0.12 | 13.2 |
| **Leu** | CTC | 0.20 | 19.6 |
| **Met** | ATG* | I | 22.0 |
| **Asn** | AAT | 0.44 | 17.0 |
| **Asn** | AAC* | 0.56 | 19.1 |
| **Pro** | CCG | 0.11 | 6.9 |
| **Pro** | CCA | 0.27 | 16.9 |
| **Pro** | CCT | D.29 | 17.5 |
| **Pro** | CCC* | 0.33 | 19.8 |
| **Gln** | CAG* | 0.73 | 34.2 |
| **Gln** | CAA | 0.27 | 12.3 |
| **Arg** | AGG | 0.22 | 12.0 |
| **Arg** | AGA* | 0.21 | 12.1 |
| **Arg** | CGG | 0.19 | 11.4 |
| **Arg** | CGA | 0.10 | 6.2 |
| **Arg** | CGT | 0.09 | 4.5 |
| **Arg** | CGC | 0.19 | 10.4 |
| **Ser** | AGT | 0.14 | 12.1 |
| **Ser** | AGC* | 0.25 | 19.5 |
| **Ser** | TCG | 0.06 | 4.4 |
| **Ser** | TCA | 0.15 | 12.2 |
| **Ser** | TCT | 0.18 | 15.2 |
| **Ser** | TCC | 0.23 | 17.7 |
| **Thr** | ACG | 0.12 | 6.1 |
| **Thr** | ACA | 0.27 | 15.1 |
| **Thr** | ACT | 0.23 | 13.1 |
| **Thr** | ACC* | 0.38 | 18.9 |
| **Val** | GTG* | 0.48 | 28.1 |
| **Val** | GTA | 0.10 | 7.1 |
| **Val** | GTT | 0.17 | 11.0 |
| **Val** | GTC | 0.25 | 14.5 |
| **Trp** | TGG* | I | 13.2 |
| **Tyr** | TAT | 0.42 | 12.2 |
| **Tyr** | TAC* | 0.58 | 15.3 |
| **Stop** | TGA* | 0.61 | 1.6 |
| **Stop** | TAG | 0.17 | 0.8 |
| **Stop** | TAA | 0.22 | 1.0 |

| | | | |
|---|---|---|---|
| *: most frequent codon | | | |

In a preferred embodiment, the present invention provides an RNA comprising at least one coding sequence, wherein the coding sequence comprises a nucleic acid sequence selected from the group consisting of SEQ ID NOs: 2017 - 2520: 4570 - 4572, or a fragment or variant of any one of said nucleic acid sequences.

According to a further embodiment, the at least one coding sequence of the RNA according to the invention comprises or consists of a nucleic acid sequence having a sequence identity of at least 5%, 10%, 20%, 30%, 40%, 50%, 60%, 70%. 80%, 85%, 86%. 87%, 88%, 89%, 90%, 91%, 92%, 93%, 94%, 95%. 96%, 97%, 98%, or 99%, preferably of at least 70%, more preferably of at least 80%, even more preferably at least 85%, even more preferably of at least 90% and most preferably of at least 95% or even 97%, with a nucleic acid sequence selected from the group consisting of SEQ ID NOs: 2017 - 2520, 4570 - 4572 or a fragment or variant of any one of said nucleic acid sequences.

### Codon-optimized sequences:

As described above it is preferred according to the invention, that all codons of the wild-type sequence which code for a tRNA, which is relatively rare in the cell, are exchanged for a codon which codes for a tRNA, which is relatively frequent in the cell and which, in each case, carries the same amino acid as the relatively rare tRNA. Therefore it is particularly preferred that the most frequent codons are used for each encoded amino acid (see Table 2, most frequent codons are marked with asterisks). Such an optimization procedure increases the codon adaptation index (CAI) and ultimately maximises the CAI. In the context of the invention, sequences with increased or maximized CAI are typically referred to as "codon-optimized" sequences and/or CAI increased and/or maximized sequences. According to a preferred embodiment, the RNA of the present invention comprises at least one coding sequence, wherein the coding sequence is codon-optimized as described herein. More preferably, the codon adaptation index (CAI) of the at least one coding sequence is at least 0.5, at least D.8, at least 0.9 or at least 0.95. Most preferably, the codon adaptation index (CAI) of the at least one coding sequence is 1.

For example, in the case of the amino acid alanine (Ala) present in the amino acid sequence encoded by the at least one coding sequence of the RNA according to the invention, the wild type coding sequence is adapted in a way that the most frequent human codon "GCC" is always used for said amino acid, or for the amino acid Cysteine (Cys), the wild type sequence is adapted in a way that the most frequent human codon "TGC" is always used for said amino acid etc.

In a preferred embodiment, the present invention provides an RNA comprising at least one coding sequence, wherein the coding sequence comprises a nucleic acid sequence selected from the group consisting of SEQ ID NOs: 2521 - 3024: 4573 - 4575, or a fragment or variant of any one of said nucleic acid sequences.

According to a further embodiment, the at least one coding sequence of the RNA according to the invention comprises or consists of a nucleic acid sequence having a sequence identity of at least 5%, 10%, 20%, 30%. 40%. 50%, 60%. 70%, 80%, 85%, 86%, 87%, 88%. 89%, 90%, 91%, 92%, 93%, 94%, 95%, 96%, 97%, 98%, or 99%, preferably of at least 70%, more preferably of at least 80%, even more preferably at least 85%, even more preferably of at least 90% and most preferably of at least 95% or even 97%, with a nucleic acid sequence selected from the group consisting of SEQ ID NOs: 2521 - 3024: 4573 - 4575, or a fragment or variant of any one of said nucleic acid sequences.

### C-optimized sequences:

According to another embodiment, the RNA of the composition of the present invention may be modified by modifying, preferably increasing, the cytosine (C) content of the RNA. preferably of the coding region of the aRNA.

In a particularly preferred embodiment of the present invention, the C content of the coding region of the RNA of the present invention is modified, preferably increased, compared to the C content of the coding region of the respective wild-type RNA, i.e. the unmodified RNA. The amino acid sequence encoded by the at least one coding sequence of the RNA of the present invention is preferably not modified as compared to the amino acid sequence encoded by the respective wild-type mRNA.

In a preferred embodiment of the present invention, the modified RNA is modified such that at least 10%, 20%, 30%, 40%, 50%, 60%, 70% or 80%, or at least 90% of the theoretically possible maximum cytosine-content or even a maximum cytosine-content is achieved.

In further preferred embodiments, at least 10%, 20%, 30%. 40%, 50%, 60%, 70%, 80%, 90% or even 100% of the codons of the target RNA wild type sequence, which are "cytosine content optimizable" are replaced by codons having a higher cytosine-content than the ones present in the wild type sequence.

In a further preferred embodiment, some of the codons of the wild type coding sequence may additionally be modified such that a codon for a relatively rare tRNA in the cell is exchanged by a codon for a relatively frequent tRNA in the cell, provided that the substituted codon for a relatively frequent tRNA carries the same amino acid as the relatively rare tRNA of the original wild type codon. Preferably, all of the codons for a relatively rare tRNA are replaced by a codon for a relatively frequent tRNA in the cell, except codons encoding amino acids, which are exclusively encoded by codons not containing any cytosine, or except for glutamine (Gln), which is encoded by two codons each containing the same number of cytosines.

In a further preferred embodiment of the present invention, the modified target RNA is modified such that at least 80%, or at least 90% of the theoretically possible maximum cytosine-content or even a maximum cytosine-content is achieved by means of codons, which code for relatively frequent tRNAs in the cell, wherein the amino acid sequence remains unchanged.

Due to the naturally occurring degeneracy of the genetic code, more than one codon may encode a particular amino acid. Accordingly, 18 out of 20 naturally occurring amino acids are encoded by more than one codon (with Tryp and Met being an exception), e.g. by 2 codons (e.g. Cys, Asp, Glu), by three codons (e.g. Ile), by 4 codons (e.g. Al. Gly, Pro) or by 6 codons (e.g. Leu. Arg, Ser). However, not all codons encoding the same amino acid are utilized with the same frequency under in vivo conditions. Depending on each single organism, a typical codon usage profile is established.

The term 'cytosine content-optimizable codon' as used within the context of the present invention refers to codons. which exhibit a lower content of cytosines than other codons encoding the same amino acid. Accordingly, any wild type codon, which may be replaced by another codon encoding the same amino acid and exhibiting a higher number of cytosines within that codon. is considered to be cytosine-optimizable (C-optimizable). Any such substitution of a C-optimizable wild type codon by the specific C-optimized codon within a wild type coding region increases its overall C-content and reflects a C-enriched modified mRNA sequence. According to a preferred embodiment, the RNA of the present invention, preferably the at least one coding sequence of the RNA of the present invention comprises or consists of a C-maximized RNA sequence containing C-optimized codons for all potentially C-optimizable codons. Accordingly, 100% or all of the theoretically replaceable C-optimizable codons are preferably replaced by C-optimized codons over the entire length of the coding region.

In this context. cytosine-content optimizable codons are codons, which contain a lower number of cytosines than other codons coding for the same amino acid.

Any of the codons GCG, GCA, GCU codes for the amino acid Ala, which may be exchanged by the codon GCC encoding the same amino acid, and/or
the codon UGU that codes for Cys may be exchanged by the codon UGC encoding the same amino acid. and/or
the codon GAU which codes for Asp may be exchanged by the codon GAC encoding the same amino acid, and/or
the codon that UUU that codes for Phe may be exchanged for the codon UUC encoding the same amino acid, and/or
any of the codons GGG, GGA, GGU that code Gly may be exchanged by the codon GGC encoding the same amino acid. and/or
the codon CAU that codes for His may be exchanged by the codon CAC encoding the same amino acid. and/or
any of the codons ALIA, AUU that code for lie may be exchanged by the codon AUC, and/or
any of the codons UUG, UUA. CUG, CUA, CUU coding for Leu may be exchanged by the codon CUC encoding the same amino acid. and/or
the codon AAU that codes for Asn may be exchanged by the codon AAC encoding the same amino acid. and/or
any of the codons CCG. CCA. CCU coding for Pro may be exchanged by the codon CCC encoding the same amino acid, and/or
any of the codons AGG, AGA, CGG, CGA, CGU coding for Arg may be exchanged by the codon CGC encoding the same amino acid, and/or
any of the codons AGU, AGC. UCG. UCA, UCU coding for Ser may be exchanged by the codon UCC encoding the same amino acid, and/or
any of the codons ACG, ACA, ACU coding for Thr may be exchanged by the codon ACC encoding the same amino acid. and/or
any of the codons GUG, GUA, GUU coding for Val may be exchanged by the codon GUC encoding the same amino acid. and/or
the codon UAU coding for Tyr may be exchanged by the codon UAC encoding the same amino acid.

In any of the above instances, the number of cytosines is increased by 1 per exchanged codon. Exchange of all non C-optimized codons (corresponding to C-optimizable codons) of the coding region results in a C-maximized coding sequence. In the context of the invention, at least 70%, preferably at least 80%, more preferably at least 90%, of the non C-optimized codons within the at least one coding region of the RNA according to the invention are replaced by C-optimized codons.

It may be preferred that for some amino acids the percentage of C-optimizable codons replaced by C-optimized codons is less than 70%, while for other amino acids the percentage of replaced codons is higher than 70% to meet the overall percentage of C-optimization of at least 70% of all C-optimizable wild type codons of the coding region.

Preferably, in a C-optimized RNA of the invention, at least 50% of the C-optimizable wild type codons for any given amino acid are replaced by C-optimized codons, e.g. any modified C-enriched RNA preferably contains at least 50% C-optimized codons at C-optimizable wild type codon positions encoding any one of the above mentioned amino acids Ala, Cys, Asp. Phe, Gly, His, Ile, Leu. Asn. Pro. Arg, Ser, Thr, Val and Tyr, preferably at least 60%.

In this context codons encoding amino acids, which are not cytosine content-optimizable and which are, however, encoded by at least two codons, may be used without any further selection process. However, the codon of the wild type sequence that codes for a relatively rare tRNA in the cell, e.g. a human cell, may be exchanged for a codon that codes for a relatively frequent tRNA in the cell, wherein both code for the same amino acid. Accordingly, the relatively rare codon GAA coding for Glu may be exchanged by the relative frequent codon GAG coding for the same amino acid. and/or
the relatively rare codon AAA coding for Lys may be exchanged by the relative frequent codon AAG coding for the same amino acid, and/or
the relatively rare codon CAA coding for Gln may be exchanged for the relative frequent codon CAG encoding the same amino acid.

In this context, the amino acids Met (AUG) and Trp (UGG), which are encoded by only one codon each, remain unchanged. Stop codons are not cytosine-content optimized, however, the relatively rare stop codons amber, ochre (UAA, UAG) may be exchanged by the relatively frequent stop codon opal (UGA).

The single substitutions listed above may be used individually as well as in all possible combinations in order to optimize the cytosine-content of the modified RNA compared to the wild type mRNA sequence.

Accordingly, the at least one coding sequence as defined herein may be changed compared to the coding region of the respective wild type RNA in such a way that an amino acid encoded by at least two or more codons, of which one comprises one additional cytosine, such a codon may be exchanged by the C-optimized codon comprising one additional cytosine, wherein the amino acid is preferably unaltered compared to the wild type sequence.

In a preferred embodiment. the present invention provides an RNA comprising at least one coding sequence, wherein the coding sequence comprises a nucleic acid sequence selected from the group consisting of SEQ ID NOs: 1513 - 2016: 4567 - 4569, or a fragment or variant of any one of said nucleic acid sequences.

According to a further embodiment, the at least one coding sequence of the RNA according to the invention comprises or consists of a nucleic acid sequence having a sequence identity of at least 5%, 10%, 20%, 30%, 40%, 50%, 60%, 70%, 80%, 85%, 86%, 87%, 88%, 89%. 90%, 91%, 92%, 93%, 94%, 95%, 96%. 97%, 98%, or 99%, preferably of at least 70%, more preferably of at least 80%, even more preferably at least 85%, even more preferably of at least 90% and most preferably of at least 95% or even 97%, with a nucleic acid sequence selected from the group consisting of SEQ ID NOs: 1513 - 2016: 4567 - 4569, or a fragment or variant of any one of said nucleic acid sequences.

According to a particularly preferred embodiment, the invention provides an RNA, preferably an mRNA, comprising at least one coding sequence as defined herein, wherein the G/C content of the at least one coding sequence of the RNA is increased compared to the G/C content of the corresponding coding sequence of the corresponding wild-type RNA, and/or
wherein the C content of the at least one coding sequence of the RNA is increased compared to the C content of the corresponding coding sequence of the corresponding wild-type RNA, and/or
wherein the codons in the at least one coding sequence of the RNA are adapted to human codon usage, wherein the codon adaptation index (CAI) is preferably increased or maximised in the at least one coding sequence of the RNA,
and wherein the amino acid sequence encoded by the RNA is preferably not being modified compared to the amino acid sequence encoded by the corresponding wild-type RNA.

According to another preferred embodiment of the invention, a modified RNA as defined herein. can be modified by the addition of a so-called '5' cap' structure, which preferably stabilizes the RNA as described herein. A 5'-cap is an entity, typically a modified nucleotide entity, which generally "caps" the 5'-end of a mature mRNA. A 5'-cap may typically be formed by a modified nucleotide, particularly by a derivative of a guanine nucleotide. Preferably, the 5'-cap is linked to the 5'-terminus via a 5'-5'-triphosphate linkage. A 5'-cap may be methylated, e.g. m7GpppN, wherein N is the terminal 5' nucleotide of the nucleic acid carrying the 5'-cap, typically the 5'-end of an mRNA. m7GpppN is the 5'-cap structure, which naturally occurs in mRNA transcribed by polymerase II and is therefore preferably not considered as modification comprised in a modified mRNA in this context. Accordingly, a modified RNA of the present invention may comprise a m7GpppN as 5'-cap, but additionally the modified RNA typically comprises at least one further modification as defined herein.

Further examples of 5'cap structures include glyceryl, inverted deoxy abasic residue (moiety), 4',5' methylene nucleotide, 1-(beta-O-erythrofuranosyl) nucleotide, 4'-thio nucleotide, carbocyclic nucleotide, 1,5-anhydrohexitol nucleotide, L-nucleotides, alpha-nucleotide, modified base nucleotide, threo-pentofuranosyl nucleotide, acyclic 3',4'-seco nucleotide, acyclic 3,4-dihydroxybutyl nucleotide, acyclic 3.5 dihydroxypentyl nucleotide, 3'-3'-inverted nucleotide moiety, 3'-3'-inverted abasic moiety. 3'-2'-inverted nucleotide moiety. 3'-2'-inverted abasic moiety, 1,4-butanediol phosphate, 3'-phosphoramidate, hexylphosphate, aminohexyl phosphate, 3'-phosphate, 3'phosphorothioate, phosphorodithioate, or bridging or non-bridging methylphosphonate moiety. These modified 5'-cap structures are regarded as at least one modification in this context.

Particularly preferred modified 5'-cap structures are cap1 (methylation of the ribose of the adjacent nucleotide of m7G), cap2 (additional methylation of the ribose of the 2nd nucleotide downstream of the m7G), cap3 (additional methylation of the ribose of the 3rd nucleotide downstream of the m7G), cap4 (methylation of the ribose of the 4th nucleotide downstream of the m7G), ARCA (anti-reverse cap analogue, modified ARCA (e.g. phosphothioate modified ARCA), inosine, NI-methyl-guanosine. 2'-fluoro-guanosine, 7-deaza-guanosine, 8-oxo-guanosine, 2-amino-guanosine, LNA-guanosine, and 2-azido-guanosine. Accordingly, the RNA according to the invention preferably comprises a 5'-cap structure.

In a preferred embodiment, the RNA according to the invention comprises at least one 5'- or 3'-UTR element. In this context. an UTR element comprises or consists of a nucleic acid sequence, which is derived from the 5'- or 3'-UTR of any naturally occurring gene or which is derived from a fragment, a homolog or a variant of the 5'- or 3'-UTR of a gene. Preferably, the 5'- or 3'-UTR element used according to the present invention is heterologous to the at least one coding sequence of the RNA of the invention. Even if 5'- or 3'-UTR elements derived from naturally occurring genes are preferred, also synthetically engineered UTR elements may be used in the context of the present invention.

The term '3'UTR element' typically refers to a nucleic acid sequence, which comprises or consists of a nucleic acid sequence that is derived from a 3'UTR or from a variant of a 3'UTR. A 3'UTR element in the sense of the present invention may represent the 3'UTR of an RNA, preferably an mRNA. Thus, in the sense of the present invention. preferably, a 3'UTR element may be the 3'UTR of an RNA. preferably of an mRNA, or it may be the transcription template for a 3'UTR of an RNA. Thus, a 3'UTR element preferably is a nucleic acid sequence which corresponds to the 3'UTR of an RNA, preferably to the 3'UTR of an mRNA, such as an mRNA obtained by transcription of a genetically engineered vector construct. Preferably, the 3'UTR element fulfils the function of a 3'UTR or encodes a sequence which fulfils the function of a 3'UTR.

According to a preferred embodiment, the RNA. preferably an mRNA, according to the invention comprises a 5'-cap structure and/or at least one 3'-untranslated region element (3'-LITR element), preferably as defined herein. More preferably, the RNA further comprises a 5'-UTR element as defined herein.

According to a further preferred embodiment, the RNA of the present invention may contain a poly-A tail on the 3' terminus of typically about 10 to 200 adenosine nucleotides, preferably about 10 to 100 adenosine nucleotides, more preferably about 40 to 80 adenosine nucleotides or even more preferably about 50 to 70 adenosine nucleotides.

Preferably, the poly(A) sequence in the RNA of the present invention is derived from a DNA template by RNA in vitro transcription. Alternatively, the poly(A) sequence may also be obtained in vitro by common methods of chemical-synthesis without being necessarily transcribed from a DNA-progenitor. Moreover, poly(A) sequences. or poly(A) tails may be generated by enzymatic polyadenylation of the RNA according to the present invention using commercially available polyadenylation kits and corresponding protocols known in the art.

Alternatively, the RNA as described herein optionally comprises a polyadenylation signal, which is defined herein as a signal, which conveys polyadenylation to a (transcribed) RNA by specific protein factors (e.g. cleavage and polyadenylation specificity factor (CPSF), cleavage stimulation factor (CstF), cleavage factors I and II (CF I and CF II). poly(A) polymerase (PAP)). In this context, a consensus polyadenylation signal is preferred comprising the NN(LI/T)ANA consensus sequence. In a particularly preferred aspect, the polyadenylation signal comprises one of the following sequences: AA(U/T)AAA or A(U/T)(U/T)AAA (wherein uridine is usually present in RNA and thymidine is usually present in DNA).

According to a further preferred embodiment, the RNA of the present invention may contain a poly(C) tail on the 3' terminus of typically about 10 to 200 cytosine nucleotides, preferably about 10 to 100 cytosine nucleotides, more preferably about 20 to 70 cytosine nucleotides or even more preferably about 20 to 60 or even 10 to 40 cytosine nucleotides.

In a further preferred embodiment, the RNA according to the invention further comprises at least one 3'UTR element. Preferably, the at least one 3'UTR element comprises or consists of a nucleic acid sequence derived from the 3'UTR of a chordate gene, preferably a vertebrate gene, more preferably a mammalian gene, most preferably a human gene, or from a variant of the 3'UTR of a chordate gene. preferably a vertebrate gene. more preferably a mammalian gene, most preferably a human gene.

Preferably, the RNA of the present invention comprises a 3'UTR element, which may be derivable from a gene that relates to an mRNA with an enhanced half-life (that provides a stable mRNA), for example a 3'UTR element as defined and described below. Preferably, the 3'-UTR element is a nucleic acid sequence derived from a 3'-UTR of a gene, which preferably encodes a stable mRNA, or from a homolog, a fragment or a variant of said gene

In a particularly preferred embodiment, the 3'UTR element comprises or consists of a nucleic acid sequence, which is derived from a 3'UTR of a gene selected from the group consisting of an albumin gene. an α-globin gene. a β-globin gene. a tyrosine hydroxylase gene, a lipoxygenase gene, and a collagen alpha gene, such as a collagen alpha 1(I) gene, or from a variant of a 3'UTR of a gene selected from the group consisting of an albumin gene. an α-globin gene. a β-globin gene, a tyrosine hydroxylase gene, a lipoxygenase gene, and a collagen alpha gene, such as a collagen alpha 1(I) gene according to SEQ ID No. 1369-1390 of the patent application WO2013/143700, whose disclosure is incorporated herein by reference, or from a homolog, a fragment or a variant thereof. In a particularly preferred embodiment, the 3'UTR element comprises or consists of a nucleic acid sequence which is derived from a 3'UTR of an albumin gene, preferably a vertebrate albumin gene. more preferably a mammalian albumin gene, most preferably a human albumin gene according to SEQ ID NO: 4549 or the corresponding RNA sequence SEQ ID NO: 4550.

Human albumin 3'UTR SEQ ID NO: 4549:

CATCACATTTAAAAGCATCTCAGCCTACCATGAGAATAAGAGAAAGAAAATGAAGATCAAAAGCTTATTCATCTGTTTTTCTTTTTCGTTGGTGTAAAGCCAACACCC
TGTCTAAAAAACATAAATTTCTTTAATCATTTTGCCTCTTTTCTCTGTGCTTCAATTAATAAAAAATGGAAAGAATCT (corresponding to SEQ ID NO: 1369 of
the patent application WO2013/143700 ).

In this context it is particularly preferred that the RNA according to the invention comprises a 3'-UTR element comprising a corresponding RNA sequence derived from the nucleic acids according to SEQ ID NO: 1368-1390 of the patent application WO2013/143700 or a fragment, homolog or variant thereof.

Most preferably the 3'-UTR element comprises the nucleic acid sequence derived from a fragment of the human albumin gene according to SEQ ID NO: SEQ ID NO: 4551 or SEQ ID NO: 4553:
albumin7 3'UTR

CATCACATTTAAAAGCATCTCAGCCTACCATGAGAATAAGAGAAAGAAAATGAAGATCAATAGCTTATTCATCTCTTTTTCTTTTTCGTTGGTGTAAAGCCAACACCC
TGTCTAAAAAACATAAATTTCTTTAATCATTTTGCCTCTTTTCTCTGTGCTTCAATTAATAAAAAATGGAAAGAACCT (SEQ ID NO: 4551 corresponding to SEQ
ID No: 1376 of the patent application WO2013/143700)

In this context. it is particularly preferred that the 3'-UTR element of the RNA according to the present invention comprises or consists of a corresponding RNA sequence of the nucleic acid sequence according to SEQ ID NO: 4551 or SEQ ID NO: 4553 as shown in SEQ ID NO: 4552 or SEQ ID NO: 4554.

In another particularly preferred embodiment, the 3'UTR element comprises or consists of a nucleic acid sequence which is derived from a 3'UTR of an α-globin gene, preferably a vertebrate α-or β-globin gene. more preferably a mammalian α-or β-globin gene, most preferably a human α-or β-globin gene according to SEQ ID NO: 4541, SEQ ID NO: 4543 or SEQ ID NO: 4545 or the corresponding RNA sequences SEQ ID NO: 4542. SEQ ID NO: 4544 or SEQ ID NO: 4546. SEQ ID NO: 3543, 3536 or 3538:
3'-UTR of Homo sapiens hemoglobin, alpha 1 (HBA1) 
GCTGGAGCCTCGGTGGCCATGCTTCTTGCCCCTTGGGCCTCCCCCCAGCCCCTCCTCCCCTTCCTGCACCCGTACCCCCGTGGTCTTTGAATAAAGTCTGAGTGG
GCGGC (SEQ ID NO: 4541 corresponding to SEQ ID NO: 1370 of the patent application WO2013/143700)
3'-UTR of Homo sapiens hemoglobin, alpha 2 (HBA2) 
GCTGGAGCCTCGGTAGCCGTTCCTCCTGCCCGCTGGGCCTCCCAACGGGCCCTCCTCCCCTCCTTGCACCGGCCCTTCCTGGTCTTTGAATAAAGTCTGAGTGGG
CAG (SEQ ID NO: 4543 corresponding to SEQ ID NO: 1371 of the patent application WO2013/143700)
 3'-UTR of Homo sapiens hemoglobin, beta (HBB)

GCTCGCTTTCTTGCTGTCCAATTTCTATTAAAGGTTCCTTTGTTCCCTAAGTCCAACTACTAAACTGGGGGATATTATGAAGGGCCTTGAGCATCTGGATTCTGCCTA
ATAAAAAACATTTATTTTCATTGC (SEQ ID NO: 4545 corresponding to SEQ ID NO: 1372 of the patent application WO2013/143700)

For example, the 3'UTR element may comprise or consist of the center, α-complex-binding portion of the 3'UTR of an α-globin gene, such as of a human α-globin gene, or a homolog. a fragment, or a variant of an α-globin gene. preferably according to SEQ ID NO: 4547:
Center, α-complex-binding portion of the 3'UTR of an α-globin gene (also named herein as "muag") GCCCGATGGGCCTCCCAACGGGCCCTCCTCCCCTCCTTGCACCG (SEQ ID NO: 4547 corresponding to SEQ ID NO: 1393 of the patent application WO2013/143700).

In this context it is particularly preferred that the 3'-UTR element of the RNA according to the invention comprises or consists of a corresponding RNA sequence of the nucleic acid sequence according to SEQ ID NO: 4547 as shown in SEQ ID NO: 4548. or a homolog, a fragment or variant thereof.

The term 'a nucleic acid sequence which is derived from the 3'UTR of a [...] gene' preferably refers to a nucleic acid sequence which is based on the 3'UTR sequence of a [...] gene or on a part thereof, such as on the 3'UTR of an albumin gene. an α-globin gene, a β-globin gene. a tyrosine hydroxylase gene, a lipoxygenase gene, or a collagen alpha gene, such as a collagen alpha 1(I) gene, preferably of an albumin gene or on a part thereof. This term includes sequences corresponding to the entire 3'UTR sequence, i.e. the full length 3'UTR sequence of a gene, and sequences corresponding to a fragment of the 3'UTR sequence of a gene, such as an albumin gene, α-globin gene, β-globin gene, tyrosine hydroxylase gene, lipoxygenase gene, or collagen alpha gene, such as a collagen alpha 1(I) gene, preferably of an albumin gene.

The term 'a nucleic acid sequence which is derived from a variant of the 3'UTR of a [...] gene' preferably refers to a nucleic acid sequence. which is based on a variant of the 3'UTR sequence of a gene, such as on a variant of the 3'UTR of an albumin gene, an α-globin gene, a β-globin gene, a tyrosine hydroxylase gene, a lipoxygenase gene, or a collagen alpha gene, such as a collagen alpha 1(I) gene, or on a part thereof as described above. This term includes sequences corresponding to the entire sequence of the variant of the 3'UTR of a gene, i.e. the full length variant 3'UTR sequence of a gene, and sequences corresponding to a fragment of the variant 3'UTR sequence of a gene. A fragment in this context preferably consists of a continuous stretch of nucleotides corresponding to a continuous stretch of nucleotides in the full-length variant 3'UTR, which represents at least 20%. preferably at least 30%, more preferably at least 40%. more preferably at least 50%, even more preferably at least 60%, even more preferably at least 70%, even more preferably at least 80%, and most preferably at least 90% of the full-length variant 3'UTR. Such a fragment of a variant, in the sense of the present invention, is preferably a functional fragment of a variant as described herein.

In a particularly preferred embodiment, the at least one mRNA of the inventive composition comprises at least one 5'-untranslated region element (5'UTR element). Preferably, the at least one 5'UTR element comprises or consists of a nucleic acid sequence, which is derived from the 5'UTR of a TOP gene or which is derived from a fragment, homolog or variant of the 5'UTR of a TOP gene.

It is particularly preferred that the 5'UTR element does not comprise a TOP-motif or a 5'TOP, as defined above.

In some embodiments, the nucleic acid sequence of the 5'UTR element, which is derived from a 5'UTR of a TOP gene. terminates at its 3'-end with a nucleotide located at position 1, 2, 3, 4, 5, B, 7, 8, 9 or 10 upstream of the start codon (e.g. A(U/T)G) of the gene or mRNA it is derived from. Thus, the 5'UTR element does not comprise any part of the protein coding region. Thus, preferably, the only protein coding part of the at least one mRNA of the inventive composition is provided by the coding region.

The nucleic acid sequence derived from the 5'UTR of a TOP gene is preferably derived from a eukaryotic TOP gene, preferably a plant or animal TOP gene, more preferably a chordate TOP gene, even more preferably a vertebrate TOP gene, most preferably a mammalian TOP gene, such as a human TOP gene.

For example, the 5'UTR element is prefereably selected from 5'-UTR elements comprising or consisting of a nucleic acid sequence, which is derived from a nucleic acid sequence selected from the group consisting of SEQ ID Nos: 1-1363, SEQ ID NO: 1395, SEQ ID NO: 1421 and SEQ ID NO: 1422 of the patent application WO2013/143700, whose disclosure is incorporated herein by reference. from the homologs of SEQ ID NOs: 1-1363, SEQ ID NO: 1395, SEQ ID NO: 1421 and SEQ ID NO: 1422 of the patent application WO2013/143700, from a variant thereof, or preferably from a corresponding RNA sequence. The term "homologs of SEQ ID NOs: 1-1363, SEQ ID NO: 1395, SEQ ID NO: 1421 and SEQ ID NO: 1422 of the patent application WD2013/143700" refers to sequences of other species than homo sapiens, which are homologous to the sequences according to SEQ ID NOs: 1-1363, SEQ ID NO: 1395, SEQ ID NO: 1421 and SEQ ID NO: 1422 of the patent application WO2013/143700.

In a preferred embodiment, the 5'UTR element of the RNA according to the invention comprises or consists of a nucleic acid sequence, which is derived from a nucleic acid sequence extending from nucleotide position 5 (i.e. the nucleotide that is located at position 5 in the sequence) to the nucleotide position immediately 5' to the start codon (located at the 3' end of the sequences), e.g. the nucleotide position immediately 5' to the ATG sequence, of a nucleic acid sequence selected from SEQ ID NOs: 1-1363, SEQ ID N0: 1395, SEQ ID NO: 1421 and SEQ ID NO: 1422 of the patent application WO2013/143700, from the homologs of SEQ ID NOs: 1-1363, SEQ ID NO: 1395, SEQ ID NO: 1421 and SEQ ID NO: 1422 of the patent application WO2013/143700 from a variant thereof, or a corresponding RNA sequence. It is particularly preferred that the 5' UTR element is derived from a nucleic acid sequence extending from the nucleotide position immediately 3' to the 5'TOP to the nucleotide position immediately 5' to the start codon (located at the 3' end of the sequences), e.g. the nucleotide position immediately 5' to the ATG sequence, of a nucleic acid sequence selected from SEQ ID NOs: 1-1363, SEQ ID NO: 1395. SEQ ID NO: 1421 and SEQ ID NO: 1422 of the patent application WO2013/143700, from the homologs of SEQ ID NOs 1-1363, SEQ ID NO: 1395, SEQ ID NO: 1421 and SEQ ID NO: 1422 of the patent application WO2013/143700. from a variant thereof, or a corresponding RNA sequence.

In a particularly preferred embodiment, the 5'UTR element comprises or consists of a nucleic acid sequence, which is derived from a 5'UTR of a TOP gene encoding a ribosomal protein or from a variant of a 5'UTR of a TOP gene encoding a ribosomal protein. For example, the 5'UTR element comprises or consists of a nucleic acid sequence, which is derived from a 5'UTR of a nucleic acid sequence according to any of SEQ ID NOs: 67, 170, 193, 244, 259, 554, 650, 675, 700, 721, 913, 1016, 1063, 1120, 1138, and 1284-1360 of the patent application WO2013/143700, a corresponding RNA sequence, a homolog thereof, or a variant thereof as described herein, preferably lacking the 5'TOP motif. As described above, the sequence extending from position 5 to the nucleotide immediately 5' to the ATG (which is located at the 3'end of the sequences) corresponds to the 5'UTR of said sequences.

Preferably, the 5'UTR element comprises or consists of a nucleic acid sequence, which is derived from a 5'UTR of a TOP gene encoding a ribosomal Large protein (RPL) or from a homolog or variant of a 5'UTR of a TOP gene encoding a ribosomal Large protein (RPL). For example, the 5'UTR element comprises or consists of a nucleic acid sequence, which is derived from a 5'UTR of a nucleic acid sequence according to any of SEQ ID NOs: 67, 259, 1284-1318, 1344, 1346, 1348-1354, 1357, 1358, 1421 and 1422 of the patent application WO2013/143700, a corresponding RNA sequence, a homolog thereof, or a variant thereof as described herein, preferably lacking the 5'TOP motif.

In a particularly preferred embodiment, the 5'UTR element comprises or consists of a nucleic acid sequence which is derived from the 5'UTR of a ribosomal protein Large 32 gene, preferably from a vertebrate ribosomal protein Large 32 (L32) gene, more preferably from a mammalian ribosomal protein Large 32 (L32) gene. most preferably from a human ribosomal protein Large 32 (L32) gene, or from a variant of the 5'UTR of a ribosomal protein Large 32 gene, preferably from a vertebrate ribosomal protein Large 32 (L32) gene, more preferably from a mammalian ribosomal protein Large 32 (L32) gene. most preferably from a human ribosomal protein Large 32 (L32) gene, wherein preferably the 5'UTR element does not comprise the 5'TOP of said gene.

Accordingly, in a particularly preferred embodiment, the 5'UTR element comprises or consists of a nucleic acid sequence, which has an identity of at least about 40%, preferably of at least about 50%, preferably of at least about 60%, preferably of at least about 70%, more preferably of at least about 80%, more preferably of at least about 90%. even more preferably of at least about 95%. even more preferably of at least about 99% to the nucleic acid sequence according to SEQ ID NO: 4537 or SEQ ID NO: 4538 (5'-UTR of human ribosomal protein Large 32 lacking the 5' terminal oligopyrimidine tract: GGCGCTGCCTACGGAGGTGGCAGCCATCTCCTTCTCGGCATC; corresponding to SEQ ID NO: 1368 of the patent application WO2013/143700) or preferably to a corresponding RNA sequence, or wherein the at least one 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 4537 or more preferably to a corresponding RNA sequence (SEQ ID NO: 4538). wherein, preferably, the fragment is as described above, i.e. being a continuous stretch of nucleotides representing at least 20% etc. of the full-length 5'LITR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

In some embodiments, the RNA according to the invention comprises a 5'UTR element, which comprises or consists of a nucleic acid sequence, which is derived from the 5'UTR of a vertebrate TOP gene, such as a mammalian, e.g. a human TOP gene. selected from RPSA, RPS2, RPS3, RPS3A, RPS4. RPS5, RPS6, RPS7, RPSS, RPS9, RPS10, RPS11, RPS12, RPS13, RPS14, RPS15, RPS15A, RPS16, RPS17, RPS18, RPS19, RPS20, RPS21, RPS23, RPS24, RPS25, RPS26, RPS27, RPS27A, RPS28, RPS29, RPS30, RPL3. RPL4. RPL5. RPLB, RPL7, RPL7A. RPL8, RPL9, RPL10, RPL10A, RPL11, RPL12, RPL13, RPL13A, RPL14, RPL15, RPL17, RPL18, RPL18A, RPL19, RPL21, RPL22. RPL23, RPL23A, RPL24, RPL2B. RPL27, RPL27A, RPL28, RPL29, RPL30, RPL31, RPL32, RPL34, RPL35, RPL35A, RPL36, RPL36A, RPL37, RPL37A, RPL38, RPL38, RPL40. RPL41, RPLP0, RPLP1, RPLP2. RPLP3. RPLPD. RPLP1, RPLP2, EEFIAI, EEF1B2, EEF1D, EEF1G, EEF2. EIF3E, EIF3F, EIF3H. EIF2S3, EIF3C, EIF3K, EIF3E1P. EIF4A2, PABPC1, HNRNPA1, TPT1, TUBB1, UBA52, NPM1, ATP5G2, GN82L1, NME2, UQCRB, or from a homolog or variant thereof, wherein preferably the 5'UTR element does not comprise a TOP-motif or the 5'TOP of said genes, and wherein optionally the 5'UTR element starts at its 5'-end with a nucleotide located at position 1, 2, 3, 4, 5, 6, 7, 8, 9 or 10 downstream of the 5'terminal oligopyrimidine tract (TOP) and wherein further optionally the 5'UTR element which is derived from a 5'UTR of a TOP gene terminates at its S'-end with a nucleotide located at position 1, 2, 3. 4, 5, 6, 7, 8, 9 or 10 upstream of the start codon (A(U/I)G) of the gene it is derived from.

In further particularly preferred embodiments, the 5'UTR element comprises or consists of a nucleic acid sequence, which is derived from the 5'UTR of a ribosomal protein Large 32 gene (RPL32), a ribosomal protein Large 35 gene (RPL35), a ribosomal protein Large 21 gene (RPL21), an ATP synthase, H+ transporting, mitochondrial F1 complex, alpha subunit I, cardiac muscle (ATP5A1) gene, an hydroxysteroid (17-beta) dehydrogenase 4 gene (HSD17B4). an androgen-induced I gene (AIG1), cytochrome c oxidase subunit Vlc gene (CUX6C), or a N-acylsphingosine amidohydrolase (acid ceramidase) I gene (ASAHI) or from a variant thereof, preferably from a vertebrate ribosomal protein Large 32 gene (RPL32), a vertebrate ribosomal protein Large 35 gene (RPL35), a vertebrate ribosomal protein Large 21 gene (RPL21), a vertebrate ATP synthase, H+ transporting, mitochondrial F1 complex, alpha subunit I, cardiac muscle (ATP5AI) gene. a vertebrate hydroxysteroid (17-beta) dehydrogenase 4 gene (HSD17B4), a vertebrate androgen-induced I gene (AIGI), a vertebrate cytochrome c oxidase subunit Vlc gene (COX6C), or a vertebrate N-acylsphingosine amidohydrolase (acid ceramidase) I gene (ASAHI) or from a variant thereof, more preferably from a mammalian ribosomal protein Large 32 gene (RPL32), a ribosomal protein Large 35 gene (RPL35), a ribosomal protein Large 21 gene (RPL21), a mammalian ATP synthase. H+ transporting, mitochondrial FI complex, alpha subunit I, cardiac muscle (ATP5A1) gene, a mammalian hydroxysteroid (17-beta) dehydrogenase 4 gene (HSD17B4), a mammalian androgen-induced I gene (AlGI), a mammalian cyto-chrome c oxidase subunit Vlc gene (COX6C), or a mammalian N-acylsphingosine amidohydrolase (acid ceramidase) I gene (ASAHI) or from a variant thereof, most preferably from a human ribosomal protein Large 32 gene (RPL32), a human ribosomal protein Large 35 gene (RPL35), a human ribosomal protein Large 21 gene (RPL21), a human ATP synthase. H+ transporting, mitochondrial FI complex, alpha subunit I, cardiac muscle (ATP5A1) gene. a human hydroxysteroid (17-beta) dehydrogenase 4 gene (HSD17B4). a human androgen-induced I gene (AIG1), a human cytochrome c oxidase subunit Vlc gene (COX6C), or a human N-acylsphingosine amidohydrolase (acid ceramidase) I gene (ASAHI) or from a variant thereof, wherein preferably the 5'UTR element does not comprise the 5'TOP of said gene.

Accordingly, in a particularly preferred embodiment, the 5'UTR element comprises or consists of a nucleic acid sequence, which has an identity of at least about 40%, preferably of at least about 50%. preferably of at least about 60%, preferably of at least about 70%. more preferably of at least about 80%, more preferably of at least about 90%, even more preferably of at least about 95%, even more preferably of at least about 99% to the nucleic acid sequence according to SEQ ID NO: 1308, or SEQ ID NOs: 1412-1420 of the patent application WO2013/143700, or a corresponding RNA sequence, or wherein the at least one 5'UTR element comprises or consists of a fragment of a nucleic acid sequence which has an identity of at least about 40%, preferably of at least about 50%, preferably of at least about 60%. preferably of at least about 70%, more preferably of at least about 80%. more preferably of at least about 90%, even more preferably of at least about 95%, even more preferably of at least about 99% to the nucleic acid sequence according to SEQ ID NO: 1368, or SEQ ID NOs: 1412-1420 of the patent application WO2013/143700, wherein, preferably, the fragment is as described above, i.e. being a continuous stretch of nucleotides representing at least 20% etc. of the full-length 5'UTR. Preferably, the fragment exhibits a length of at least about 20 nucleotides or more, preferably of at least about 30 nucleotides or more. more preferably of at least about 40 nucleotides or more. Preferably, the fragment is a functional fragment as described herein.

Accordingly, in a particularly preferred embodiment, the 5'UTR element comprises or consists of a nucleic acid sequence, which has an identity of at least about 40%. preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SEQ ID NO: 4539 or SEQ ID NO: 4540 (5'-UTR of ATPSAI lacking the 5' terminal oligopyrimidine tract: GCGGCTCGGCCATTTTGTCCCAGTCAGTCCGGAGGCTGCGGCTGCAGAAGTACCGCCTGCGGAGTAACTGCAAAG; corresponding to SEQ ID No. 1414 of the patent application WO2013/143700) or preferably to a corresponding RNA sequence, or wherein the at least one 5'UTR element comprises or consists of a fragment of a nucleic acid sequence which has an identity of at least about 40%, preferably of at least about 50%, preferably of at least about 60%, preferably of at least about 70%, more preferably of at least about 80%, more preferably of at least about 90%, even more preferably of at least about 95%, even more preferably of at least about 99% to the nucleic acid sequence according to SED ID NO: 4539or more preferably to a corresponding RNA sequence (SEQ ID NO: 4540). wherein, preferably, the fragment is as described above, i.e. being a continuous stretch of nucleotides representing at least 20% etc. of the full-length 5'UIR. Preferably, the fragment exhibits a length of at least about 20 nucleotides or more, preferably of at least about 30 nucleotides or more, more preferably of at least about 40 nucleotides or more. Preferably, the fragment is a functional fragment as described herein.

Preferably, the at least one 5'UTR element and the at least one 3'UTR element act synergistically to increase protein production from the at least one mRNA of the inventive composition as described above.

In a particularly preferred embodiment, the RNA according to the invention comprises a histone stem-loop sequence/structure. Such histone stem-loop sequences are preferably selected from histone stem-loop sequences as disclosed in WO 2012/019780, the disclosure of which is incorporated herewith by reference.

A histone stem-loop sequence, suitable to be used within the present invention, is preferably selected from at least one of the following formulae (I) or (II):
formula (I) (stem-loop sequence without stem bordering elements): 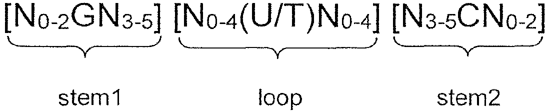
formula (II) (stem-loop sequence with stem bordering elements): 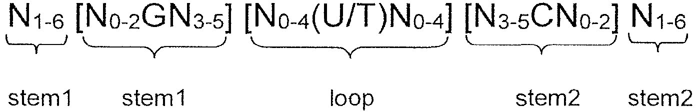 bordering element bordering element
wherein:
   - stem1 or stem2 bordering elements N₁₋₆: is a consecutive sequence of 1 to 6, preferably of 2 to 6, more preferably of 2 to 5. even more preferably of 3 to 5, most preferably of 4 to 5 or 5 N. wherein each N is independently from another selected from a nucleotide selected from A, U, T, G and C. or a nucleotide analogue thereof:
   - stem1 [N₀₋₂GN₃₋₅]: is reverse complementary or partially reverse complementary with element stem2, and is a consecutive sequence between of 5 to 7 nucleotides;
   wherein N₀₋₂ is a consecutive sequence of 0 to 2. preferably of 0 to 1, more preferably of 1 N, wherein each N is independently from another selected from a nucleotide selected from A, U, T, G and C or a nucleotide analogue thereof:
   wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof, and
   wherein G is guanosine or an analogue thereof, and may be optionally replaced by a cytidine or an analogue thereof, provided that its complementary nucleotide cytidine in stem2 is replaced by guanosine;
   - loop sequence [N₀₋₄(U/T)N₀₋₄]: is located between elements stem1 and stem2, and is a consecutive sequence of 3 to 5 nucleotides, more preferably of 4 nucleotides;
   wherein each N₀₋₄ is independent from another a consecutive sequence of 0 to 4. preferably of 1 to 3, more preferably of 1 to 2 N, wherein each N is independently from another selected from a nucleotide selected from A, U, T, G and C or a nucleotide analogue thereof; and
   wherein U/T represents uridine, or optionally thymidine:
   - stem2 [N₃₋₅CN₀₋₂]: is reverse complementary or partially reverse complementary with element stem1, and is a consecutive sequence between of 5 to 7 nucleotides:
   wherein N₃₋₅ is a consecutive sequence of 3 to 5, preferably of 4 to 5, more preferably of 4 N, wherein each N is independently from another selected from a nucleotide selected from A. U, T, G and C or a nucleotide analogue thereof;
   wherein N₀₋₂ is a consecutive sequence of 0 to 2, preferably of 0 to 1. more preferably of 1 N, wherein each N is independently from another selected from a nucleotide selected from A. U, T, G or C or a nucleotide analogue thereof; and
   wherein C is cytidine or an analogue thereof, and may be optionally replaced by a guanosine or an analogue thereof provided that its complementary nucleoside guanosine in stem1 is replaced by cytidine;
wherein
stem1 and stem2 are capable of base pairing with each other forming a reverse complementary sequence, wherein base pairing may occur between stem1 and stem2, e.g. by Watson-Crick base pairing of nucleotides A and U/T or G and C or by non-Watson-Crick base pairing e.g. wobble base pairing, reverse Watson-Crick base pairing, Hoogsteen base pairing, reverse Hoogsteen base pairing or are capable of base pairing with each other forming a partially reverse complementary sequence, wherein an incomplete base pairing may occur between stem1 and stem2, on the basis that one are more bases in one stem do not have a complementary base in the reverse complementary sequence of the other stem.

According to a further preferred embodiment, the RNA according to the invention may comprise at least one histone stem-loop sequence according to at least one of the following specific formulae (Ia) or (Ila):
formula (Ia) (stem-loop sequence without stem bordering elements): 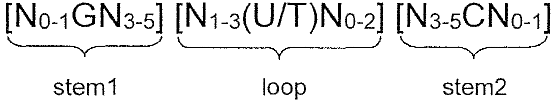
formula (IIa) (stem-loop sequence with stem bordering elements): 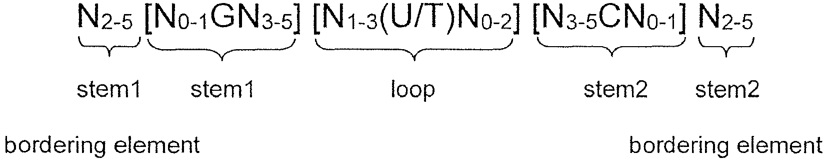
wherein:
   N, C, G, T and U are as defined above.

According to a further more particularly preferred embodiment, the RNA according to the invention may comprise at least one histone stem-loop sequence according to at least one of the following specific formulae (Ib) or (IIb):
formula (lb) (stem-loop sequence without stem bordering elements): 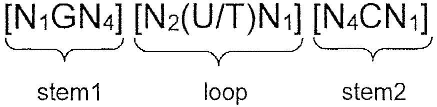
formula (IIb) (stem-loop sequence with stem bordering elements): 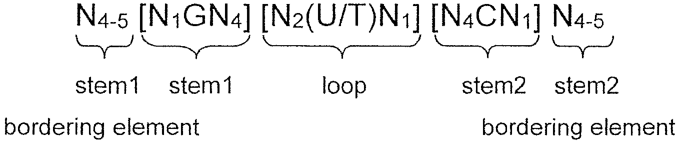
wherein:
   N, C, G, T and U are as defined above.

A particularly preferred histone stem-loop sequence is the sequence CAAAGGCTCTTTTCAGAGCCACCA (according to SEQ ID NO: 3547) or more preferably the corresponding RNA sequence CAAAGGCUCUUUUCAGAGCCACCA (according to SEQ ID NO: 4556).

According to another particularly preferred embodiment, the RNA according to the invention may additionally or alternatively encode a secretory signal peptide. Such signal peptides are sequences, which typically exhibit a length of about 15 to 30 amino acids and are preferably located at the N-terminus of the encoded peptide, without being limited thereto. Signal peptides as defined herein preferably allow the transport of the antigen, antigenic protein or antigenic peptide as encoded by the at least one mRNA of the composition into a defined cellular compartiment. preferably the cell surface, the endoplasmic reticulum (ER) or the endosomal-lysosomal compartiment. Examples of secretory signal peptide sequences as defined herein include, without being limited thereto, signal sequences of classical or non-classical MHC-molecules (e.g. signal sequences of MHC I and II molecules, e.g. of the MHC class I molecule HLA-A*0201). signal sequences of cytokines or immunoglobulines as defined herein, signal sequences of the invariant chain of immunoglobulines or antibodies as defined herein, signal sequences of Lamp1, Tapasin. Erp57, Calretikulin, Calnexin, and further membrane associated proteins or of proteins associated with the endoplasmic reticulum (ER) or the endosomal-lysosomal compartiment. Most preferably, signal sequences of MHC class I molecule HLA-A*0201 may be used according to the present invention. For example, a signal peptide derived from HLA-A is preferably used in order to promote secretion of the encoded antigen as defined herein or a fragment or variant thereof. More preferably, an HLA-A signal peptide is fused to an encoded antigen as defined herein or to a fragment or variant thereof:
Any of the above modifications may be applied to the RNA of the present invention. and further to any RNA as used in the context of the present invention and may be, if suitable or necessary, be combined with each other in any combination, provided, these combinations of modifications do not interfere with each other in the respective at least one mRNA. A person skilled in the art will be able to take his choice accordingly.

The RNA, preferably an mRNA. according to the invention, which comprises at least one coding sequence as defined herein, may preferably comprise a 5' UTR and/or a 3' UTR preferably containing at least one histone stem-loop. Where, in addition to the antigen as defined herein or a fragment or variant thereof, a further peptide or protein is encoded by the at least one coding sequence of the RNA according to the invention, the encoded peptide or protein is preferably no histone protein, no reporter protein and/or no marker or selection protein, as defined herein. The 3' UTR of the RNA according to the invention preferably comprises also a poly(A) and/or a poly(C) sequence as defined herein. The single elements of the 3' UTR may occur therein in any order from 5' to 3' along the sequence of the RNA of the present invention. In addition, further elements as described herein, may also be contained. such as a stabilizing sequence as defined herein (e.g. derived from the UTR of a globin gene), IRES sequences, etc. Each of the elements may also be repeated in the RNA according to the invention at least once (particularly in di- or multicistronic constructs), preferably twice or more. As an example, the single elements may be present in the RNA according to the invention in the following order:
5' - coding region - histone stem-loop - poly(A)/(C) sequence - 3': or
5' - coding region - poly(A)/(C) sequence - histone stem-loop - 3'; or
5' - coding region - histone stem-loop - polyadenylation signal - 3'; or
5' - coding region - polyadenylation signal- histone stem-loop - 3'; or
5' - coding region - histone stem-loop - histone stem-loop - poly(A)/(C) sequence - 3'; or
5' - coding region - histone stem-loop - histone stem-loop - polyadenylation signal- 3'; or
5' - coding region - stabilizing sequence - poly(A)/(C) sequence - histone stem-loop - 3'; or
5' - coding region - stabilizing sequence - poly(A)/(C) sequence - poly(A)/(C) sequence - histone stem-loop - 3': etc.

According to a further embodiment, the RNA. preferably an mRNA. of the present invention preferably comprises at least one of the following structural elements: a 5'- and/or 3'- untranslated region element (UTR element), particularly a 5'-UTR element, which preferably comprises or consists of a nucleic acid sequence which is derived from the 5'-UTR of a TOP gene or from a fragment, homolog or a variant thereof, or a 5'- and/or 3'-UTR element which may preferably be derivable from a gene that provides a stable mRNA or from a homolog, fragment or variant thereof: a histone-stem-loop structure, preferably a histone-stem-loop in its 3' untranslated region; a 5'-cap structure; a poly-A tail: or a poly(C) sequence.

According to some embodiments, it is particularly preferred that - if, in addition to an antigen as defined herein or a fragment or variant thereof, a further peptide or protein is encoded by the at least one coding sequence as defined herein - the encoded peptide or protein is preferably no histone protein, no reporter protein (e.g. Luciferase, GFP, EGFP. β-Galactosidase, particularly EGFP) and/or no marker or selection protein (e.g. alpha-Globin, Galactokinase and Xanthine:Guanine phosphoribosyl transferase (GPT)). In a preferred embodiment, the RNA according to the invention does not comprise a reporter gene or a marker gene. Preferably, the RNA according to the invention does not encode, for instance, luciferase: green fluorescent protein (GFP) and its variants (such as eGFP. RFP or BFP); α-globin; hypoxanthine-guanine phosphoribosyltransferase (HGPRT); β-galactosidase; galactokinase; alkaline phosphatase; secreted embryonic alkaline phosphatase (SEAP)) or a resistance gene (such as a resistance gene against neomycin, puromycin, hygromycin and zeocin). In a preferred embodiment, the RNA according to the invention does not encode luciferase. In another embodiment, the RNA according to the invention does not encode GFP or a variant thereof.

According to a preferred embodiment, the RNA according to the present invention comprises, preferably in 5' to 3' direction, the following elements:
a) a 5'-CAP structure, preferably m7GpppN,
b) at least one coding sequence comprising or consisting of any one of the nucleic acid sequences defined in the fifth column (column 'C') of Table 1, or a fragment or variant thereof,
c) a poly(A) tail. preferably consisting of 10 to 200, 10 to 100, 40 to 80 or 50 to 70 adenosine nucleotides,
d) a poly(C) tail. preferably consisting of 10 to 200, 10 to 100, 20 to 70. 20 to 60 or 10 to 40 cytosine nucleotides, and
e) a histone stem-loop, preferably comprising the RNA sequence according to SEQ ID NO: 4556.

More preferably, the RNA according to the invention comprises, preferably in 5' to 3' direction, the following elements:
a) a 5'-CAP structure, preferably m7GpppN,
b) at least one coding sequence comprising or consisting of any one of the nucleic acid sequences defined in the fifth column (column 'C') of Table 1, or a fragment or variant thereof,
c) a 3'-UTR element comprising a nucleic acid sequence, which is derived from an α-globin gene. preferably comprising the corresponding RNA sequence of the nucleic acid sequence according to SEQ ID NO: 4547, or a homolog. a fragment or a variant thereof.
d) a poly(A) tail. preferably consisting of ID to 200, 10 to 100, 40 to 80 or 50 to 70 adenosine nucleotides,
e) a poly(C) tail. preferably consisting of ID to 200, ID to 100, 20 to 70, 20 to 60 or ID to 40 cytosine nucleotides, and
f) a histone stem-loop, preferably comprising the RNA sequence according to SEQ ID NO: 4556.

In a further embodiment, the RNA according to the invention comprises, preferably in 5' to 3' direction, the following elements:
a) a 5'-CAP structure. preferably m7GpppN,
b) a 5'-UTR element, which preferably comprises or consists of a nucleic acid sequence, which is derived from the 5'-UTR of a TOP gene. preferably comprising an RNA sequence corresponding to the nucleic acid sequence according to SEQ ID NO: 4537, or a homolog, a fragment or a variant thereof,
c) at least one coding sequence comprising or consisting of any one of the nucleic acid sequences defined in the fifth column (column 'C') of Table 1, or a fragment or variant thereof,
d) a 3'-UTR element comprising a nucleic acid sequence, which is preferably derived from an α-globin gene, preferably comprising the corresponding RNA sequence of the nucleic acid sequence according to SEQ ID NO: 4547, or a homolog, a fragment or a variant thereof; and/or
   a 3'-UTR element comprising a nucleic acid sequence, which is derived from an albumin gene. preferably comprising the corresponding RNA sequence of the nucleic acid sequence according to SEQ ID NO: 4551, or a homolog, a fragment or a variant thereof.
e) a poly(A) tail. preferably consisting of 10 to 200, 10 to 100, 40 to 80 or 50 to 70 adenosine nucleotides,
f) a poly(C) tail, preferably consisting of 10 to 200, 10 to 100, 20 to 70, 20 to 60 or 10 to 40 cytosine nucleotides, and
g) a histone stem-loop, preferably comprising the RNA sequence according to SEQ ID NO: 4556.

The RNA according to the present invention may be prepared using any method known in the art, including synthetic methods such as e.g. solid phase RNA synthesis, as well as *in vitro* methods, such as RNA *in vitro* transcription reactions.

In a further aspect, the present invention concerns a composition comprising the RNA comprising at least one coding sequence as defined herein and a pharmaceutically acceptable carrier. The composition according to the invention is preferably provided as a pharmaceutical composition or as a vaccine.

According to a preferred embodiment, the (pharmaceutical) composition or the vaccine according to the invention comprises the RNA of the present invention, wherein the at least one coding sequence of the RNA encodes any one of the tumor antigens as defined in Table 1, preferably as defined in the first ('Gene Name'). second (Protein Accession No.') or third column ('A') of Table 1, or a fragment or variant of any one of these antigens.

Preferably, the (pharmaceutical) composition or the vaccine according to the invention comprises the RNA of the present invention, wherein the at least one coding sequence of the RNA comprises or consists of a nucleic acid sequence encoding a tumor antigen, or a fragment or variant of a tumor antigen, wherein the tumor antigen preferably comprises or consists of any one of the amino acid sequences defined in Table 1 herein, preferably in the third column (column 'A') of Table 1, or a fragment or variant of any one of these sequences.

Preferably, the (pharmaceutical) composition or the vaccine according to the invention comprises the RNA of the present invention, wherein the at least one coding sequence of the RNA comprises or consists of a nucleic acid sequence encoding a tumor antigen, or a fragment or variant of a tumor antigen, wherein the tumor antigen preferably comprises or consists of an amino acid sequence having a sequence identity of at least 5%, 10%. 20%, 30%, 40%, 50%, 60%, 70%, 80%, 85%, 86%. 87%, 88%, 89%, 90%. 91%, 92%, 93%, 94%. 95%, 96%, 97%, 98%, or 99%, preferably of at least 70%. more preferably of at least 80%. even more preferably at least 85%, even more preferably of at least 90% and most preferably of at least 95% or even 97%, with any one of the amino acid sequences defined in Table 1 herein, preferably in the third column (column 'A') of Table 1, or a fragment or variant of any one of these sequences.

More preferably, the (pharmaceutical) composition or the vaccine according to the invention comprises the RNA of the present invention, wherein the at least one coding sequence of the RNA comprises or consists of a nucleic acid sequence encoding a tumor antigen, or a fragment or variant of a tumor antigen, wherein the tumor antigen preferably comprises or consists of an amino acid sequence having a sequence identity of at least 80% with any one of the amino acid sequences defined in Table I herein, preferably in the third column (column 'A') of Table 1, or a fragment or variant of any one of these sequences.

In preferred embodiments, the (pharmaceutical) composition or the vaccine according to the invention comprises the RNA of the present invention, wherein the at least one coding sequence of the RNA comprises or consists of any one of the nucleic acid sequences defined in Table 1, preferably in the fourth or fifth column (column 'B' or 'C', respectively) of Table 1, or a fragment or variant of any one of these sequences.

According to another embodiment, the (pharmaceutical) composition or the vaccine according to the invention comprises the RNA of the present invention, wherein the at least one coding sequence of the RNA comprises or consists of a nucleic acid sequence having a sequence identity of at least 5%, 10%, 20%, 30%, 40%, 50%, 60%, 70%, 80%, 85%, 86%, 87%, 88%, 89%, 90%, 91%, 92%, 93%, 94%, 95%, 96%, 97%, 98%, or 99%, preferably of at least 70%, more preferably of at least 80%. even more preferably at least 85%. even more preferably of at least 90% and most preferably of at least 95% or even 97%, with any one of the nucleic acid sequences defined in Table 1, preferably in the fourth or fifth column (column 'B' or 'C', respectively) of Table 1, or a fragment or variant of any one of these sequences.

According to a particularly preferred embodiment, the (pharmaceutical) composition or the vaccine according to the invention comprises the RNA of the present invention, wherein the at least one coding sequence of the RNA comprises or consists of a nucleic acid sequence having a sequence identity of at least 80% with any one of the nucleic acid sequences defined in Table 1, preferably in the fourth or fifth column (column 'B' or 'C', respectively) of Table 1, or a fragment or variant of any one of these sequences.

More preferably, the (pharmaceutical) composition or the vaccine according to the invention comprises the RNA of the present invention, wherein the at least one coding sequence of the RNA comprises or consists of any one of the nucleic acid sequences defined in the fifth column ('C') of Table 1, or a fragment or variant of any one of these sequences.

According to a further embodiment, the (pharmaceutical) composition or the vaccine according to the invention comprises the RNA of the present invention, wherein the at least one coding sequence of the RNA comprises or consists of a nucleic acid sequence having a sequence identity of at least 5%, 10%, 20%. 30%. 40%. 50%, 60%. 70%, 80%. 85%, 86%, 87%, 88%, 89%, 90%, 91%, 92%, 93%. 94%, 95%, 96%, 97%, 98%, or 99%, preferably of at least 70%, more preferably of at least 80%, even more preferably at least 85%. even more preferably of at least 90% and most preferably of at least 95% or even 97%, with any one of the nucleic acid sequences defined in the fifth column ('C') of Table 1, or a fragment or variant of any one of these sequences.

According to a particularly preferred embodiment, the (pharmaceutical) composition or the vaccine according to the invention comprises the RNA of the present invention, wherein the at least one coding sequence of the RNA comprises or consists of a nucleic acid sequence having a sequence identity of at least 80% with any one of the nucleic acid sequences defined in the fifth column ('C') of Table 1, or a fragment or variant of any one of these sequences.

In the context of the present invention, the (pharmaceutical) composition or vaccine may encode one or more of the tumor antigens defined herein or a fragment or variant thereof.

The (pharmaceutical) composition or vaccine according to the invention may thus comprise the RNA of the present invention, wherein the RNA encodes one specific antigen of the antigens defined herein or a fragment or a variant thereof. In that embodiment, the (pharmaceutical) composition or vaccine preferably comprises the RNA according to the invention comprising the at least one coding sequence as defined herein encoding the antigen or a fragment or variant thereof.

Alternatively, the (pharmaceutical) composition or vaccine of the present invention may comprise at least one RNA according to the invention, wherein the at least one RNA encodes at least two, three, four, five, six, seven, eight, nine, ten, eleven or twelve distinct antigens as defined herein or a fragment or variant thereof. Preferably, the (pharmaceutical) composition or vaccine comprises several classes of the RNA according to the invention, wherein each RNA species encodes one of the antigens or a fragment or variant thereof. In another embodiment, the RNA comprised in the (pharmaceutical) composition or vaccine is a bi- or multicistronic RNA as defined herein, which encodes the at least two, three, four. five. six, seven, eight, nine, ten, eleven or twelve distinct antigens. Mixtures between these embodiments are also envisaged, such as compositions comprising more than one RNA species, wherein at least one RNA species may be monocistronic, while at least one other RNA species may be bi- or multicistronic.

The (pharmaceutical) composition or vaccine according to the present invention, preferably the at least one coding sequence of the RNA comprised therein, may thus comprise any combination of the nucleic acid sequences as defined herein.

In a preferred embodiment of the composition according to the invention, the RNA is complexed with one or more cationic or polycationic compounds, preferably with cationic or polycationic polymers, cationic or polycationic peptides or proteins, e.g. protamine, cationic or polycationic polysaccharides and/or cationic or polycationic lipids.

According to a preferred embodiment, the RNA of the composition according to the present invention may be complexed with lipids to form one or more liposomes, lipoplexes, or lipid nanoparticles. Therefore, in one embodiment, the inventive composition comprises liposomes, lipoplexes, and/or lipid nanoparticles comprising the at least one mRNA.

Lipid-based formulations have been increasingly recognized as one of the most promising delivery systems for RNA due to their biocompatibility and their ease of large-scale production. Cationic lipids have been widely studied as synthetic materials for delivery of RNA. After mixing together, nucleic acids are condensed by cationic lipids to form lipid/nucleic acid complexes known as lipoplexes. These lipid complexes are able to protect genetic material from the action of nucleases and deliver it into cells by interacting with the negatively charged cell membrane. Lipoplexes can be prepared by directly mixing positively charged lipids at physiological pH with negatively charged nucleic acids.

Conventional liposomes consist of a lipid bilayer that can be composed of cationic, anionic, or neutral (phospho)lipids and cholesterol, which encloses an aqueous core. Both the lipid bilayer and the aqueous space can incorporate hydrophobic or hydrophilic compounds, respectively. Liposome characteristics and behaviour in vivo can be modified by addition of a hydrophilic polymer coating, e.g. polyethylene glycol (PEG), to the liposome surface to confer steric stabilization. Furthermore, liposomes can be used for specific targeting by attaching ligands (e.g.. antibodies, peptides, and carbohydrates) to its surface or to the terminal end of the attached PEG chains (Front Pharmacol. 2015 Dec 1;6:286).

Liposomes are colloidal lipid-based and surfactant-based delivery systems composed of a phospholipid bilayer surrounding an aqueous compartment. They may present as spherical vesicles and can range in size from 20 nm to a few microns. Cationic lipid-based liposomes are able to complex with negatively charged nucleic acids via electrostatic interactions, resulting in complexes that offer biocompatibility, low toxicity, and the possibility of the large-scale production required for in vivo clinical applications. Liposomes can fuse with the plasma membrane for uptake; once inside the cell, the liposomes are processed via the endocytic pathway and the genetic material is then released from the endosome/carrier into the cytoplasm. Liposomes have long been perceived as drug delivery vehicles because of their superior biocompatibility, given that liposomes are basically analogs of biological membranes, and can be prepared from both natural and synthetic phospholipids (Int J Nanomedicine. 2014: 9: 1833-1843).

Cationic liposomes have been traditionally the most commonly used non-viral delivery systems for oligonucleotides, including plasmid DNA, antisense oligos, and siRNA/small hairpin RNA-shRNA). Cationic lipids, such as DOTAP, (I.2-dioleoyl-3-trimethylammonium-propane) and DOTMA (N-[1-(2,3-dioleoyloxy)propyl]-N,N,N-trimethyl-ammonium methyl sulfate) can form complexes or lipoplexes with negatively charged nucleic acids to form nanoparticles by electrostatic interaction, providing high in vitro transfection efficiency. Furthermore, neutral lipid-based nanoliposomes for RNA delivery as e.g. neutral 1.2-dioleoyl-sn-glycero-3- phosphatidylcholine (DOPC)-based nanoliposomes were developed. (Adv Drug Deliv Rev. 2014 Feb; 66: 110-116.).

Therefore, in one embodiment the RNA of the composition according to the present invention is complexed with cationic lipids and/or neutral lipids and thereby forms liposomes, lipid nanoparticles, lipoplexes or neutral lipid-based nanoliposomes.

In a preferred embodiment, the composition according to the invention comprises the RNA according to the invention that is formulated together with a cationic or polycationic compound and/or with a polymeric carrier. Accordingly, in a further embodiment of the invention, it is preferred that the RNA as defined herein or any other nucleic acid comprised in the inventive (pharmaceutical) composition or vaccine is associated with or complexed with a cationic or polycationic compound or a polymeric carrier, optionally in a weight ratio selected from a range of about 6:1 (w/w) to about 0.25:1 (w/w), more preferably from about 5:1 (w/w) to about 0.5:1 (w/w). even more preferably of about 4:1 (w/w) to about 1:1 (w/w) or of about 3:1 (w/w) to about 1:1 (w/w). and most preferably a ratio of about 3:1 (w/w) to about 2:1 (w/w) of mRNA or nucleic acid to cationic or polycationic compound and/or with a polymeric carrier; or optionally in a nitrogen/phosphate (N/P) ratio of mRNA or nucleic acid to cationic or polycationic compound and/or polymeric carrier in the range of about 0.1-10, preferably in a range of about 0.3-4 or 0.3-1, and most preferably in a range of about 0.5-1 or 0.7-1, and even most preferably in a range of about 0.3-0.9 or 0.5-0.9. More preferably, the N/P ratio of the at least one mRNA to the one or more polycations is in the range of about 0.1 to 10, including a range of about 0.3 to 4. of about 0.5 to 2. of about 0.7 to 2 and of about 0.7 to 1.5.

Therein, the RNA as defined herein or any other nucleic acid comprised in the (pharmaceutical) composition or vaccine according to the invention can also be associated with a vehicle, transfection or complexation agent for increasing the transfection efficiency and/or the immunostimulatory properties of the RNA according to the invention or of optionally comprised further included nucleic acids. Cationic or polycationic compounds, being particularly preferred agents in this context include protamine, nucleoline, spermine or spermidine, or other cationic peptides or proteins, such as poly-L-lysine (PLL), poly-arginine, basic polypeptides, cell penetrating peptides (CPPs), including HIV-binding peptides. HIV-1 Tat (HIV). Tat-derived peptides. Penetratin, VP22 derived or analog peptides. HSV VP22 (Herpes simplex). MAP, KALA or protein transduction domains (PTDs), PpTG20, prolin-rich peptides, arginine-rich peptides, lysine-rich peptides. MPG-peptide(s), Pep-I. L-oligomers, Calcitonin peptide(s), Antennapedia-derived peptides (particularly from *Drosophila antennapedia*). pAntp. plsl, FGF, Lactoferrin, Transportan, Buforin-2, Bac715-24, SynB, SynB(1), pVEC, hCT-derived peptides, SAP, or histones. More preferably, the RNA according to the invention is complexed with one or more polycations. preferably with protamine or oligofectamine, most preferably with protamine. In this context protamine is particularly preferred.

Additionally, preferred cationic or polycationic proteins or peptides may be selected from the following proteins or peptides having the following total formula (III):

(Arg)ₗ;(Lys)ₘ;(His)ₙ:(Orn)ₙ;(Xaa)ₓ. (formula (III))

wherein l + m + n +o + x = 8-15, and l, m, n or o independently of each other may be any number selected from 0, 1, 2, 3, 4, 5, 6, 7, 8, 9, 10, 11, 12. 13, 14 or 15, provided that the overall content of Arg. Lys. His and Orn represents at least 50% of all amino acids of the oligopeptide: and Xaa may be any amino acid selected from native (= naturally occurring) or non-native amino acids except of Arg, Lγs, His or Orn: and x may be any number selected from 0, 1, 2, 3 or 4. provided, that the overall content of Xaa does not exceed 50% of all amino acids of the oligopeptide. Particularly preferred cationic peptides in this context are e.g. Arg₇. Args, Args, H₃R₉, R₉H₃, HaRsHs. YSSR₉SSY, (RKH)₄, Y(RKH)₂R, etc. In this context the disclosure of WO 2009/030481 is incorporated herewith by reference.

Further preferred cationic or polycationic compounds, which can be used as transfection or complexation agent may include cationic polysaccharides. for example chitosan, polybrene, cationic polymers, e.g. polyethyleneimine (PEI), cationic lipids, e.g. DOTMA: [1-(2,3-sioleyloxy)propyl)]-N,N,N-trimethylammonium chloride, DMRIE, di-C14-amidine, DDTIM, SAINT, OC-Chol. BGTC, CTAP, OOPC, DODAP, DOPE: Dioleyl phosphatidylethanol-amine, DDSPA, DDDAB, DOIC, DMEPC, DOGS: Dioctadecylamidoglicylspermin. DIMRI: Dimyristo-oxypropyl dimethyl hydroxyethyl ammonium bromide, DOTAP: dioleoyloxy-3-(trimethylammonio)propane, DC-6-14: 0,0-ditetradocanoyl-N-(α-trimethylammanioacetyl)diethanolamine chloride, CLIPI: rac-[(2.3-dioctadecyloxypropyl)(2-hydroxyethyl)]-dimethylammonium chloride, CLIPS: rac-[2(2.3-dihexadecyloxypropyl-oxymethyloxy)ethyl]trimethylammoniom, CLIPS: rac-[2(2,3-dihexadecyloxypropyl-oxysuccinyloxy)ethyl]-trimethylammonium, oligofectamine, or cationic or polycationic polymers, e.g. modified polyaminoacids, such as β-aminoacid-polymers or reversed polyamides, etc., modified polyethylenes, such as PVP (poly(N-ethyl-4-vinylpyridinium bromide)). etc., modified acrylates, such as pDMAEMA (poly(dimethylaminoethyl methylacrylate)), etc., modified amidoamines such as pAMAM (poly(amidoamine)), etc., modified polybetaaminoester (PBAE), such as diamine end modified 1,4 butanediol diacrylate-co-5-amino-1-pentanol polymers, etc., dendrimers. such as polypropylamine dendrimers or pAMAM based dendrimers. etc., polyimine(s), such as PEI: poly(ethyleneimine), poly(propyleneimine), etc., polyallylamine, sugar backbone based polymers, such as cyclodextrin based polymers, dextran based polymers, chitosan, etc., silan backbone based polymers, such as PMDXA-PDMS copolymers, etc., blockpolymers consisting of a combination of one or more cationic blocks (e.g. selected from a cationic polymer as mentioned above) and of one or more hydrophilic or hydrophobic blocks (e.g. polyethyleneglycole); etc.

According to a preferred embodiment, the composition of the present invention comprises the RNA as defined herein and a polymeric carrier. A polymeric carrier used according to the invention might be a polymeric carrier formed by disulfide-crosslinked cationic components. The disulfide-crosslinked cationic components may be the same or different from each other. The polymeric carrier can also contain further components. It is also particularly preferred that the polymeric carrier used according to the present invention comprises mixtures of cationic peptides, proteins or polymers and optionally further components as defined herein, which are crosslinked by disulfide bonds as described herein. In this context, the disclosure of WO 2012/013328 is incorporated herewith by reference.

In this context. the cationic components, which form basis for the polymeric carrier by disulfide-crosslinkage, are typically selected from any suitable cationic or polycationic peptide. protein or polymer suitable for this purpose. particular any cationic or polycationic peptide. protein or polymer capable of complexing the RNA as defined herein or a further nucleic acid comprised in the composition, and thereby preferably condensing the RNA or the nucleic acid. The cationic or polycationic peptide. protein or polymer, is preferably a linear molecule, however, branched cationic or polycationic peptides, proteins or polymers may also be used.

Every disulfide-crosslinking cationic or polycationic protein, peptide or polymer of the polymeric carrier, which may be used to complex the RNA according to the invention or any further nucleic acid comprised in the (pharmaceutical) composition or vaccine of the present invention contains at least one -SH moiety, most preferably at least one cysteine residue or any further chemical group exhibiting an - SH moiety. capable of forming a disulfide linkage upon condensation with at least one further cationic or polycationic protein, peptide or polymer as cationic component of the polymeric carrier as mentioned herein.

As defined above, the polymeric carrier, which may be used to complex the RNA of the present invention or any further nucleic acid comprised in the (pharmaceutical) composition or vaccine according to the invention may be formed by disulfide-crosslinked cationic (or polycationic) components. Preferably, such cationic or polycationic peptides or proteins or polymers of the polymeric carrier, which comprise or are additionally modified to comprise at least one -SH moiety, are selected from, proteins, peptides and polymers as defined herein for complexation agent.

In a further particular embodiment, the polymeric carrier which may be used to complex the RNA as defined herein or any further nucleic acid comprised in the (pharmaceutical) composition or vaccine according to the invention may be selected from a polymeric carrier molecule according to generic formula (IV):

L-P¹-S-[S-P²-S]ₙ-S-P³-L formula (IV)

wherein,
- P¹ and P³: are different or identical to each other and represent a linear or branched hydrophilic polymer chain, each P¹ and P³ exhibiting at least one -SH-moiety, capable to form a disulfide linkage upon condensation with component P², or alternatively with (AA). (AA)ₓ, or [(AA)ₓ]_{z} if such components are used as a linker between P¹ and P² or P³ and P²) and/or with further components (e.g. (AA), (AA)ₓ, [(AA)ₓ]_{z} or L), the linear or branched hydrophilic polymer chain selected independent from each other from polyethylene glycol (PEG), poly-*N*-(2 -hydroxypropyl)methacrylamide, poly-2-(methacryloyloxy)ethyl phosphorylcholines, poly(hydroxyalkyl L-asparagine), poly(2-(methacryloyloxy)ethyl phosphorylcholine), hydroxyethylstarch or poly(hydroxyalkyl L-glutamine). wherein the hydrophilic polymer chain exhibits a molecular weight of about 1 kDa to about 100 kDa, preferably of about 2 kDa to about 25 kDa; or more preferably of about 2 kDa to about 10 kDa, e.g. about 5 kDa to about 25 kDa or 5 kDa to about 10 kDa;
- P²: is a cationic or polycationic peptide or protein, e.g. as defined above for the polymeric carrier formed by disulfide-crosslinked cationic components, and preferably having a length of about 3 to about 100 amino acids, more preferably having a length of about 3 to about 50 amino acids, even more preferably having a length of about 3 to about 25 amino acids, e.g. a length of about 3 to 10, 5 to 15, 10 to 20 or 15 to 25 amino acids, more preferably a length of about 5 to about 20 and even more preferably a length of about 10 to about 20; or
is a cationic or polycationic polymer, e.g. as defined above for the polymeric carrier formed by disulfide-crosslinked cationic components, typically having a molecular weight of about 0.5 kDa to about 30 kDa, including a molecular weight of about I kDa to about 20 kDa, even more preferably of about 1.5 kDa to about 10 kDa, or having a molecular weight of about 0.5 kDa to about 100 kDa, including a molecular weight of about 10 kDa to about 50 kDa, even more preferably of about 10 kDa to about 30 kDa;
each P² exhibiting at least two -SH-moieties, capable to form a disulfide linkage upon condensation with further components P² or component(s) P¹ and/or P³ or alternatively with further components (e.g. (AA), (AA)ₓ, or [(AA)ₓ]_{z});
- -S-S-: is a (reversible) disulfide bond (the brackets are omitted for better readability), wherein δ preferably represents sulphur or a -SH carrying moiety, which has formed a (reversible) disulfide bond. The (reversible) disulfide bond is preferably formed by condensation of -SH-moieties of either components P¹ and P², P² and P². or P² and P³, or optionally of further components as defined herein (e.g. L, (AA), (AA)ₓ, [(AA)ₓ]_{z}, etc); The -SH-moiety may be part of the structure of these components or added by a modification as defined below:
- L: is an optional ligand, which may be present or not. and may be selected independent from the other from RGD, Transferrin, Folate, a signal peptide or signal sequence, a localization signal or sequence, a nuclear localization signal or sequence (NLS), an antibody, a cell penetrating peptide. (e.g. TAT or KALA), a ligand of a receptor (e.g. cytokines, hormones, growth factors etc), small molecules (e.g. carbohydrates like mannose or galactose or synthetic ligands), small molecule agonists, inhibitors or antagonists of receptors (e.g. RGD peptidomimetic analogues), or any further protein as defined herein, etc.:
- n: is an integer, typically selected from a range of about I to 5D. preferably from a range of about 1, 2 or 3 to 30, more preferably from a range of about 1, 2, 3, 4, or 5 to 25. or a range of about 1, 2, 3, 4, or 5 to 20, or a range of about 1, 2, 3, 4, or 5 to 15, or a range of about 1, 2, 3. 4, or 5 to 10, including e.g. a range of about 4 to 9, 4 to 10, 3 to 20, 4 to 20, 5 to 20. or 10 to 20, or a range of about 3 to 15, 4 to 15, 5 to 15, or 10 to 15, or a range of about 6 to 11 or 7 to 10. Most preferably, n is in a range of about 1, 2, 3, 4, or 5 to 10, more preferably in a range of about 1, 2. 3, or 4 to 9. in a range of about 1, 2, 3, or 4 to 8, or in a range of about 1, 2, or 3 to 7.

In this context, the disclosure of WO 2011/026641 is incorporated herewith by reference. Each of hydrophilic polymers P¹ and P³ typically exhibits at least one -SH-moiety, wherein the at least one -SH-moiety is capable to form a disulfide linkage upon reaction with component P² or with component (AA) or (AA)ₓ, if used as linker between P¹ and P² or P³ and P² as defined below and optionally with a further component, e.g. L and/or (AA) or (AA)ₓ. e.g. if two or more -SH-moieties are contained. The following subformulae "P¹-S-S-P²" and "P²-S-S-P³" within generic formula (IV) above (the brackets are omitted for better readability), wherein any of S, P¹ and P³ are as defined herein, typically represent a situation, wherein one-SH-moiety of hydrophilic polymers P¹ and P³ was condensed with one -SH-moiety of component P² of generic formula (IV) above, wherein both sulphurs of these -SH-moieties form a disulfide bond -S-S- as defined herein in formula (IV). These -SH-moieties are typically provided by each of the hydrophilic polymers P¹ and P³, e.g. via an internal cysteine or any further (modified) amino acid or compound which carries a -SH moiety. Accordingly, the subformulae "P¹-S-S-P²" and "P²-S-S-P³" may also be written as "P¹-Cys-Cys-P²" and "P²-Cys-Cys-P³", if the -SH- moiety is provided by a cysteine. wherein the term Cys-Cys represents two cysteines coupled via a disulfide bond, not via a peptide bond. In this case, the term "-S-S-" in these formulae may also be written as "-S-Cys", as "-Cys-S" or as "-Cys-Cys-". In this context, the term "-Cys-Cys-" does not represent a peptide bond but a linkage of two cysteines via their -SH-moieties to form a disulfide bond. Accordingly, the term "-Cys-Cys-" also may be understood generally as "-(Cys-S)-(S-Cys)-", wherein in this specific case S indicates the sulphur of the -SH-moiety of cysteine. Likewise, the terms "-S-Cys" and "-Cys-S" indicate a disulfide bond between a -SH containing moiety and a cysteine. which may also be written as "-S-(S-Cys)" and "-(Cys-S)-S". Alternatively, the hydrophilic polymers P¹ and P³ may be modified with a -SH moiety, preferably via a chemical reaction with a compound carrying a -SH moiety, such that each of the hydrophilic polymers P¹ and P³ carries at least one such -SH moiety. Such a compound carrying a -SH moiety may be e.g. an (additional) cysteine or any further (modified) amino acid, which carries a -SH moiety. Such a compound may also be any non-amino compound or moiety, which contains or allows to introduce a -SH moiety into hydrophilic polymers P¹ and P³ as defined herein. Such non-amino compounds may be attached to the hydrophilic polymers P¹ and P³ of formula (IV) of the polymeric carrier according to the present invention via chemical reactions or binding of compounds, e.g. by binding of a 3-thio propionic acid or thioimolane, by amide formation (e.g. carboxylic acids, sulphonic acids, amines, etc), by Michael addition (e.g maleinimide moieties, α,β-unsatured carbonyls, etc), by click chemistry (e.g. azides or alkines), by alkene/alkine methatesis (e.g. alkenes or alkines), imine or hydrozone formation (aldehydes or ketons, hydrazins, hydroxylamins, amines), complexation reactions (avidin, biotin, protein G) or components which allow Sₙ-type substitution reactions (e.g halogenalkans, thiols, alcohols, amines, hydrazines, hydrazides, sulphonic acid esters, oxyphosphonium salts) or other chemical moieties which can be utilized in the attachment of further components. A particularly preferred PEG derivate in this context is alpha-Methoxy-omega-mercapto polyethylene glycol). In each case, the SH-moiety, e.g. of a cysteine or of any further (modified) amino acid or compound, may be present at the terminal ends or internally at any position of hydrophilic polymers P¹ and P³. As defined herein, each of hydrophilic polymers P¹ and P³ typically exhibits at least one -SH-moiety preferably at one terminal end. but may also contain two or even more -SH-moieties, which may be used to additionally attach further components as defined herein, preferably further functional peptides or proteins e.g. a ligand, an amino acid component (AA) or (AA)ₓ, antibodies, cell penetrating peptides or enhancer peptides (e.g. TAT, KALA), etc.

Preferably, the inventive composition comprises at least one RNA as defined herein, which is complexed with one or more polycations, and at least one free RNA, wherein the at least one complexed RNA is preferably identical to the at least one free RNA. In this context, it is particularly preferred that the composition of the present invention comprises the RNA according to the invention that is complexed at least partially with a cationic or polycationic compound and/or a polymeric carrier, preferably cationic proteins or peptides. In this context, the disclosure of WO 2010/037539 and WO 2012/113513 is incorporated herewith by reference. Partially means that only a part of the RNA as defined herein is complexed in the composition according to the invention with a cationic compound and that the rest of the RNA as defined herein is (comprised in the inventive (pharmaceutical) composition or vaccine) in uncomplexed form ("free"). Preferably, the molar ratio of the complexed RNA to the free RNA is selected from a molar ratio of about 0.001:1 to about 1:0,001, including a ratio of about 1:1. More preferably the ratio of complexed RNA to free RNA (in the (pharmaceutical) composition or vaccine of the present invention) is selected from a range of about 5:1 (w/w) to about 1:10 (w/w). more preferably from a range of about 4:1 (w/w) to about 1:8 (w/w). even more preferably from a range of about 3:1 (w/w) to about 1:5 (w/w) or 1:3 (w/w). and most preferably the ratio of complexed mRNA to free mRNA in the inventive pharmaceutical composition or vaccine is selected from a ratio of about 1:1 (w/w).

The complexed RNA in the (pharmaceutical) composition or vaccine according to the present invention, is preferably prepared according to a first step by complexing the RNA according to the invention with a cationic or polycationic compound and/or with a polymeric carrier, preferably as defined herein. in a specific ratio to form a stable complex. In this context, it is highly preferable, that no free cationic or polycationic compound or polymeric carrier or only a negligibly small amount thereof remains in the component of the complexed RNA after complexing the RNA. Accordingly, the ratio of the RNA and the cationic or polycationic compound and/or the polymeric carrier in the component of the complexed RNA is typically selected in a range so that the RNA is entirely complexed and no free cationic or polycationic compound or polymeric carrier or only a negligibly small amount thereof remains in the composition.

Preferably the ratio of the RNA as defined herein to the cationic or polycationic compound and/or the polymeric carrier, preferably as defined herein, is selected from a range of about 6:1 (w/w) to about 0,25:1 (w/w), more preferably from about 5:1 (w/w) to about 0,5:1 (w/w), even more preferably of about 4:1 (w/w) to about 1:1 (w/w) or of about 3:1 (w/w) to about 1:1 (w/w). and most preferably a ratio of about 3:1 (w/w) to about 2:1 (w/w). Alternatively, the ratio of the RNA as defined herein to the cationic or polycationic compound and/or the polymeric carrier, preferably as defined herein, in the component of the complexed mRNA. may also be calculated on the basis of the nitrogen/phosphate ratio (N/P-ratio) of the entire complex. In the context of the present invention, an N/P-ratio is preferably in the range of about 0.1-10, preferably in a range of about 0.3-4 and most preferably in a range of about 0.5-2 or 0.7-2 regarding the ratio of RNA : cationic or polycationic compound and/or polymeric carrier, preferably as defined herein, in the complex, and most preferably in a range of about 0,7-1,5, 0,5-1 or 0.7-1, and even most preferably in a range of about 0,3-0,9 or 0,5-0,9, preferably provided that the cationic or polycationic compound in the complex is a cationic or polycationic cationic or polycationic protein or peptide and/or the polymeric carrier as defined above. In this specific embodiment the complexed RNA as defined herein is also emcompassed in the term "adjuvant component".

In other embodiments, the composition according to the invention comprising the RNA as defined herein may be administered naked without being associated with any further vehicle, transfection or complexation agent.

It has to be understood and recognized, that according to the present invention, the inventive composition may comprise at least one naked RNA as defined herein, preferably an mRNA, and/or at least one formulated/complexed RNA as defined herein, preferably an mRNA. wherein every formulation and/or complexation as disclosed above may be used.

According to another embodiment, the (pharmaceutical) composition or vaccine according to the invention may comprise an adjuvant. which is preferably added in order to enhance the immunostimulatory properties of the composition. In this context, an adjuvant may be understood as any compound, which is suitable to support administration and delivery of the composition according to the invention. Furthermore, such an adjuvant may, without being bound thereto, initiate or increase an immune response of the innate immune system, i.e. a non-specific immune response. In other words, when administered, the composition according to the invention typically initiates an adaptive immune response due to an antigen as defined herein or a fragment or variant thereof, which is encoded by the at least one coding sequence of the inventive RNA contained in the composition of the present invention. Additionally, the composition according to the invention may generate an (supportive) innate immune response due to addition of an adjuvant as defined herein to the composition according to the invention.

Such an adjuvant may be selected from any adjuvant known to a skilled person and suitable for the present case, i.e. supporting the induction of an immune response in a mammal. Preferably, the adjuvant may be selected from the group consisting of, without being limited thereto. TDM, MDP, muramyl dipeptide. pluronics, alum solution, aluminium hydroxide, ADJUMER^{™} (polyphosphazene); aluminium phosphate gel; glucans from algae; algammulin; aluminium hydroxide gel (alum); highly protein-adsorbing aluminium hydroxide gel; low viscosity aluminium hydroxide gel; AF or SPT (emulsion of squalane (5%). Tween 80 (0,2%), Pluronic L121 (1.25%), phosphate-buffered saline, pH 7.4); AVRIDINE^{™} (propanediamine); BAY R1005^{™} ((N-(2-deoxy-2-L-leucylamino-b-D-glucopyranosyl)-N-octadecyl-dodecanoyl-amide hydroacetate); CALCITRIOL^{™} (1-alpha,25-dihydroxy-vitamin D3); calcium phosphate gel; CAPT^{™} (calcium phosphate nanoparticles): cholera holotoxin, cholera-toxin-Al-protein-A-D-fragment fusion protein, sub-unit B of the cholera toxin; CRL 1005 (block copolymer P1205); cytokine-containing liposomes; DDA (dimethyldioctadecylammonium bromide): DHEA (dehydroepiandrosterone); DMPC (dimyristoylphosphatidylcholine); DMPG (dimyristoylphosphatidylglycerol); DOC/alum complex (deoxycholic acid sodium salt); Freund's complete adjuvant: Freund's incomplete adjuvant; gamma inulin: Gerbu adjuvant (mixture of: i) N-acetylglucosaminyl-(PI-4)-N-acetylmuramyl-L-alanyl-D-glutamine (GMDP), ii) dimethyldioctadecylammonium chloride (DDA), iii) zinc-L-proline salt complex (ZnPro-8); GM-CSF); GMDP (N-acetylglucosaminyl-(bl-4)-N-acetylmuramyl-L-alanyl-D-isoglutamine); imiquimod (I-(2-methypropyl)-1H-imidazo[4.5-c]quinoline-4-amine); ImmTher^{™} (N-acetylglucosaminyl-N-acetylmuramyl-L-Ala-D-isoGlu-L-Ala-glycerol dipalmitate); DRVs (immunoliposomes prepared from dehydration-rehydration vesicles); interferon-gamma; interleukin-Ibeta; interleukin-2; interleukin-7; interleukin-12; tICCMS^{™}: ISCOPREP 7.0.3.^{™}: liposomes; LOXORIBINE^{™} (7-allyl-8-oxogoaoosine); LT oral adjuvant (E.coli labile enterotoxin-protoxin): microspheres and microparticles of any composition; MF59^{™}; (squalene-water emulsion); MONTANIDE ISA 51^{™} (purified incomplete Freund's adjuvant); MONTANIDE ISA 720^{™} (metabolisable oil adjuvant); MPL^{™} (3-Q-desacyl-4'-monophosphoryl lipid A); MTP-PE and MTP-PE liposomes ((N-acetyl-L-alanyl-D-isoglutaminyl-L-alanine-2-(1,2-dipalmitoyl-sn-glycero-3-(hydroxyphosphoryloxy))-ethylamide, monosodium salt); MURAMETIDE^{™} (Nac-Mur-L-Ala-D-Gln-OCH3); MURAPALMITINE^{™} and D-MURAPALMITINE^{™} (Nac-Mur-L-Thr-D-isoGln-sn-glycepoldipalmitoyl): NAGO (neuraminidase-galactose oxidase); nanospheres or nanoparticles of any composition; NISVs (non-ionic surfactant vesicles); PLEURAN^{™} (β-glucan): PLGA, PGA and PLA (homo- and co-polymers of lactic acid and glycolic acid; microspheres/nanospheres); PLURONIC·L121^{™}; PMMA (polymethyl methacrylate); PODDS^{™} (proteinoid microspheres); polyethylene carbamate derivatives; poly-rA: poly-rU (polyadenylic acid-polyuridylic acid complex); polysorbate 80 (Tween 80); protein cochleates (Avanti Polar Lipids, Inc., Alabaster, AL); STIMULON^{™} (QS-21): Quil-A (Quil-A saponin): S-28463 (4-amino-otec-dimethyl-2-ethoxymethyl-1H-imidazo[4,5 c]quinoline-I-ethanol); SAF-1^{™} ("Syntex adjuvant formulation"); Sendai proteoliposomes and Sendai-containing lipid matrices: Span-85 (sorbitan trioleate); Specol (emulsion of Marcol 52, Span 85 and Tween 85); squalene or Robane^{®} (2,6.10,15.19.23-hexamethyltetracosan and 2,6,10,15,19,23-hexamethyl-2,6,10,14,18,22-tetracosahexane); stearyltyrosine (octadecyltyrosine hydrochloride); Theramid^{®} (N-acetylglucosaminyl-N-acetylmuramyl-L-Ala-D-isoGlu-L-Ala-dipalmitoxypropylamide); Theronyl-MDP (Termurtide^{™} or [thr 1]-MDP; N-acetylmuramyl-L-threonyl-D-isoglutamine); Ty particles (Ty-VLPs or virus-like particles); Walter-Reed liposomes (liposomes containing lipid A adsorbed on aluminium hydroxide), and lipopeptides, including Pam3Cys, in particular aluminium salts, such as Adju-phos, Alhydrogel. Rehydragel; emulsions, including CFA, SAF, IFA, MF59, Provax, TiterMax, Montanide, Vaxfectin; copolymers, including Optivax (CRL1005), L121, Poloaxmer4010), etc.; liposomes, including Stealth, cochleates, including BIORAL; plant derived adjuvants, including QS21, Quil A. Iscomatrix, ISCOM; adjuvants suitable for costimulation including Tomatine, biopolymers, including PLG, PMM, Inulin; microbe derived adjuvants, including Romurtide, DETOX, MPL, CWS, Mannose, CpG nucleic acid sequences. CpG7909, ligands of human TLR 1-10, ligands of murine TLR 1-13, ISS-1018, IC31, Imidazoquinolines. Ampligen. Ribi529, IMOxine, IRIVs, VLPs, cholera toxin, heat-labile toxin, Pam3Cys, Flagellin, GPI anchor, LNFPIII/Lewis X, antimicrobial peptides. UC-1V150, RSV fusion protein, cdiGMP; and adjuvants suitable as antagonists including CGRP neuropeptide.

Particularly preferred, an adjuvant may be selected from adjuvants, which support induction of a Thl-immune response or maturation of naïve T-cells, such as GM-CSF. IL-12, IFNγ, any immunostimulatory nucleic acid as defined above. preferably an immunostimulatory RNA. CpG DNA, etc.

In a further preferred embodiment it is also possible that the inventive composition contains besides the antigen-providing RNA further components which are selected from the group comprising: further antigens (e.g. in the form of a peptide or protein) or further antigen-encoding nucleic acids; a further immunotherapeutic agent; one or more auxiliary substances; or any further compound, which is known to be immunostimulating due to its binding affinity (as ligands) to human Toll-like receptors; and/or an adjuvant nucleic acid, preferably an immunostimulatory RNA (isRNA).

The composition of the present invention can additionally contain one or more auxiliary substances in order to increase its immunogenicity or immunostimulatory capacity, if desired. A synergistic action of the RNA as defined herein and of an auxiliary substance, which may be optionally contained in the inventive composition, is preferably achieved thereby. Depending on the various types of auxiliary substances, various mechanisms can come into consideration in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS. GP96, etc.) or cytokines, such as GM-CFS, which allow an immune response to be enhanced and/or influenced in a targeted manner. Particularly preferred auxiliary substances are cytokines. such as monokines, lymphokines, interleukins or chemokines. that further promote the innate immune response, such as IL-1, IL-2, IL-3, IL-4, IL-5, IL-6, IL-7, IL-8, IL-9, IL-10, IL-12, IL-13, IL-14. IL-15, IL-16, IL-17, IL-18, IL-19, IL-29, IL-21, IL-22, IL-23, IL-24, IL-25, IL-26, IL-27, IL-28, IL-29, IL-36, IL-31, IL-32, IL-33, IFN-alpha, IFN-beta, IFN-gamma, GM-CSF, G-CSF, M-CSF, LT-beta or TNF-alpha, growth factors, such as hGH.

Suitable adjuvants may also be selected from cationic or polycationic compounds wherein the adjuvant is preferably prepared upon complexing the RNA of the composition according to the invention with the cationic or polycationic compound. Associating or complexing the RNA of the composition with cationic or polycationic compounds as defined herein preferably provides adjuvant properties and confers a stabilizing effect to the RNA of the composition. In particular, such preferred cationic or polycationic compounds are selected from cationic or polycationic peptides or proteins, including protamine, nucleoline, spermin or spermidine, or other cationic peptides or proteins, such as poly-L-lysine (PLL), poly-arginine, basic polypeptides, cell penetrating peptides (CPPs), including HIV-binding peptides. Tat, HIV-1 Tat (HIV), Tat-derived peptides, Penetratin, VP22 derived or analog peptides. HSV VP22 (Herpes simplex), MAP, KALA or protein transduction domains (PTDs, PpT620, prolin-rich peptides, arginine-rich peptides, lysine-rich peptides, MPG-peptide(s), Pep-I, L-oligomers. Calcitonin peptide(s), Antennapedia-derived peptides (particularly from Drosophila antennapedia). pAntp, plsl, FGF, Lactoferrin, Transportan, Buforin-2, Bac715-24, SynB. SynB(1), pVEC, hCT-derived peptides, SAP, protamine, spermine, spermidine, or histones. Further preferred cationic or polycationic compounds may include cationic polysaccharides, for example chitosan, polybrene, cationic polymers, e.g. polyethyleneimine (PEI), cationic lipids, e.g. DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC. DODAP, DOPE: Dioleyl phosphatidylethanol-amine. DOSPA, DDDAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(α-trimethylammonioacetyl)diethanolamine chloride, CLIPI: rac-[(2,3-dioctadecyloxypropyl)(2-hydroxyethyl)]-dimethylammonium chloride, CLIP6: rac-[2(2.3-dihexadecyloxypropyl-oxymethyloxy)ethyl]-trimethylammonium, CLIP9: rac-[2(2,3-dihexadecyloxypropyl-oxysuccinyloxy)ethyl]-trimethylammonium, oligofectamine, or cationic or polycationic polymers, e.g. modified polyaminoacids, such as β-aminoacid-polymers or reversed polyamides, etc., modified polyethylenes, such as PVP (poly(N-ethyl-4-vinylpyridinium bromide)). etc., modified acrylates, such as pDMAEMA (poly(dimethylaminoethyl methylacrylate)), etc., modified Amidoamines such as pAMAM (poly(amidoamine)). etc., modified polybetaaminoester (PBAE), such as diamine end modified 1.4 butanediol diacrylate-co-5-amino-1-pentanol polymers, etc., dendrimers. such as polypropylamine dendrimers or pAMAM based dendrimers. etc., polyimine(s), such as PEI: poly(ethyleneimine), poly(propyleneimine), etc., polyallylamine, sugar backbone based polymers, such as cyclodextrin based polymers, dextran based polymers, Chitosan, etc., silan backbone based polymers, such as PMOXA-PDMS copolymers, etc., blockpolymers consisting of a combination of one or more cationic blocks (e.g. selected of a cationic polymer as mentioned above) and of one or more hydrophilic- or hydrophobic blocks (e.g polyethyleneglycole); etc.

Additionally, preferred cationic or polycationic proteins or peptides, which can be used as an adjuvant by complexing the RNA of the composition according to the invention, may be selected from following proteins or peptides having the following total formula (III): (Arg)ₗ;(Lys)ₘ:(His)ₙ:(Orn)ₒ;(Xaa)ₓ, wherein l + m + n +o + x = 8-15, and l, m, n or o independently of each other may be any number selected from 0, 1, 2, 3, 4, 5, 6, 7, 8, 9, 10, 11, 12, 13, 14 or 15, provided that the overall content of Arg, Lys. His and Orn represents at least 50% of all amino acids of the oligopeptide; and Xaa may be any amino acid selected from native (= naturally occurring) or non-native amino acids except of Arg, Lys. His or Orn; and x may be any number selected from 0, 1, 2, 3 or 4. provided, that the overall content of Xaa does not exceed 50% of all amino acids of the oligopeptide. Particularly preferred oligoarginines in this context are e.g. Arg_{7,} Args. Args. Arg₇, H₃R₉, R₉H₃, H₃R₉H₃, YSSR₉SSY, (RKH)₄, Y(RKH)₂R, etc.

The ratio of the RNA to the cationic or polycationic compound in the adjuvant component may be calculated on the basis of the nitrogen/phosphate ratio (N/P-ratio) of the entire RNA complex, i.e. the ratio of positively charged (nitrogen) atoms of the cationic or polycationic compound to the negatively charged phosphate atoms of the nucleic acids. For example, 1 pg of RNA typically contains about 3 nmol phosphate residues, provided the RNA exhibits a statistical distribution of bases. Additionally, I pg of peptide typically contains about x nmol nitrogen residues, dependent on the molecular weight and the number of basic amino acids. When exemplarily calculated for (Arg)₉ (molecular weight 1424 g/mol. 9 nitrogen atoms). 1 pg (Arg)₉ contains about 700 pmol (Arg)₉ and thus 700 x 9=6300 pmol basic amino acids = 6.3 nmol nitrogen atoms. For a mass ratio of about 1:1 RNA/(Arg)9 an N/P ratio of about 2 can be calculated. When exemplarily calculated for protamine (molecular weight about 4250 g/mol. 21 nitrogen atoms. when protamine from salmon is used) with a mass ratio of about 2:1 with 2 pg RNA. 6 nmol phosphate are to be calulated for the RNA: 1 pg protamine contains about 235 pmol protamine molecues and thus 235 x 21 = 4935 pmol basic nitrogen atoms = 4.9 nmol nitrogen atoms. For a mass ratio of about 2:1 RNA/protamine an N/P ratio of about 0,81 can be calculated. For a mass ratio of about 8:1 RNA/protamine an N/P ratio of about 0.2 can be calculated. In the context of the present invention, an N/P-ratio is preferably in the range of about 0.1-10, preferably in a range of about 0,3-4 and most preferably in a range of about 0.5-2 or 0.7-2 regarding the ratio of RNA: peptide in the complex, and most preferably in the range of about 0,7-1,5.

In a preferred embodiment, the composition of the present invention is obtained in two separate steps in order to obtain both, an efficient immunostimulatory effect and efficient translation of the RNA according to the invention. Therein, a so called "adjuvant component" is prepared by complexing - in a first step - an RNA as defined herein of the adjuvant component with a cationic or polycationic compound in a specific ratio to form a stable complex. In this context, it is important, that no free cationic or polycationic compound or only a neglibly small amount remains in the adjuvant component after complexing the RNA. Accordingly, the ratio of the RNA and the cationic or polycationic compound in the adjuvant component is typically selected in a range that the RNA is entirely complexed and no free cationic or polycationic compound or only a neglectably small amount remains in the composition. Preferably the ratio of the adjuvant component, i.e. the ratio of the RNA to the cationic or polycationic compound is selected from a range of about 6:1 (w/w) to about 0,25:1 (w/w), more preferably from about 5:1 (w/w) to about 0.5:1 (w/w). even more preferably of about 4:1 (w/w) to about 1:1 (w/w) or of about 3:1 (w/w) to about 1:1 (w/w), and most preferably a ratio of about 3:1 (w/w) to about 2:1 (w/w).

According to a preferred embodiment, the RNA of the invention comprising at least one coding sequence as defined herein is added in a second step to the complexed RNA of the adjuvant component in order to form the (immunostimulatory) composition of the invention. Therein, the RNA of the composition according to the invention is added as free RNA, preferably as free mRNA, which is not complexed by other compounds. Prior to addition, the free RNA is not complexed and will preferably not undergo any detectable or significant complexation reaction upon the addition of the adjuvant component. This is due to the strong binding of the cationic or polycationic compound to the above described RNA according to the invention comprised in the adjuvant component. In other words, when the RNA comprising at least one coding sequence as defined herein is added to the "adjuvant component", preferably no free or substantially no free cationic or polycationic compound is present, which could form a complex with the free RNA. Accordingly, an efficient translation of the RNA of the composition is possible in vivo. Therein, the free RNA, preferably an mRNA, may occur as a mono-, di-, or multicistronic (m)RNA, i.e. an RNA which carries the coding sequences of one or more proteins. Such coding sequences in di-. or even multicistronic mRNA may be separated by at least one IRES sequence, e.g. as defined herein.

In a particularly preferred embodiment, the free RNA as defined herein, which is comprised in the composition of the present invention, may be identical or different to the RNA as defined herein, which is comprised in the adjuvant component of the composition, depending on the specific requirements of therapy. Even more preferably, the free RNA, which is comprised in the composition according to the invention, is identical to the RNA of the adjuvant component of the inventive composition.

In a particularly preferred embodiment, the composition according to the invention comprises the RNA of the invention, which encodes at least one antigen as defined herein and wherein said RNA is present in the composition partially as free RNA and partially as complexed RNA. Preferably, the RNA as defined herein, preferably an mRNA. is complexed as described above and the same (m)RNA is then added as free RNA, wherein preferably the compound, which is used for complexing the RNA is not present in free form in the composition at the moment of addition of the free RNA component.

The ratio of the first component (i.e. the adjuvant component comprising or consisting of the RNA as defined herein complexed with a cationic or polycationic compound) and the second component (i.e. the free RNA as defined herein) may be selected in the inventive composition according to the specific requirements of a particular therapy. Typically, the ratio of the RNA in the adjuvant component and the at least one free RNA (RNA in the adjuvant component: free RNA) of the composition according to the invention is selected such that a significant stimulation of the innate immune system is elicited due to the adjuvant component. In parallel, the ratio is selected such that a significant amount of the free RNA can be provided *in vivo* leading to an efficient translation and concentration of the expressed protein *in vivo,* e.g. the at least one antigen as defined herein. Preferably the ratio of the RNA in the adjuvant component: free RNA in the inventive composition is selected from a range of about 5:1 (w/w) to about 1:10 (w/w). more preferably from a range of about 4:1 (w/w) to about 1:8 (w/w). even more preferably from a range of about 3:1 (w/w) to about 1:5 (w/w) or 1:3 (w/w). and most preferably the ratio of RNA in the adjuvant component: free RNA in the inventive composition is selected from a ratio of about 1:1 (w/w).

Additionally or alternatively, the ratio of the first component (i.e. the adjuvant component comprising or consisting of the RNA complexed with a cationic or polycationic compound) and the second component (i.e. the free RNA) may be calculated on the basis of the nitrogen/phosphate ratio (N/P-ratio) of the entire RNA complex. In the context of the present invention, an N/P-ratio is preferably in the range of about 0.1-10, preferably in a range of about 0,3-4 and most preferably in a range of about 0.5-2 or 0.7-2 regarding the ratio of RNA : peptide in the complex, and most preferably in the range of about 0.7-1.5.

Additionally or alternatively, the ratio of the first component (i.e. the adjuvant component comprising or consisting of the RNA complexed with a cationic or polycationic compound) and the second component (i.e. the free RNA) may also be selected in the composition according to the invention on the basis of the molar ratio of both RNA to each other, i.e. the RNA of the adjuvant component, being complexed with a cationic or polycationic compound and the free RNA of the second component. Typically, the molar ratio of the RNA of the adjuvant component to the free RNA of the second component may be selected such, that the molar ratio suffices the above (w/w) and/or N/P-definitions. More preferably, the molar ratio of the RNA of the adjuvant component to the free RNA of the second component may be selected e.g. from a molar ratio of about 0001:1, 0.01:1, 0.1:1, 0.2:1, 0.3:1, 0.4:1. 0.5:1. 0.6:1, 0.7:1, 0.8:1, 0.9:1, 1:1, 1:0.9, 1:0.8, 1:0.7, 1:0.6, 1:0.5, 1:0.4, 1:0.3, 1:0.2, 1:0.1, 1:0.01, 1:0.001, etc. or from any range formed by any two of the above values, e.g. a range selected from about 0.001:1 to 1:0.001, including a range of about 0.01:1 to 1:0.001, 0.1:1 to 1:0.001, 0.2:1 to 1:0.001, 0.3:1 to 1:0.001, 0.4:1 to 1:0.001, 0.5:1 to 1:0.001, 0.6:1 to 1:0.001, 0.7:1 to 1:0.001, 0.8:1 to 1:0.001, 0.9:1 to 1:0.001, 1:1 to 1:0.001, 1:0.9 to 1:0.001, 1:0.8 to 1:0.001, 1:0.7 to 1:0.001,1:0.6 to 1:0.001, 1:0.5 to 1:0.001.1:0.4 to 1:0.001, 1:0.3 to 1:0.001, 1:0.2 to 1:0.001, 1:0.1 to 1:0.001, 1:0.01 to 1:0.001, or a range of about 0.01:1 to 1:0.01, 0.1:1 to 1:0.01, 0.2:1 to 1:0.01, 0.3:1 to 1:0.01, 0.4:1 to 1:0.01, 0.5:1 to 1:0.01, 0.6:1 to 1:0.01, 0.7:1 to 1:0.01, 0.8:1 to 1:0.01, 0.9:1 to 1:0.01, 1:1 to 1:0.01, 1:0.9 to 1:0.01, 1:0.8 to 1:0.01, 1:0.7 to 1:0.01, 1:0.6 to 1:0.01, 1:0.5 to 1:0.01, 1:0.4 to 1:0.01, 1:0.3 to 1:0.01, 1:0.2 to 1:0.01, 1:0.1 to 1:0.01, 1:0.01 to 1:0.01, or including a range of about 0.001:1 to 1:0.01, 0.001:1 to 1:0.1, 0.001:1 to 1:0.2, 0.001:1 to 1:0.3, 0.001:1 to 1:0.4, 0.001:1 to 1:0.5, 0.001:1 to 1:0.6, 0.001:1 to 1:0.7, 0.001:1 to 1:0.8, 0.001:1 to 1:0.9, 0.001:1 to 1:1, 0.001 to 0.9:1, 0.001 to 0.8:1, 0.001 to 0.7:1, 0.001 to 0.6:1, 0.001 to 0.5:1, 0.001 to 0.4:1, 0.001 to 0.3:1, 0.001 to 0.2:1, 0.001 to 0.1:1, or a range of about 0.01:1 to 1:0.01, 0.01:1 to 1:0.1, 0.01:1 to 1:0.2, 0.01:1 to 1:0.3, 0.01:1 to 1:0.4, 0.01:1 to 1:0.5, 0.01:1 to 1:0.6, 0.01:1 to 1:0.7, 0.01:1 to 1:0.8, 0.01:1 to 1:0.9, 0.01:1 to 1:1, 0.001 to 0.9:1, 0.001 to 0.8:1, 0.001 to 0.7:1, 0.001 to 0.6:1, 0.001 to 0.5:1. 0.001 to 0.4:1, 0.001 to 0.3:1, 0.001 to 0.2:1, 0.001 to 0.1:1, etc.

Even more preferably, the molar ratio of the RNA of the adjuvant component to the free RNA of the second component may be selected e.g. from a range of about 0.01:1 to 1:0.01. Most preferably, the molar ratio of the RNA of the adjuvant component to the free RNA of the second component may be selected e.g. from a molar ratio of about 1:1. Any of the above definitions with regard to (w/w) and/or N/P ratio may also apply.

Suitable adjuvants may furthermore be selected from nucleic acids having the formula (Va): GₗXₘGₙ, wherein: G is guanosine, uracil or an analogue of guanosine or uracil; X is guanosine, uracil, adenosine, thymidine, cytosine or an analogue of the above-mentioned nucleotides: I is an integer from 1 to 40, wherein when I = 1 G is guanosine or an analogue thereof, when I > 1 at least 50% of the nucleotides are guanosine or an analogue thereof; m is an integer and is at least 3; wherein when m = 3 X is uracil or an analogue thereof, when m > 3 at least 3 successive uracils or analogues of uracil occur: n is an integer from 1 to 40, wherein when n = I G is guanosine or an analogue thereof, when n > 1 at least 50% of the nucleotides are guanosine or an analogue thereof, or formula (Vb): (NᵤGₗXₘGₙNᵥ)ₐ, wherein: G is guanosine (guanine), uridine (uracil) or an analogue of guanosine (guanine) or uridine (uracil), preferably guanosine (guanine) or an analogue thereof: X is guanosine (guanine), uridine (uracil), adenosine (adenine), thymidine (thymine), cytidine (cytosine), or an analogue of these nucleotides (nucleosides). preferably uridine (uracil) or an analogue thereof; N is a nucleic acid sequence having a length of about 4 to 50, preferably of about 4 to 40. more preferably of about 4 to 30 or 4 to 20 nucleic acids, each N independently being selected from guanosine (guanine), uridine (uracil), adenosine (adenine), thymidine (thymine), cytidine (cytosine) or an analogue of these nucleotides (nucleosides); a is an integer from 1 to 20, preferably from 1 to 15, most preferably from 1 to 10; 1 is an integer from 1 to 40, wherein when l = 1, G is guanosine (guanine) or an analogue thereof, when I > 1, at least 50% of these nucleotides (nucleosides) are guanosine (guanine) or an analogue thereof: m is an integer and is at least 3: wherein when m = 3, X is uridine (uracil) or an analogue thereof, and when m > 3, at least 3 successive uridines (uracils) or analogues of uridine (uracil) occur: n is an integer from I to 40, wherein when n = 1, G is guanosine (guanine) or an analogue thereof, when n > 1, at least 50% of these nucleotides (nucleosides) are guanosine (guanine) or an analogue thereof: u,v may be independently from each other an integer from 0 to 50, preferably wherein when u = D, v ≥ 1, or when v = 0, u ≥ 1: wherein the nucleic acid molecule of formula (Vb) has a length of at least 50 nucleotides, preferably of at least 100 nucleotides, more preferably of at least 150 nucleotides, even more preferably of at least 200 nucleotides and most preferably of at least 250 nucleotides.

Other suitable adjuvants may furthermore be selected from nucleic acids having the formula (VI): CₗXₘCₙ, wherein: C is cytosine, uracil or an analogue of cytosine or uracil; X is guanosine, uracil, adenosine, thymidine, cytosine or an analogue of the above-mentioned nucleotides; I is an integer from I to 40, wherein when I = 1 C is cytosine or an analogue thereof, when I > I at least 50% of the nucleotides are cytosine or an analogue thereof; m is an integer and is at least 3; wherein when m = 3 X is uracil or an analogue thereof, when m > 3 at least 3 successive uracils or analogues of uracil occur: n is an integer from I to 40, wherein when n = I C is cytosine or an analogue thereof, when n > I at least 50% of the nucleotides are cytosine or an analogue thereof.

In this context the disclosure of WO002008014979 and WO2009095226 is also incorporated herein by reference.

In a further aspect, the present invention provides a vaccine, which is based on the RNA according to the invention comprising at least one coding sequence as defined herein. The vaccine according to the invention is preferably a (pharmaceutical) composition as defined herein.

Accordingly, the vaccine according to the invention is based on the same components as the (pharmaceutical) composition described herein. Insofar. it may be referred to the description of the (pharmaceutical) composition as provided herein. Preferably, the vaccine according to the invention comprises the RNA as defined herein and a pharmaceutically acceptable carrier. In embodiments, where the vaccine comprises more than one RNA component (such as a plurality of RNAs according to the invention, wherein each encodes a distinct antigen). the vaccine may be provided in physically separate form and may be administered by separate administration steps. The vaccine according to the invention may correspond to the (pharmaceutical) composition as described herein, especially where the mRNA components are provided by one single composition. However, the inventive vaccine may also be provided physically separated. For instance, in embodiments, wherein the vaccine comprises more than one RNA species, these RNA species may be provided such that, for example, two, three, four, five or six separate compositions, which may contain at least one RNA species each (e.g. three distinct mRNA species), each encoding distinct antigens, are provided, which may or may not be combined. Also, the inventive vaccine may be a combination of at least two distinct compositions, each composition comprising at least one mRNA encoding at least one of the antigens defined herein. Alternatively, the vaccine may be provided as a combination of at least one mRNA, preferably at least two, three, four. five, six or more mRNAs, each encoding one of the antigens defined herein. The vaccine may be combined to provide one single composition prior to its use or it may be used such that more than one administration is required to administer the distinct mRNA species encoding any of the antigen combinations as defined herein. If the vaccine contains at least one mRNA molecule, typically at least two mRNA molecules, encoding of the antigen combinations defined herein, it may e.g. be administered by one single administration (combining all mRNA species), by at least two separate administrations. Accordingly; any combination of mono-, bi- or multicistronic mRNAs encoding the at least one antigen or any combination of antigens as defined herein (and optionally further antigens), provided as separate entities (containing one mRNA species) or as combined entity (containing more than one mRNA species), is understood as a vaccine according to the present invention. According to a particularly preferred embodiment of the inventive vaccine. the at least one antigen, preferably a combination as defined herein of at least two, three, four, five, six or more antigens encoded by the inventive composition as a whole, is provided as an individual (monocistronic) mRNA, which is administered separately.

As with the (pharmaceutical) composition according to the present invention, the entities of the vaccine may be provided in liquid and or in dry (e.g. lyophilized) form. They may contain further components, in particular further components allowing for its pharmaceutical use. The vaccine or the (pharmaceutical) composition may, e.g., additionally contain a pharmaceutically acceptable carrier and/or further auxiliary substances and additives and/or adjuvants.

The vaccine or (pharmaceutical) composition typically comprises a safe and effective amount of the RNA according to the invention as defined herein, encoding an antigen as defined herein or a fragment or variant thereof or a combination of antigens, preferably as defined herein. As used herein, "safe and effective amount" means an amount of the RNA that is sufficient to significantly induce a positive modification of cancer or a disease or disorder related to cancer. At the same time, however, a "safe and effective amount" is small enough to avoid serious side-effects, that is to say to permit a sensible relationship between advantage and risk. The determination of these limits typically lies within the scope of sensible medical judgment. In relation to the vaccine or (pharmaceutical) composition of the present invention, the expression "safe and effective amount" preferably means an amount of the RNA (and thus of the encoded antigen) that is suitable for stimulating the adaptive immune system in such a manner that no excessive or damaging immune reactions are achieved but, preferably, also no such immune reactions below a measurable level. Such a "safe and effective amount" of the RNA of the (pharmaceutical) composition or vaccine as defined herein may furthermore be selected in dependence of the type of RNA, e.g. monocistronic. bi- or even multicistronic RNA, since a bi- or even multicistronic RNA may lead to a significantly higher expression of the encoded antigen(s) than the use of an equal amount of a monocistronic RNA. A "safe and effective amount" of the RNA of the (pharmaceutical) composition or vaccine as defined above will furthermore vary in connection with the particular condition to be treated and also with the age and physical condition of the patient to be treated, the severity of the condition, the duration of the treatment, the nature of the accompanying therapy, of the particular pharmaceutically acceptable carrier used. and similar factors, within the knowledge and experience of the accompanying doctor. The vaccine or composition according to the invention can be used according to the invention for human and also for veterinary medical purposes, as a pharmaceutical composition or as a vaccine.

In a preferred embodiment, the RNA of the (pharmaceutical) composition, vaccine or kit of parts according to the invention is provided in lyophilized form. Preferably, the lyophilized RNA is reconstituted in a suitable buffer, advantageously based on an aqueous carrier, prior to administration, e.g. Ringer-Lactate solution, which is preferred, Ringer solution, a phosphate buffer solution. In a preferred embodiment, the (pharmaceutical) composition, the vaccine or the kit of parts according to the invention contains at least two, three, four, five, six or more RNAs. preferably mRNAs which are provided separately in lyophilized form (optionally together with at least one further additive) and which are preferably reconstituted separately in a suitable buffer (such as Ringer-Lactate solution) prior to their use so as to allow individual administration of each of the (monocistronic) RNAs.

The vaccine or (pharmaceutical) composition according to the invention may typically contain a pharmaceutically acceptable carrier. The expression "pharmaceutically acceptable carrier" as used herein preferably includes the liquid or non-liquid basis of the inventive vaccine. If the inventive vaccine is provided in liquid form, the carrier will be water, typically pyrogen-free water; isotonic saline or buffered (aqueous) solutions, e.g phosphate, citrate etc. buffered solutions. Particularly for injection of the inventive vaccine. water or preferably a buffer, more preferably an aqueous buffer, may be used, containing a sodium salt, preferably at least 50 mM of a sodium salt, a calcium salt, preferably at least 0,01 mM of a calcium salt, and optionally a potassium salt, preferably at least 3 mM of a potassium salt. According to a preferred embodiment, the sodium, calcium and, optionally, potassium salts may occur in the form of their halogenides, e.g. chlorides, iodides, or bromides, in the form of their hydroxides, carbonates, hydrogen carbonates, or sulfates, etc. Without being limited thereto, examples of sodium salts include e.g. NaCl, NaI, NaBr, Na₂CO₃. NaHCO₃, Na₂SO₄, examples of the optional potassium salts include e.g. KCl, Kt. KBr, K₂CO₃. KHCO₃. K₂SO₄, and examples of calcium salts include e.g. CaCl₂, CaI₂, CaBr₂, CaCO₃, CaSO₄, Ca(0H)z. Furthermore, organic anions of the aforementioned cations may be contained in the buffer. According to a more preferred embodiment, the buffer suitable for injection purposes as defined above, may contain salts selected from sodium chloride (NaCl), calcium chloride (CaCl₂) and optionally potassium chloride (KCI), wherein further anions may be present additional to the chlorides. CaCl₂ can also be replaced by another salt like KCI. Typically, the salts in the injection buffer are present in a concentration of at least 50 mM sodium chloride (NaCl), at least 3 mM potassium chloride (KCI) and at least 0,01 mM calcium chloride (CaCl₂). The injection buffer may be hypertonic, isotonic or hypotonic with reference to the specific reference medium, i.e. the buffer may have a higher, identical or lower salt content with reference to the specific reference medium, wherein preferably such concentrations of the afore mentioned salts may be used. which do not lead to damage of cells due to osmosis or other concentration effects. Reference media are e.g. in "in vivo" methods occurring liquids such as blood, lymph, cytosolic liquids, or other body liquids, or e.g. liquids, which may be used as reference media in "in vitro" methods, such as common buffers or liquids. Such common buffers or liquids are known to a skilled person. Ringer-Lactate solution is particularly preferred as a liquid basis.

However, one or more compatible solid or liquid fillers or diluents or encapsulating compounds may be used as well, which are suitable for administration to a person. The term "compatible" as used herein means that the constituents of the inventive vaccine are capable of being mixed with the RNA according to the invention as defined herein. in such a manner that no interaction occurs, which would substantially reduce the pharmaceutical effectiveness of the inventive vaccine under typical use conditions. Pharmaceutically acceptable carriers, fillers and diluents must, of course, have sufficiently high purity and sufficiently low toxicity to make them suitable for administration to a person to be treated. Some examples of compounds which can be used as pharmaceutically acceptable carriers, fillers or constituents thereof are sugars, such as, for example, lactose, glucose, trehalose and sucrose; starches, such as, for example, corn starch or potato starch; dextrose; cellulose and its derivatives, such as, for example, sodium carboxymethylcellulose, ethylcellulose, cellulose acetate; powdered tragacanth; malt; gelatin; tallow: solid glidants, such as, for example, stearic acid, magnesium stearate; calcium sulfate; vegetable oils, such as, for example, groundnut oil, cottonseed oil, sesame oil, olive oil, corn oil and oil from theobroma; polyols, such as, for example, polypropylene glycol, glycerol, sorbitol. mannitol and polyethylene glycol; alginic acid.

The choice of a pharmaceutically acceptable carrier is determined, in principle, by the manner, in which the pharmaceutical composition or vaccine according to the invention is administered. The composition or vaccine can be administered, for example, systemically or locally. Routes for systemic administration in general include, for example, transdermal, oral, parenteral routes, including subcutaneous, intravenous, intramuscular, intraarterial, intradermal and intraperitoneal injections and/or intranasal administration routes. Routes for local administration in general include, for example, topical administration routes but also intradermal, transdermal, subcutaneous, or intramuscular injections or intralesional, intracranial, intrapulmonal, intracardial, and sublingual injections. More preferably, composition or vaccines according to the present invention may be administered by an intradermal, subcutaneous, or intramuscular route, preferably by injection, which may be needle-free and/or needle injection. Compositions/vaccines are therefore preferably formulated in liquid or solid form. The suitable amount of the vaccine or composition according to the invention to be administered can be determined by routine experiments. e.g. by using animal models. Such models include, without implying any limitation, rabbit, sheep, mouse, rat, dog and non-human primate models. Preferred unit dose forms for injection include sterile solutions of water, physiological saline or mixtures thereof. The pH of such solutions should be adjusted to about 7.4. Suitable carriers for injection include hydrogels, devices for controlled or delayed release, polylactic acid and collagen matrices. Suitable pharmaceutically acceptable carriers for topical application include those which are suitable for use in lotions, creams, gels and the like. If the inventive composition or vaccine is to be administered perorally, tablets, capsules and the like are the preferred unit dose form. The pharmaceutically acceptable carriers for the preparation of unit dose forms which can be used for oral administration are well known in the prior art. The choice thereof will depend on secondary considerations such as taste, costs and storability, which are not critical for the purposes of the present invention. and can be made without difficulty by a person skilled in the art.

The inventive vaccine or composition can additionally contain one or more auxiliary substances in order to further increase the immunogenicity. A synergistic action of the RNA according to the invention and of an auxiliary substance, which may be optionally be co-formulated (or separately formulated) with the inventive vaccine or composition as described above, is preferably achieved thereby. Depending on the various types of auxiliary substances, various mechanisms may play a role in this respect. For example, compounds that permit the maturation of dendritic cells (DCs), for example lipopolysaccharides, TNF-alpha or CD40 ligand, form a first class of suitable auxiliary substances. In general, it is possible to use as auxiliary substance any agent that influences the immune system in the manner of a "danger signal" (LPS, GP96, etc.) or cytokines, such as GM-CFS. which allow an immune response produced by the immune-stimulating adjuvant according to the invention to be enhanced and/or influenced in a targeted manner. Particularly preferred auxiliary substances are cytokines. such as monokines, lymphokines, interleukins or chemokines. that - additional to induction of the adaptive immune response by the encoded at least one antigen - promote the innate immune response, such as IL-1, IL-2, IL-3, IL-4, IL-5, IL-6, IL-7, IL-8, IL-9, IL-10, IL-12, IL-13, IL-14, IL-15, IL-16, IL-17, IL-18, IL-19. IL-20, IL-21, IL-22, IL-23, IL-24, IL-25, IL-2G, IL-27, IL-28, IL-29, IL-30, IL-31, IL-32, IL-33, INF-alpha, IFN-beta, INF-gamma, GM-CSF, G-CSF, M-CSF, LT-beta or TNF-alpha, growth factors, such as hGH. Preferably, such immunogenicity increasing agents or compounds are provided separately (not co-formulated with the inventive vaccine or composition) and administered individually.

Further additives which may be included in the inventive vaccine or composition are emulsifiers, such as, for example, Tween: wetting agents, such as, for example, sodium lauryl sulfate; colouring agents; taste-imparting agents, pharmaceutical carriers; tablet-forming agents; stabilizers; antioxidants; preservatives.

The inventive vaccine or composition can also additionally contain any further compound, which is known to be immune-stimulating due to its binding affinity (as ligands) to human Toll-like receptors TLR1, TLR2, TLR3. TLR4, TLR5, TLR6. TLR7, TLR8, TLR9, TLR10, or due to its binding affinity (as ligands) to murine Toll-like receptors TLR1, TLR2, TLR3, TLR4, TLR5, TLR6, TLR7, TLR8, TLR9, TLR10, TLR11, TLR12 or TLRI3.

Another class of compounds, which may be added to an inventive vaccine or composition in this context. may be CpG nucleic acids, in particular CpG-RNA or CpG-DNA. A CpG-RNA or CpG-DNA can be a single-stranded CpG-DNA (ss CpG-DNA), a double-stranded CpG-DNA (dsDNA), a single-stranded CpG-RNA (ss CpG-RNA) or a double-stranded CpG-RNA (ds CpG-RNA). The CpG nucleic acid is preferably in the form of CpG-RNA, more preferably in the form of single-stranded CpG-RNA (ss CpG-RNA). The CpG nucleic acid preferably contains at least one or more (mitogenic) cytosine/guanine dinucleotide sequence(s) (CpG motif(s)). According to a first preferred alternative, at least one CpG motif contained in these sequences. that is to say the C (cytosine) and the G (guanine) of the CpG motif, is unmethylated. All further cytosines or guanines optionally contained in these sequences can be either methylated or unmethylated. According to a further preferred alternative, however, the C (cytosine) and the G (guanine) of the CpG motif can also be present in methylated form.

According to one aspect of the present invention, the RNA, the (pharmaceutical) composition or the vaccine may be used according to the invention (for the preparation of a medicament) for the treatment or prophylaxis of cancer and diseases or disorders related thereto.

As used herein, the term "cancer" refers to the broad class of disorders and malignancies characterized by hyperproliferative cell growth, either *in vitro*(e.g., transformed cells) or *in vivo.* Conditions which can be treated or prevented by the compositions and methods of the invention include, e.g., a variety of neoplasms, including benign or malignant tumours, a variety of hyperplasias, or the like. Compositions and methods of the invention can achieve the inhibition and/or reversion of undesired hyperproliferative cell growth involved in such conditions. Specific examples of cancer include Acute Lymphoblastic Leukemia, Adult; Acute Lymphoblastic Leukemia. Childhood; Acute Myeloid Leukemia, Adult; Adrenocortical Carcinoma; Adrenocortical Carcinoma, Childhood; AIDS-Related Lymphoma: AIDS-Related Malignancies: Anal Cancer: Astrocytoma, Childhood Cerebellar; Astrocytoma, Childhood Cerebral: Bile Duct Cancer, Extrahepatic; Bladder Cancer; Bladder Cancer, Childhood; Bone Cancer, Osteosarcoma/Malignant Fibrous Histiocytoma; Brain Stem Glioma, Childhood: Brain Tumor. Adult; Brain Tumor, Brain Stem Glioma, Childhood; Brain Tumor, Cerebellar Astrocytoma, Childhood: Brain Tumor, Cerebral Astrocytoma/Malignant Glioma. Childhood; Brain Tumor, Ependymoma, Childhood: Brain Tumor, Medulloblastoma. Childhood: Brain Tumor, Supratentorial Primitive Neuroectodermal Tumors, Childhood; Brain Tumor, Visual Pathway and Hypothalamic Glioma, Childhood: Brain Tumor, Childhood (Other); Breast Cancer: Breast Cancer and Pregnancy: Breast Cancer, Childhood: Breast Cancer, Male; Bronchial Adenomas/Carcinoids, Childhood: Carcinoid Tumor, Childhood; Carcinoid Tumor, Gastrointestinal; Carcinoma, Adrenocortical: Carcinoma, Islet Cell; Carcinoma of Unknown Primary; Central Nervous System Lymphoma, Primary; Cerebellar Astrocytoma. Childhood; Cerebral Astrocytoma/Malignant Glioma. Childhood; Cervical Cancer; Childhood Cancers: Chronic Lymphocytic Leukemia; Chronic Myelogenous Leukemia; Chronic Myeloproliferative Disorders: Clear Cell Sarcoma of Tendon Sheaths: Colon Cancer; Colorectal Cancer, Childhood: Cutaneous T-Cell Lymphoma; Endometrial Cancer: Ependymoma, Childhood; Epithelial Cancer, Ovarian: Esophageal Cancer; Esophageal Cancer, Childhood; Ewing's Family of Tumors; Extracranial Germ Cell Tumor. Childhood; Extragonadal Germ Cell Tumor; Extrahepatic Bile Duct Cancer; Eye Cancer, Intraocular Melanoma; Eye Cancer, Retinoblastoma: Gallbladder Cancer: Gastric (Stomach) Cancer: Gastric (Stomach) Cancer, Childhood; Gastrointestinal Carcinoid Tumor; Germ Cell Tumor, Extracranial. Childhood: Germ Cell Tumor, Extragonadal; Germ Cell Tumor, Ovarian; Gestational Trophoblastic Tumor; Glioma. Childhood Brain Stem; Glioma. Childhood Visual Pathway and Hypothalamic: Hairy Cell Leukemia; Head and Neck Cancer; Hepatocellular (Liver) Cancer, Adult (Primary); Hepatocellular (Liver) Cancer, Childhood (Primary); Hodgkin's Lymphoma, Adult; Hodgkin's Lymphoma, Childhood; Hodgkin's Lymphoma During Pregnancy: Hypopharyngeal Cancer; Hypothalamic and Visual Pathway Glioma, Childhood; Intraocular Melanoma; Islet Cell Carcinoma (Endocrine Pancreas): Kaposi's Sarcoma; Kidney Cancer: Laryngeal Cancer; Laryngeal Cancer, Childhood: Leukemia. Acute Lymphoblastic. Adult; Leukemia. Acute Lymphoblastic, Childhood: Leukemia. Acute Myeloid, Adult; Leukemia. Acute Myeloid, Childhood: Leukemia, Chronic Lymphocytic; Leukemia. Chronic Myelogenous: Leukemia. Hairy Cell; Lip and Oral Cavity Cancer; Liver Cancer, Adult (Primary); Liver Cancer, Childhood (Primary): Lung Cancer, Non-Small Cell; Lung Cancer, Small Cell: Lymphoblastic Leukemia, Adult Acute: Lymphoblastic Leukemia, Childhood Acute: Lymphocytic Leukemia. Chronic; Lymphoma, AIDS- Related; Lymphoma. Central Nervous System (Primary); Lymphoma. Cutaneous T-Cell; Lymphoma, Hodgkin's. Adult: Lymphoma, Hodgkin's; Childhood: Lymphoma. Hodgkin's During Pregnancy; Lymphoma, Non-Hodgkin's. Adult: Lymphoma, Non-Hodgkin's, Childhood; Lymphoma. Non-Hodgkin's During Pregnancy: Lymphoma, Primary Central Nervous System: Macroglobulinemia, Waldenstrom's: Male Breast Cancer; Malignant Mesothelioma, Adult; Malignant Mesothelioma, Childhood: Malignant Thymoma; Medulloblastoma, Childhood; Melanoma: Melanoma, Intraocular; Merkel Cell Carcinoma; Mesothelioma, Malignant: Metastatic Squamous Neck Cancer with Occult Primary: Multiple Endocrine Neoplasia Syndrome. Childhood: Multiple Myeloma/Plasma Cell Neoplasm: Mycosis Fungoides: Myelodysplasia Syndromes: Myelogenous Leukemia. Chronic; Myeloid Leukemia. Childhood Acute; Myeloma, Multiple; Myeloproliferative Disorders, Chronic: Nasal Cavity and Paranasal Sinus Cancer; Nasopharyngeal Cancer: Nasopharyngeal Cancer, Childhood: Neuroblastoma; Neurofibroma: Non-Hodgkin's Lymphoma, Adult; Non-Hodgkin's Lymphoma, Childhood: Non-Hodgkin's Lymphoma During Pregnancy: Non- Small Cell Lung Cancer; Oral Cancer, Childhood; Oral Cavity and Lip Cancer: Oropharyngeal Cancer; Osteosarcoma/Malignant Fibrous Histiocytoma of Bone: Ovarian Cancer, Childhood: Ovarian Epithelial Cancer: Ovarian Germ Cell Tumor; Ovarian Low Malignant Potential Tumor; Pancreatic Cancer: Pancreatic Cancer, Childhood', Pancreatic Cancer, Islet Cell: Paranasal Sinus and Nasal Cavity Cancer; Parathyroid Cancer; Penile Cancer; Pheochromocytoma: Pineal and Supratentorial Primitive Neuroectodermal Tumors, Childhood: Pituitary Tumor; Plasma Cell Neoplasm/Multiple Myeloma: Pleuropulmonary Blastoma; Pregnancy and Breast Cancer; Pregnancy and Hodgkin's Lymphoma: Pregnancy and Non-Hodgkin's Lymphoma; Primary Central Nervous System Lymphoma; Primary Liver Cancer, Adult; Primary Liver Cancer. Childhood; Prostate Cancer: Rectal Cancer; Renal Cell (Kidney) Cancer; Renal Cell Cancer, Childhood: Renal Pelvis and Ureter, Transitional Cell Cancer: Retinoblastoma; Rhabdomyosarcoma, Childhood: Salivary Gland Cancer: Salivary Gland'Cancer, Childhood; Sarcoma, Ewing's Family of Tumors: Sarcoma. Kaposi's; Sarcoma (Osteosarcoma)/Malignant Fibrous Histiocytoma of Bone: Sarcoma, Rhabdomyosarcoma, Childhood: Sarcoma, Soft Tissue, Adult; Sarcoma, Soft Tissue, Childhood: Sezary Syndrome; Skin Cancer; Skin Cancer, Childhood: Skin Cancer (Melanoma); Skin Carcinoma, Merkel Cell: Small Cell Lung Cancer: Small Intestine Cancer; Soft Tissue Sarcoma, Adult: Soft Tissue Sarcoma, Childhood; Squamous Neck Cancer with Occult Primary, Metastatic; Stomach (Gastric) Cancer; Stomach (Gastric) Cancer, Childhood: Supratentorial Primitive Neuroectodermal Tumors. Childhood: T-Cell Lymphoma, Cutaneous; Testicular Cancer: Thymoma. Childhood; Thymoma, Malignant; Thyroid Cancer; Thyroid Cancer, Childhood; Transitional Cell Cancer of the Renal Pelvis and Ureter; Trophoblastic Tumor, Gestational: Unknown Primary Site. Cancer of, Childhood; Unusual Cancers of Childhood; Ureter and Renal Pelvis, Transitional Cell Cancer; Urethral Cancer: Uterine Sarcoma; Vaginal Cancer: Visual Pathway and Hypothalamic Glioma. Childhood: Vulvar Cancer: Waldenstrom's Macro globulinemia: and Wilms' Tumor.

In this context, also included in the present invention are methods of treating or preventing cancer and diseases or disorders related thereto, preferably as defined herein. by administering to a subject in need thereof a pharmaceutically effective amount of the RNA, the pharmaceutical composition or the vaccine according to the invention. Such a method typically comprises an optional first step of preparing the RNA. the composition or the vaccine of the present invention, and a second step, comprising administering (a pharmaceutically effective amount of) said composition or vaccine to a patient/subject in need thereof. A subject in need thereof will typically be a mammal. In the context of the present invention, the mammal is preferably selected from the group comprising, without being limited thereto, e.g. goat, cattle, swine, dog. cat, donkey, monkey, ape. a rodent such as a mouse, hamster, rabbit and, particularly, human, wherein the mammal typically suffers from cancer, and/or diseases or disorders related thereto (as defined herein).

The invention also relates to the use of the RNA. the composition or the vaccine according to the invention, preferably for eliciting an immune response in a mammal, preferably for the treatment or prophylaxis of cancer or a related condition as defined herein.

The subject receiving the pharmaceutical composition or the vaccine according to the invention may be a patient with cancer, preferably as defined herein, or a related condition, receiving chemotherapy (e.g. first-line or second-line chemotherapy), radiotherapy, chemoradiation (combination of chemotherapy and radiotherapy), tyrosine kinase inhibitors (e.g. EGFR tyrosine kinase inhibitors). antibody therapy and/or inhibitory and/or stimulatory checkpoint molecules (e.g. CTLA4 inhibitors, PDI pathway inhibitors), or a patient, who has achieved partial response or stable disease after having received one or more of the treatments specified above.

The present invention furthermore comprises the use of the (pharmaceutical) composition or the RNA according to the invention as defined herein for modulating, preferably for inducing or enhancing, an immune response in a mammal as defined herein, more preferably for treating and/or for supporting the treatment or prophylaxis of cancer. preferably as defined herein, or of diseases or disorders related thereto. In this context. support of the treatment or prophylaxis of cancer may be any combination of a conventional cancer therapy method of such as surgery, radiation therapy. chemotherapy (e.g. first-line or second-line chemotherapy), chemoradiation, treatment with tyrosine kinase inhibitors, treatment with inhibitory and/or stimulatory checkpoint molecules, preferably CTLA4 inhibitors, PD-1 pathway inhibitors, antibody therapy or any combination of these, and a therapy using the RNA or the pharmaceutical composition as defined herein. Support of the treatment or prophylaxis of cancer may be also envisaged in any of the other embodiments defined herein. Accordingly, any use of the RNA. the (pharmaceutical) composition or the vaccine according to the invention in co-therapy with any other approach, preferably one or more of the above therapeutic approaches, in particular in combination with surgery, radiation therapy, chemotherapy, chemoradiation, and/or treatment with kinase inhibitors, inhibitory and/or stimulatory checkpoint molecules or antibodies is within the scope of the present invention.

In this context, antibodies and/or inhibitory and/or stimulatory checkpoint molecules used for combination with the RNA. the vaccine or the (pharmaceutical) composition according to the invention may be encoded by a nucleic acid, preferably an RNA and most preferably by an mRNA.

For administration, preferably any of the administration routes may be used as defined herein. In particular, an administration route is used, which is suitable for treating or preventing cancer, preferably a cancer disease as defined herein or diseases or disorders related thereto. by inducing or enhancing an adaptive immune response on the basis of an antigen encoded by the RNA according to the invention. Administration of the composition and/or the vaccine according to the invention may then occur prior, concurrent and/or subsequent to administering another composition and/or vaccine as defined herein, which may - in addition - contain another RNA or combination of RNAs encoding a different antigen or combination of antigens, wherein each antigen encoded by the RNA according to the invention is preferably suitable for the treatment or prophylaxis of cancer and diseases or disorders related thereto. In this context. a treatment as defined herein may also comprise the modulation of a disease associated to cancer and of diseases or disorders related thereto.

According to a preferred embodiment of this aspect of the invention, the (pharmaceutical) composition or the vaccine according to the invention is administered by injection. Any suitable injection technique known in the art may be employed. Preferably, the inventive composition is administered by injection, preferably by needle-less injection, for example by jet-injection.

In one embodiment, the inventive composition comprises at least two, three, four, five, six, seven, eight, nine, ten, eleven, twelve or more RNAs as defined herein, each of which is preferably injected separately, preferably by needle-less injection. Alternatively, the inventive composition comprises at least two, three, four, five, six, seven, eight, nine, ten, eleven, twelve or more RNAs, wherein the at least two, three, four, five, six, seven, eight, nine, ten, eleven, twelve or more RNAs are administered, preferably by injection as defined herein, as a mixture.

The immunization protocol for the immunization of a subject against an antigen or a combination of at least two, three, four, five, six, seven, eight, nine, ten, eleven, twelve or more antigens as defined herein typically comprises a series of single doses or dosages of the (pharmaceutical) composition or the vaccine according to the invention. A single dosage, as used herein, refers to the initial/first dose, a second dose or any further doses, respectively, which are preferably administered in order to "boost" the immune reaction. In this context, each single dosage preferably comprises the administration of the same antigen or the same combination of antigens as defined herein, wherein the interval between the administration of two single dosages can vary from at least one day, preferably 2, 3, 4, 5, 6 or 7 days. to at least one week, preferably 2, 3, 4, 5, 6, 7 or 8 weeks. The intervals between single dosages may be constant or vary over the course of the immunization protocol, e.g. the intervals may be shorter in the beginning and longer towards the end of the protocol. Depending on the total number of single dosages and the interval between single dosages, the immunization protocol may extend over a period of time, which preferably lasts at least one week, more preferably several weeks (e.g. 2, 3, 4, 5, 6, 7, 8, 9, 10, 11 or 12 weeks), even more preferably several months (e.g. 3, 4, 5, 6, 7, 8, 9, 10, 11, 12, 18 or 24 months). Each single dosage preferably encompasses the administration of an antigen, preferably of a combination of at least two, three, four, five, six, seven, eight, nine, ten, eleven, twelve or more antigens as defined herein and may therefore involve at least one, preferably 1, 2, 3, 4, 5, 6, 7, 8, 9, 10, 11 or 12 injections. In some cases, the composition or the vaccine according to the invention is administered as a single dosage typically in one injection. In the case, where the vaccine according to the invention comprises separate RNA formulations encoding distinct antigens as defined herein, the minimum number of injections carried out during the administration of a single dosage corresponds to the number of separate components of the vaccine. In certain embodiments, the administration of a single dosage may encompass more than one injection for each component of the vaccine (e.g. a specific RNA formulation comprising an RNA encoding, for instance, one antigen as defined herein). For example, parts of the total volume of an individual component of the vaccine may be injected into different body parts, thus involving more than one injection. In a more specific example, a single dosage of a vaccine comprising four separate RNA formulations, each of which is administered in two different body parts, comprises eight injections. Typically, a single dosage comprises all injections required to administer all components of the vaccine, wherein a single component may be involve more than one injection as outlined above. In the case, where the administration of a single dosage of the vaccine according to the invention encompasses more than one injection, the injection are carried out essentially simultaneously or concurrently, i.e. typically in a time-staggered fashion within the time-frame that is required for the practitioner to carry out the single injection steps, one after the other. The administration of a single dosage therefore preferably extends over a time period of several minutes, e.g. 2, 3, 4, 5, 10, 15, 30 or 60 minutes.

Administration of the RNA as defined herein, the (pharmaceutical) composition or the vaccine according to the invention may be carried out in a time staggered treatment. A time staggered treatment may be e.g. administration of the RNA. the composition or the vaccine prior. concurrent and/or subsequent to a conventional therapy of cancer or diseases or disorders related thereto. e.g. by administration of the RNA. the composition or the vaccine prior, concurrent and/or subsequent to a therapy or an administration of a therapeutic suitable for the treatment or prophylaxis of cancer or diseases or disorders related thereto. Such time staggered treatment may be carried out using e.g. a kit, preferably a kit of parts as defined herein.

Time staggered treatment may additionally or alternatively also comprise an administration of the RNA as defined herein, the (pharmaceutical) composition or the vaccine according to the invention in a form. wherein the RNA encoding an antigen as defined herein or a fragment or variant thereof, preferably forming part of the composition or the vaccine, is administered parallel, prior or subsequent to another RNA encoding an antigen as defined above, preferably forming part of the same inventive composition or vaccine. Preferably, the administration (of all RNAs) occurs within an hour, more preferably within 30 minutes, even more preferably within 15, 10, 5, 4, 3, or 2 minutes or even within I minute. Such time staggered treatment may be carried out using e.g. a kit, preferably a kit of parts as defined herein.

In a preferred embodiment, the pharmaceutical composition or the vaccine of the present invention is administered repeatedly, wherein each administration preferably comprises individual administration of the at least one mRNA of the inventive composition or vaccine. At each time point of administration, the at least one mRNA may be administered more than once (e.g. 2 or 3 times). In a particularly preferred embodiment of the invention, at least two, three, four, five, six or more RNAs (each encoding a distinct one of the antigens as defined herein) are administered at each time point, wherein each RNA is administered twice by injection, distributed over the four limbs.

According to another aspect of the present invention, the present invention also provides a kit, in particular a kit of parts, comprising the RNA as defined herein, the (pharmaceutical) composition, and/or the vaccine according to the invention, optionally a liquid vehicle for solubilising and optionally technical instructions with information on the administration and dosage of the RNA. the composition and/or the vaccine. The technical instructions may contain information about administration and dosage of the RNA. the composition and/or the vaccine. Such kits, preferably kits of parts, may be applied e.g. for any of the above mentioned applications or uses, preferably for the use of the RNA according to the invention (for the preparation of an inventive medicament, preferably a vaccine) for the treatment or prophylaxis of cancer or diseases or disorders related thereto. The kits may also be applied for the use of the RNA. the composition or the vaccine as defined herein (for the preparation of an inventive vaccine) for the treatment or prophylaxis of cancer or diseases or disorders related thereto, wherein the RNA, the composition and/or the vaccine may be capable of inducing or enhancing an immune response in a mammal as defined above. Such kits may further be applied for the use of the RNA. the composition or the vaccine as defined herein (for the preparation of an inventive vaccine) for modulating, preferably for eliciting, e.g. to induce or enhance, an immune response in a mammal as defined above, and preferably for supporting treatment or prophylaxis of cancer or diseases or disorders related thereto. Kits of parts, as a special form of kits. may contain one or more identical or different compositions and/or one or more identical or different vaccines as described herein in different parts of the kit. Kits of parts may also contain an (e.g. one) composition. an (e.g. one) vaccine and/or the RNA according to the invention in different parts of the kit, e.g. each part of the kit containing an RNA as defined herein, preferably encoding a distinct antigen. Preferably, the kit or the kit of parts contains as a part a vehicle for solubilising the RNA according to the invention, the vehicle preferably being Ringer-lactate solution. Any of the above kits may be used in a treatment or prophylaxis as defined above.

In another embodiment of this aspect, the kit according to the present invention may additionally contain at least one adjuvant. In a further embodiment, the kit according to the present invention may additionally contain at least one further pharmaceutically active component, preferably a therapeutic compound suitable for treatment and/or prophylaxis of cancer or a related disorder. Moreover, in another embodiment, the kit may additionally contain parts and/or devices necessary or suitable for the administration of the composition or the vaccine according to the invention, including needles, applicators, patches, injection-devices.

### Figures:

The figures shown in the following are merely illustrative and shall describe the present invention in a further way. These figures shall not be construed to limit the present invention thereto.
- **Figure 1**:: shows the presence of IgG2a antibodies specific for the cancer antigen NY-ESO-1 in mice that were vaccinated with NY-ESO-1 mRNA vaccine. A detailed description of the experiment is provided in the example section (see Example 2).
- **Figure 2:**: shows the induction of antigen-specific T-lymphocytes directed against the cancer antigen NY-ESO-1 in mice that were vaccinated with NY-ESO-1 mRNA vaccine. A detailed description of the experiment is provided in the example section (see Example 2).
- **Figure 3:**: shows the presence of IgG2a antibodies specific for the cancer antigen PSA in mice that were vaccinated with PSA mRNA vaccine. A detailed description of the experiment is provided in the example section (see Example 3).
- **Figure 4**:: shows the induction of antigen-specific T-lymphocytes directed against the cancer antigen PSA in mice that were vaccinated with PSA mRNA vaccine. A detailed description of the experiment is provided in the example section (see Example 3).
- **Figure 5:**: shows the induction of antigen-specific T-lymphocytes directed against the cancer antigen PSM (PSMA) in mice that were vaccinated with PSM (PSMA) mRNA vaccine. A detailed description of the experiment is provided in the example section (see Example 4).
- **Figure 6**:: shows the induction of antigen-specific T-lymphocytes directed against the cancer antigen MAGE-A2 in mice that were vaccinated with MAGE-A2 mRNA vaccine. A detailed description of the experiment is provided in the example section (see Example 5).
- **Figure 7:**: shows the induction of antigen-specific T-lymphocytes directed against the cancer antigen MAGE-C2 in mice that were vaccinated with MAGE-C2 mRNA vaccine. A detailed description of the experiment is provided in the example section (see Example 5).
- **Figure 8:**: shows the induction of antigen-specific T-lymphocytes directed against the cancer antigen MAGE-A3 in mice that were vaccinated with MAGE-A3 mRNA vaccine. A detailed description of the experiment is provided in the example section (see Example 5).
- Figure 9:: shows the induction of antigen-specific T-lymphocytes directed against the cancer antigen PMEL (human gp100, hgp100) in mice that were vaccinated with PMEL mRNA vaccine. A detailed description of the experiment is provided in the example section (see Example 6).

### Examples

The Examples shown in the following are merely illustrative and shall describe the present invention in a further way. These Examples shall not be construed to limit the present invention thereto.

### Example 1: Preparation of mRNA cancer vaccine

### 1.1 Preparation of DNA and mRNA constructs

For the present examples, DNA sequences encoding cancer antigens, are prepared and used for subsequent RNA *in vitro* transcription reactions.

Most DNA sequences were prepared by modifying the wild type encoding DNA sequences by introducing a GC-optimized sequence. Sequences were introduced into a pUC19 derived vector and modified to comprise stabilizing sequences derived from alpha-globin-3'-UTR, a stretch of 30 cytosines, and a stretch of 64 adenosines at the 3'-terminal end (poly-A-tail).

The following constructs, coding for the indicated antigens, are used in the present example:
NY-ESO-1 (SEQ ID NO: 458G), PSA (SEQ ID NO: 4590). PSM (SEQ ID NO: 4589). MAGE-A2 (SEQ ID NO: 4588), MAGE-A3 (SEQ ID NO: 4587). MAGE-C2 (SEQ ID NO: 4585) and PMEL (SEQ ID NO: 4591).

The obtained plasmid DNA constructs were transformed and propagated in bacteria (*Escherichia coli*) using common protocols known in the art.

### 1.2. RNA in vitro transcription

The DNA plasmids prepared according to paragraph I are enzymatically linearized using EcoR1 and transcribed *in vitro* using DNA dependent T7 RNA polymerase in the presence of a nucleotide mixture and CAP analog (m7GpppG) under suitable buffer conditions. The obtained mRNAs purified using PureMessenger 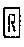 (CureVac, Tübingen, Germany; WO 2008/077592 AI) are used for *in vivo* vaccination experiments (see **Examples 2-6).**

1.3. Preparation of protamine complexed mRNA ("RNActive 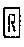 formulation");

The obtained antigen mRNA constructs were complexed with protamine prior to use in *in vivo* vaccination experiments. The mRNA complexation consists of a mixture of 50% free mRNA and 50% mRNA complexed with protamine at a weight ratio of 2:1. First, mRNA is complexed with protamine by addition of protamine-Ringer's lactate solution to mRNA, After incubation for 10 minutes, when the complexes are stably generated, free mRNA is added, and the final concentration of the vaccine is adjusted with Ringer's lactate solution.

### Example 2: Vaccination experiment with NY-ESO-1 mRNA vaccine

To test the NY-ESO-1 mRNA vaccine obtained according to **Example 1** *in vivo.* C57BL/6 mice were intradermally injected with NY-ESO-1 mRNA vaccine. The immune responses of NY-ESO-1 vaccinated mice were analysed (NY-ESO-1 specific immune response and NY-ESO1 specific cellular immune response). A detailed description of the experiment is provided below.

### 2.1. Detection of an NY-ESD-1-specific immune response (B-cell immune response):

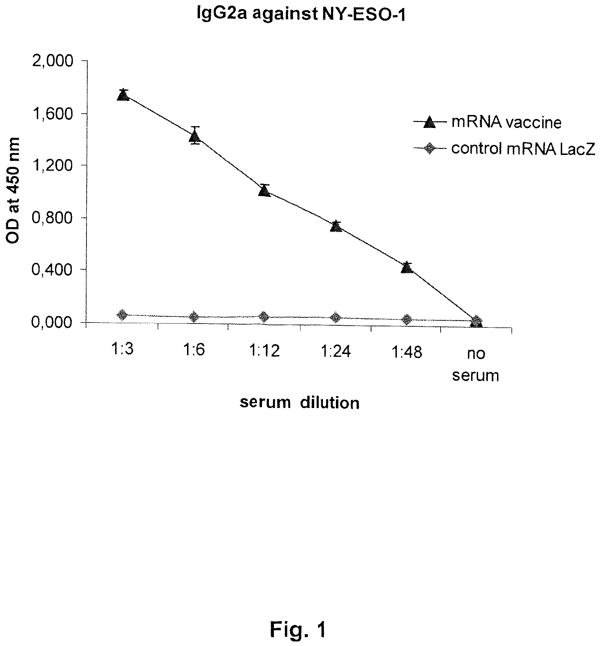

For vaccination 5 mice (C57 BL/B) per group were intradermally injected 8 times within 3 weeks with NY-ESO-1 mRNA vaccine. As negative control lacZ mRNA was injected. Detection of an antigen-specific immune response (B-cell immune response) was carried out by detecting NY-ESD-1 specific antibodies. Therefore, blood samples were taken from the vaccinated mice one week after the last vaccination and sera were prepared. MaxiSorb plates (Nalgene Nunc International) were coated with the antigenic protein (0.5 pg/well). After blocking with 1×PBS containing 0.05% Tween-20 and 1% BSA the plates were incubated with diluted mouse serum (1:30, 1:90, 1:270, 1:810). Subsequently a biotin-coupled secondary antibody (anti-mouse-lgG2a, Pharmingen) was added. After washing, the plate was incubated with horseradish peroxidase-streptavidin and subsequently the conversion of the ABIS substrate (2,2'-azino-bis(3-ethyl-benzthiazoline-6-sulfonic acid) was measured. The result of the experiment is shown in Figure 1.

### 2.2. Detection of an NY-ESO-1-specific cellular immune response (CTL) by ELISPOT

2 weeks after the last vaccination mice were sacrificed, the spleens were removed and the splenocytes were isolated. The splenocytes were restimulated for 7 days in the presence of NY-ESO-1 peptides (peptide library); a peptide library of HIV antigen was used as control. To determine an antigen-specific cellular immune response IFNgamma secretion was measured after re-stimulation with peptide. For detection of IFNgamma a coat multiscreen plate (Millipore) was incubated overnight with coating buffer (0.1 M Carbonat-Bicarbonat Buffer pH 9,6, 10,59 g/l Na₂CO₃, 8,4g/l NaHCO₃) comprising antibody against IFNgamma (BD Pharmingen, Heidelberg, Germany). After coating effector cells (5 × 10⁵/well) were incubated with the antigen-specific peptide library, an unspecific peptide. DMSO or medium as control for 24h in the plate. Subsequently the plate was washed with 1xPBS and incubated with a biotin-coupled secondary antibody. After washing with 1xPBS/0.05% Tween-20 the substrate (5-Bromo-4-Cloro-3-Indolyl Phosphate/Nitro Blue Tetrazolium Liquid Substrate System from Sigma Aldrich. Taufkirchen. Germany) was added to the plate and the conversion of the substrate could be detected visually. The results are shown in Figure 1.

### Results:

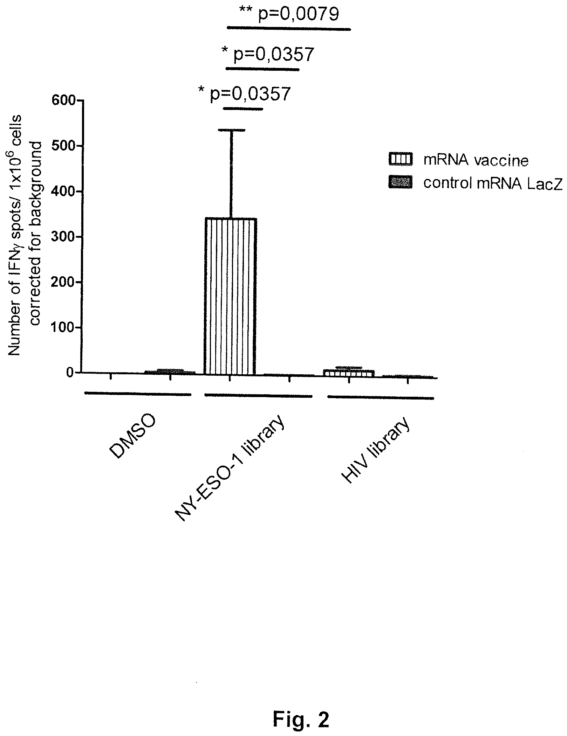

The data shows that we detected antigen specific IgG2a antibodies in serum of mice vaccinated with the NY-ESO-1 mRNA vaccine demonstrating that the mRNAs are functional and immunogenic *in vivo.* The result is shown in Figure 1. The presence of antigen-specific cytotoxic T-cells in splenocytes from NY-ESO-1 vaccinated mice was investigated using the ELISPOT technique. As shown in Figure 2, the stimulation with NY-ESO-1 peptide library led to high IFN-gamma secretion in splenocytes from mice vaccinated with NY-ESO-1 mRNA vaccine and not in splenocytes from control mice, vaccinated with mRNA coding for irrelevant protein beta-galctosidase. None of the splenocytes reacted to the HIV-derived control peptide library. The result is shown in Figure 2.

Overall, the results shown in Figure 1 and Figure 2 demonstrate that the inventive NY-ESO-1 mRNA vaccine induces antigen specific B-cell and T-cell responses *in vivo.*

### Example 3: Vaccination experiment with PSA mRNA vaccine

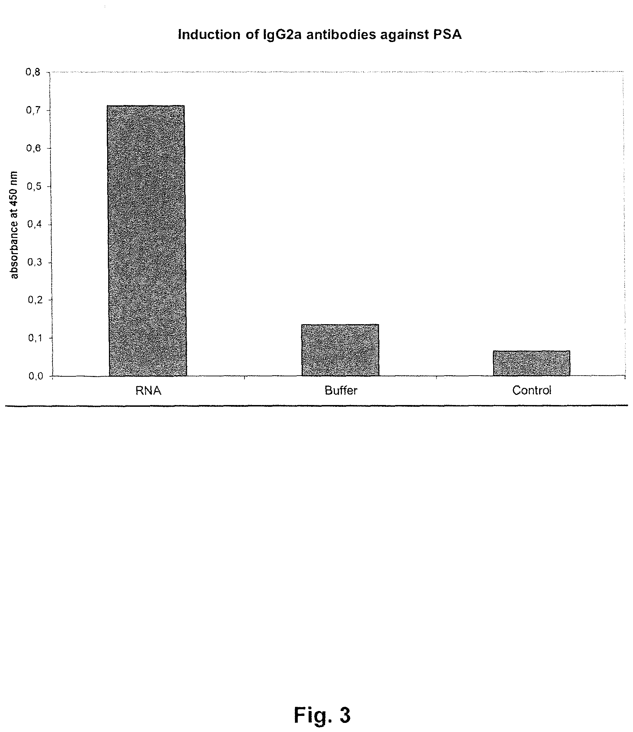

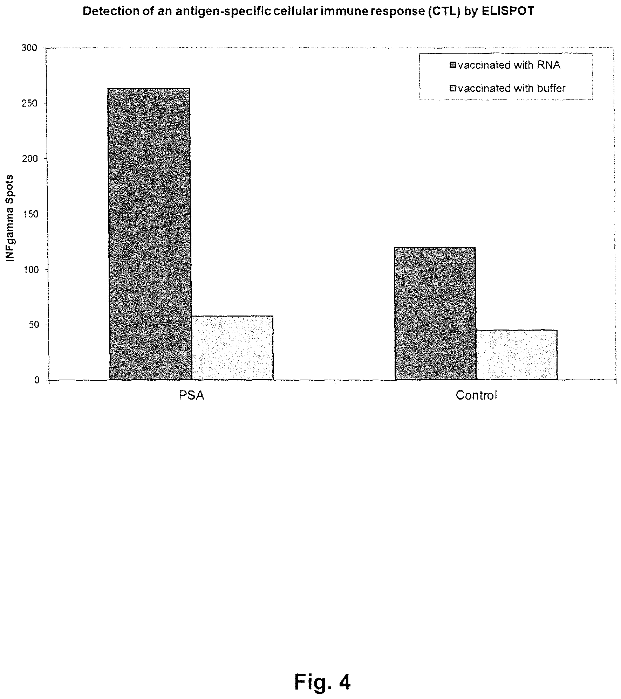

To test the PSA mRNA vaccine obtained according to Example I *in vivo,* C57BL/6 mice were intradermally injected with PSA mRNA vaccine. The immune responses of PSA vaccinated mice were analysed (PSA specific immune response and PSA specific cellular immune response). The results of the experiment are shown in Figures 3 and 4. A detailed description of the experiment is provided below.

### 3.1. Detection of an PSA-specific immune response (B-cell immune response):

For vaccination 4 mice (C57 BL/6) per group were intradermally injected 4 times within 3 weeks with 5 pg PSA mRNA vaccine. As negative control. an injection buffer was applied. Detection of an antigen-specific immune response (B-cell immune response) was carried out by detecting PSA specific antibodies. The detection of antigen-specific immune response (B-cell immune response) was performed according to Example 2.2. The results are shown in Figure 3.

### 3.2. Detection of an PSA-specific cellular immune response by ELISPOT:

2 months after the last vaccination mice were sacrificed, the spleens were removed and the splenocytes were isolated. The splenocytes were incubated for 7 days in presence of IL-4 to select dendritic cells. To determine an antigen-specific cellular immune response INFgamma secretion was measured after re-stimulation. The detection of an antigen-specific cellular immune response by ELISPOT was essentially performed according to Example 2.3. The results are shown in Figure 4.

### Results:

The data shows that we detected antigen specific lgG2a antibodies in serum of mice vaccinated with the PSA mRNA vaccine demonstrating that the mRNAs are functional and immunogenic *in vivo.* The result is shown in Figure 3. The presence of antigen-specific cytotoxic T-cells in splenocytes from PSA vaccinated mice was investigated using the ELISPOT technique. As shown in Figure 4, the stimulation with PSA peptide library led to high IFN-gamma secretion in splenocytes from mice vaccinated with PSA mRNA vaccine and not in splenocytes from control mice, vaccinated with buffer. None of the splenocytes reacted to the control peptide library. The result is shown in Figure 4.

Overall, the results shown in Figure 3 and Figure 4 demonstrate that the inventive PSA mRNA vaccine induces antigen specific B-cell and T-cell responses *in vivo.*

### Example 4: Vaccination experiment with PSM mRNA vaccine

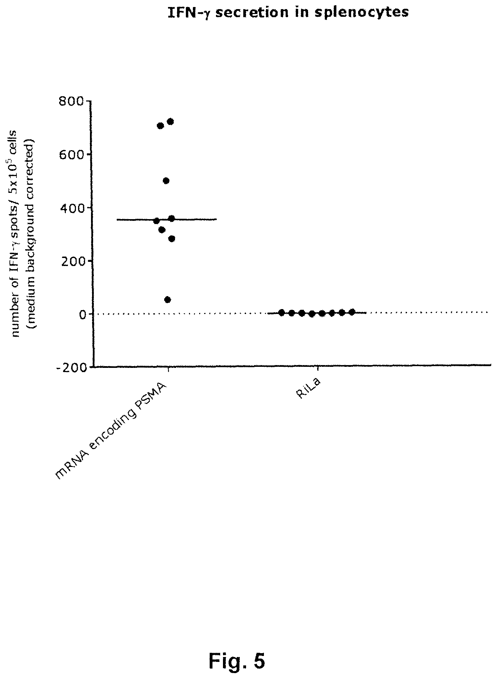

in test the PSM mRNA vaccine obtained according to Example I *in vivo.* C57BL/6 mice were intradermally injected with PSM mRNA vaccine. The immune responses of PSM vaccinated mice were analysed (PSM specific cellular immune response). The result of the experiment is shown in Figure 5. A detailed description of the experiment is provided below.

### 4.1. Detection of an PSM-specific cellular immune response by ELISPOT:

For vaccination 8 mice (C57 BL/6) per group were intradermally injected 5 times with 32 pg PSA mRNA vaccine. As negative control. ringer lactate (RiLa) buffer was applied. After the last vaccination mice were sacrificed, the spleens were removed and the splenocytes were isolated. For re-stimulation 3 × 10⁷ splenocytes were incubated for 5 days with a PSM peptide library (1 pg/ml per peptide) in presence of IL-2 (30 U/ml). The detection of an antigen-specific cellular immune response by ELISPOT was essentially performed according to Example 2.3. The result is shown in Figure 5.

### Results:

The presence of antigen-specific cytotoxic T-cells in splenocytes from PSM vaccinated mice was investigated using the ELISPOT technique. As shown in Figure 5, the stimulation with PSM peptide library led to high IFN-gamma secretion in splenocytes from mice vaccinated with PSM mRNA vaccine and not in splenocytes from control mice, vaccinated with RiLa buffer. The result is shown in Figure 5.

Overall, the results shown in Figure 5 demonstrate that the inventive PSM mRNA vaccine induces antigen specific T-cell responses *in vivo.*

### Example 5: Vaccination experiment with MAGE-A2, MAGE-A3 and MAGE-C2 mRNA vaccines

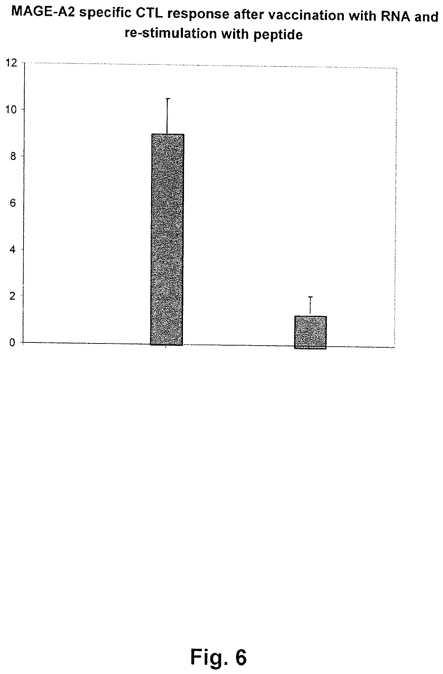

To test the MAGE-A2, MAGE-A3 and MAGE-C2 mRNA vaccine obtained according to Example 1 *in vivo,* C57BL/6 mice were intradermally injected with MAGE-A2, MAGE-A3 and MAGE-C2 mRNA vaccines. The immune responses of vaccinated mice were analysed (antigen specific cellular immune response). The results of the experiments are shown in Figures 6-8. A detailed description of the experiment is provided below.

### 5.1. Detection of an antigen-specific cellular immune response by ELISPOT:

For the present example, MAGE-A2. MAGE-A3 and MAGE-C2 mRNA vaccines were applied with application regimens as provided below:
   - MAGE-A2: C57BL/6 mice were vaccinated intradermally with mRNA encoding human MAGE-A2. (20 pg/mouse/vaccination day. 5 mice per group). Ringer lactate was used as control. After 8 vaccinations splenocytes were isolated and analyzed by ELISpot assay.
   - MAGE-A3: C57BL/6 mice were vaccinated intradermally with mRNA encoding human MAGE-A2. (32 pg/mouse/vaccination day, 8 mice per group). Ringer lactate was used as control. After 5 vaccinations splenocytes were isolated and analyzed by ELISpot assay.
   - MAGE-C2: C57BL/6 mice were vaccinated intradermally with mRNA encoding human MAGE-A2. (20 pg/mouse/vaccination day, 5 mice per group). Ringer lactate was used as control. After 8 vaccinations splenocytes were isolated and analyzed by ELlspot assay.

After the last vaccination the mice were sacrificed, the spleens were removed and the splenocytes were isolated. For re-stimulation 3 × 10⁷ splenocytes were incubated for 5 days with the respective antigen-specific peptide or peptide library (1 µg/ml per peptide) in presence of IL-2 (30 U/ml). To determine an antigen-specific cellular immune response IFNy secretion was measured after re-stimulation with peptide. The detection of an antigen-specific cellular immune response by ELISPOT was performed according to Example 2.3. The result is shown in Figures 6-8.

### Results:

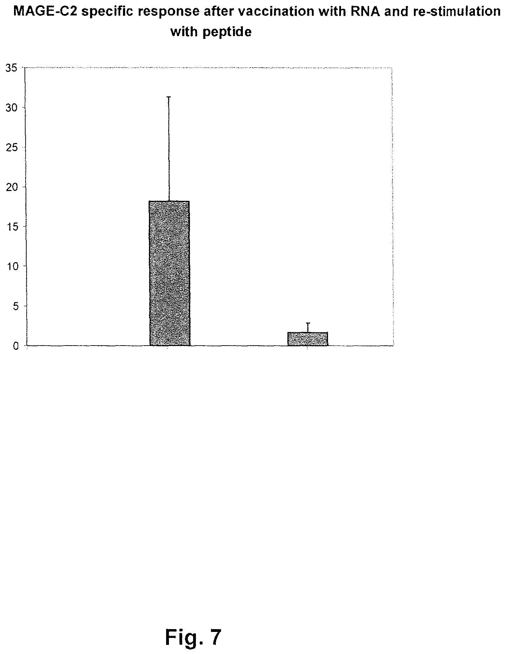

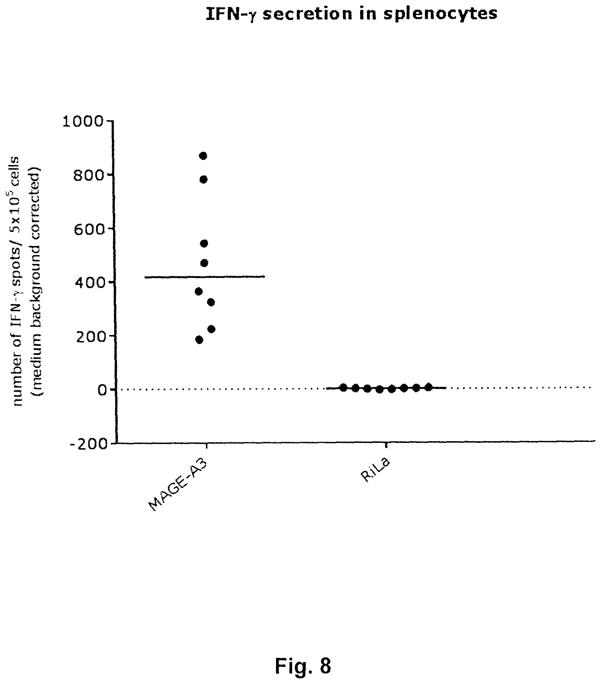

The presence of antigen-specific cytotoxic T-cells in splenocytes from MAGE-A2, MAGE-A3 and MAGE-C2 vaccinated mice was investigated using the ELISPOT technique. As shown in Figure 6, the stimulation with MAGE-A2 peptide library led to high IFN-gamma secretion in splenocytes from mice vaccinated with MAGE-A2 mRNA vaccine. Stimulation with a control peptide library did not lead to IFN-gamma secretion. As shown in Figure 7, the stimulation with MAGE-C2 peptide library led to high IFN-gamma secretion in splenocytes from mice vaccinated with MAGE-C2 mRNA vaccine. Stimulation with a control peptide library did not lead to IFN-gamma secretion. As shown in Figure 8, the stimulation with MAGE-A3 peptide library led to high IFN-gamma secretion in splenocytes from mice vaccinated with MAGE-A3 mRNA vaccine and not in splenocytes from control mice, vaccinated with RiLa buffer.

Overall, the results shown in Figures 6-8 demonstrate that the inventive MAGE-A2, MAGE-A3 and MAGE-C2 mRNA vaccine induce antigen specific T-cell responses *in vivo.*

### Example 6: Vaccination experiment with PMEL mRNA vaccine

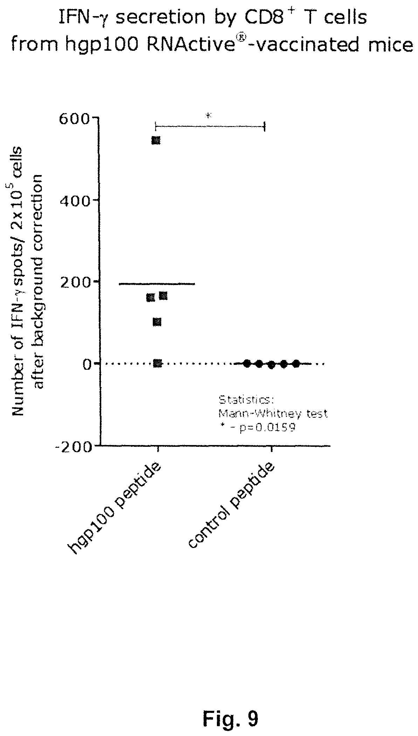

To test the PMEL mRNA vaccine obtained according to Example 1 *in vivo,* C57BL/6 mice were intradermally injected with PMEL mRNA vaccines. The immune response of vaccinated mice was analysed (PMEL specific cellular immune response). The result of the experiment is shown in Figure 9. A detailed description of the experiment is provided below.

### 6.1. Detection of a PMEL-specific cellular immune response by ELISPOT:

8 mice (C57 BL/B) per group were intradermally injected 4 times within 3 weeks with 64 pg PMEL mRNA vaccine. As negative control, RiLa injection buffer was injected. After the last vaccination the mice were sacrificed, the spleens were removed and the splenocytes were isolated. The detection of an antigen-specific cellular immune response by ELISPDT was performed according to Example 2.3. The result is shown in Figure 9.

### Results:

The presence of PMEL-specific cytotoxic T-cells in splenocytes from PMEL vaccinated mice was investigated using the ELISPOT technique. As shown in Figure 9, the stimulation with PMEL peptide library led to high IFN-gamma secretion in splenocytes from mice vaccinated with PMEL mRNA vaccine. Stimulation with a control peptide library did not lead to IFN-gamma secretion.

### Items

1. RNA comprising at least one coding sequence, wherein the coding sequence comprises a nucleic acid sequence selected from the group consisting of SEQ ID NOs: 505 - 4033; 4561 - 4591, or a fragment or variant of any one of said nucleic acid sequences.
2. The RNA according to item I. wherein the at least one coding sequence encodes a tumor antigen, or a fragment or variant of a tumor antigen, wherein the tumor antigen is preferably selected from the group consisting of 1AO1_HLA-A/m: 1AO2; 5T4; ACRBP; AFP; AKAP4; alpha-actinin-_4/m; alpha-methylacyl-coenzyme_A_racemase; ANDR; ART-4; ARTCI/m; AURKB: B2MG: B3GN5; B4GNI; B7H4; BAGE-1; BASI: BCL-2; bcr/abl; beta-catenin/m: BING-4; BIRC7; BRCAI/m; BY55; calreticulin; CAMEL; CASP-8/m; CASPA: cathepsin_B: cathepsin_L: CDIA; CDIB; CD1C; CD1D; CD1E; CD20; CD22; CD276; CD33: CD3E; CD3Z: CD44_Isoform_1; CD44_lsoform_B; CD4: CD52; CD55; CD56: CD80; CD8B: CD8A: CDC27/m: CDE30; CDK4/m: CDKN2A/m; CEA: CEAM6; CH312; CLCA2; CML28: CML66; COA-1/m; coactosin-like_protein; collagen XXIII; COX-2; CP1B1; CSAG2; CT45AI: CT55; CT- 9/BRD6: CTAG2_Isoform_LAGE-1A; CTAG2_Isoform_LAGE-1B; CTCFL; Cten: cyclin_B1; cyclin_D1: cyp-B; DAM-IO: DEPIA; E7: EFIA2; EFTLID2/m: EGFR: EGLN3; ELF2/m; EMMPRIN; EpCam; EphA2; EphA3; ErbB3; ERBB4; ERG; ETVB; EWS; EZH2; FABP7; FCGR3A_Version_I; FCGR3A_Version_2; FGF5; FGFR2; fibronectin; FOS: FOXP3; FUTI; G250; GAGE-1: GAGE-2; GAGE-3; GAGE-4: GAGE-5; GAGE-G; GAGE7b: GAGE-8 (GAGE-2D): GASR: GnT-V; GPC3; GPNMB/m; GRM3; HAGE; hepsin: Her2/neu: HLA-A2/m: homeobox_NKX3.1; HOM-TES-85: HPG1; HS71A; HS71B; HST-2; hTERT; iCE; IF2B3; IL10; IL-13Ra2; IL2-RA: IL2-RB: IL2-RG: IL-5; IMP3; ITA5; ITB1; ITB6; kallikrein-2: kallikrein-3; kallikrein-4: KI20A; KIAA0205: KIF2C: KK-LC-1; LDLR; LGMN: LIRB2; LYGK; MAGAS: MAGAB; MAGAB; MAGE-AID; MAGE-A12; MAGE-A1: MAGE-A2: MAGE-A3; MAGE-A4; MAGE-A6: MAGE-A9; MAGE-B10; MAGE-B16: MAGE-B17; MAGE- B1; MAGE-B2; MAGE-B3; MAGE-B4; MAGE-B5; MAGE-86; MAGE-C1; MAGE-C2; MAGE-C3; MAGE-D1; MAGE-D2; MAGE-D4: MAGE-_E1; MAGE-E1_(MAGE1): MAGE-E2; MAGE-FI; MAGE-HI; MAGEL2: mammaglobin_A; MART-1/melan-A; MART-2; MC1_R: M-CSF; mesothelin; MITF; MMP1_1; MMP7; MUC-1: MUM-1/m: MUM-2/m; MYCN; MYO1A; MYO1B: MYO1C; MYO1D; MYO1E; MYO1F; MYO1G; MYOIH; NA17; NA88-A; Neo-PAP; NFYC/m: NGEP: NPM; NRCAM: NSE; NUF2; NY-ESO-1; DA1; OGT; OS-9; osteocalcin; osteopontin; p53; PAGE-4; PAI-1; PAI-2; PAP; PATE; PAX3; PAX5; PDILI; PDCD1; PDEF; PECAI; PGCB; PGFRB; Pim-1 - Kinase; Pin-1; PLACI; PMEL: PML: POTEF; POTE; PRAME: PRDX5/m: PRM2; prostein: proteinase-3; PSA; PSB9; PSCA; PSGR: PSM: PTPRC; RAB8A; RAGE-1; RARA; RASH; RASK; RASN; RGS5; RHAMM/CD168: RHOC; RSSA: RU1; RU2; RUNXI; S-100; SAGE; SART- 1; SART-2; SART-3; SEPR: SERPIN85; SIA7F: SIA8A; SIAT9; SIRT2/m; SOX10; SP17; SPNXA: SPXN3; SSX-1; SSX-2: SSX3; SSX-4; STIAI: STAG2; STAMP-1; STEAP-1: Survivin-2B; survivin: SYCPI; SYT-SSX-I; SYT-SSX-2; TARP: TCRg; TF2AA: TGFBI: TGFR2; TGM-4; TIE2: TKTL1; TPI/m; TRGV11; TRGV9; TRPC1; TRP-p8; TSG10; TSPYI: TVC_(TRGV3); TXIO1; tyrosinase; TYRPI; TYRP2; UPA: VEGFRI: WT1: and XAGEI.
3. The RNA according to item 1 or 2. wherein the at least one coding sequence encodes a tumor antigen comprising an amino acid sequence selected from the group consisting of SEQ ID NOs: I-504: 4558 - 4560, or a fragment or variant of any one of said amino acid sequences.
4. The RNA according to any one of items 1 to 3. wherein the RNA is mono-, bi-, or multicistronic.
5. The RNA according to any one of items 1 to 4, wherein the RNA is an mRNA. a viral RNA or a replicon RNA.
6. The RNA according to any one of items 1 to 5, wherein the RNA is a modified RNA, preferably a stabilized RNA.
7. The RNA according to any one of items 1 to 6, wherein
   the G/C content of the at least one coding sequence of the RNA is increased compared to the G/C content of the corresponding coding sequence of the corresponding wild-type RNA, and/or wherein
   the C content of the at least one coding sequence of the RNA is increased compared to the C content of the corresponding coding sequence of the corresponding wild-type RNA, and/or wherein
   the codons in the at least one coding sequence of the RNA are adapted to human codon usage, wherein the codon adaptation index (CAI) is preferably increased or maximised in the at least one coding sequence of the RNA.
   wherein the amino acid sequence encoded by the RNA is preferably not being modified compared to the amino acid sequence encoded by the corresponding wild-type RNA.
8. The RNA according to item 7. wherein the at least one coding sequence of the RNA comprises a nucleic acid sequence selected from the group consisting of SEQ ID NOs: 1009 - 4033: 4564 - 4591, or a fragment or variant of any one of said nucleic acid sequences.
9. The RNA according to any one of items I to 8, wherein the at least one coding sequence comprises a nucleic acid sequence, which is identical or at least 80% identical to a nucleic acid sequence selected from the group consisting of SEQ ID NOs: 505 - 4033; 4561 - 4591, or a fragment or variant of any one of said sequences.
10. The RNA according to any one of items 1 to 9, wherein the at least one coding sequence encodes a tumor antigen comprising an amino acid sequence, which is identical to or at least 80% identical to an amino acid sequence selected from the group consisting of SEQ ID NOs: 1 - 504; 4558 - 4560, or a fragment or variant of any one of said sequences.
11. The RNA according to any one of items 1 to 10, which comprises a 5'-CAP structure and/or at least one 3'-untranslated region element (3'-UTR element).
12. The RNA according to any one of items 1 to 11, which comprises at least one histone stem-loop.
13. The RNA according to item 12. wherein the at least one histone stem-loop comprises a nucleic acid sequence according to the following formulae (I) or (II):
   formula (I) (stem-loop sequence without stem bordering elements): 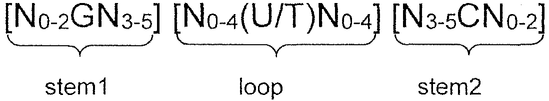
   formula (II) (stem-loop sequence with stem bordering elements): 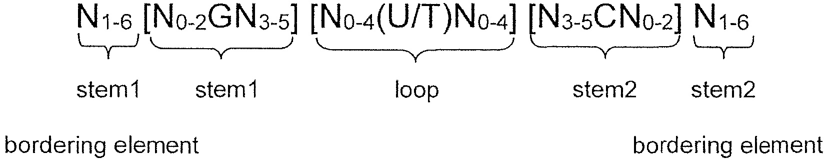
   wherein:
      - stem) or stem2 bordering elements N₁₋₆: is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof;
      - stem) [N₀₋₂GN₃₋₅]: is reverse complementary or partially reverse complementary with element stem2. and is a consecutive sequence between of 5 to 7 nucleotides;
      wherein N₀₋₂ is a consecutive sequence of 0 to 2, preferably of 0 to 1, more preferably of I N, wherein each N is independently from another selected from a nucleotide selected from A, U. T, G and C or a nucleotide analogue thereof;
      wherein N₃₋₅ is a consecutive sequence of 3 to 5. preferably of 4 to 5, more preferably of 4 N, wherein each N is independently from another selected from a nucleotide selected from A, U. T, G and C or a nucleotide analogue thereof, and
      wherein G is guanosine or an analogue thereof, and may be optionally replaced by a cytidine or an analogue thereof, provided that its complementary nucleotide cytidine in stem2 is replaced by guanosine;
      - loop sequence [N₀₋₄(U/T)N₀₋₄]: is located between elements stem1 and stem2, and is a consecutive sequence of 3 to 5 nucleotides, more preferably of 4 nucleotides:
      wherein each N₀₋₄, is independent from another a consecutive sequence of 0 to 4, preferably of I to 3. more preferably of 1 to 2 N, wherein each N is independently from another selected from a nucleotide selected from A. U, T. G and C or a nucleotide analogue thereof; and
      wherein U/T represents uridine, or optionally thymidine:
      - stem2 [N₃₋₅CN₀₋₂]: is reverse complementary or partially reverse complementary with element stem!. and is a consecutive sequence between of 5 to 7 nucleotides;
      wherein Ns-s is a consecutive sequence of 3 to 5, preferably of 4 to 5, more preferably of 4 N. wherein each N is independently from another selected from a nucleotide selected from A, U, T, G and C or a nucleotide analogue thereof;
      wherein N₀₋₂ is a consecutive sequence of 0 to 2, preferably of 0 to 1, more preferably of I N. wherein each N is independently from another selected from a nucleotide selected from A. U, T, G and C or a nucleotide analogue thereof; and
      wherein C is cytidine or an analogue thereof, and may be optionally replaced by a guanosine or an analogue thereof provided that its complementary nucleotide guanosine in stem) is replaced by cytidine;
   wherein
   stem1 and stem2 are capable of base pairing with each other
   forming a reverse complementary sequence, wherein base pairing may occur between stem1 and stem2, or
   forming a partially reverse complementary sequence, wherein an incomplete base pairing may occur between stem1 and stem2.
14. The RNA according to item I2 or 13, wherein the at least one histone stem-loop comprises a nucleic acid sequence according to the following formulae (Ia) or (Ila):
   formula (la) (stem-loop sequence without stem bordering elements): 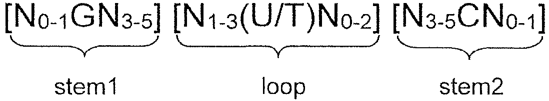
   formula (IIa) (stem-loop sequence with stem bordering elements): 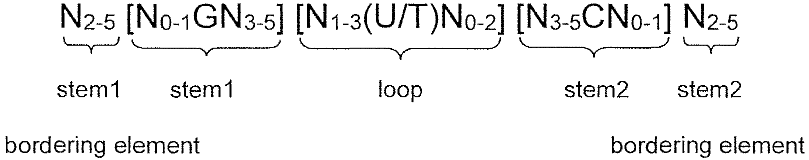
15. The RNA according to any one of items 12 to 14, wherein the at least one histone stem loop comprises a nucleic acid sequence according to SEQ ID NO: 4555 and most preferably a RNA sequence according to SEQ ID NO: 4556.
16. The RNA according to any one of items 1 to 15, wherein the at least one RNA comprises a poly(A) sequence, preferably comprising 10 to 200, 10 to 100, 40 to 80 or 50 to 70 adenosine nucleotides, and/or a poly(C) sequence, preferably comprising 10 to 200, 10 to 100, 20 to 70, 20 to 60 or 10 to 40 cytosine nucleotides.
17. The RNA according to any one of items I to 16, which comprises, preferably in 5' to 3' direction, the following elements:
   a) a 5'-CAP structure, preferably m7GpppN.
   b) at least one coding sequence as defined in item 8,
   c) a poly(A) tail. preferably consisting of 10 to 200, 10 to 100, 40 to 80 or 50 to 70 adenosine nucleotides.
   d) a poly(C) tail. preferably consisting of 10 to 200, 10 to 100, 20 to 70. 20 to 60 or 10 to 40 cytosine nucleotides, and
   e) a histone stem-loop, preferably comprising the RNA sequence according to SEQ ID NO: 4556.
18. The RNA according to any one of items 11 to 17, which comprises a 3'-UTR element and wherein the 3'-UTR element comprises a nucleic acid sequence derived from a 3'-UTR of a gene, which preferably encodes a stable mRNA, or from a homolog, a fragment or a variant of said gene.
19. The RNA according to item 18. wherein the 3'-UTR element comprises a nucleic acid sequence derived from a 3'-UTR of a gene selected from the group consisting of an albumin gene, an α-globin gene, a β-globin gene, a tyrosine hydroxylase gene, a lipoxygenase gene, and a collagen alpha gene, or from a homolog, a fragment or a variant thereof.
20. The RNA according to item 18 or 19, wherein the 3'-UTR element comprises a nucleic acid sequence derived from a 3'UTR of an α-globin gene, preferably comprising the corresponding RNA sequence of the nucleic acid sequence according to SEQ ID NO: 4547, a homolog, a fragment, or a variant thereof;
21. The RNA according to any of items 1 to 20, which comprises, preferably in 5' to 3' direction, the following elements:
   a) a 5'-CAP structure, preferably m7GpppN,
   b) at least one coding sequence as defined in item 8.
   c) a 3'-UTR element comprising a nucleic acid sequence. which is derived from an α-globin gene, preferably comprising the corresponding RNA sequence of the nucleic acid sequence according to SEQ ID NO: 4547, or a homolog, a fragment or a variant thereof,
   d) a poly(A) tail, preferably consisting of 10 to 200, 10 to 100, 40 to 80 or 50 to 70 adenosine nucleotides,
   e) a poly(C) tail. preferably consisting of 10 to 200, 10 to 100, 20 to 70, 20 to 60 or ID to 40 cytosine nucleotides, and
   f) a histone stem-loop, preferably comprising the RNA sequence according to SEQ ID NO: 4556.
22. The RNA according to any one of item 18 or 19, wherein the at least one 3'-UTR element comprises a nucleic acid sequence, which is derived from the 3'-UTR of a vertebrate albumin gene or from a variant thereof, preferably from the 3'-UTR of a mammalian albumin gene or from a variant thereof, more preferably from the 3'-UTR of a human albumin gene or from a variant thereof, even more preferably from the 3'-UTR of the human albumin gene according to GenBank Accession number NM_000477.5, or from a fragment or variant thereof.
23. The RNA according to item 22, wherein the 3-'UTR element is derived from a nucleic acid sequence according to SEQ ID NO: 4551 or SEQ ID NO: 4553, preferably from a corresponding RNA sequence, or a homolog, a fragment or a variant thereof.
24. The RNA according to any one of items I to 23, wherein the RNA comprises a 5'-UTR element.
25. The RNA according to item 24, wherein the 5'-UTR element comprises a nucleic acid sequence, which is derived from the 5'-UTR of a TOP gene, preferably from a corresponding RNA sequence, or a homolog, a fragment, or a variant thereof, preferably lacking the 5'TOP motif.
26. The RNA according to item 25. wherein the 5'-UTR element comprises a nucleic acid sequence, which is derived from a 5'-UTR of a TOP gene encoding a ribosomal protein, preferably from a corresponding RNA sequence, or from a homolog, a fragment or a variant thereof, preferably lacking the 5'TOP motif.
27. The RNA according to item 25 or 26. wherein the 5'-UTR element comprises a nucleic acid sequence, which is derived from a 5'-UTR of a TOP gene encoding a ribosomal Large protein (RPL). or from a homolog, a fragment or variant thereof, preferably lacking the 5'TOP motif.
28. The RNA according to any one of items 25 to 27, wherein the 5'-UTR element comprises an RNA sequence corresponding to the nucleic acid sequence according to SEQ ID NO: 4537. or a homolog, a fragment or a variant thereof.
29. The RNA according to any one of items 1 to 28, which comprises, preferably in 5' to 3' direction, the following elements:
   a) a 5'-CAP structure, preferably m7GpppN,
   b) a 5'-UTR element, which comprises or consists of a nucleic acid sequence, which is derived from the 5'-UTR of a TOP gene, preferably comprising an RNA sequence corresponding to the nucleic acid sequence according to SEM ID NO: 4537. or a homolog, a fragment or a variant thereof.
   c) at least one coding sequence as defined in item 8.
   d) a 3'-UTR element comprising a nucleic acid sequence, which is derived from an α-globin gene, preferably comprising the corresponding RNA sequence of the nucleic acid sequence according to SEQ ID NO: 4547. or a homolog, a fragment or a variant thereof; and/or
      a 3'-UTR element comprising a nucleic acid sequence, which is derived from an albumin gene, preferably comprising the corresponding RNA sequence of the nucleic acid sequence according to SEQ ID NO: 4551 or SEQ ID NO: 4553, or a homolog, a fragment or a variant thereof,
   e) a poly(A) tail. preferably consisting of 10 to 200, 10 to 100, 40 to 80 or 50 to 70 adenosine nucleotides,
   f) a poly(C) tail. preferably consisting of 10 to 200, 10 to 100, 20 to 70, 20 to 60 or 10 to 40 cytosine nucleotides, and
   g) a histone stem-loop, preferably comprising the RNA sequence according to SEQ ID NO: 4556
30. Composition comprising the RNA according to any one of items 1 to 29 and a pharmaceutically acceptable carrier.
31. The composition according to item 30, wherein the RNA is complexed with one or more cationic or polycationic compounds, preferably with cationic or polycationic polymers, cationic or polycationic peptides or proteins, e.g. protamine, cationic or polycationic polysaccharides and/or cationic or polycationic lipids item
32. The composition according to item 31, wherein the N/P ratio of the RNA to the one or more cationic or polycationic peptides or proteins is in the range of about 0.1 to 10. including a range of about 0.3 to 4, of about 0.5 to 2. of about 0.7 to 2 and of about 0.7 to 1.5.
33. The composition according to any one of items 30 to 32 comprising at least one RNA. which is complexed with one or more cationic or polycationic compounds, and at least one free RNA.
34. The composition according to item 33, wherein the at least one complexed RNA is identical to the at least one free RNA.
35. The composition according to item 33 or 34, wherein the molar ratio of the complexed RNA to the free RNA is selected from a molar ratio of about 0.001:1 to about 1:0.001, including a ratio of about 1:1.
36. The composition according to any one of items 30 to 35, wherein the RNA is complexed with one or more lipids, thereby forming liposomes, lipid nanoparticles and/or lipoplexes.
37. The composition according to any one of items 30 to 36, wherein the composition comprises at least one adjuvant.
38. The composition according to item 37, wherein the adjuvant is selected from the group consisting of:
   cationic or polycationic compounds, comprising cationic or polycationic peptides or proteins, including protamine, nucleoline. spermin or spermidine, poly-L-lysine (PLL), poly-arginine, basic polypeptides, cell penetrating peptides (CPPs). including HIV-binding peptides, Tat, HIV-1 Tat (HIV), Tat-derived peptides. Penetratin, VP22 derived or analog peptides, HSV VP22 (Herpes simplex), MAP, KALA or protein transduction domains (PTDs, PpT620, proline-rich peptides, arginine-rich peptides, lysine-rich peptides, MPG-peptide(s). Pep-I. L-oligomers, Calcitonin peptide(s), Antennapedia-derived peptides (particularly from Drosophila antennapedia). pAntp, plsl, FGF, Lactoferrin, Transportan, Buforin-2, Bac715-24, SynB, SynB(1), pVEC, hCT-derived peptides, SAP, protamine, spermine, spermidine, or histones, cationic polysaccharides, including chitosan, polybrene, cationic polymers, including polyethyleneimine (PEI), cationic lipids, including DOTMA: [1-(2,3-sioleyloxy)propyl)]-N,N,N-trimethylammonium chloride, DMRIE, di-C14-amidine, DOTIM. SAINT, DC-Chol, BGTC, CTAP, DOPC, DODAP, DOPE: Dioleyl phosphatidylethanol-amine, DOSPA, DDDAB, DOIC, DMEPC, DOGS: Oioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: O,O-ditetradecanoyl-N-(α-trimethylammonioacetyl)diethanolamine chloride, CLIPI: rac-[(2,3-dioctadecyloxypropyl)(2-hydroxyethyl)]-dimethylammonium chloride, CLIPS: rac-[2(2,3-dihexadecyloxypropyl-oxymethyloxy)ethyl]-trimethylammonium, CLIPS: rac-[2(2,3-dihexadecyloxypropyl-oxysuccinyloxy)ethyl]-trimethylammonium, oligofectamine, or cationic or polycationic polymers, including modified polyaminoacids, including β-aminoacid-polymers or reversed polyamides, modified polyethylenes, including PVP (poly(N-ethyl-4-vinylpyridinium bromide)), modified acrylates, including pDMAEMA (poly(dimethylaminoethyl methylacrylate)), modified Amidoamines including pAMAM (poly(amidoamine)), modified polybetaaminoester (PBAE), including diamine end modified 1.4 butanediol diacrylate-co-5-amino-1-pentanol polymers, dendrimers, including polypropylamine dendrimers or pAMAM based dendrimers, polyimine(s), including PEI: poly(ethyleneimine), poly(propyleneimine), polyallylamine, sugar backbone based polymers, including cyclodextrin based polymers, dextran based polymers, Chitosan, etc., silan backbone based polymers , such as PMOXA-PDMS copolymers, etc., block polymers consisting of a combination of one or more cationic blocks selected from a cationic polymer as mentioned before, and of one or more hydrophilic- or hydrophobic blocks (e.g polyethyleneglycole);
      or
   cationic or polycationic proteins or peptides, selected from the following proteins or peptides having the following total formula (III): (Arg)ₗ;(Lys)ₘ;(His)ₙ;(Orn)ₒ;(Xaa)ₓ. wherein l + m + n +o + x = 8-15, and l, m, n or o independently of each other may be any number selected from 0, 1, 2, 3, 4, 5, 6, 7, 8, 9, 10, 11, 12, 13, 14 or 15, provided that the overall content of Arg, Lys, His and Orn represents at least 50% of all amino acids of the oligopeptide: and Xaa may be any amino acid selected from native (= naturally occurring) or non-native amino acids except from Arg. Lys, His or Orn; and x may be any number selected from 0, 1. 2, 3 or 4, provided, that the overall content of Xaa does not exceed 50% of all amino acids of the oligopeptide: or
   nucleic acids having the formula (V): GₗXₘGₙ. wherein: G is guanosine. uracil or an analogue of guanosine or uracil; X is guanosine. uracil, adenosine, thymidine, cytosine or an analogue of the above-mentioned nucleotides; I is an integer from 1 to 40, wherein, when l = 1 G is guanosine or an analogue thereof, when l > 1 at least 50% of the nucleotides are guanosine or an analogue thereof; m is an integer and is at least 3: wherein when m = 3 X is uracil or an analogue thereof, when m > 3 at least 3 successive uracils or analogues of uracil occur; n is an integer from 1 to 40, wherein when n = 1 G is guanosine or an analogue thereof, when n > 1 at least 50% of the nucleotides are guanosine or an analogue thereof;
      or
   nucleic acids having the formula (VI): CₗXₘCₙ, wherein: C is cytosine, uracil or an analogue of cytosine or uracil: X is guanosine, uracil, adenosine, thymidine, cytosine or an analogue of the above-mentioned nucleotides: l is an integer from 1 to 4D, wherein when l = 1 C is cytosine or an analogue thereof, when l > 1 at least 50% of the nucleotides are cytosine or an analogue thereof: m is an integer and is at least 3: wherein when m = 3 X is uracil or an analogue thereof, when m > 3 at least 3 successive uracils or analogues of uracil occur; n is an integer from 1 to 40, wherein when n = 1 C is cytosine or an analogue thereof, when n > 1 at least 50% of the nucleotides are cytosine or an analogue thereof;
      or
   adjuvants selected from the group consisting of:
      TDM, MDP, muramyl dipeptide, pluronics, alum solution, aluminium hydroxide. ADJUMER^{™} (polyphosphazene): aluminium phosphate gel; glucans from algae; algammulin: aluminium hydroxide gel (alum); highly protein-adsorbing aluminium hydroxide gel; low viscosity aluminium hydroxide gel: AF or SPT (emulsion of squalane (5%). Tween 80 (0.2%), Pluronic L121 (1.25%). phosphate-buffered saline, pH 7.4); AVRIDINE^{™} (propanediamine); BAY R1005^{™} ((N-(2-deoxy-2-L-leucylamino-b-D-glucopyranosyl)-N-octadecyl-dodecanoyl-amide hydroacetate); CALCITRIOL^{™} (1-alpha,25-dihydroxy-vitamin D3): calcium phosphate gel; CAP^{™} (calcium phosphate nanoparticles); cholera holotoxin, cholera-toxin-A1-protein-A-D-fragment fusion protein, sub-unit B of the cholera toxin: CRL 1005 (block copolymer P1205); cytokine-containing liposomes; DDA (dimethyldioctadecylammonium bromide); DHEA (dehydroepiandrosterone): DMPC (dimyristoylphosphatidylcholine): DMPG (dimyristoylphosphatidylglycerol): DOC/alum complex (deoxycholic acid sodium salt); Freund's complete adjuvant: Freund's incomplete adjuvant: gamma inulin; Gerbu adjuvant (mixture of: i) N-acetylglucosaminyl-(PI-4)-N-acetylmuramyl-L-alanyl-D-glutamine (GMDP), ii) dimethyldioctadecylammonium chloride (DDA). iii) zinc-L-proline salt complex (ZnPro-8); GM-CSF); GMDP (N-acetylglucosaminyl-(b1-4)-N-acetylmuramyl-L-alanyl-D-isoglutamine); imiquimod (1-(2-methypropyl)-1H-imidazo[4.5-c]quinolino-4-amine); ImmTher^{™} (N-acetylglucosaminyl-N-acetylmuramyl-L-Ala-D-isoGlu-L-Ala-glycerol dipalmitate); DRVs (immunoliposomes prepared from dehydration-rehydration vesicles); interferon-gamma: interleukin-lbeta; interleukin-2; interleukin-7; interleukin-12; ISCOMS^{™}: ISCOPREP 7,0.3.^{™}; liposomes: LOXORIBINE^{™} (7-allyl-8-oxoguanosine); LT oral adjuvant (E.coli labile enterotoxin-protoxin); microspheres and microparticles of any composition; MF59^{™}; (squalene-water emulsion); MONTANIDE ISA 51^{™} (purified incomplete Freund's adjuvant); MONTANIDE ISA 720^{™} (metabolisable oil adjuvant); MPL^{™} (3-Q-desacyl-4'-monophosphoryl lipid A); MTP-PE and MTP-PE liposomes ((N-acetyl-L-alanyl-0-isoglutaminyl-L-alanine-2-(1,2-dipalmitoyi-sn-glycero-3-(hydroxyphosphoryloxy))-ethylamide, monosodium salt); MURAMETIDE^{™} (Nac-Mur-L-Ala-D-Gln-OCH3); MURAPALMITINE^{™} and D-MURAPALMITINE^{™} (Nac-Mur-L-Thr-D-isoGln-sn-glyceroldipalmitoyl); NAGO (neuraminidase-galactose oxidase); nanospheres or nanoparticles of any composition; NISVs (non-ionic surfactant vesicles): PLEURAN^{™} (β-glucan): PLGA. PGA and PLA (homo- and co-polymers of lactic acid and glycolic acid: microspheres/nanospheres); PLURONIC L121^{™}; PMMA (polymethyl methacrylate); PDDDS^{™} (proteinoid microspheres); polyethylene carbamate derivatives: poly-rA: poly-rU (polyadenylic acid-polyuridylic acid complex); polysorbate 80 (Tween 80): protein cochleates (Avanti Polar lipids, Inc., Alabaster, AL): STIMULON^{™} (QS-21); Quil-A (Quil-A saponin); S-28463 (4-amino-otec-dimethyl-2-ethoxymethyl-1H-imidazo[4,5c]quinoline-1-ethanol); SAF-1^{™} ("Syntex adjuvant formulation"); Sendai proteoliposomes and Sendai-containing lipid matrices: Span-85 (sorbitan trioleate); Specol (emulsion of Marcol 52, Span 85 and Tween 85): squalene or Robane^{®} (2,6,10,15,19,23-hexamethyltetracosan and 2,6,10,15.19,23-hexamethyl-2,6,10,14,18,22-tetracosahexane); stearyltyrosine (octadecyltyrosine hydrochloride); Theramid^{®} (N-acetylglucasaminyl-N-acetylmuramyl-L-Ala-D-isoGlu-L-Ala-dipalmitoxypropylamide); Theronyl-MDP (Termurtide^{™} or [thr 1]-MDP; N-acetylmuramyl-L-threanyl-D-isoglutamine); Ty particles (Ty-VLPs or virus-like particles): Walter-Reed liposomes (liposomes containing lipid A adsorbed on aluminium hydroxide), and lipopeptides, including Pam3Cys, in particular aluminium salts, such as Adju-phos, Alhydrogel, Rehydragel; emulsions, including CFA, SAF, IFA, MF59, Provax, TiterMax, Montanide, Vaxfectin; copolymers, including Optivax (CRL1005), L121, Poloaxmer4010), etc.; liposomes, including Stealth, cochleates. including BIORAL; plant derived adjuvants, including QS21, Quil A, Iscomatrix, ISCOM; adjuvants suitable for costimulation including Tomatine, biopolymers, including PLG, PMM, Inulin; microbe derived adjuvants, including Romurtide, DETOX, MPL, CWS, Mannose, CpG nucleic acid sequences, CpG7909, ligands of human TLR 1-10, ligands of murine TLR 1-13, ISS-1018, IC31, Imidazoquinolines, Ampligen, Ribi529, IMOxine, IRIVs, VLPs, cholera toxin. heat-labile toxin. Pam3Cys, Flagellin, GPI anchor. LNFPIII/Lewis X, antimicrobial peptides. UC-IVI50, RSV fusion protein. cdiGMP; and adjuvants suitable as antagonists including CGRP neuropeptide.
39. Vaccine comprising the RNA according to any one of items 1 to 29 and a pharmaceutically acceptable carrier, or comprising the composition according to any one of items 30 to 38.
40. The vaccine according to item 39, wherein the RNA according to any one of items 1 to 29 or the composition according to any one of items 30 to 38 elicits an adaptive immune response.
41. The vaccine according to item 39 or 40 comprising an adjuvant.
42. Kit, preferably kit of parts, comprising the RNA according to any one of items 1 to 29, the composition according to any one of items 30 to 38 or the vaccine according to any one of items 39 to 41, and optionally a liquid vehicle for solubilising and optionally technical instructions with information on the administration and dosage of the RNA. the composition and/or the vaccine.
43. The kit according to item 42, wherein the kit contains as a part Ringer-Lactate solution.
44. The kit according to item 42 or 43, wherein the kit contains as a part an adjuvant.
45. The kit according to any one of items 42 to 44, wherein the kit contains as a part at least one inhibitory and/or stimulatory checkpoint molecule.
46. The RNA according to any one of items I to 29, the composition according to any one of items 30 to 38, the vaccine according to any one of items 39 to 41, or the kit according to any one of items 42 to 45 for use in the treatment or prophylaxis of cancer.
47. The RNA according to any one of items 1 to 29, the composition according to any one of items 30 to 38, the vaccine according to any one of items 39 to 41, or the kit according to any one of items 42 to 45 for use according to item 46. wherein the cancer is selected from the group consisting of Acute Lymphoblastic Leukemia. Adult; Acute Lymphoblastic Leukemia. Childhood; Acute Myeloid Leukemia, Adult; Adrenocortical Carcinoma; Adrenocortical Carcinoma, Childhood: AIDS-Related Lymphoma: AIDS-Related Malignancies; Anal Cancer; Astrocytoma. Childhood Cerebellar; Astrocytoma. Childhood Cerebral; Bile Duct Cancer, Extrahepatic; Bladder Cancer; Bladder Cancer, Childhood: Bone Cancer, Osteosarcoma/Malignant Fibrous Histiocytoma; Brain Stem Glioma. Childhood: Brain Tumor, Adult; Brain Tumor, Brain Stem Glioma, Childhood: Brain Tumor, Cerebellar Astrocytoma, Childhood; Brain Tumor, Cerebral Astrocytoma/Malignant Glioma, Childhood: Brain Tumor, Ependymoma, Childhood: Brain Tumor. Medulloblastoma. Childhood; Brain Tumor. Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy: Breast Cancer, Childhood; Breast Cancer, Male; Bronchial Adenomas/Carcinoids, Childhood: Carcinoid Tumor. Childhood; Carcinoid Tumor. Gastrointestinal; Carcinoma, Adrenocortical; Carcinoma. Islet Cell; Carcinoma of Unknown Primary: Central Nervous System Lymphoma, Primary: Cerebellar Astrocytoma. Childhood: Cerebral Astrocytoma/Malignant Glioma, Childhood: Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood; Cutaneous T-Cell Lymphoma: Endometrial Cancer; Ependymoma, Childhood; Epithelial Cancer, Ovarian; Esophageal Cancer; Esophageal Cancer, Childhood; Ewing's Family of Tumors; Extracranial Germ Cell Tumor. Childhood: Extragonadal Germ Cell Tumor: Extrahepatic Bile Duct Cancer; Eye Cancer, Intraocular Melanoma; Eye Cancer. Retinoblastoma; Gallbladder Cancer: Gastric (Stomach) Cancer; Gastric (Stomach) Cancer, Childhood: Gastrointestinal Carcinoid Tumor; Germ Cell Tumor, Extracranial, Childhood; Germ Cell Tumor. Extragonadal; Germ Cell Tumor. Ovarian; Gestational Trophoblastic Tumor: Glioma. Childhood Brain Stem; Glioma. Childhood Visual Pathway and Hypothalamic; Hairy Cell Leukemia; Head and Neck Cancer; Hepatocellular (Liver) Cancer, Adult (Primary): Hepatocellular (Liver) Cancer, Childhood (Primary); Hodgkin's Lymphoma, Adult; Hodgkin's Lymphoma, Childhood: Hodgkin's Lymphoma During Pregnancy; Hypopharyngeal Cancer; Hypothalamic and Visual Pathway Glioma. Childhood; Intraocular Melanoma; Islet Cell Carcinoma (Endocrine Pancreas): Kaposi's Sarcoma; Kidney Cancer; Laryngeal Cancer; Laryngeal Cancer, Childhood: Leukemia, Acute Lymphoblastic, Adult; Leukemia, Acute Lymphoblastic, Childhood: Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute: Lymphoblastic Leukemia, Childhood Acute: Lymphocytic Leukemia, Chronic: Lymphoma, AIDS- Related: Lymphoma, Central Nervous System (Primary); Lymphoma, Cutaneous T-Cell; Lymphoma, Hodgkin's, Adult: Lymphoma, Hodgkin's: Childhood; Lymphoma, Hodgkin's During Pregnancy; Lymphoma. Non-Hodgkin's, Adult: Lymphoma, Non-Hodgkin's, Childhood; Lymphoma, Non-Hodgkin's During Pregnancy; Lymphoma, Primary Central Nervous System; Macroglobulinemia. Waldenstrom's: Male Breast Cancer; Malignant Mesothelioma, Adult; Malignant Mesothelioma, Childhood: Malignant Thymoma: Medulloblastoma, Childhood; Melanoma; Melanoma, Intraocular; Merkel Cell Carcinoma; Mesothelioma, Malignant; Metastatic Squamous Neck Cancer with Occult Primary: Multiple Endocrine Neoplasia Syndrome, Childhood: Multiple Myeloma/Plasma Cell Neoplasm; Mycosis Fungoides: Myelodysplasia Syndromes; Myelogenous Leukemia, Chronic: Myeloid Leukemia, Childhood Acute; Myeloma, Multiple; Myeloproliferative Disorders, Chronic: Nasal Cavity and Paranasal Sinus Cancer; Nasopharyngeal Cancer; Nasopharyngeal Cancer, Childhood: Neuroblastoma; Neurofibroma; Non-Hodgkin's Lymphoma, Adult; Non- Hodgkin's Lymphoma, Childhood; Non-Hodgkin's Lymphoma During Pregnancy: Non- Small Cell Lung Cancer; Oral Cancer, Childhood: Oral Cavity and Lip Cancer; Oropharyngeal Cancer: Osteosarcoma/Malignant Fibrous Histiocytoma of Bone: Ovarian Cancer, Childhood: Ovarian Epithelial Cancer; Ovarian Germ Cell Tumor; Ovarian Low Malignant Potential Tumor; Pancreatic Cancer; Pancreatic Cancer, Childhood', Pancreatic Cancer, Islet Cell; Paranasal Sinus and Nasal Cavity Cancer; Parathyroid Cancer; Penile Cancer; Pheochromocytoma: Pineal and Supratentorial Primitive Neuroectodermal Tumors, Childhood; Pituitary Tumor; Plasma Cell Neoplasm/Multiple Myeloma: Pleuropulmonary Blastoma; Pregnancy and Breast Cancer; Pregnancy and Hodgkin's Lymphoma; Pregnancy and Non-Hodgkin's Lymphoma; Primary Central Nervous System Lymphoma: Primary Liver Cancer, Adult: Primary Liver Cancer, Childhood: Prostate Cancer; Rectal Cancer; Renal Cell (Kidney) Cancer: Renal Cell Cancer, Childhood: Renal Pelvis and Ureter, Transitional Cell Cancer: Retinoblastoma: Rhabdomyosarcoma, Childhood; Salivary Gland Cancer; Salivary Gland'Cancer, Childhood; Sarcoma, Ewing's Family of Tumors; Sarcoma, Kaposi's; Sarcoma (Osteosarcoma)/Malignant Fibrous Histiocytoma of Bone: Sarcoma. Rhabdomyosarcoma, Childhood; Sarcoma, Soft Tissue, Adult; Sarcoma, Soft Tissue, Childhood: Sezary Syndrome; Skin Cancer; Skin Cancer, Childhood: Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood: Squamous Neck Cancer with Occult Primary, Metastatic; Stomach (Gastric) Cancer; Stomach (Gastric) Cancer, Childhood: Supratentorial Primitive Neuroectodermal Tumors, Childhood; T-Cell Lymphoma, Cutaneous; Testicular Cancer: Thymoma. Childhood: Thymoma, Malignant: Thyroid Cancer; Thyroid Cancer, Childhood: Transitional Cell Cancer of the Renal Pelvis and Ureter; Trophoblastic Tumor. Gestational: Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer: Visual Pathway and Hypothalamic Glioma. Childhood: Vulvar Cancer; Waldenstrom's Macro globulinemia; and Wilms' Tumor.
48. The RNA according to any one of items 1 to 29, the composition according to any one of items 30 to 38, the vaccine according to any one of items 39 to 41, or the kit according to any one of items 42 to 45 for use according to item 46 or 47, wherein the RNA. the composition, the vaccine or the pharmaceutically active component of the kit is administered by injection, preferably by needleless injection, more preferably by jet injection.
49. The RNA according to any one of items I to 29, the composition according to any one of items 30 to 38, the vaccine according to any one of items 39 to 41, or the kit according to any one of items 42 to 45 for use according to any one of items 46 to 48, wherein the treatment or prophylaxis comprises the administration of a further pharmaceutical compound.
50. The RNA according to any one of items 1 to 29, the composition according to any one of items 30 to 38, the vaccine according to any one of items 39 to 41, or the kit according to any one of items 42 to 45 for use according to item 49, wherein the further pharmaceutical compound is a chemotherapeutic agent or a kinase inhibitor.
51. The RNA according to any one of items I to 29, the composition according to any one of items 30 to 38, the vaccine according to any one of items 39 to 41, or the kit according to any one of items 42 to 45 for use according to item 49 or 50, wherein the treatment or prophylaxis further comprises radiation therapy.
52. The RNA according to any one of items 1 to 29, the composition according to any one of items 30 to 38, the vaccine according to any one of items 39 to 41, or the kit according to any one of items 42 to 45 for use according to any one of items 48 to 51, wherein the treatment or prophylaxis further comprises the administration of at least one inhibitory and/or stimulatory checkpoint molecule.
53. Method of treating or preventing a disorder, wherein the method comprises administering to a subject in need thereof an effective amount of the RNA according to any one of items 1 to 29, the composition according to any one of items 30 to 38, the vaccine according to any one of items 39 to 41, or the kit according to any one of items 42 to 45.
54. The method according to item 53, wherein the disorder is cancer.
55. The method according to item 54. wherein the cancer is selected from the group consisting of Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma: AIDS-Related Malignancies: Anal Cancer; Astrocytoma. Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma: Brain Stem Glioma, Childhood; Brain Tumor. Adult; Brain Tumor, Brain Stem Glioma, Childhood; Brain Tumor, Cerebellar Astrocytoma. Childhood; Brain Tumor, Cerebral Astrocytoma/Malignant Glioma, Childhood; Brain Tumor, Ependymoma. Childhood; Brain Tumor, Medulloblastoma. Childhood; Brain Tumor, Supratentorial Primitive Neuroectodermal Tumors, Childhood: Brain Tumor, Visual Pathway and Hypothalamic Glioma. Childhood: Brain Tumor, Childhood (Other); Breast Cancer; Breast Cancer and Pregnancy; Breast Cancer, Childhood; Breast Cancer, Male; Bronchial Adenomas/Carcinoids, Childhood: Carcinoid Tumor, Childhood; Carcinoid Tumor. Gastrointestinal: Carcinoma, Adrenocortical: Carcinoma, Islet Cell; Carcinoma of Unknown Primary: Central Nervous System Lymphoma, Primary: Cerebellar Astrocytoma, Childhood; Cerebral Astrocytoma/Malignant Glioma. Childhood: Cervical Cancer; Childhood Cancers; Chronic Lymphocytic Leukemia; Chronic Myelogenous Leukemia: Chronic Myeloproliferative Disorders; Clear Cell Sarcoma of Tendon Sheaths: Colon Cancer: Colorectal Cancer, Childhood; Cutaneous T-Cell Lymphoma: Endometrial Cancer; Ependymoma, Childhood; Epithelial Cancer, Ovarian: Esophageal Cancer; Esophageal Cancer, Childhood; Ewing's Family of Tumors; Extracranial Germ Cell Tumor. Childhood; Extragonadal Germ Cell Tumor; Extrahepatic Bile Duct Cancer: Eye Cancer. Intraocular Melanoma; Eye Cancer, Retinoblastoma; Gallbladder Cancer; Gastric (Stomach) Cancer; Gastric (Stomach) Cancer, Childhood: Gastrointestinal Carcinoid Tumor; Germ Cell Tumor, Extracranial, Childhood; Germ Cell Tumor, Extragonadal; Germ Cell Tumor. Ovarian; Gestational Trophoblastic Tumor; Glioma. Childhood Brain Stem; Glioma. Childhood Visual Pathway and Hypothalamic: Hairy Cell Leukemia: Head and Neck Cancer; Hepatocellular (Liver) Cancer, Adult (Primary); Hepatocellular (Liver) Cancer, Childhood (Primary); Hodgkin's Lymphoma. Adult: Hodgkin's Lymphoma, Childhood: Hodgkin's Lymphoma During Pregnancy: Hypopharyngeal Cancer; Hypothalamic and Visual Pathway Glioma, Childhood; Intraocular Melanoma; Islet Cell Carcinoma (Endocrine Pancreas); Kaposi's Sarcoma: Kidney Cancer; Laryngeal Cancer; Laryngeal Cancer, Childhood: Leukemia. Acute Lymphoblastic, Adult: Leukemia, Acute Lymphoblastic, Childhood; Leukemia, Acute Myeloid, Adult: Leukemia, Acute Myeloid, Childhood; Leukemia. Chronic Lymphocytic; Leukemia. Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia. Adult Acute: Lymphoblastic Leukemia, Childhood Acute: Lymphocytic Leukemia. Chronic: Lymphoma, AIDS- Related: Lymphoma, Central Nervous System (Primary); Lymphoma. Cutaneous T-Cell; Lymphoma, Hodgkin's, Adult; Lymphoma, Hodgkin's: Childhood; Lymphoma, Hodgkin's During Pregnancy; Lymphoma, Non-Hodgkin's, Adult; Lymphoma, Non-Hodgkin's, Childhood; Lymphoma, Non-Hodgkin's During Pregnancy; Lymphoma. Primary Central Nervous System; Macroglobulinemia. Waldenstrom's; Male Breast Cancer: Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant: Metastatic Squamous Neck Cancer with Occult Primary: Multiple Endocrine Neoplasia Syndrome, Childhood: Multiple Myeloma/Plasma Cell Neoplasm; Mycosis Fungoides: Myelodysplasia Syndromes; Myelogenous Leukemia. Chronic: Myeloid Leukemia. Childhood Acute: Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Neurofibroma; Non-Hodgkin's Lymphoma, Adult; Non- Hodgkin's Lymphoma, Childhood; Non-Hodgkin's Lymphoma During Pregnancy; Non- Small Cell Lung Cancer; Oral Cancer, Childhood: Oral Cavity and Lip Cancer; Oropharyngeal Cancer: Osteosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood', Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma: Pleuropulmonary Blastoma; Pregnancy and Breast Cancer; Pregnancy and Hodgkin's Lymphoma; Pregnancy and Non-Hodgkin's Lymphoma; Primary Central Nervous System Lymphoma; Primary Liver Cancer, Adult; Primary Liver Cancer, Childhood: Prostate Cancer; Rectal Cancer; Renal Cell (Kidney) Cancer: Renal Cell Cancer, Childhood: Renal Pelvis and Ureter, Transitional Cell Cancer: Retinoblastoma; Rhabdomyosarcoma, Childhood; Salivary Gland Cancer; Salivary Gland'Cancer. Childhood; Sarcoma, Ewing's Family of Tumors; Sarcoma. Kaposi's: Sarcoma (Osteosarcoma)/Malignant Fibrous Histiocytoma of Bone: Sarcoma, Rhabdomyosarcoma, Childhood: Sarcoma, Soft Tissue, Adult: Sarcoma, Soft Tissue, Childhood: Sezary Syndrome; Skin Cancer; Skin Cancer, Childhood: Skin Cancer (Melanoma); Skin Carcinoma. Merkel Cell: Small Cell Lung Cancer; Small Intestine Cancer; Soft Tissue Sarcoma, Adult; Soft Tissue Sarcoma, Childhood; Squamous Neck Cancer with Occult Primary, Metastatic: Stomach (Gastric) Cancer; Stomach (Gastric) Cancer, Childhood: Supratentorial Primitive Neuroectodermal Tumors, Childhood: T-Cell Lymphoma, Cutaneous: Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational: Unknown Primary Site, Cancer of. Childhood; Unusual Cancers of Childhood; Ureter and Renal Pelvis, Transitional Cell Cancer; Urethral Cancer; Uterine Sarcoma; Vaginal Cancer: Visual Pathway and Hypothalamic Glioma, Childhood; Vulvar Cancer; Waldenstrom's Macro globulinemia; and Wilms' Tumor.

## Claims

1. RNA comprising at least one coding sequence, wherein the coding sequence comprises a nucleic acid sequence selected from the group consisting of SEQ ID NOs:780, 1284, 1788, 2292, 2796, 3300, 3804, 4308, 768, 1272, 1776, 2280, 2784, 3288, 3792, 4286, 774, 775, 1278, 1279, 1782, 1783, 2286, 2287, 2790, 2791, 3294, 3295, 3788, 3799, 4302, 4303, 773, 1277,1781, 2285, 2788, 3293, 3797, 4301 and 4587, or a fragment or variant of any one of said nucleic acid sequences.

2. The RNA according to claim 1, wherein the at least one coding sequence encodes a tumor antigen, or a fragment or variant of a tumor antigen, wherein the tumor antigen is selected from the group consisting of MAGE-A9; MAGAB; MAGE-A4 and MAGE-A3.

3. The RNA according to claim 1 or 2, wherein the at least one coding sequence encodes a tumor antigen comprising an amino acid sequence selected from the group consisting of SEQ ID NOs: 276, 264, 270, 271 and 269, or a fragment or variant of any one of said amino acid sequences.

4. The RNA according to any one of claims 1 to 3, wherein the RNA is mono-, bi-, or multicistronic.

5. The RNA according to any one of claims 1 to 4, wherein the RNA is an mRNA, a viral RNA or a replicon RNA.

6. The RNA according to any one of claims 1 to 5, wherein the RNA is a modified RNA, preferably a stabilized RNA.

7. The RNA according to claim 6, wherein the at least one coding sequence of the RNA comprises a nucleic acid sequence selected from the group consisting of SEQ ID NOs: 1284, 1788, 2292, 2796, 3300, 3804, 4308, 1272, 1776, 2280, 2784, 3288, 3792, 4296,1278,1279,1782,1783, 2286, 2287, 2790, 2791, 3294, 3295, 3798, 3799, 4302, 4303,1277, 1781, 2285, 2789, 3293, 3797, 4301 and 4587, or a fragment or variant of any one of said nucleic acid sequences.

8. The RNA according to any one of claims 1 to 7, which comprises a 5'-CAP structure and/or at least one 3'-untranslated region element (3'-UTR element).

9. The RNA according to any one of claims 1 to 8, which comprises at least one histone stem-loop wherein the at least one histone stem-loop preferably comprises a nucleic acid sequence according to the following formulae (I) or (II):
formula (I) (stem-loop sequence without stem bordering elements): 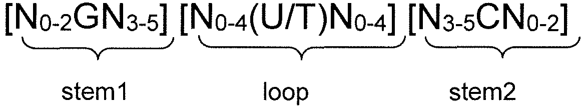
formula (II) (stem-loop sequence with stem bordering elements): 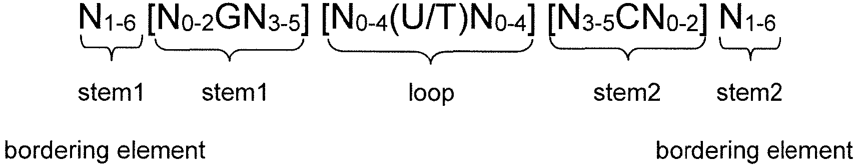
wherein:
stem1 or stem2 bordering elements N₁₋₆ is a consecutive sequence of 1 to 6, preferably of 2 to 6, more preferably of 2 to 5, even more preferably of 3 to 5, most preferably of 4 to 5 or 5 N, wherein each N is independently from another selected from a nucleotide selected from A, U, T, G and C, or a nucleotide analogue thereof:
stem1 [N₀₋₂GN₃₋₅] is reverse complementary or partially reverse complementary with element stem2, and is a consecutive sequence between of 5 to 7 nucleotides;
wherein N₀₋₂ is a consecutive sequence of 0 to 2, preferably of 0 to 1, more preferably of 1 N, wherein each N is independently from another selected from a nucleotide selected from A, U, T, G and C or a nucleotide analogue thereof;
wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof, and
wherein G is guanosine or an analogue thereof, and may be optionally replaced by a cytidine or an analogue thereof, provided that its complementary nucleotide cytidine in stem2 is replaced by guanosine:
loop sequence [N₀₋₄(U/T)N₀₋₄] is located between elements stem1 and stem2, and is a consecutive sequence of 3 to 5 nucleotides, more preferably of 4 nucleotides;
wherein each N₀₋₄ is independent from another a consecutive sequence of 0 to 4, preferably of I to 3, more preferably of I to 2 N, wherein each N is independently from another selected from a nucleotide selected from A, U, T, G and C or a nucleotide analogue thereof; and
wherein U/T represents uridine, or optionally thymidine:
stem2 [N₃₋₅CN₀₋₂] is reverse complementary or partially reverse complementary with element stem1. and is a consecutive sequence between of 5 to 7 nucleotides;
wherein N₃₋₅ is a consecutive sequence of 3 to 5, preferably of 4 to 5, more preferably of 4 N, wherein each N is independently from another selected from a nucleotide selected from A, U, T, G and C or a nucleotide analogue thereof;
wherein N₀₋₂ is a consecutive sequence of 0 to 2, preferably of 0 to 1, more preferably of 1 N, wherein each N is independently from another selected from a nucleotide selected from A, U, T, G and C or a nucleotide analogue thereof; and
wherein C is cytidine or an analogue thereof, and may be optionally replaced by a guanosine or an analogue thereof provided that its complementary nucleotide guanosine in stem1 is replaced by cytidine;
wherein
stem1 and stem2 are capable of base pairing with each other
forming a reverse complementary sequence, wherein base pairing may occur between stem1 and stem2, or
forming a partially reverse complementary sequence, wherein an incomplete base pairing may occur between stem1 and stem2, and/or
wherein the at least one histone stem-loop comprises a nucleic acid sequence according to the following formulae (la) or (Ila):
formula (la) (stem-loop sequence without stem bordering elements): 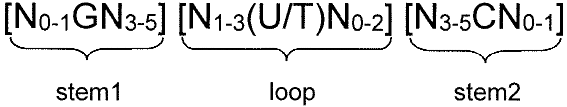
formula (IIa) (stem-loop sequence with stem bordering elements): 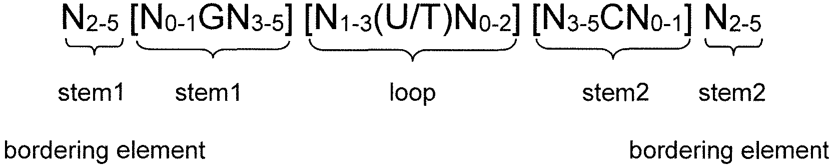
and/or wherein the at least one histone stem loop comprises a nucleic acid sequence according to SEQ ID NO: 4555 and most preferably a RNA sequence according to SEQ ID N0: 4556.

10. The RNA according to any one of claims 1 to 9, which comprises, preferably in 5' to 3' direction, the following elements:
a) a 5'-CAP structure, preferably m7GpppN,
b) at least one coding sequence as defined in claim 7,
c) a poly(A) tail. preferably consisting of 10 to 200, 10 to 100, 40 to 80 or 50 to 70 adenosine nucleotides,
d) a poly(C) tail. preferably consisting of 10 to 200, 10 to 100, 20 to 70, 20 to 60 or 10 to 40 cytosine nucleotides, and
e) a histone stem-loop, preferably comprising the RNA sequence according to SEQ ID NO: 4556.

11. The RNA according to any one of claims 1 to 10, wherein the RNA comprises a 5'-UTR element,
wherein the 5'-UTR element preferably comprises a nucleic acid sequence, which is derived from the 5'-UTR of a TOP gene, preferably from a corresponding RNA sequence, or a homolog, a fragment, or a variant thereof, preferably lacking the 5'TOP motif.

12. Composition comprising the RNA according to any one of claims 1 to 11 and a pharmaceutically acceptable carrier.

13. The composition according to claim 12, wherein the RNA is complexed with one or more cationic or polycationic compounds, preferably with cationic or polycationic polymers, cationic or polycationic peptides or proteins, e.g. protamine, cationic or polycationic polysaccharides and/or cationic or polycationic lipids.

14. The composition according to claim 12 or 13, wherein the RNA is complexed with one or more lipids, thereby forming liposomes, lipid nanoparticles and/or lipoplexes.

15. The composition according to any one of claims 12 to 14, wherein the composition comprises at least one adjuvant,
wherein the adjuvant is preferably selected from the group consisting of:
cationic or polycationic compounds, comprising cationic or polycationic peptides or proteins, including protamine, nucleoline, spermin or spermidine, poly-L-lysine (PLL), poly-arginine, basic polypeptides, cell penetrating peptides (CPPs), including HIV-binding peptides, Tat, HIV-1 Tat (HIV), Tat-derived peptides, Penetratin, VP22 derived or analog peptides. HSV VP22 (Herpes simplex), MAP, KALA or protein transduction domains (PTDs, PpT620, proline-rich peptides, arginine-rich peptides, lysine-rich peptides, MPG-peptide(s), Pop-1, L-oligomers, Calcitonin peptide(s), Antennapedia-derived peptides (particularly from Drosophila antennapedia), pAntp, plsl, FGF, Lactoferrin, Transportan, Buforin-2, Bac715-24, SynB, SynB(1), pVEC, hCT-derived peptides, SAP, protamine, spermine, spermidine, or histones, cationic polysaccharides, including chitosan, polybrene, cationic polymers, including polyethyleneimine (PEI), cationic lipids, including DOTMA: [1-(2,3-sioleyloxy)propyl)]-N,N,N-trimethylammonium chloride, DMRIE, di-C14-amidine, DOTIM, SAINT, DC-Chol, BGTC, CTAP, DOPC, DODAP, DOPE: Dioleyl phosphatidylethanol-amine, DOSPA, DODAB, DOIC, DMEPC, DOGS: Dioctadecylamidoglicylspermin, DIMRI: Dimyristo-oxypropyl dimethyl hydroxyethyl ammonium bromide, DOTAP: dioleoyloxy-3-(trimethylammonio)propane, DC-6-14: 0,0-ditetradecanoyl-N-(α-trimethylammonioacetyl)diethanolamino chloride, CLIP1: rac-[(2,3-dioctadecyloxypropyl)(2-hydroxyethyl)]-dimethylammonium chloride, CLIPS: rac-[2(2,3-dihexadecyloxypropyl-oxymethyloxy)ethyl]-trimethylammonium, CLIPS: rac-[2(2,3-dihexadecyloxypropyl-oxysuccinyloxy)ethyl]-trimethylammonium, oligofectamine, or cationic or polycationic polymers, including modified polyaminoacids, including β-aminoacid-polymers or reversed polyamides, modified polyethylenes, including PVP (poly(N-ethyl-4-vinylpyridinium bromide)), modified acrylates, including pDMAEMA (poly(dimethylaminoethyl methylacrylate)), modified Amidoamines including pAMAM (poly(amidoamine)), modified polybetaaminoester (PBAE), including diamine end modified 1.4 butanediol diacrylate-co-5-amino-1-pentanol polymers, dendrimers, including polypropylamine dendrimers or pAMAM based dendrimers, polyimine(s), including PEI: poly(ethyleneimine), poly(propyleneimine), polyallylamine, sugar backbone based polymers, including cyclodextrin based polymers, dextran based polymers, Chitosan, etc., silan backbone based polymers, such as PMOXA-PDMS copolymers, etc., block polymers consisting of a combination of one or more cationic blocks selected from a cationic polymer as mentioned before, and of one or more hydrophilic- or hydrophobic blocks (e.g polyethyleneglycole);
or
cationic or polycationic proteins or peptides, selected from the following proteins or peptides having the following total formula (III): (Arg)ₗ;(Lys)ₘ:(His)ₙ;(Orn)ₒ;(Xaa)ₓ, wherein I + m + n +o + x = 8-15, and l, m, n or o independently of each other may be any number selected from 0, 1, 2, 3, 4, 5, 6, 7, 8. 9, 10, 11, 12, 13, 14 or 15, provided that the overall content of Arg, Lys. His and Orn represents at least 50% of all amino acids of the oligopeptide; and Xaa may be any amino acid selected from native (= naturally occurring) or non-native amino acids except from Arg, Lys, His or Orn; and x may be any number selected from 0, 1, 2, 3 or 4, provided, that the overall content of Xaa does not exceed 50% of all amino acids of the oligopeptide; or nucleic acids having the formula (V): GₗXₘGₙ, wherein: G is guanosine. uracil or an analogue of guanosine or uracil; X is guanosine, uracil, adenosine, thymidine, cytosine or an analogue of the above-mentioned nucleotides: I is an integer from I to 40, wherein, when I = I G is guanosine or an analogue thereof, when I > 1 at least 50% of the nucleotides are guanosine or an analogue thereof; m is an integer and is at least 3; wherein when m = 3 X is uracil or an analogue thereof, when m > 3 at least 3 successive uracils or analogues of uracil occur; n is an integer from 1 to 40, wherein when n = 1 G is guanosine or an analogue thereof, when n > 1 at least 50% of the nucleotides are guanosine or an analogue thereof;
or
nucleic acids having the formula (VI): CₗXₘCₙ, wherein: C is cytosine, uracil or an analogue of cytosine or uracil: X is guanosine, uracil, adenosine, thymidine, cytosine or an analogue of the above-mentioned nucleotides; I is an integer from 1 to 40, wherein when l = 1 C is cytosine or an analogue thereof, when I > 1 at least 50% of the nucleotides are cytosine or an analogue thereof; m is an integer and is at least 3; wherein when m = 3 X is uracil or an analogue thereof, when m > 3 at least 3 successive uracils or analogues of uracil occur; n is an integer from I to 40. wherein when n = 1 C is cytosine or an analogue thereof, when n > 1 at least 50% of the nucleotides are cytosine or an analogue thereof;
or
adjuvants selected from the group consisting of:
TDM, MDP, muramyl dipeptide, pluronics, alum solution, aluminium hydroxide, ADJUMER^{™} (polyphosphazene): aluminium phosphate gel; glucans from algae; algammulin; aluminium hydroxide gel (alum); highly protein-adsorbing aluminium hydroxide gel: low viscosity aluminium hydroxide gel; AF or SPT (emulsion of squalane (5%), Tween 80 (0.2%), Pluronic L121 (1.25%), phosphate-buffered saline, pH 7.4); AVRIDINE^{™} (propanediamine); BAY R1005^{™} ((N-(2-deoxy-2-L-leucylamino-b-D-glucopyranosyl)-N-octadecyl-dodecanoyl-amide hydroacetate); CALCITRIOL^{™} (1-alpha,25-dihydroxy-vitamin D3): calcium phosphate gel; CAP^{™} (calcium phosphate nanoparticles): cholera holotoxin, cholera-toxin-A1-protein-A-D-fragment fusion protein, sub-unit B of the cholera toxin; CRL 1005 (block copolymer P1205); cytokine-containing liposomes: DDA (dimethyldioctadecylammonium bromide); DHEA (dehydroepiandrosterone): DMPC (dimyristoylphosphatidylcholine); DMPG (dimyristoylphosphatidylglycerol): DOC/alum complex (deoxycholic acid sodium salt); Freund's complete adjuvant: Freund's incomplete adjuvant: gamma inulin: Gerbu adjuvant (mixture of: i) N-acetylglucosaminyl-(P1-4)-N-acetylmuramyl-L-alanyl-D-glutamine (GMDP), ii) dimethyldioctadecylammonium chloride (DDA). iii) zinc-L-proline salt complex (ZnPro-8); GM-CSF); GMDP (N-acetylglucosaminyl-(b1-4)-N-acetylmuramyl-L-alanyl-D-isoglutamine); imiquimod (1-(2-methypropyl)-1H-imidazo[4,5-c]quinoline-4-amine): ImmTher^{™} (N-acetylglucosaminyl-N-acetylmuramyl-L-Ala-D-isoGlu-L-Ala-glycerol dipalmitate); DRVs (immunoliposomes prepared from dehydration-rehydration vesicles); interferon-gamma; interleukin-1beta; interleukin-2; interleukin-7: interleukin-12: ISCOMS^{™}; ISCOPREP 7.0.3.^{™}; liposomes; LOXORIBINE^{™} (7-allyl-8-oxoguanosine); LT oral adjuvant (E.coli labile enterotoxin-protoxin): microspheres and microparticles of any composition; MF59^{™}; (squalene-water emulsion); MONTANIDE ISA 51^{™} (purified incomplete Freund's adjuvant): MONTANIDE ISA 720^{™} (metabolisable oil adjuvant): MPL^{™} (3-Q-desacyl-4'-monophosphoryl lipid A); MTP-PE and MTP-PE liposomes ((N-acetyl-L-alanyl-D-isaglutaminyl-L-alanine-2-(1,2-dipalmitoyl-sn-glycero-3-(hydroxyphosphoryloxy))-ethylamide, monosodium salt); MURAMETIDE^{™} (Nac-Mur-L-Ala-D-Gln-OCH3): MURAPALMITINE^{™} and D-MURAPALMITINE^{™} (Nac-Mur-L-Thr-D-isoGIn-sn-glyceroldipalmitoyl); NAGO (neuraminidase-galactose oxidase); nanospheres or nanoparticles of any composition; NISVs (non-ionic surfactant vesicles); PLEURAN^{™} (β-glucan); PLGA, PGA and PLA (homo- and co-polymers of lactic acid and glycolic acid; microspheres/nanospheres); PLURONIC L121^{™}; PMMA (polymethyl methacrylate); PODDS^{™} (proteinoid microspheres); polyethylene carbamate derivatives; poly-rA: poly-rU (polyadenylic acid-polyuridylic acid complex); polysorbate 80 (Tween 80); protein cochleates (Avanti Polar Lipids, Inc., Alabaster, AL); STIMULON^{™} (QS-21); Quil-A (Quil-A saponin); S-28463 (4-amino-otec-dimethyl-2-ethoxymethyl-1H-imidazo[4,5 c)quinoline-1-ethanol); SAF-1^{™} ("Syntex adjuvant formulation"); Sendai proteoliposomes and Sendai-containing lipid matrices; Span-85 (sorbitan trioleate); Specol (emulsion of Marcol 52. Span 85 and Tween 85): squalene or Robane 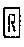 (2,6,10,15,19,23-hexamethyltetracosan and 2,6,10,15,19,23-hexamethyl-1,6,10,14,18.22-tetracosahexane); stearyltyrosine (octadecyltyrosine hydrochloride); Theramid 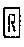 (N-acetylglucosamioyl-N-acetylmuramyl-L-Ala-D-isoGlu-L-Ala-dipalmitoxypropylamide); Theronyl-MDP (Termurtide^{™} or [thr 1]-MDP; N-acetylmuramyl-L-threonyl-D-isoglutamine); Ty particles (Ty-VLPs or virus-like particles); Walter-Reed liposomes (liposomes containing lipid A adsorbed on aluminium hydroxide), and lipopeptides, including Pam3Cys, in particular aluminium salts, such as Adju-phos, Alhydrogel, Rehydragel; emulsions, including CFA, SAF, IFA, MF59, Provax, TiterMax, Montanide, Vaxfectin; copolymers, including Optivax (ERL1005), L121, Poloaxmer4010), etc.; liposomes, including Stealth, cochleates, including BIORAL; plant derived adjuvants, including QS21, Quil A, Iscomatrix, ISCOM; adjuvants suitable for costimulation including Tomatine, biopolymers, including PLG, PMM, Inulin: microbe derived adjuvants, including Romurtide, DETOX, MPL, CWS, Mannose, CpG nucleic acid sequences, CpG7909, ligands of human TLR 1-10, ligands of murine TLR 1-13, ISS-1018, IC31, Imidazoquinolines, Ampligen, Ribi529, IMOxine, IRIVs, VLPs, cholera toxin, heat-labile toxin, Pam3Cys, Flagellin, GPI anchor. LNFPIII/Lewis X, antimicrobial peptides, UC-1V150, RSV fusion protein, cdiGMP; and adjuvants suitable as antagonists including CGRP neuropeptide.

16. Vaccine comprising the RNA according to any one of claims 1 to 11 and a pharmaceutically acceptable carrier, or comprising the composition according to any one of claims 12 to 15,
wherein the vaccine preferably comprises an adjuvant.

17. Kit, preferably kit of parts, comprising the RNA according to any one of claims 1 to 11, the composition according to any one of claims 12 to 15 or the vaccine according to claim 16, and optionally a liquid vehicle for solubilising and optionally technical instructions with information on the administration and dosage of the RNA. the composition and/or the vaccine,
wherein the kit preferably contains as a part Ringer-Lactate solution, and/or
wherein the kit preferably contains as a part an adjuvant. and/or
wherein the kit preferably contains as a part at least one inhibitory and/or stimulatory checkpoint molecule.

18. The RNA according to any one of claims 1 to 17, the composition according to any one of claims 12 to 15, the vaccine according to claim 16, or the kit according to claim 17 for use in the treatment or prophylaxis of cancer,
wherein the cancer is preferably selected from the group consisting of Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma; AIDS-Related Malignancies; Anal Cancer; Astrocytoma, Childhood Cerebellar; Astrocytoma, Childhood Cerebral; Bile Duct Cancer, Extrahepatic: Bladder Cancer; Bladder Cancer, Childhood; Bone Cancer, Osteosarcoma/Malignant Fibrous Histiocytoma; Brain Stem Glioma, Childhood; Brain Tumor, Adult; Brain Tumor, Brain Stem Glioma, Childhood; Brain Tumor, Cerebellar Astrocytoma, Childhood: Brain Tumor, Cerebral Astrocytoma/Malignant Glioma, Childhood; Brain Tumor, Ependymoma, Childhood; Brain Tumor, Medulloblastoma, Childhood; Brain Tumor, Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy; Breast Cancer, Childhood; Breast Cancer, Male; Bronchial Adenomas/Carcinoids, Childhood: Carcinoid Tumor, Childhood: Carcinoid Tumor, Gastrointestinal; Carcinoma, Adrenocortical; Carcinoma. Islet Cell; Carcinoma of Unknown Primary: Central Nervous System Lymphoma, Primary: Cerebellar Astrocytoma, Childhood: Cerebral Astrocytoma/Malignant Glioma, Childhood: Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood: Cutaneous T-Cell Lymphoma: Endometrial Cancer; Ependymoma, Childhood; Epithelial Cancer, Ovarian; Esophageal Cancer; Esophageal Cancer, Childhood; Ewing's Family of Tumors; Extracranial Germ Cell Tumor, Childhood; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma. Childhood Brain Stem; Glioma. Childhood Visual Pathway and Hypothalamic: Hairy Cell Leukemia; Head and Neck Cancer; Hepatocellular (Liver) Cancer, Adult (Primary); Hepatocellular (Liver) Cancer, Childhood (Primary); Hodgkin's Lymphoma, Adult; Hodgkin's Lymphoma, Childhood; Hodgkin's Lymphoma During Pregnancy; Hypopharyngeal Cancer; Hypothalamic and Visual Pathway Glioma, Childhood; Intraocular Melanoma; Islet Cell Carcinoma (Endocrine Pancreas); Kaposi's Sarcoma; Kidney Cancer; Laryngeal Cancer; Laryngeal Cancer, Childhood: Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute: Lymphoblastic Leukemia, Childhood Acute: Lymphocytic Leukemia, Chronic; Lymphoma, AIDS- Related: Lymphoma, Central Nervous System (Primary); Lymphoma, Cutaneous T-Cell; Lymphoma, Hodgkin's, Adult; Lymphoma, Hodgkin's: Childhood; Lymphoma, Hodgkin's During Pregnancy; Lymphoma, Non-Hodgkin's, Adult; Lymphoma, Non-Hodgkin's, Childhood; Lymphoma, Non-Hodgkin's During Pregnancy; Lymphoma, Primary Central Nervous System: Macroglobulinemia, Waldenstrom's; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary: Multiple Endocrine Neoplasia Syndrome, Childhood; Multiple Myeloma/Plasma Cell Neoplasm; Mycosis Fungoides; Myelodysplasia Syndromes; Myelogenous Leukemia, Chronic; Myeloid Leukemia. Childhood Acute: Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Neurofibroma; Non-Hodgkin's Lymphoma, Adult; Non- Hodgkin's Lymphoma, Childhood; Non-Hodgkin's Lymphoma During Pregnancy; Non- Small Cell Lung Cancer; Oral Cancer, Childhood; Oral Cavity and Lip Cancer; Oropharyngeal Cancer: Osteosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood', Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin's Lymphoma; Pregnancy and Non-Hodgkin's Lymphoma; Primary Central Nervous System Lymphoma; Primary Liver Cancer, Adult: Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood: Salivary Gland Cancer; Salivary Gland'Cancer, Childhood; Sarcoma, Ewing's Family of Tumors; Sarcoma, Kaposi's; Sarcoma (Osteosarcoma)/Malignant Fibrous Histiocytoma of Bone; Sarcoma, Rhabdomyosarcoma, Childhood: Sarcoma, Soft Tissue, Adult; Sarcoma, Soft Tissue, Childhood; Sezary Syndrome; Skin Cancer; Skin Cancer, Childhood: Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial Primitive Neuroectodermal Tumors, Childhood; T-Cell Lymphoma, Cutaneous: Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood: Vulvar Cancer; Waldenstrom's Macro globulinemia; and Wilms' Tumor.

19. The RNA according to any one of claims 1 to 11, the composition according to any one of claims 12 to 15, the vaccine according to claim 16, or the kit according to claim 17 for use according to claim 18, wherein the treatment or prophylaxis comprises the administration of a further pharmaceutical compound,
wherein the further pharmaceutical compound is preferably a chemotherapeutic agent or a kinase inhibitor, and/or
wherein the treatment or prophylaxis preferably further comprises radiation therapy, and/or
wherein the treatment or prophylaxis preferably further comprises the administration of at least one inhibitory and/or stimulatory checkpoint molecule.
